(19) Europäisches Patentamt
European Patent Office
Office européen des brevets

(11) EP 2 668 288 B1

# (12) EUROPEAN PATENT SPECIFICATION

(45) Date of publication and mention of the grant of the patent:
**20.01.2016 Bulletin 2016/03**

(21) Application number: **12701010.6**

(22) Date of filing: **26.01.2012**

(51) Int Cl.:
***C12Q 1/68*** *(2006.01)*

(86) International application number:
**PCT/EP2012/051241**

(87) International publication number:
**WO 2012/101219 (02.08.2012 Gazette 2012/31)**

## (54) COMPLEX MIRNA SETS AS NOVEL BIOMARKERS FOR LUNG DISEASES

KOMPLEXE MIRNA-SÄTZE ALS NEUE BIOMARKER FÜR LUNGENERKRANKUNGEN

ENSEMBLES DE MIARN COMPLEXES EN TANT QUE NOUVEAUX BIOMARQUEURS POUR DES MALADIES PULMONAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.01.2011 US 201161437293 P**

(43) Date of publication of application:
**04.12.2013 Bulletin 2013/49**

(73) Proprietor: **Comprehensive Biomarker Center GmbH**
**69120 Heidelberg (DE)**

(72) Inventors:
- **KELLER, Andreas**
  **66346 Püttlingen (DE)**
- **BEIER, Markus**
  **69469 Weinheim (DE)**

(74) Representative: **Geling, Andrea**
**ZSP Patentanwälte Partnerschaftsgesellschaft**
**Radlkoferstrasse 2**
**81373 München (DE)**

(56) References cited:
**EP-A1- 2 341 145** **WO-A1-2009/070653**
**US-B2- 7 709 616**

- **KELLER ANDREAS ET AL: "miRNAs in lung cancer - Studying complex fingerprints in patient's blood cells by microarray experiments", BMC CANCER, BIOMED CENTRAL, LONDON, GB, vol. 9, no. 1, 6 October 2009 (2009-10-06) , page 353, XP021062692, ISSN: 1471-2407, DOI: 10.1186/1471-2407-9-353**
- **BONS ET AL: "Potential biomarkers for diagnosis of sarcoidosis using proteomics in serum", RESPIRATORY MEDICINE, BAILLIERE TINDALL, LONDON, GB, vol. 101, no. 8, 20 July 2007 (2007-07-20) , pages 1687-1695, XP022154569, ISSN: 0954-6111, DOI: 10.1016/J.RMED. 2007.03.002**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 668 288 B1

SEQUENCE LISTING

<110> febit holding GmbH, et al.

<120> Complex miRNA sets as novel biomarkers for lung diseases

<130> 505-21 PCT

<150> US 61/437,293
<151< 2011-01-28

<160> 391

<170> PatentIn version 3.5

<210> 1
<211> 22
<212> RNA
<213> Homo sapiens

<400> 1
aauugcacgg uauccaucug ua 22

<210> 2
<211> 21
<212> RNA
<213> Homo sapiens

<400> 2
cuagacugaa gcuccuugag g 21

<210> 3
<211> 23
<212> RNA
<213> Homo sapiens

<400> 3
caaagugcuu acagugcagg uag 23

<210> 4
<211> 23
<212> RNA
<213> Homo sapiens

<400> 4
caaagugcuc auagugcagg uag 23

<210> 5
<211> 23
<212> RNA
<213> Homo sapiens

<400> 5
agcagcauug uacagggcua uga 23

<210> 6
<211> 23
<212> RNA
<213> Homo sapiens

<400> 6
aaaagugcuu acagugcagg uag 23

<210> 7
<211> 21
<212> RNA
<213> Homo sapiens

<400> 7
uacaguacug ugauaacuga a 21

<210> 8
<211> 23
<212> RNA
<213> Homo sapiens

<400> 8
uaaagugcuu auagugcagg uag 23

<210> 9
<211> 22
<212> RNA
<213> Homo sapiens

<400> 9
agagguagua gguugcauag uu 22

<210> 10
<211> 19
<212> RNA
<213> Homo sapiens

<400> 10
aaaagcuggg uugagagga 19

<210> 11
<211> 20
<212> RNA
<213> Homo sapiens

<400> 11
acugccccag gugcugcugg 20

<210> 12
<211> 22
<212> RNA
<213> Homo sapiens

<400> 12
uagcagcaca ucaugguuua ca 22

<210> 13
<211> 22
<212> RNA
<213> Homo sapiens

<400> 13
ucagugcauc acagaacuuu gu 22

<210> 14
<211> 22
<212> RNA
<213> Homo sapiens

<400> 14
ugucuuacuc ccucaggcac au 22

<210> 15
<211> 22
<212> RNA
<213> Homo sapiens

<400> 15
uguaaacauc cccgacugga ag 22

<210> 16
<211> 22
<212> RNA
<213> Homo sapiens

<400> 16
uucaaguaau ccaggauagg cu 22

<210> 17
<211> 23
<212> RNA
<213> Homo sapiens

<400> 17
agcagcauug uacagggcua uca 23

<210> 18
<211> 22
<212> RNA
<213> Homo sapiens

<400> 18
aaaagcuggg uugagagggc aa 22

<210> 19
<211> 22
<212> RNA
<213> Homo sapiens

<400> 19
uggcucaguu cagcaggaac ag 22

<210> 20
<211> 23
<212> RNA
<213> Homo sapiens

<400> 20
uaaggugcau cuagugcaga uag 23

<210> 21
<211> 20

<212> RNA
<213> Homo sapiens

<400> 21
aaaagcuggg uugagagggu 20

<210> 22
<211> 22
<212> RNA
<213> Homo sapiens

<400> 22
ugcggggcua gggcuaacag ca 22

<210> 23
<211> 23
<212> RNA
<213> Homo sapiens

<400> 23
caaagugcug uucgugcagg uag 23

<210> 24
<211> 23
<212> RNA
<213> Homo sapiens

<400> 24
uguaaacauc cuacacucuc agc 23

<210> 25
<211> 21
<212> RNA
<213> Homo sapiens

<400> 25
uaaagugcug acagugcaga u 21

<210> 26
<211> 24
<212> RNA
<213> Homo sapiens

<400> 26
uuuggcaaug guagaacuca cacu 24

<210> 27
<211> 22
<212> RNA
<213> Homo sapiens

<400> 27
aaaagcuggg uugagagggc ga 22

<210> 28
<211> 17
<212> RNA
<213> Homo sapiens

<400> 28
ucucgcuggg gccucca 17

<210> 29
<211> 22
<212> RNA
<213> Homo sapiens

<400> 29
uguaaacauc cuugacugga ag 22

<210> 30
<211> 23
<212> RNA
<213> Homo sapiens

<400> 30
uauucauuua uccccagccu aca 23

<210> 31
<211> 22
<212> RNA
<213> Homo sapiens

<400> 31
uagcagcacg uaaauauugg cg 22

<210> 32
<211> 22
<212> RNA
<213> Homo sapiens

<400> 32
uacccauugc auaucggagu ug 22

<210> 33
<211> 22
<212> RNA
<213> Homo sapiens

<400> 33
cagugcaaug uuaaaagggc au 22

<210> 34
<211> 22
<212> RNA
<213> Homo sapiens

<400> 34
ucagugcacu acagaacuuu gu 22

<210> 35
<211> 22
<212> RNA
<213> Homo sapiens

<400> 35
acugcugagc uagcacuucc cg 22

<210> 36
<211> 23
<212> RNA
<213> Homo sapiens

<400> 36
ugaggggcag agagcgagac uuu 23

<210> 37
<211> 22
<212> RNA
<213> Homo sapiens

<400> 37
acugcaguga aggcacuugu ag 22

<210> 38
<211> 22
<212> RNA
<213> Homo sapiens

<400> 38
ugagguagua gguuguauag uu 22

<210> 39
<211> 21
<212> RNA
<213> Homo sapiens

<400> 39
gccccugggc cuauccuaga a 21

<210> 40
<211> 21
<212> RNA
<213> Homo sapiens

<400> 40
cugaccuaug aauugacagc c 21

<210> 41
<211> 23
<212> RNA
<213> Homo sapiens

<400> 41
uagcaccauu ugaaaucagu guu 23

<210> 42
<211> 22
<212> RNA
<213> Homo sapiens

<400> 42
uguaaacauc cucgacugga ag 22

<210> 43
<211> 22

<212> RNA
<213> Homo sapiens

<400> 43
aaggagcuca cagucuauug ag 22

<210> 44
<211> 21
<212> RNA
<213> Homo sapiens

<400> 44
cggcggggac ggcgauuggu c 21

<210> 45
<211> 18
<212> RNA
<213> Homo sapiens

<400> 45
aucccaccuc ugccacca 18

<210> 46
<211> 22
<212> RNA
<213> Homo sapiens

<400> 46
uucaccaccu ucuccaccca gc 22

<210> 47
<211> 22
<212> RNA
<213> Homo sapiens

<400> 47
ccucccacac ccaaggcuug ca 22

<210> 48
<211> 23
<212> RNA
<213> Homo sapiens

<400> 48
uaauccuugc uaccugggug aga 23

<210> 49
<211> 22
<212> RNA
<213> Homo sapiens

<400> 49
ugucaguuug ucaaauaccc ca 22

<210> 50
<211> 21
<212> RNA
<213> Homo sapiens

<400> 50
uagcagcaca gaaauauugg c 21

<210> 51
<211> 23
<212> RNA
<213> Homo sapiens

<400> 51
ucucacacag aaaucgcacc cgu 23

<210> 52
<211> 22
<212> RNA
<213> Homo sapiens

<400> 52
cauugcacuu gucucggucu ga 22

<210> 53
<211> 21
<212> RNA
<213> Homo sapiens

<400> 53
agcuacaucu ggcuacuggg u 21

<210> 54
<211> 17
<212> RNA
<213> Homo sapiens

<400> 54
ucccaccgcu gccaccc 17

<210> 55
<211> 23
<212> RNA
<213> Homo sapiens

<400> 55
aacauucaac gcugucggug agu 23

<210> 56
<211> 22
<212> RNA
<213> Homo sapiens

<400> 56
uguaacagca acuccaugug ga 22

<210> 57
<211> 23
<212> RNA
<213> Homo sapiens

<400> 57
cccaguguuc agacuaccug uuc 23

<210> 58
<211> 22
<212> RNA
<213> Homo sapiens

<400> 58
ucccugagac ccuaacuugu ga 22

<210> 59
<211> 23
<212> RNA
<213> Homo sapiens

<400> 59
ucccuguccu ccaggagcuc acg 23

<210> 60
<211> 22
<212> RNA
<213> Homo sapiens

<400> 60
ucaggcucag uccccucccg au 22

<210> 61
<211> 22
<212> RNA
<213> Homo sapiens

<400> 61
cacgcucaug cacacaccca ca 22

<210> 62
<211> 22
<212> RNA
<213> Homo sapiens

<400> 62
uagcaccauc ugaaaucggu ua 22

<210> 63
<211> 22
<212> RNA
<213> Homo sapiens

<400> 63
uagcagcaca uaaugguuug ug 22

<210> 64
<211> 21
<212> RNA
<213> Homo sapiens

<400> 64
ucgaggagcu cacagucuag u 21

<210> 65
<211> 23

<212> RNA
<213> Homo sapiens

<400> 65
ugagugugug ugugugagug ugu 23

<210> 66
<211> 22
<212> RNA
<213> Homo sapiens

<400> 66
ucugcccccu ccgcugcugc ca 22

<210> 67
<211> 23
<212> RNA
<213> Homo sapiens

<400> 67
aaugacacga ucacucccgu uga 23

<210> 68
<211> 20
<212> RNA
<213> Homo sapiens

<400> 68
uacaguauag augauguacu 20

<210> 69
<211> 22
<212> RNA
<213> Homo sapiens

<400> 69
ucguaccgug aguaauaaug cg 22

<210> 70
<211> 22
<212> RNA
<213> Homo sapiens

<400> 70
ggauaucauc auauacugua ag 22

<210> 71
<211> 22
<212> RNA
<213> Homo sapiens

<400> 71
auauaauaca accugcuaag ug 22

<210> 72
<211> 22
<212> RNA
<213> Homo sapiens

<400> 72
uagcuuauca gacugauguu ga 22

<210> 73
<211> 23
<212> RNA
<213> Homo sapiens

<400> 73
cagugcaaua guauugucaa agc 23

<210> 74
<211> 22
<212> RNA
<213> Homo sapiens

<400> 74
ugagguagua guuugugcug uu 22

<210> 75
<211> 22
<212> RNA
<213> Homo sapiens

<400> 75
ugagguagua gauuguauag uu 22

<210> 76
<211> 23
<212> RNA
<213> Homo sapiens

<400> 76
acugcccuaa gugcuccuuc ugg 23

<210> 77
<211> 22
<212> RNA
<213> Homo sapiens

<400> 77
uauggcacug guagaauuca cu 22

<210> 78
<211> 21
<212> RNA
<213> Homo sapiens

<400> 78
gugccagcug caguggggga g 21

<210> 79
<211> 23
<212> RNA
<213> Homo sapiens

<400> 79
uagcagcggg aacaguucug cag 23

<210> 80
<211> 25
<212> RNA
<213> Homo sapiens

<400> 80
aggcaccagc caggcauugc ucagc 25

<210> 81
<211> 19
<212> RNA
<213> Homo sapiens

<400> 81
aagcagcugc cucugaggc 19

<210> 82
<211> 22
<212> RNA
<213> Homo sapiens

<400> 82
aacccguaga uccgaacuug ug 22

<210> 83
<211> 24
<212> RNA
<213> Homo sapiens

<400> 83
ucccugagac ccuuuaaccu guga 24

<210> 84
<211> 23
<212> RNA
<213> Homo sapiens

<400> 84
guccaguuuu cccaggaauc ccu 23

<210> 85
<211> 22
<212> RNA
<213> Homo sapiens

<400> 85
cugcgcaagc uacugccuug cu 22

<210> 86
<211> 22
<212> RNA
<213> Homo sapiens

<400> 86
cagcagcaau ucauguuuug aa 22

<210> 87
<211> 22

<212> RNA
<213> Homo sapiens

<400> 87
aacauucaac cugucgguga gu 22

<210> 88
<211> 21
<212> RNA
<213> Homo sapiens

<400> 88
ucagugcaug acagaacuug g 21

<210> 89
<211> 22
<212> RNA
<213> Homo sapiens

<400> 89
acagcaggca cagacaggca gu 22

<210> 90
<211> 24
<212> RNA
<213> Homo sapiens

<400> 90
aauccuugga accuaggugu gagu 24

<210> 91
<211> 22
<212> RNA
<213> Homo sapiens

<400> 91
caugccuuga guguaggacc gu 22

<210> 92
<211> 26
<212> RNA
<213> Homo sapiens

<400> 92
gugagggcau gcaggccugg augggg 26

<210> 93
<211> 23
<212> RNA
<213> Homo sapiens

<400> 93
ccugcagcga cuugauggcu ucc 23

<210> 94
<211> 22
<212> RNA
<213> Homo sapiens

<400> 94
ugcuaugcca acauauugcc au 22

<210> 95
<211> 22
<212> RNA
<213> Homo sapiens

<400> 95
uacccagagc augcagugug aa 22

<210> 96
<211> 21
<212> RNA
<213> Homo sapiens

<400> 96
cccggagcca ggaugcagcu c 21

<210> 97
<211> 22
<212> RNA
<213> Homo sapiens

<400> 97
ugagccccug ugccgccccc ag 22

<210> 98
<211> 22
<212> RNA
<213> Homo sapiens

<400> 98
ccucuagaug gaagcacugu cu 22

<210> 99
<211> 24
<212> RNA
<213> Homo sapiens

<400> 99
uucuccaaaa gaaagcacuu ucug 24

<210> 100
<211> 21
<212> RNA
<213> Homo sapiens

<400> 100
ugucuugcag gccgucaugc a 21

<210> 101
<211> 21
<212> RNA
<213> Homo sapiens

<400> 101
cucccacaug caggguuugc a 21

<210> 102
<211> 23
<212> RNA
<213> Homo sapiens

<400> 102
uacugcauca ggaacugauu gga 23

<210> 103
<211> 21
<212> RNA
<213> Homo sapiens

<400> 103
aaggcagggc ccccgcuccc c 21

<210> 104
<211> 27
<212> RNA
<213> Homo sapiens

<400> 104
auugaucauc gacacuucga acgcaau 27

<210> 105
<211> 21
<212> RNA
<213> Homo sapiens

<400> 105
uucacagugg cuaaguucug c 21

<210> 106
<211> 21
<212> RNA
<213> Homo sapiens

<400> 106
uucaaguaau ucaggauagg u 21

<210> 107
<211> 21
<212> RNA
<213> Homo sapiens

<400> 107
uucacagugg cuaaguuccg c 21

<210> 108
<211> 22
<212> RNA
<213> Homo sapiens

<400> 108
ugagguagua guuuguacag uu 22

<210> 109
<211> 22

<212> RNA
<213> Homo sapiens

<400> 109
uuauaauaca accugauaag ug 22

<210> 110
<211> 24
<212> RNA
<213> Homo sapiens

<400> 110
uugcagcugc cugggaguga cuuc 24

<210> 111
<211> 22
<212> RNA
<213> Homo sapiens

<400> 111
uacugcagac guggcaauca ug 22

<210> 112
<211> 22
<212> RNA
<213> Homo sapiens

<400> 112
uuaugguuug ccugggacug ag 22

<210> 113
<211> 21
<212> RNA
<213> Homo sapiens

<400> 113
cggggcagcu caguacagga u 21

<210> 114
<211> 23
<212> RNA
<213> Homo sapiens

<400> 114
uuuggcacua gcacauuuuu gcu 23

<210> 115
<211> 26
<212> RNA
<213> Homo sapiens

<400> 115
gaugaugaug gcagcaaauu cugaaa 26

<210> 116
<211> 21
<212> RNA
<213> Homo sapiens

<400> 116
ugagaugaag cacuguagcu c 21

<210> 117
<211> 21
<212> RNA
<213> Homo sapiens

<400> 117
uuggccacaa uggguuagaa c 21

<210> 118
<211> 22
<212> RNA
<213> Homo sapiens

<400> 118
ugccuacuga gcugaaacac ag 22

<210> 119
<211> 22
<212> RNA
<213> Homo sapiens

<400> 119
uggugggcac agaaucugga cu 22

<210> 120
<211> 24
<212> RNA
<213> Homo sapiens

<400> 120
agccuggaag cuggagccug cagu 24

<210> 121
<211> 21
<212> RNA
<213> Homo sapiens

<400> 121
ucuaguaaga guggcagucg a 21

<210> 122
<211> 22
<212> RNA
<213> Homo sapiens

<400> 122
ggugcagugc ugcaucucug gu 22

<210> 123
<211> 22
<212> RNA
<213> Homo sapiens

<400> 123
uggugggccg cagaacaugu gc 22

<210> 124
<211> 21
<212> RNA
<213> Homo sapiens

<400> 124
ugcaggacca agaugagccc u 21

<210> 125
<211> 23
<212> RNA
<213> Homo sapiens

<400> 125
uaaggugcau cuagugcagu uag 23

<210> 126
<211> 23
<212> RNA
<213> Homo sapiens

<400> 126
agugccugag ggaguaagag ccc 23

<210> 127
<211> 22
<212> RNA
<213> Homo sapiens

<400> 127
cuauacgacc ugcugccuuu cu 22

<210> 128
<211> 22
<212> RNA
<213> Homo sapiens

<400> 128
acacagggcu guugugaaga cu 22

<210> 129
<211> 20
<212> RNA
<213> Homo sapiens

<400> 129
gugcauugcu guugcauugc 20

<210> 130
<211> 19
<212> RNA
<213> Homo sapiens

<400> 130
gagccaguug gacaggagc 19

<210> 131
<211> 21

<212> RNA
<213> Homo sapiens

<400> 131
aggcaagaug cuggcauagc u 21

<210> 132
<211> 21
<212> RNA
<213> Homo sapiens

<400> 132
gcaguccaug ggcauauaca c 21

<210> 133
<211> 22
<212> RNA
<213> Homo sapiens

<400> 133
ucuacaaagg aaagcgcuuu cu 22

<210> 134
<211> 22
<212> RNA
<213> Homo sapiens

<400> 134
ggaggggucc cgcacuggga gg 22

<210> 135
<211> 22
<212> RNA
<213> Homo sapiens

<400> 135
cggcaacaag aaacugccug ag 22

<210> 136
<211> 21
<212> RNA
<213> Homo sapiens

<400> 136
gaagugugcc gugguguguc u 21

<210> 137
<211> 22
<212> RNA
<213> Homo sapiens

<400> 137
cugccaauuc cauaggucac ag 22

<210> 138
<211> 22
<212> RNA
<213> Homo sapiens

<400> 138
ccaauauuac ugugcugcuu ua 22

<210> 139
<211> 22
<212> RNA
<213> Homo sapiens

<400> 139
aaucaugugc agugccaaua ug 22

<210> 140
<211> 21
<212> RNA
<213> Homo sapiens

<400> 140
gugcauugua guugcauugc a 21

<210> 141
<211> 21
<212> RNA
<213> Homo sapiens

<400> 141
aggaggcagc gcucucagga c 21

<210> 142
<211> 21
<212> RNA
<213> Homo sapiens

<400> 142
uuaggccgca gaucugggug a 21

<210> 143
<211> 23
<212> RNA
<213> Homo sapiens

<400> 143
ccucagggcu guagaacagg gcu 23

<210> 144
<211> 22
<212> RNA
<213> Homo sapiens

<400> 144
uauugcacau uacuaaguug ca 22

<210> 145
<211> 22
<212> RNA
<213> Homo sapiens

<400> 145
uccagcauca gugauuuugu ug 22

<210> 146
<211> 21
<212> RNA
<213> Homo sapiens

<400> 146
acucuagcug ccaaaggcgc u 21

<210> 147
<211> 22
<212> RNA
<213> Homo sapiens

<400> 147
cuggcccucu cugcccuucc gu 22

<210> 148
<211> 22
<212> RNA
<213> Homo sapiens

<400> 148
cagugccucg gcagugcagc cc 22

<210> 149
<211> 21
<212> RNA
<213> Homo sapiens

<400> 149
agaccuggcc cagaccucag c 21

<210> 150
<211> 22
<212> RNA
<213> Homo sapiens

<400> 150
cucuagaggg aagcgcuuuc ug 22

<210> 151
<211> 22
<212> RNA
<213> Homo sapiens

<400> 151
ggcuacaaca caggacccgg gc 22

<210> 152
<211> 22
<212> RNA
<213> Homo sapiens

<400> 152
uaacagucua cagccauggu cg 22

<210> 153
<211> 22

<212> RNA
<213> Homo sapiens

<400> 153
ugagaacuga auuccauagg cu 22

<210> 154
<211> 23
<212> RNA
<213> Homo sapiens

<400> 154
uagugcaaua uugcuuauag ggu 23

<210> 155
<211> 24
<212> RNA
<213> Homo sapiens

<400> 155
agcagaagca gggagguucu ccca 24

<210> 156
<211> 22
<212> RNA
<213> Homo sapiens

<400> 156
cucuagaggg aagcgcuuuc ug 22

<210> 157
<211> 22
<212> RNA
<213> Homo sapiens

<400> 157
caggccauau ugugcugccu ca 22

<210> 158
<211> 22
<212> RNA
<213> Homo sapiens

<400> 158
ugagguagga gguuguauag uu 22

<210> 159
<211> 22
<212> RNA
<213> Homo sapiens

<400> 159
acaguagucu gcacauuggu ua 22

<210> 160
<211> 21
<212> RNA
<213> Homo sapiens

<400> 160
cuccagaggg augcacuuuc u 21

<210> 161
<211> 18
<212> RNA
<213> Homo sapiens

<400> 161
ugcuuccuuu cagagggu 18

<210> 162
<211> 22
<212> RNA
<213> Homo sapiens

<400> 162
acaguagucu gcacauuggu ua 22

<210> 163
<211> 22
<212> RNA
<213> Homo sapiens

<400> 163
ugucuacuac uggagacacu gg 22

<210> 164
<211> 22
<212> RNA
<213> Homo sapiens

<400> 164
uuuuucauua uugcuccuga cc 22

<210> 165
<211> 22
<212> RNA
<213> Homo sapiens

<400> 165
cgucaacacu ugcugguuuc cu 22

<210> 166
<211> 22
<212> RNA
<213> Homo sapiens

<400> 166
uccauuacac uacccugccu cu 22

<210> 167
<211> 22
<212> RNA
<213> Homo sapiens

<400> 167
aagcccuuac cccaaaaagu au 22

<210> 168
<211> 22
<212> RNA
<213> Homo sapiens

<400> 168
aagugcuguc auagcugagg uc 22

<210> 169
<211> 22
<212> RNA
<213> Homo sapiens

<400> 169
ggagacgcgg cccuguugga gu 22

<210> 170
<211> 19
<212> RNA
<213> Homo sapiens

<400> 170
aggcugcgga auucaggac 19

<210> 171
<211> 22
<212> RNA
<213> Homo sapiens

<400> 171
ugaccgauuu cuccuggugu uc 22

<210> 172
<211> 23
<212> RNA
<213> Homo sapiens

<400> 172
uaagugcuuc cauguuuuag uag 23

<210> 173
<211> 22
<212> RNA
<213> Homo sapiens

<400> 173
cguguucaca gcggaccuug au 22

<210> 174
<211> 22
<212> RNA
<213> Homo sapiens

<400> 174
caaaaaucuc aauuacuuuu gc 22

<210> 175
<211> 22

<212> RNA
<213> Homo sapiens

<400> 175
cgucuuaccc agcaguguuu gg 22

<210> 176
<211> 20
<212> RNA
<213> Homo sapiens

<400> 176
caccaggcau uguggucucc 20

<210> 177
<211> 22
<212> RNA
<213> Homo sapiens

<400> 177
aucgugcauc cuuuuagagu gu 22

<210> 178
<211> 22
<212> RNA
<213> Homo sapiens

<400> 178
augggugaau uuguagaagg au 22

<210> 179
<211> 22
<212> RNA
<213> Homo sapiens

<400> 179
aaaacuguaa uuacuuuugu ac 22

<210> 180
<211> 21
<212> RNA
<213> Homo sapiens

<400> 180
auccuugcua ucugggugcu a 21

<210> 181
<211> 22
<212> RNA
<213> Homo sapiens

<400> 181
caaccuggag gacuccaugc ug 22

<210> 182
<211> 21
<212> RNA
<213> Homo sapiens

<400> 182
ucacuccucu ccucccgucu u 21

<210> 183
<211> 22
<212> RNA
<213> Homo sapiens

<400> 183
ucgugucuug uguugcagcc gg 22

<210> 184
<211> 22
<212> RNA
<213> Homo sapiens

<400> 184
aaagugcauc cuuuuagagg uu 22

<210> 185
<211> 22
<212> RNA
<213> Homo sapiens

<400> 185
uagaguuaca cccugggagu ua 22

<210> 186
<211> 22
<212> RNA
<213> Homo sapiens

<400> 186
aaaccugugu uguucaagag uc 22

<210> 187
<211> 22
<212> RNA
<213> Homo sapiens

<400> 187
aucgugcauc ccuuuagagu gu 22

<210> 188
<211> 22
<212> RNA
<213> Homo sapiens

<400> 188
aguauucugu accagggaag gu 22

<210> 189
<211> 21
<212> RNA
<213> Homo sapiens

<400> 189
cauuauuacu uuugguacgc g 21

<210> 190
<211> 21
<212> RNA
<213> Homo sapiens

<400> 190
aauauuauac agucaaccuc u 21

<210> 191
<211> 21
<212> RNA
<213> Homo sapiens

<400> 191
aguuaaugaa uccuggaaag u 21

<210> 192
<211> 19
<212> RNA
<213> Homo sapiens

<400> 192
gugucugcuu ccuguggga 19

<210> 193
<211> 22
<212> RNA
<213> Homo sapiens

<400> 193
cuccugacuc cagguccugu gu 22

<210> 194
<211> 21
<212> RNA
<213> Homo sapiens

<400> 194
aaaacgguga gauuuuguuu u 21

<210> 195
<211> 23
<212> RNA
<213> Homo sapiens

<400> 195
uuauugcuua agaauacgcg uag 23

<210> 196
<211> 27
<212> RNA
<213> Homo sapiens

<400> 196
cacuguaggu gauggugaga gugggca 27

<210> 197
<211> 22

<212> RNA
<213> Homo sapiens

<400> 197
auauuaccau uagcucaucu uu 22

<210> 198
<211> 21
<212> RNA
<213> Homo sapiens

<400> 198
ugauauguuu gauauugggu u 21

<210> 199
<211> 22
<212> RNA
<213> Homo sapiens

<400> 199
accuugccuu gcugcccggg cc 22

<210> 200
<211> 22
<212> RNA
<213> Homo sapiens

<400> 200
caaagaauuc uccuuuuggg cu 22

<210> 201
<211> 21
<212> RNA
<213> Homo sapiens

<400> 201
cuccguuugc cuguuucgcu g 21

<210> 202
<211> 22
<212> RNA
<213> Homo sapiens

<400> 202
acccuaucaa uauugucucu gc 22

<210> 203
<211> 22
<212> RNA
<213> Homo sapiens

<400> 203
uuuucaacuc uaaugggaga ga 22

<210> 204
<211> 22
<212> RNA
<213> Homo sapiens

<400> 204
agacuuccca uuugaaggug gc 22

<210> 205
<211> 22
<212> RNA
<213> Homo sapiens

<400> 205
gaaaucaagc gugggugaga cc 22

<210> 206
<211> 21
<212> RNA
<213> Homo sapiens

<400> 206
uguucaugua gauguuuaag c 21

<210> 207
<211> 21
<212> RNA
<213> Homo sapiens

<400> 207
ugguagacua uggaacguag g 21

<210> 208
<211> 22
<212> RNA
<213> Homo sapiens

<400> 208
aaagugcuuc cuuuuagagg gu 22

<210> 209
<211> 22
<212> RNA
<213> Homo sapiens

<400> 209
aaaaguaauu gugguuuugg cc 22

<210> 210
<211> 22
<212> RNA
<213> Homo sapiens

<400> 210
aaagugcauc cuuuuagagu gu 22

<210> 211
<211> 23
<212> RNA
<213> Homo sapiens

<400> 211
uaaaucccau ggugccuucu ccu 23

<210> 212
<211> 21
<212> RNA
<213> Homo sapiens

<400> 212
cacauuacac ggucgaccuc u 21

<210> 213
<211> 21
<212> RNA
<213> Homo sapiens

<400> 213
aguuaggauu aggucgugga a 21

<210> 214
<211> 22
<212> RNA
<213> Homo sapiens

<400> 214
ugcaacuuac cugagucauu ga 22

<210> 215
<211> 22
<212> RNA
<213> Homo sapiens

<400> 215
gaauguugcu cggugaaccc cu 22

<210> 216
<211> 21
<212> RNA
<213> Homo sapiens

<400> 216
acucuuuccc uguugcacua c 21

<210> 217
<211> 22
<212> RNA
<213> Homo sapiens

<400> 217
aaaguucuga gacacuccga cu 22

<210> 218
<211> 25
<212> RNA
<213> Homo sapiens

<400> 218
aaaggauucu gcugucgguc ccacu 25

<210> 219
<211> 19

<212> RNA
<213> Homo sapiens

<400> 219
uggauuuuug gaucaggga 19

<210> 220
<211> 21
<212> RNA
<213> Homo sapiens

<400> 220
uaaaguaaau augcaccaaa a 21

<210> 221
<211> 22
<212> RNA
<213> Homo sapiens

<400> 221
agggcuuagc ugcuugugag ca 22

<210> 222
<211> 20
<212> RNA
<213> Homo sapiens

<400> 222
uaaagagccc uguggagaca 20

<210> 223
<211> 19
<212> RNA
<213> Homo sapiens

<400> 223
agguugacau acguuuccc 19

<210> 224
<211> 22
<212> RNA
<213> Homo sapiens

<400> 224
uccgagccug ggucucccuc uu 22

<210> 225
<211> 22
<212> RNA
<213> Homo sapiens

<400> 225
auaagacgaa caaaagguuu gu 22

<210> 226
<211> 23
<212> RNA
<213> Homo sapiens

<400> 226
agguuacccg agcaacuuug cau 23

<210> 227
<211> 21
<212> RNA
<213> Homo sapiens

<400> 227
aauaauacau gguugaucuu u 21

<210> 228
<211> 21
<212> RNA
<213> Homo sapiens

<400> 228
cgcgggugcu uacugacccu u 21

<210> 229
<211> 22
<212> RNA
<213> Homo sapiens

<400> 229
aauccuuugu cccuggguga ga 22

<210> 230
<211> 22
<212> RNA
<213> Homo sapiens

<400> 230
uuugugaccu gguccacuaa cc 22

<210> 231
<211> 23
<212> RNA
<213> Homo sapiens

<400> 231
ucuuugguua ucuagcugua uga 23

<210> 232
<211> 21
<212> RNA
<213> Homo sapiens

<400> 232
agggagggac gggggcugug c 21

<210> 233
<211> 22
<212> RNA
<213> Homo sapiens

<400> 233
cagugcaaug augaaagggc au 22

<210> 234
<211> 22
<212> RNA
<213> Homo sapiens

<400> 234
uguaaacauc cuacacucag cu 22

<210> 235
<211> 22
<212> RNA
<213> Homo sapiens

<400> 235
cuuggcaccu agcaagcacu ca 22

<210> 236
<211> 17
<212> RNA
<213> Homo sapiens

<400> 236
ucccuguucg ggcgcca 17

<210> 237
<211> 23
<212> RNA
<213> Homo sapiens

<400> 237
ugugcaaauc caugcaaaac uga 23

<210> 238
<211> 22
<212> RNA
<213> Homo sapiens

<400> 238
uggagagaaa ggcaguuccu ga 22

<210> 239
<211> 21
<212> RNA
<213> Homo sapiens

<400> 239
cauaaaguag aaagcacuac u 21

<210> 240
<211> 23
<212> RNA
<213> Homo sapiens

<400> 240
uggugcggag agggcccaca gug 23

<210> 241
<211> 22

<212> RNA
<213> Homo sapiens

<400> 241
cugugcgugu gacagcggcu ga 22

<210> 242
<211> 22
<212> RNA
<213> Homo sapiens

<400> 242
cucggcgcgg ggcgcgggcu cc 22

<210> 243
<211> 22
<212> RNA
<213> Homo sapiens

<400> 243
gugagucucu aagaaaagag ga 22

<210> 244
<211> 21
<212> RNA
<213> Homo sapiens

<400> 244
aucacauugc cagggauuuc c 21

<210> 245
<211> 22
<212> RNA
<213> Homo sapiens

<400> 245
ucuacagugc acgugucucc ag 22

<210> 246
<211> 22
<212> RNA
<213> Homo sapiens

<400> 246
uccuguacug agcugccccg ag 22

<210> 247
<211> 21
<212> RNA
<213> Homo sapiens

<400> 247
agggggaaag uucuauaguc c 21

<210> 248
<211> 21
<212> RNA
<213> Homo sapiens

<400> 248
ccccaccucc ucucuccuca g 21

<210> 249
<211> 22
<212> RNA
<213> Homo sapiens

<400> 249
ugggucuuug cgggcgagau ga 22

<210> 250
<211> 21
<212> RNA
<213> Homo sapiens

<400> 250
aggggugcua ucugugauug a 21

<210> 251
<211> 22
<212> RNA
<213> Homo sapiens

<400> 251
cuugguucag ggaggguccc ca 22

<210> 252
<211> 20
<212> RNA
<213> Homo sapiens

<400> 252
guagaggaga uggcgcaggg 20

<210> 253
<211> 21
<212> RNA
<213> Homo sapiens

<400> 253
caacaccagu cgaugggcug u 21

<210> 254
<211> 17
<212> RNA
<213> Homo sapiens

<400> 254
ucgccuccuc cucuccc 17

<210> 255
<211> 22
<212> RNA
<213> Homo sapiens

<400> 255
agaucgaccg uguuauauuc gc 22

<210> 256
<211> 21
<212> RNA
<213> Homo sapiens

<400> 256
ugguucuaga cuugccaacu a 21

<210> 257
<211> 23
<212> RNA
<213> Homo sapiens

<400> 257
acuccauuug uuuugaugau gga 23

<210> 258
<211> 17
<212> RNA
<213> Homo sapiens

<400> 258
ucauauugcu ucuuucu 17

<210> 259
<211> 21
<212> RNA
<213> Homo sapiens

<400> 259
aaagugcuuc cuuuuugagg g 21

<210> 260
<211> 22
<212> RNA
<213> Homo sapiens

<400> 260
cuauacaacc uacugccuuc cc 22

<210> 261
<211> 22
<212> RNA
<213> Homo sapiens

<400> 261
aaucauacac gguugaccua uu 22

<210> 262
<211> 22
<212> RNA
<213> Homo sapiens

<400> 262
uugggaucau uuugcaucca ua 22

<210> 263
<211> 20

<212> RNA
<213> Homo sapiens

<400> 263
aaaguagcug uaccauuugc 20

<210> 264
<211> 22
<212> RNA
<213> Homo sapiens

<400> 264
ugaaggucua cugugugcca gg 22

<210> 265
<211> 21
<212> RNA
<213> Homo sapiens

<400> 265
caaagguauu ugugguuuuu g 21

<210> 266
<211> 23
<212> RNA
<213> Homo sapiens

<400> 266
caagucuuau uugagcaccu guu 23

<210> 267
<211> 23
<212> RNA
<213> Homo sapiens

<400> 267
guuugcacgg gugggccuug ucu 23

<210> 268
<211> 23
<212> RNA
<213> Homo sapiens

<400> 268
ugauuguagc cuuuuggagu aga 23

<210> 269
<211> 23
<212> RNA
<213> Homo sapiens

<400> 269
ccaguuaccg cuuccgcuac cgc 23

<210> 270
<211> 22
<212> RNA
<213> Homo sapiens

<400> 270
cuauacaguc uacugucuuu cc 22

<210> 271
<211> 22
<212> RNA
<213> Homo sapiens

<400> 271
gaaagugcuu ccuuuuagag gc 22

<210> 272
<211> 22
<212> RNA
<213> Homo sapiens

<400> 272
aaaaguaauu gcgguuuuug cc 22

<210> 273
<211> 22
<212> RNA
<213> Homo sapiens

<400> 273
uucuccaaaa gggagcacuu uc 22

<210> 274
<211> 21
<212> RNA
<213> Homo sapiens

<400> 274
ccaccaccgu gucugacacu u 21

<210> 275
<211> 22
<212> RNA
<213> Homo sapiens

<400> 275
ucuauacaga cccuggcuuu uc 22

<210> 276
<211> 22
<212> RNA
<213> Homo sapiens

<400> 276
ccuauucuug guuacuugca cg 22

<210> 277
<211> 23
<212> RNA
<213> Homo sapiens

<400> 277
acuuaaacgu ggauguacuu gcu 23

<210> 278
<211> 22
<212> RNA
<213> Homo sapiens

<400> 278
uucccuuugu cauccuaugc cu 22

<210> 279
<211> 23
<212> RNA
<213> Homo sapiens

<400> 279
gaagugcuuc gauuuugggg ugu 23

<210> 280
<211> 22
<212> RNA
<213> Homo sapiens

<400> 280
gggguuccug gggaugggau uu 22

<210> 281
<211> 17
<212> RNA
<213> Homo sapiens

<400> 281
uaauugcuuc cauguuu 17

<210> 282
<211> 24
<212> RNA
<213> Homo sapiens

<400> 282
acugggggcu uucgggcucu gcgu 24

<210> 283
<211> 22
<212> RNA
<213> Homo sapiens

<400> 283
cuagguaugg ucccagggau cc 22

<210> 284
<211> 19
<212> RNA
<213> Homo sapiens

<400> 284
ugggcguauc uguaugcua 19

<210> 285
<211> 22

<212> RNA
<213> Homo sapiens

<400> 285
aaaaguaauc gcgguuuuug uc 22

<210> 286
<211> 21
<212> RNA
<213> Homo sapiens

<400> 286
uuuugcaccu uuuggaguga a 21

<210> 287
<211> 22
<212> RNA
<213> Homo sapiens

<400> 287
ggagaaauua uccuuggugu gu 22

<210> 288
<211> 22
<212> RNA
<213> Homo sapiens

<400> 288
guagauucuc cuucuaugag ua 22

<210> 289
<211> 22
<212> RNA
<213> Homo sapiens

<400> 289
aacgcacuuc ccuuuagagu gu 22

<210> 290
<211> 22
<212> RNA
<213> Homo sapiens

<400> 290
aagugccucc uuuuagagug uu 22

<210> 291
<211> 22
<212> RNA
<213> Homo sapiens

<400> 291
aacaauaucc uggugcugag ug 22

<210> 292
<211> 22
<212> RNA
<213> Homo sapiens

<400> 292
aaaaguaauu gcggucuuug gu 22

<210> 293
<211> 22
<212> RNA
<213> Homo sapiens

<400> 293
aggcauugac uucucacuag cu 22

<210> 294
<211> 23
<212> RNA
<213> Homo sapiens

<400> 294
aaacucuacu uguccuucug agu 23

<210> 295
<211> 23
<212> RNA
<213> Homo sapiens

<400> 295
uauggcuuuu cauuccuaug uga 23

<210> 296
<211> 22
<212> RNA
<213> Homo sapiens

<400> 296
agaauugugg cuggacaucu gu 22

<210> 297
<211> 22
<212> RNA
<213> Homo sapiens

<400> 297
aaauuauugu acaucggaug ag 22

<210> 298
<211> 22
<212> RNA
<213> Homo sapiens

<400> 298
ugauugguac gucugugggu ag 22

<210> 299
<211> 22
<212> RNA
<213> Homo sapiens

<400> 299
gagugccuuc uuuuggagcg uu 22

<210> 300
<211> 22
<212> RNA
<213> Homo sapiens

<400> 300
aaaaguaauu gcggauuuug cc 22

<210> 301
<211> 22
<212> RNA
<213> Homo sapiens

<400> 301
cuauacggcc uccuagcuuu cc 22

<210> 302
<211> 22
<212> RNA
<213> Homo sapiens

<400> 302
uagguuaucc guguugccuu cg 22

<210> 303
<211> 22
<212> RNA
<213> Homo sapiens

<400> 303
cuuagcaggu uguauuauca uu 22

<210> 304
<211> 22
<212> RNA
<213> Homo sapiens

<400> 304
uucacauugu gcuacugucu gc 22

<210> 305
<211> 21
<212> RNA
<213> Homo sapiens

<400> 305
uugcucacug uucuucccua g 21

<210> 306
<211> 22
<212> RNA
<213> Homo sapiens

<400> 306
ccuauucuug auuacuuguu uc 22

<210> 307
<211> 23

<212> RNA
<213> Homo sapiens

<400> 307
uaagugcuuc cauguuuugg uga 23

<210> 308
<211> 23
<212> RNA
<213> Homo sapiens

<400> 308
uauggcuuuu uauuccuaug uga 23

<210> 309
<211> 22
<212> RNA
<213> Homo sapiens

<400> 309
agagguugcc cuuggugaau uc 22

<210> 310
<211> 22
<212> RNA
<213> Homo sapiens

<400> 310
aaaaugguuc ccuuuagagu gu 22

<210> 311
<211> 26
<212> RNA
<213> Homo sapiens

<400> 311
aaguaguugg uuuguaugag augguu 26

<210> 312
<211> 21
<212> RNA
<213> Homo sapiens

<400> 312
ccacaccgua ucugacacuu u 21

<210> 313
<211> 22
<212> RNA
<213> Homo sapiens

<400> 313
auaauacaug guuaaccucu uu 22

<210> 314
<211> 22
<212> RNA
<213> Homo sapiens

<400> 314
ugccugucua cacuugcugu gc 22

<210> 315
<211> 22
<212> RNA
<213> Homo sapiens

<400> 315
uagguaguuu ccuguuguug gg 22

<210> 316
<211> 22
<212> RNA
<213> Homo sapiens

<400> 316
uccuucauuc caccggaguc ug 22

<210> 317
<211> 22
<212> RNA
<213> Homo sapiens

<400> 317
caacuagacu gugagcuucu ag 22

<210> 318
<211> 22
<212> RNA
<213> Homo sapiens

<400> 318
accguggcuu ucgauuguua cu 22

<210> 319
<211> 23
<212> RNA
<213> Homo sapiens

<400> 319
aaagugcugc gacauuugag cgu 23

<210> 320
<211> 22
<212> RNA
<213> Homo sapiens

<400> 320
gggggucccc ggugcucgga uc 22

<210> 321
<211> 22
<212> RNA
<213> Homo sapiens

<400> 321
aagugcuucc uuuuagaggg uu 22

<210> 322
<211> 22
<212> RNA
<213> Homo sapiens

<400> 322
uagguaguuu cauguuguug gg 22

<210> 323
<211> 22
<212> RNA
<213> Homo sapiens

<400> 323
caaaguuuaa gauccuugaa gu 22

<210> 324
<211> 22
<212> RNA
<213> Homo sapiens

<400> 324
aaaaguauuu gcggguuuug uc 22

<210> 325
<211> 21
<212> RNA
<213> Homo sapiens

<400> 325
agugaaugau ggguucugac c 21

<210> 326
<211> 22
<212> RNA
<213> Homo sapiens

<400> 326
aaaaguaauu gcgaguuuua cc 22

<210> 327
<211> 21
<212> RNA
<213> Homo sapiens

<400> 327
guguugaaac aaucucuacu g 21

<210> 328
<211> 22
<212> RNA
<213> Homo sapiens

<400> 328
acugauuucu uuugguguuc ag 22

<210> 329
<211> 23

<212> RNA
<213> Homo sapiens

<400> 329
aagugccgcc aucuuuugag ugu 23

<210> 330
<211> 18
<212> RNA
<213> Homo sapiens

<400> 330
cgggcguggu gguggggg 18

<210> 331
<211> 22
<212> RNA
<213> Homo sapiens

<400> 331
uauugcacuu gucccggccu gu 22

<210> 332
<211> 22
<212> RNA
<213> Homo sapiens

<400> 332
gagcuuauuc auaaaagugc ag 22

<210> 333
<211> 21
<212> RNA
<213> Homo sapiens

<400> 333
gugggcgggg gcaggugugu g 21

<210> 334
<211> 22
<212> RNA
<213> Homo sapiens

<400> 334
uuaucagaau cuccaggggu ac 22

<210> 335
<211> 21
<212> RNA
<213> Homo sapiens

<400> 335
aucacauugc cagggauuac c 21

<210> 336
<211> 22
<212> RNA
<213> Homo sapiens

<400> 336
ucggccugac cacccacccc ac 22

<210> 337
<211> 22
<212> RNA
<213> Homo sapiens

<400> 337
uauugcacuc gucccggccu cc 22

<210> 338
<211> 22
<212> RNA
<213> Homo sapiens

<400> 338
uagcaccauu ugaaaucggu ua 22

<210> 339
<211> 25
<212> RNA
<213> Homo sapiens

<400> 339
gggcgacaaa gcaagacucu uucuu 25

<210> 340
<211> 22
<212> RNA
<213> Homo sapiens

<400> 340
agaucagaag gugauugugg cu 22

<210> 341
<211> 23
<212> RNA
<213> Homo sapiens

<400> 341
ugugcuugcu cgucccgccc gca 23

<210> 342
<211> 22
<212> RNA
<213> Homo sapiens

<400> 342
ccucugaaau ucaguucuuc ag 22

<210> 343
<211> 22
<212> RNA
<213> Homo sapiens

<400> 343
uagcaaaaac ugcaguuacu uu 22

<210> 344
<211> 24
<212> RNA
<213> Homo sapiens

<400> 344
aaagacauag gauagaguca ccuc 24

<210> 345
<211> 22
<212> RNA
<213> Homo sapiens

<400> 345
accuggcaua caauguagau uu 22

<210> 346
<211> 21
<212> RNA
<213> Homo sapiens

<400> 346
aucauagagg aaaauccacg u 21

<210> 347
<211> 22
<212> RNA
<213> Homo sapiens

<400> 347
caaagcgcuc cccuuuagag gu 22

<210> 348
<211> 23
<212> RNA
<213> Homo sapiens

<400> 348
aggaccugcg ggacaagauu cuu 23

<210> 349
<211> 21
<212> RNA
<213> Homo sapiens

<400> 349
gaaagcgcuu cucuuuagag g 21

<210> 350
<211> 22
<212> RNA
<213> Homo sapiens

<400> 350
caucuuaccg gacagugcug ga 22

<210> 351
<211> 22

<212> RNA
<213> Homo sapiens

<400> 351
uauagggauu ggagccgugg cg 22

<210> 352
<211> 18
<212> RNA
<213> Homo sapiens

<400> 352
gcaugggugg uucagugg 18

<210> 353
<211> 20
<212> RNA
<213> Homo sapiens

<400> 353
auuccuagaa auuguucaua 20

<210> 354
<211> 23
<212> RNA
<213> Homo sapiens

<400> 354
ggcagguucu cacccucucu agg 23

<210> 355
<211> 21
<212> RNA
<213> Homo sapiens

<400> 355
ucccacguug uggcccagca g 21

<210> 356
<211> 23
<212> RNA
<213> Homo sapiens

<400> 356
ugaguaccgc caugucuguu ggg 23

<210> 357
<211> 22
<212> RNA
<213> Homo sapiens

<400> 357
cuccuauaug augccuuucu uc 22

<210> 358
<211> 22
<212> RNA
<213> Homo sapiens

<400> 358
ugagguagua aguuguauug uu 22

<210> 359
<211> 22
<212> RNA
<213> Homo sapiens

<400> 359
uaauacuguc ugguaaaacc gu 22

<210> 360
<211> 20
<212> RNA
<213> Homo sapiens

<400> 360
guccgcucgg cgguggccca 20

<210> 361
<211> 22
<212> RNA
<213> Homo sapiens

<400> 361
cugggagagg guuguuuacu cc 22

<210> 362
<211> 22
<212> RNA
<213> Homo sapiens

<400> 362
uucccuuugu cauccuucgc cu 22

<210> 363
<211> 22
<212> RNA
<213> Homo sapiens

<400> 363
caaaaguaau uguggauuuu gu 22

<210> 364
<211> 22
<212> RNA
<213> Homo sapiens

<400> 364
aaaaguacuu gcggauuuug cu 22

<210> 365
<211> 20
<212> RNA
<213> Homo sapiens

<400> 365
aggaauguuc cuucuuugcc 20

<210> 366
<211> 22
<212> RNA
<213> Homo sapiens

<400> 366
caagcucgcu ucuauggguc ug 22

<210> 367
<211> 27
<212> RNA
<213> Homo sapiens

<400> 367
accuucuugu auaagcacug ugcuaaa 27

<210> 368
<211> 24
<212> RNA
<213> Homo sapiens

<400> 368
gcugguuuca uauggugguu uaga 24

<210> 369
<211> 22
<212> RNA
<213> Homo sapiens

<400> 369
uggacggaga acugauaagg gu 22

<210> 370
<211> 19
<212> RNA
<213> Homo sapiens

<400> 370
gggcgccugu gaucccaac 19

<210> 371
<211> 22
<212> RNA
<213> Homo sapiens

<400> 371
acuuuaacau ggaagugcuu uc 22

<210> 372
<211> 23
<212> RNA
<213> Homo sapiens

<400> 372
aucccuugca ggggcuguug ggu 23

<210> 373
<211> 22

<212> RNA
<213> Homo sapiens

<400> 373
aacgccauua ucacacuaaa ua 22

<210> 374
<211> 20
<212> RNA
<213> Homo sapiens

<400> 374
ugagcccugu ccucccgcag 20

<210> 375
<211> 21
<212> RNA
<213> Homo sapiens

<400> 375
aaacuacuga aaaucaaaga u 21

<210> 376
<211> 22
<212> RNA
<213> Homo sapiens

<400> 376
ugccuacuga gcugauauca gu 22

<210> 377
<211> 21
<212> RNA
<213> Homo sapiens

<400> 377
ccuggaaaca cugagguugu g 21

<210> 378
<211> 21
<212> RNA
<213> Homo sapiens

<400> 378
gcaggaacuu gugagucucc u 21

<210> 379
<211> 21
<212> RNA
<213> Homo sapiens

<400> 379
caagucacua gugguuccgu u 21

<210> 380
<211> 22
<212> RNA
<213> Homo sapiens

<400> 380
augguacccu ggcauacuga gu 22

<210> 381
<211> 22
<212> RNA
<213> Homo sapiens

<400> 381
accaucgacc guugauugua cc 22

<210> 382
<211> 23
<212> RNA
<213> Homo sapiens

<400> 382
ucugcucaua ccccaugguu ucu 23

<210> 383
<211> 23
<212> RNA
<213> Homo sapiens

<400> 383
uaagugcuuc cauguuugag ugu 23

<210> 384
<211> 22
<212> RNA
<213> Homo sapiens

<400> 384
auccgcgcuc ugacucucug cc 22

<210> 385
<211> 22
<212> RNA
<213> Homo sapiens

<400> 385
acucaaaaug ggggcgcuuu cc 22

<210> 386
<211> 19
<212> RNA
<213> Homo sapiens

<400> 386
auggauaagg cuuuggcuu 19

<210> 387
<211> 21
<212> RNA
<213> Homo sapiens

<400> 387
caucccuugc augguggagg g 21

<210> 388
<211> 22
<212> RNA
<213> Homo sapiens

<400> 388
cuuaucagau uguauuguaa uu 22

<210> 389
<211> 22
<212> RNA
<213> Homo sapiens

<400> 389
aacaucacag caagucugug cu 22

<210> 390
<211> 21
<212> RNA
<213> Homo sapiens

<400> 390
acagucugcu gagguuggag c 21

<210> 391
<211> 22
<212> RNA
<213> Homo sapiens

<400> 391
uucacaagga ggugucauuu au 22

**Description**

TECHNICAL FIELD OF THE INVENTION

The present invention relates to the use of polynucleotides for detecting miRNAs for diagnosing sarcoidosis in a blood sample from a patient. Further, the present invention relates to the use of means for diagnosing sarcoidosis comprising said polynucleotides. Furthermore, the present invention relates to a method for diagnosing sarcoidosis based on the determination of expression profiles of miRNAs representative for sarcoidosis.

BACKGROUND OF THE INVENTION

Today, biomarkers play a key role in early diagnosis, risk stratification, and therapeutic management of various diseases. However, many biomarkers were mainly discovered by candidate approach. By contrast, the recent development of high-throughput molecular technologies that allow with a reasonable effort the analysis of whole transcriptomes, proteomes, and metabolomes of individuals at risk, may lead to the discovery of novel biomarkers in an unbiased approach.

MicroRNAs (miRNAs) are a new class of biomarkers. They represent a group of regulatory elements that enable cells to fine-tune complex gene expression cascades in a wide range of biological processes. Since recently it is known that miRNAs are not only present in tissues but also in human blood both as free circulating nucleic acids and in mononuclear cells. This may be due to the fact that miRNAs expressed in diverse tissues or cells may be able to be released into circulating blood. Although the mechanism why miRNAs are found in human blood is not fully understood yet, this finding renders miRNAs to biological markers for diagnostics for various types of diseases based on blood analysis.

Various miRNA biomarkers have been proposed to indicate several diseases, e.g. diseases of the heart and cardiovascular system or multiple sclerosis. However, there is still a need for novel miRNAs as biomarkers for the detection and/or prediction of other types of diseases. Particularly, the potential role of miRNAs as biomarkers for the diagnosis and/or prognosis of lung diseases has not been systematically evaluated in detail yet. In addition, many of the miRNA biomarkers presently available for diagnosing and/or prognosing of diseases have shortcomings such as low sensitivity, no sufficient specificity or do not allow timely diagnosis. Accordingly, there is still a need to provide novel and efficient miRNAs or sets of miRNAs as biomarkers, effective methods and kits for the diagnosis and/or prognosis of diseases such as lung diseases. The term lung disease refers to many disorders affecting the lungs, such as asthma, chronic obstructive pulmonary disease (COPD), lung cancer, sarcoidosis and many other breathing problems. Lung cancer, for example, is a disease which consists of uncontrolled cell growth in tissues of the lung. This growth may lead to metastasis, which is the invasion of adjacent tissue and infiltration beyond the lungs. The vast majority of primary lung cancers are carcinomas of the lung, derived from epithelial cells. Lung cancer, the most common cause of cancer-related death in men and women, is responsible for more than 1.3 million deaths worldwide. The most common symptoms are shortness of breath, coughing (including coughing up blood), and weight loss. Chronic obstructive pulmonary disease (COPD) refers to chronic bronchitis and emphysema, a pair of commonly co-existing diseases of the lungs in which the airways become narrowed. This leads to a limitation of the flow of air to and from the lungs causing shortness of breath. In England, an estimated 842.100 of 50 million people have a diagnosis of COPD; translating into approximately one person in 59 receiving a diagnosis of COPD at some point in their lives. Several miRNA markers have been proposed to indicate lung cancer or COPD. However, many of these markers have shortcomings such as low sensitivity, no sufficient specificity or do not allow timely diagnosis. Sarcoidosis is characterized by the formation of tiny lumps of cells in various organs in the body. Particularly, sarcoidosis can occur in the lungs. The lumps are called granulomas because they look like grains of sugar or sand. These granulomas can grow and clump together, making several larger groups of lumps. If many granulomas form in the lung, they can affect how the lung works, which can cause symptoms of lung sarcoidosis. Lung sarcoidosis is not a form of cancer. At present, no miRNA markers are known to indicate lung sarcoidosis. Bons et al (Respiratory Medicine, Vol. 101, No. 8, July 20, 2007, pages 1687-1695) discloses potential biomarkers for diagnosis of sarcoidosis using proteomics in serum.

Accordingly, there is still a need to provide novel and efficient miRNAs (e.g. miRNAs signatures or sets of miRNAs) as biomarkers, effective methods and kits for the diagnosis and/or prognosis of lung diseases. Particularly, the potential role of miRNAs present in blood, e.g. human blood, as biomarkers for the diagnosis and/or prognosis of lung diseases in a patient, e.g. human, has not been systematically evaluated yet.

In the present disclosure it was assessed for the first time the expression of miRNAs on a whole-genome level in subjects suffering from a lung disease. They identified novel miRNAs which are significantly dysregulated in a biological sample, e.g. blood sample, of subjects suffering from a lung disease in comparison to healthy controls. The inventors of the present invention revealed that said single miRNAs can predict or determine a lung disease with high specificity, sensitivity and accuracy. The inventors of the present invention also pursued a multiple biomarker strategy to circumvent the above mentioned limitations by adding accuracy and predictive power. In detail, by using a machine learning approach, they identified unique miRNA signatures that can predict or determine a lung disease with even higher power, indicating that both, single miRNAs and especially complex miRNA signatures or sets derived from a biological sample, e.g. blood sample, can be used as novel biomarkers for the diagnosis and/or prognosis of a lung disease in a patient.

Further, distinguishing between the lung diseases lung cancer, sarcoidosis and/or COPD can be extremely difficult. Symptoms are frequently non-specific and often limited to wheezing, shortness of breath and/or chest pain. Many patients with lung cancer exhibit the same symptoms as patients with sarcoidosis and/or COPD. Thus, there is a need for additional diagnostic assays (differential diagnostic assays) that can assess the condition of a lung disease such as lung cancer, sarcoidosis and/or COPD in general and that can distinguish lung cancer from sarcoidosis and/or COPD, distinguish sarcoidosis from lung cancer and/or COPD and distinguish COPD from sarcoidosis and/or lung cancer in particular.

It is also possible that a patient successively or simultaneously develops lung cancer, sarcoidosis and/or COPD. Thus, there is also a need for diagnostic assays that allow the diagnosis and/or prognosis of sarcoidosis and lung cancer, sarcoidosis and COPD, lung cancer and COPD, and sarcoidosis, lung cancer and COPD at the same time.

The present disclosure revealed miRNAs (miRNAs signatures or sets of miRNAs as biomarkers that allow the differential diagnosis of sarcoidosis with respect to lung cancer and/or COPD, the differential diagnosis of lung cancer with respect to sarcoidosis and/or COPD and the differential diagnosis of COPD with respect to sarcoidosis and/or lung cancer with high specificity, sensitivity and accuracy. They also revelead miRNAs (miRNAs signatures or sets of mirNAs) that can predict or determine lung diseases such as sarcoidosis and lung cancer, sarcoidosis and COPD, lung cancer and COPD, and sarcoidosis, lung cancer and COPD with high specificity, sensitivity and accuracy.

SUMMARY OF THE INVENTION

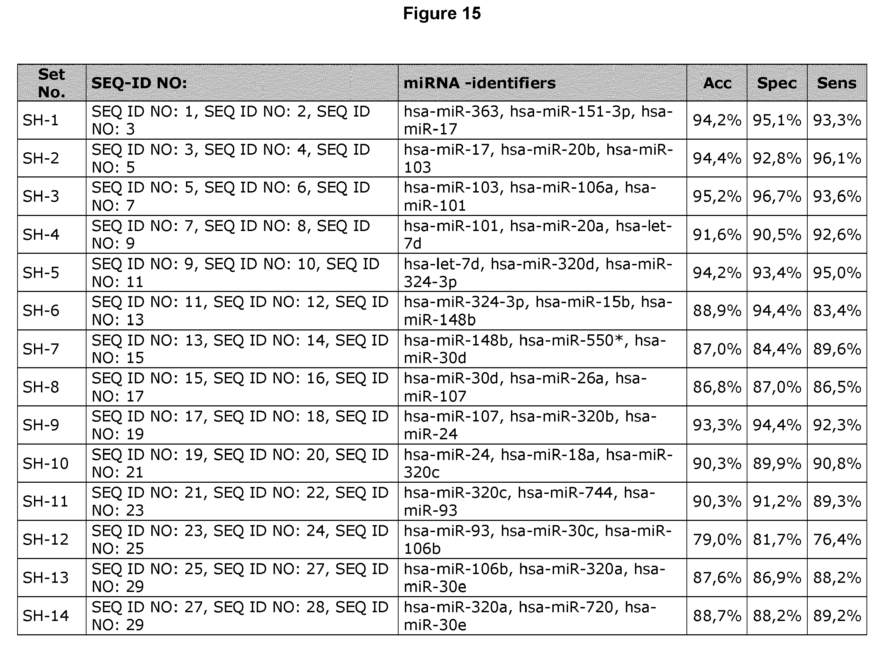

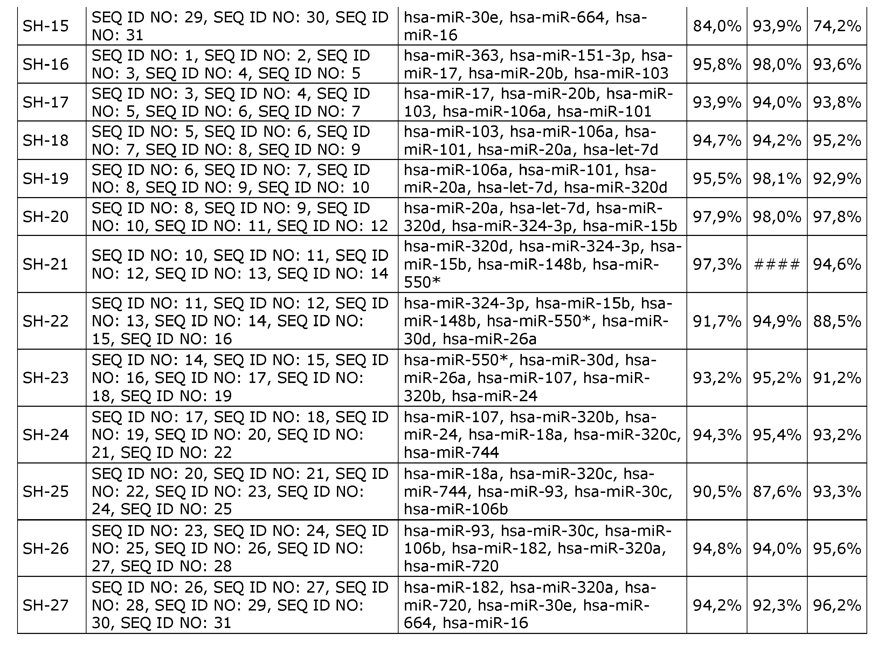

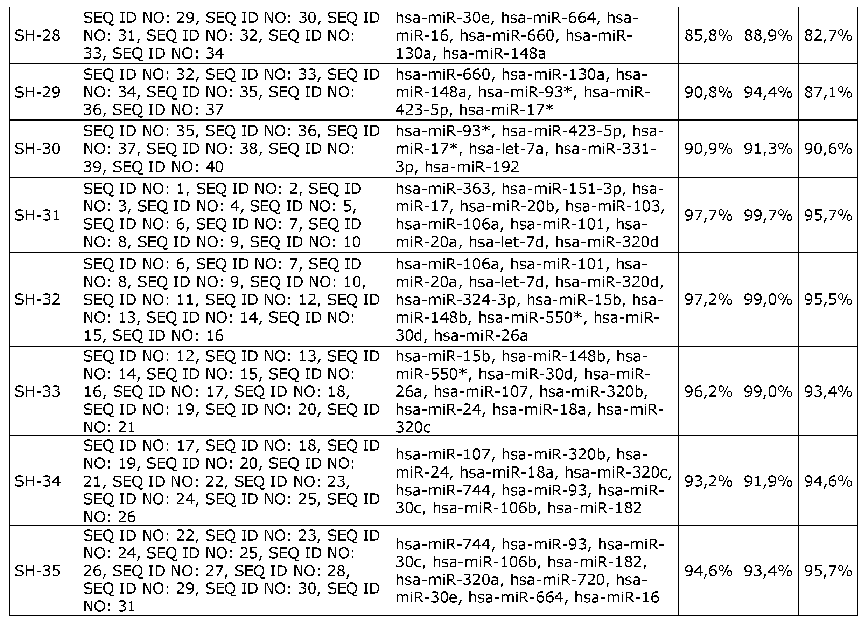

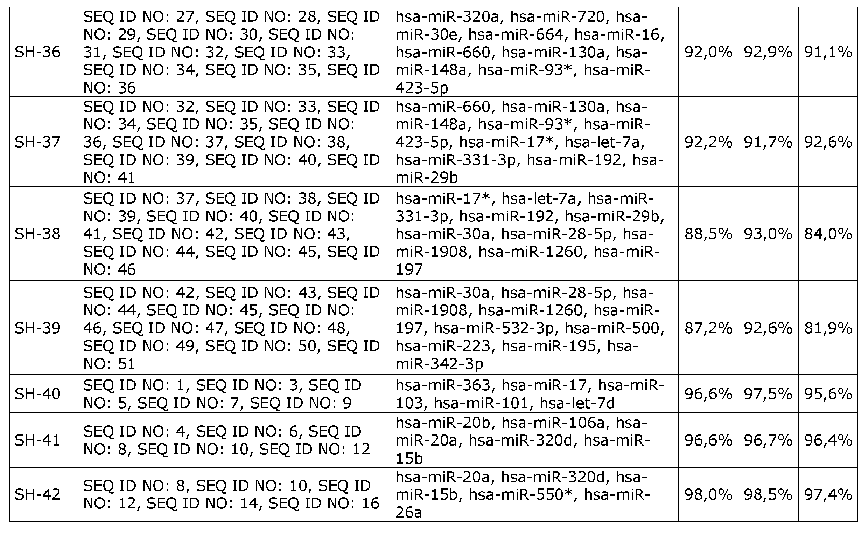

In a first aspect, the invention provides the use of polynucleotides for detecting miRNAs for diagnosing sarcoidosis in a blood sample from a patient, wherein the nucleotide sequences of said miRNAs comprise the nucleotide sequences according to Set No. 1 to 42 of Figure 15.

In a second aspect, the invention provides a method for diagnosing sarcoidosis comprising the steps of:

(i) determining an expression profile of miRNAs in a blood sample from a patient, wherein the nucleotide sequences of said miRNAs comprise the nucleotide sequences according to Set No. 1 to 42 of Figure 15, and
(ii) comparing said expression profile to a reference, wherein the comparison of said expression profile to said reference allows for the diagnosis of sarcoidosis.

In a third aspect, the invention provides the use of means for diagnosing sarcoidosis, wherein said means comprise a solid support, substrate, surface, platform or matrix and polynucleotides according to the first aspect, wherein said polynucleotides are attached to the solid support, substrate, surface, platform or matrix.

This summary of the invention does not necessarily describe all features of the invention.

DETAILED DESCRIPTION OF THE INVENTION

Preferably, the terms used herein are defined as described in "A multilingual glossary of biotechnological terms: (IUPAC Recommendations)", H.G.W. Leuenberger, B. Nagel, and H. Kölbl, Eds., Helvetica Chimica Acta, CH-4010 Basel, Switzerland, (1995).

To practice the present invention, unless otherwise indicated, conventional methods of chemistry, biochemistry, and recombinant DNA techniques are employed which are explained in the literature in the field (cf., e.g., Molecular Cloning: A Laboratory Manual, 2nd Edition, J. Sambrook et al. eds., Cold Spring Harbor Laboratory Press, Cold Spring Harbor 1989).

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used in this specification and in the appended claims, the singular forms "a", "an", and "the" include plural referents, unless the content clearly dictates otherwise. For example, the term "a test compound" also includes "test compounds".

The terms "microRNA" or "miRNA" refer to single-stranded RNA molecules of at least 10 nucleotides and of not more than 35 nucleotides covalently linked together. Preferably, the polynucleotides described herein are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The miRNAs regulate gene expression and are encoded by genes from whose DNA they are transcribed but miRNAs are not translated into protein (i.e. miRNAs are noncoding RNAs). The genes encoding miRNAs are longer than the processed mature miRNA molecules. The miRNAs are first transcribed as primary transcripts or pri-miRNAs with a cap and poly-A tail and processed to short, 70 nucleotide stem-loop structures known as pre-miRNAs in the cell nucleus. This processing is performed in animals by a protein complex known as the Microprocessor complex consisting of the nuclease Drosha and the doublestranded RNA binding protein Pasha. These pre-miRNAs are then processed to mature miRNAs in the cytoplasm by interaction with the endonuclease Dicer, which also initiates the formation of the RNA-induced silencing complex (RISC). When Dicer cleaves the pre-miRNA stem-loop, two complementary short RNA molecules are formed, but only one is integrated into the RISC. This strand is known as the guide strand and is selected by the argonaute protein, the catalytically active RNase in the RISC, on the basis of the stability of the 5' end. The remaining strand, known as the miRNA*, anti-guide (anti-strand), or passenger strand, is degraded as a RISC substrate. Therefore, the miRNA*s are derived from the same hairpin structure like the "normal" miRNAs. So if the "normal" miRNA is then later called the "mature miRNA" or "guide strand", the miRNA* is the "anti-guide strand" or "passenger strand".

The terms "microRNA*" or "miRNA*" refer to single-stranded RNA molecules of at least 10 nucleotides and of not more than 35 nucleotides covalently linked together. Preferably, the polynucleotides described herein are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally labels and/or elongated sequences (e.g. biotin stretches). The "miRNA*s", also known as the "anti-guide strands" or "passenger strands", are mostly complementary to the "mature miRNAs" or "guide strands", but have usually single-stranded overhangs on each end. There are usually one or more mispairs and there are sometimes extra or missing bases causing single-stranded "bubbles". The miRNA*s are likely to act in a regulatory fashion as the miRNAs (see also above). In the context of the present invention, the terms "miRNA" and "miRNA*" are interchangeable used. Described are (target) miRNAs suitable for the diagnosis and/or prognosis of a lung disease. Said (target) miRNAs are selected from the group consisting of SEQ ID NO: 1 to SEQ ID NO: 391.

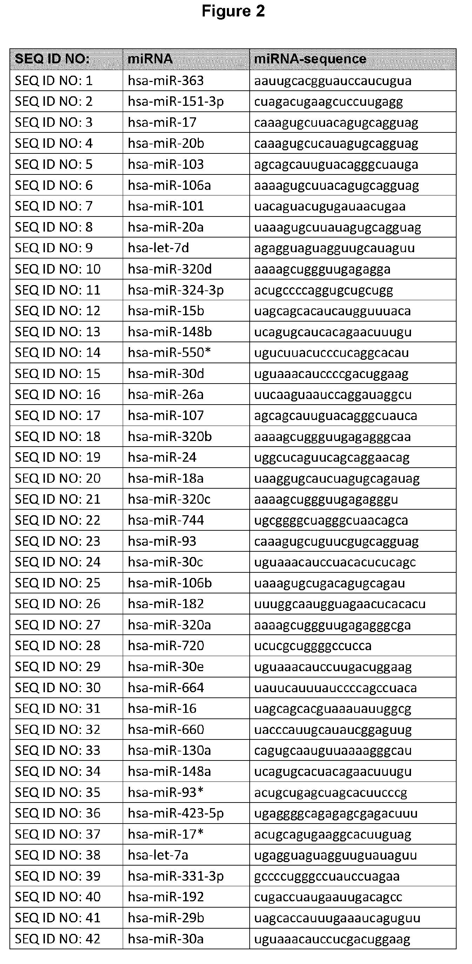

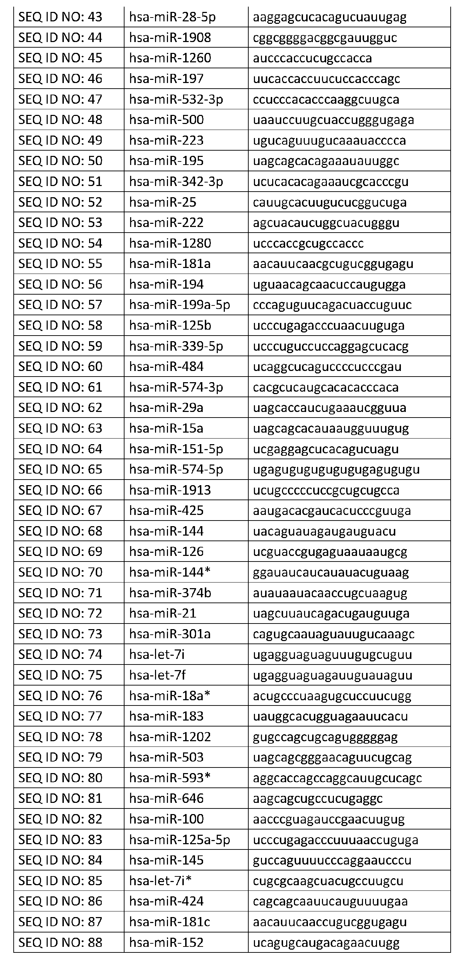

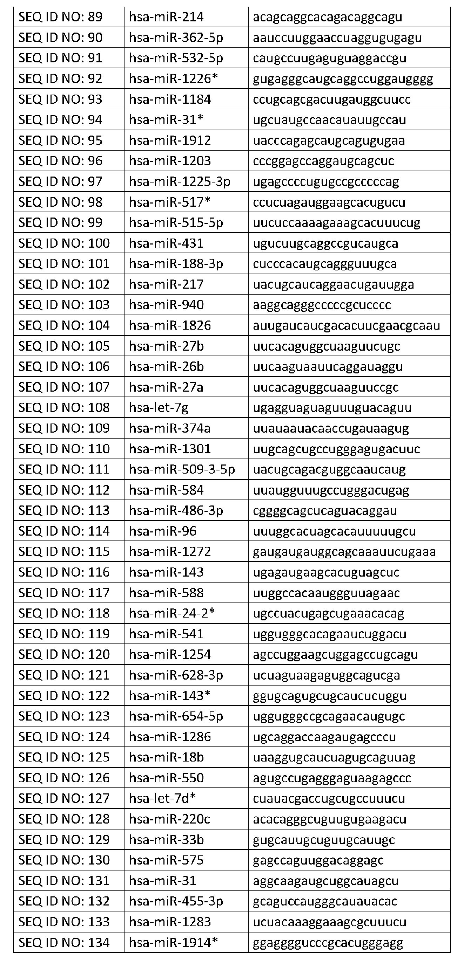

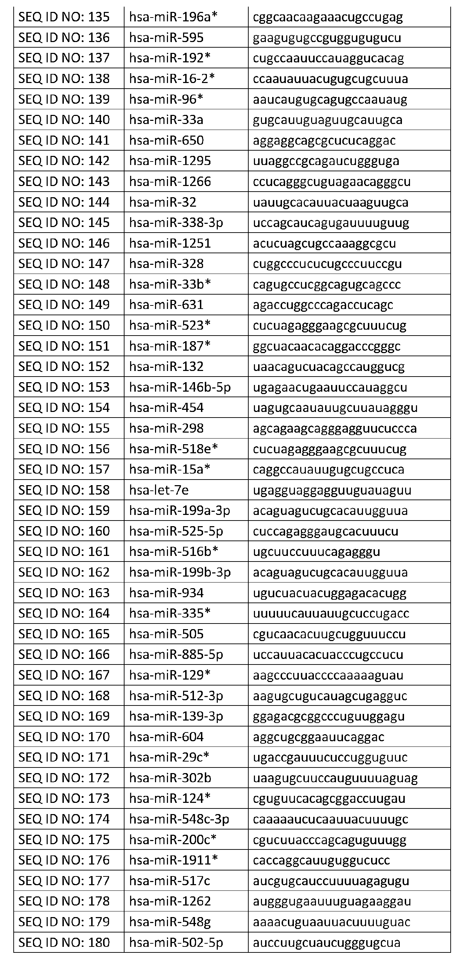

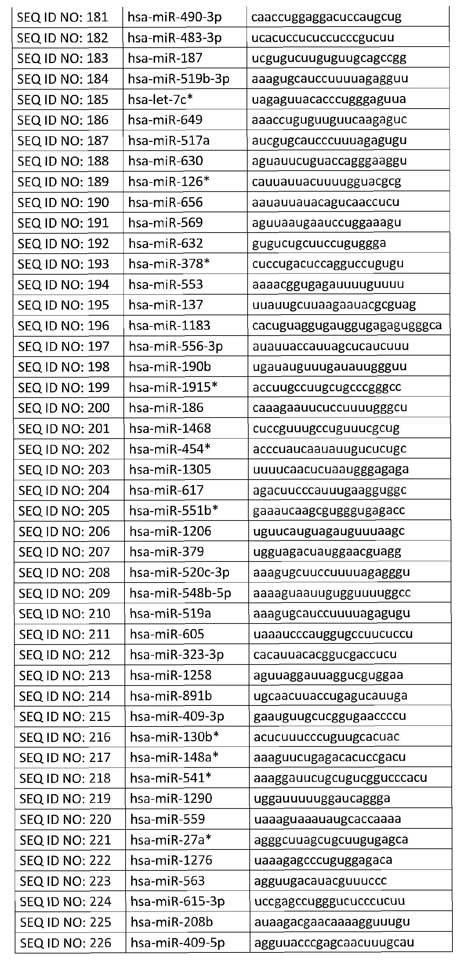

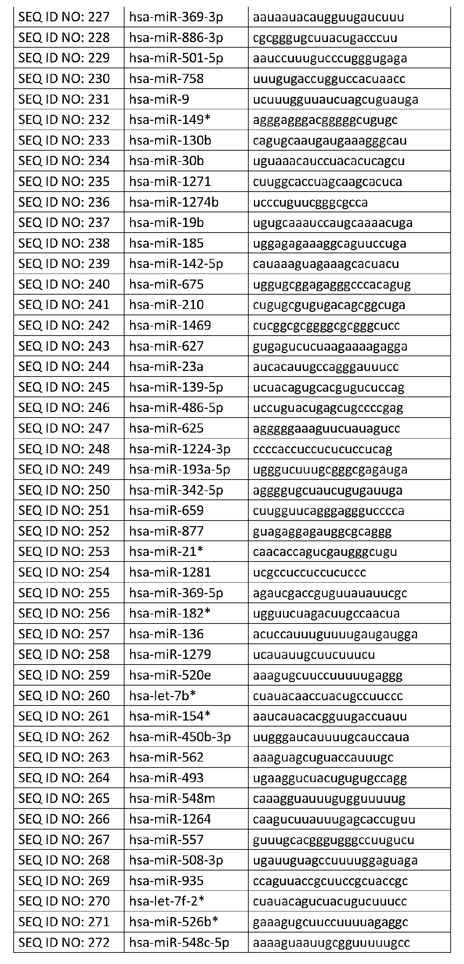

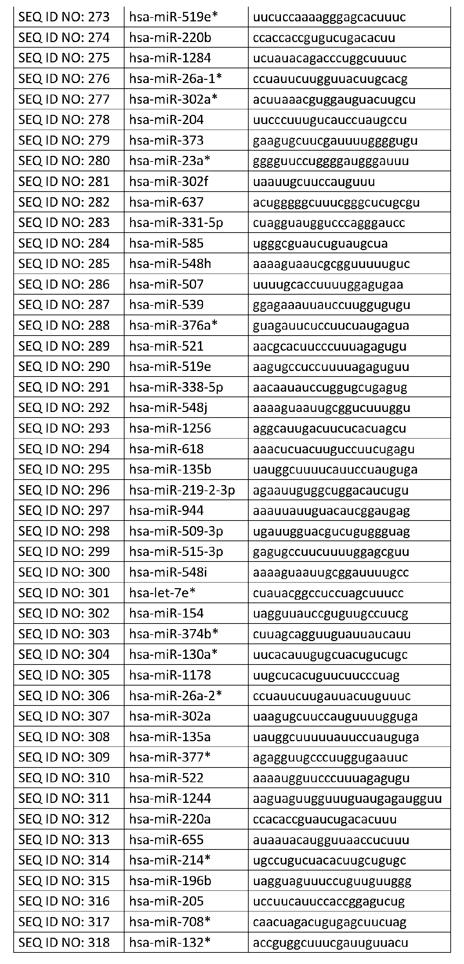

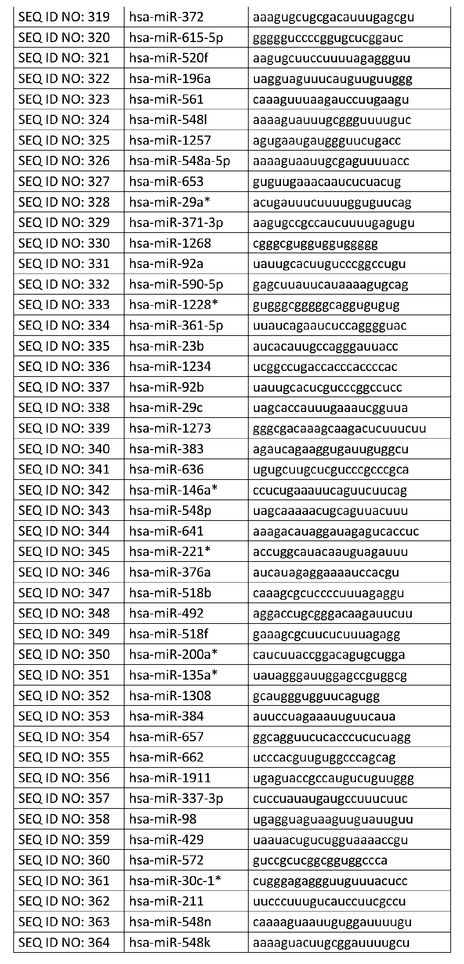

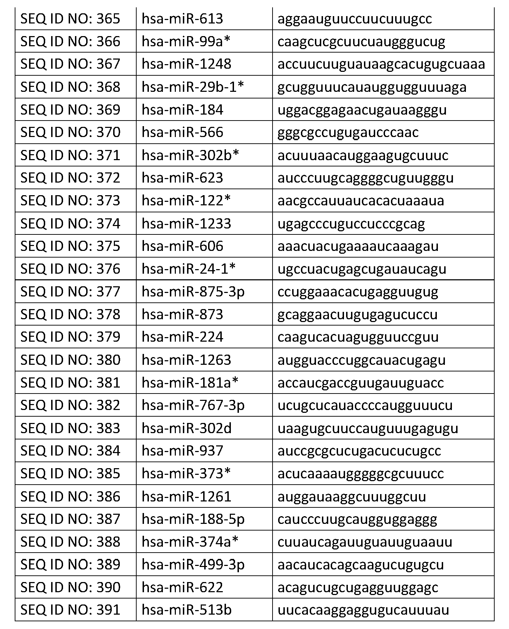

The term "miRBase" refers to a well established repository of validated miRNAs. The miRBase (www.mirbase.org) is a searchable database of published miRNA sequences and annotation. Each entry in the miRBase Sequence database represents a predicted hairpin portion of a miRNA transcript (termed mir in the database), with information on the location and sequence of the mature miRNA sequence (termed miR). Both hairpin and mature sequences are available for searching and browsing, and entries can also be retrieved by name, keyword, references and annotation. All sequence and annotation data are also available for download. The sequences of the miRNAs for diagnosis and/or prognosis of a lung disease in **Figure 2** are based on miRBase version 12.0, 13.0 and 14.0.

As used herein, the term "nucleotides" refers to structural components, or building blocks, of DNA and RNA. Nucleotides consist of a base (one of four chemicals: adenine, thymine, guanine, and cytosine) plus a molecule of sugar and one of phosphoric acid. The term "nucleosides" refers to glycosylamine consisting of a nucleobase (often referred to simply base) bound to a ribose or deoxyribose sugar. Examples of nucleosides include cytidine, uridine, adenosine, guanosine, thymidine and inosine. Nucleosides can be phosphorylated by specific kinases in the cell on the sugar's primary alcohol group (-CH2-OH), producing nucleotides, which are the molecular building blocks of DNA and RNA.

The term "polynucleotide", as used herein, means a molecule of at least 10 nucleotides and of not more than 35 nucleotides covalently linked together. Preferably, the polynucleotides described herein are molecules of 10 to 33 nucleotides or 15 to 30 nucleotides in length, more preferably of 17 to 27 nucleotides or 18 to 26 nucleotides in length, i.e. 10, 11, 12, 13, 14, 15, 16, 17, 18, 19, 20, 21, 22, 23, 24, 25, 26, 27, 28, 29, 30, 31, 32, 33, 34, or 35 nucleotides in length, not including optionally spacer elements and/or elongation elements described below. The depiction of a single strand of a polynucleotide also defines the sequence of the complementary strand. Polynucleotides may be single stranded or double stranded, or may contain portions of both double stranded and single stranded sequences. The term "polynucleotide" means a polymer of deoxyribonucleotide or ribonucleotide bases and includes DNA and RNA molecules, both sense and anti-sense strands. In detail, the polynucleotide may be DNA, both cDNA and genomic DNA, RNA, cRNA or a hybrid, where the polynucleotide sequence may contain combinations of deoxyribonucleotide or ribonucleotide bases, and combinations of bases including uracil, adenine, thymine, cytosine, guanine, inosine, xanthine, hypoxanthine, isocytosine and isoguanine. Polynucleotides may be obtained by chemical synthesis methods or by recombinant methods.

In the context of the present invention, a polynucleotide as a single polynucleotide strand provides a probe (e.g. miRNA capture probe) that is capable of binding to, hybridizing with, or detecting a target of complementary sequence, such as a nucleotide sequence of a miRNA or miRNA*, through one or more types of chemical bonds, usually through complementary base pairing, usually through hydrogen bond formation. Polynucleotides in their function as probes may bind target sequences, such as nucleotide sequences of miRNAs or miRNAs*, lacking complete complementarity with the polynucleotide sequences depending upon the stringency of the hybridization condition. There may be any number of base pair mismatches which will interfere with hybridization between the target sequence, such as a nucleotide sequence of a miRNA or miRNA*, and the single stranded polynucleotide described herein. However, if the number of mutations is so great that no hybridization can occur under even the least stringent hybridization conditions, the sequences are no complementary sequences. Described are polynucleotides in form of single polynucleotide strands as probes for binding to, hybridizing with or detecting complementary sequences of (target) miRNAs for diagnosing and/or prognosing of a lung disease. Said (target) miRNAs are selected from the group consisting of SEQ ID NO: 1 to SEQ ID NO: 391.

The polynucleotide, e.g. the polynucleotide used as a probe for detecting a miRNA or miRNA*, may be unlabeled, directly labeled, or indirectly labeled, such as with biotin to which a streptavidin complex may later bind. The polynucleotide, e.g. the polynucleotide used as a probe for detecting a miRNA or miRNA*, may also be modified, e.g. may comprise an elongation (EL) element. For use in a RAKE or MPEA assay, a polynucleotide with an elongation element may be used as a probe. The elongation element comprises a nucleotide sequence with 1 to 30 nucleotides chosen on the basis of showing low complementarity to potential target sequences, such as nucleotide sequences of miRNAs or miRNAs*, therefore resulting in no or a low degree of cross-hybridization to a target mixture. A homomeric sequence stretch $N_n$ with n = 1 to 30, i.e. 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30, and N = A or C, or T or G may be preferred. Particularly preferred is a homomeric sequence stretch $N_n$ with n = 1 to 12, i.e. 1, 2, 3, 4, 5, 6, 7, 8, 9, 10, 11, or 12, and N = A or C, or T or G. The polynucleotide, e.g. the polynucleotide used as a probe for detecting a miRNA or miRNA*, may be present in form of a tandem, i.e. in form of a polynucleotide hybrid of two different or identical polynucleotides, both in the same orientation, i.e. 5' to 3' or 3' to 5', or in different orientation, i.e. 5' to 3' and 3' to 5'. Said polynucleotide hybrid/tandem may comprise a spacer element. For use in a tandem hybridization assay, the polynucleotide hybrid/tandem as a probe may comprise a spacer (SP) element. The spacer element represents a nucleotide sequence with n = 0 to 12, i.e. 0, 1, 2, 3, 4, 5, 6, 7, 8, 9, 10, 11, or 12, nucleotides chosen on the basis of showing low complementarity to potential target sequences, such as nucleotide sequences of miRNAs or anti-miRNAs, therefore resulting in no or a low degree of cross-hybridization to a target mixture. It is preferred that n is 0, i.e. that there is no spacer between the two miRNA sequence stretches.

A non-homomeric sequence stretch $N_n$ with n = 1 to 30, i.e. 1, 2, 3, 4, 5, 6, 7, 8, 9, 10, 1 l, 12, 13, 14, 15, 16, 17, 18, 19, 20, 21, 22, 23, 24, 25, 26, 27, 28, 29, or 30, and N = A or C, or T or G may also be preferred. Particularly preferred is a non-homomeric sequence stretch that can be used as a priming site for a polymerase for a downstream amplification reaction.

For detection purposes, the miRNA(s) or miRNA*(s) may be employed unlabeled, directly labeled, or indirectly labeled, such as with biotin to which a streptavidin complex may later bind.

The term "label", as used herein, means a composition detectable by spectroscopic, photochemical, biochemical, immunochemical, chemical, or other physical means. For example, useful labels include 32P, fluorescent dyes, electron-dense reagents, enzymes (e.g., as commonly used in an ELISA), biotin, digoxigenin, or haptens and other entities which are or can be made detectable. A label may be incorporated into nucleic acids at any position, e.g. at the 3' or 5' end or internally. The polynucleotide for detecting a miRNA (polynucleotide probe) and/or the miRNAs itself may be labeled.

The term "stringent hybridization conditions", as used herein, means conditions under which a first nucleotide sequence (e.g. polynucleotide in its function as a probe for detecting a miRNA or miRNA*) will hybridize to a second nucleotide sequence (e.g. target sequence such as nucleotide sequence of a miRNA or miRNA*), such as in a complex mixture of nucleotide sequences. Stringent conditions are sequence-dependent and will be different in different circumstances. Stringent conditions may be selected to be about 5 to 10°C lower than the thermal melting point (Tm) for the specific sequence at a defined ionic strength, pH. The Tm may be the temperature (under defined ionic strength, pH, and nucleic acid concentration) at which 50% of the probes complementary to the target hybridize to the target sequence at equilibrium (as the target sequences are present in excess, at Tm, 50% of the probes are occupied at equilibrium). Stringent conditions may be those in which the salt concentration is less than about 1.0 M sodium ion, such as about 0.01 to 1.0 M sodium ion concentration (or other salts) at pH 7.0 to 8.3 and the temperature is at least about 20°C for short probes (e.g. about 10-35 nucleotides) and up to 60°C for long probes (e.g. greater than about 50 nucleotides). Stringent conditions may also be achieved with the addition of destabilizing agents such as formamide. For selective or specific hybridization, a positive signal may be at least 2 to 10 times background hybridization. Exemplary stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %, Tween 20, 0.1 x TE buffer, 0.5 mg/ml BSA, 0.1 mg/ml herring sperm DNA, incubating at 42°C with wash in 0.5x SSPE and 6x SSPE at 45°C.

The term "antisense", as used herein, refers to nucleotide sequences which are complementary to a specific DNA or RNA sequence. The term "antisense strand" is used in reference to a nucleic acid strand that is complementary to the "sense" strand.

The term "sensitivity", as used herein, means a statistical measure of how well a binary classification test correctly identifies a condition, for example how frequently it correctly classifies a lung disease into the correct type out of two or more possible types (e.g. a lung disease type such as a lung cancer type, a sarcoidosis type or a chronic obstructive pulmonary disease (COPD) type, and a healthy type). The sensitivity for class A is the proportion of cases that are determined to belong to class "A" by the test out of the cases that are in class "A". A theoretical, optimal prediction can achieve 100% sensitivity (i.e. predict all patients from the sick group as sick).

The term "specificity", as used herein, means a statistical measure of how well a binary classification test correctly identifies a condition, for example how frequently it correctly classifies a lung disease into the correct type out of two or more possible types (e.g. a lung disease type such as a lung cancer type, a sarcoidosis type or a chronic obstructive pulmonary disease (COPD) type, and a healthy type). The specificity for class A is the proportion of cases that are determined to belong to class "not A" by the test out of the cases that are in class "not A". A theoretical, optimal prediction can achieve 100% specificity (i.e. not predict anyone from the healthy group as sick).

The term "accuracy", as used herein, means a statistical measure for the correctness of classification or identification of sample types. The accuracy is the proportion of true results (both true positives and true negatives).

The term "lung disease", as used herein, encompasses any group of clinical symptoms compatible with a lung disease. The term "lung disease" refers to disorders affecting the lungs, such as asthma, lung sarcoidosis (or simply sarcoidosis), chronic obstructive pulmonary disease (COPD), infections like influenza, pneumonia and tuberculosis, lung cancer, and many other breathing problems.

Sarcoidosis is characterized by the formation of tiny lumps of cells in various organs in the body. Lung sarcoidosis is sarcoidosis that occurs in the lungs. The lumps are called granulomas because they look like grains of sugar or sand. These tiny granulomas can grow and clump together, making several larger groups of lumps. If many granulomas form in the lung, they can affect how the lung works, which can cause symptoms of lung sarcoidosis. Lung sarcoidosis is not a form of cancer. Lung sarcoidosis has an active and a non-active phase. In the active phase of lung sarcoidosis, the granulomas form and grow. In this phase, symptoms can develop, and scar tissue can form in the lungs. In the non-active phase of lung sarcoidosis, the inflammation goes down, and the granulomas stay the same size or shrink. However, the scars may remain and cause symptoms.

Lung cancer is a disease which consists of uncontrolled cell growth in tissues of the lung. This growth may lead to metastasis, which is the invasion of adjacent tissue and infiltration beyond the lungs. The vast majority of primary lung cancers are carcinomas of the lung, derived from epithelial cells. Lung cancer is the most common cause of cancer-related death in men and women. The most common symptoms are shortness of breath, coughing (including coughing up blood), and weight loss. The main types of lung cancer are small cell lung carcinoma and non-small cell lung carcinoma.

Chronic obstructive pulmonary disease (COPD), also known as chronic obstructive lung disease (COLD), chronic obstructive airway disease (COAD), chronic airflow limitation (CAL) and chronic obstructive respiratory disease (CORD), refers to chronic bronchitis and emphysema, a pair of commonly co-existing diseases of the lungs in which the airways become narrowed. This leads to a limitation of the flow of air to and from the lungs causing shortness of breath. In contrast to asthma, this limitation is poorly reversible and usually gets progressively worse over time. COPD is caused by noxious particles or gas, most commonly from tobacco smoking, which triggers an abnormal inflammatory response in the lung.

It should be noted that patients having sarcoidosis of the lungs can have or develop at the same time lung cancer and/or COPD, patients having lung cancer can have or develop at the same time COPD and/or sarcoidosis, or patients having COPD can have or develop at the same time lung cancer and/or sarcoidosis.

The term "patient", as used herein, may mean a subject (e.g. a human subject), which is suspected to be affected by a lung disease (e.g. sarcoidosis, lung cancer, and/or COPD). The patient may be diagnosed to be affected by a lung disease (e.g. sarcoidosis, lung cancer and/or COPD), i.e. diseased, or may be diagnosed to be not affected by a lung disease (e.g. sarcoidosis, lung cancer, and/or COPD), i.e. healthy. The patient may further be prognosed to develop a lung disease (e.g. sarcoidosis, lung cancer, and/or COPD), as the inventors of the present invention surprisingly found that miRNAs representative for a lung disease are already present in a biological sample (e.g. blood sample) before a lung disease occurs or during the early stage of a lung disease.

The term "patient", as used herein, may also mean a subject (e.g. a human subject), which is affected by a lung disease (e.g. sarcoidosis, lung cancer, and/or COPD), i.e. diseased. The patient may be retested for a lung disease and may be diagnosed to be still affected by a lung disease (e.g. sarcoidosis, lung cancer, and/or COPD), i.e. diseased, or not affected by a lung disease (e.g. sarcoidosis, lung cancer, and/or COPD) anymore, i.e. healthy, for example after therapeutic intervention (e.g. to evaluate the success of surgery and/or chemotherapy).

It should be noted that a patient that is diagnosed as being healthy, i.e. not suffering from a lung disease or a specific form of a lung disease (e.g. sarcoidosis, lung cancer, and/or COPD), or as staying healthy, i.e. not developing a lung disease or a specific form of a lung disease (e.g. sarcoidosis, lung cancer, and/or COPD), may possibly suffer from another disease not tested/known. For example, a patient tested for the presence of a lung disease may be diagnosed to be not affected by a lung disease, but may be affected by an acute coronary syndrome, multiples scleroses, diabetes, and/or gastric cancer which disease(s) has (have) not been tested. Further, for example, a patient tested for the presence of a specific form of a lung disease (e.g. sarcoidosis, lung cancer, and/or COPD) may suffer from an(other) specific form(s) of (a) lung disease(s) which disease(s) has (have) not been tested (e.g. lung cancer, COPD, and/or sarcoidosis).

The patient may be any mammal, including both a human and another mammal, e.g. an animal such as a rabbit, mouse, rat, or monkey. Human subjects as patients are particularly preferred.

The term "(control) subject", as used herein, may refer to a subject (e.g. human or animal subject) known to be affected by a lung disease (e.g. sarcoidosis, lung cancer, or COPD) (positive control), i.e. diseased. The term "(control) subject", as used herein, may also refer to a subject (e.g. human or animal subject) known to be not affected by a lung disease (e.g. sarcoidosis, lung cancer, or COPD) (negative control), i.e. healthy. The term "(control) subject" as used herein, may further refer to a subject (e.g. human or animal subject) having a specific stage of a lung disease (e.g. sarcoidosis, lung cancer, or COPD), for example, a subject having an early stage of a lung disease, a subject having an advanced stage of a lung disease, or a subject with subsiding symptoms of a lung disease. It should be noted that a (control) subject that is known to be healthy, i.e. not suffering from a lung disease (e.g. sarcoidosis, lung cancer, and/or COPD), may possibly suffer from another disease not tested/known.

The (control) subject may be any mammal, including both a human and another mammal, e.g. an animal such as a rabbit, mouse, rat, or monkey. Human (control) subjects are particularly preferred.

The expression levels or miRNAs were analysed (i) in biological samples, e.g. blood samples, of a cohort of controls (healthy subjects) and in biological samples, e.g. blood samples, of subjects suffering from sarcoidosis, (ii) in biological samples, e.g. blood samples, of subjects suffering from sarcoidosis and in biological samples, e.g. blood samples, of subjects suffering from lung cancer, and (iii) in biological samples, e.g. blood samples, of subjects suffering from sarcoidosis and in biological samples, e.g. blood samples, of subjects suffering from COPD. They succeeded in determining the miRNAs that are differentially regulated in biological samples, e.g. blood samples, (i) from subjects having sarcoidosis compared to a cohort of controls (healthy subjects), (ii) from subjects having sarcoidosis compared to subjects having lung cancer, and (iii) from subjects having sarcoidosis compared to subjects having COPD (see experimental section for experimental details). In total, they found 391 miRNAs which are suitable for the diagnosis and/or prognosis of a lung disease (e.g. sarcoidosis, lung cancer, and/or COPD). Said miRNAs are listed in **Figure 2.**

Additionally hypothesis tests were performed (e.g. t-test, limma-test) or other measurements (e.g. AUC, mutual information) on the expression level of the found miRNAs in all controls (healthy subjects) and subjects having a lung disease (e.g. sarcoidosis, lung cancer, or COPD). These tests resulted in a significance value (p-value) for each miRNA. This p-value is a measure for the diagnostic power of each miRNA to discriminate, for example, between two clinical conditions, e.g. absence of sarcoidosis (i.e. healthy) and presence of sarcoidosis (i.e. diseased). Since a manifold of tests are carried out, one for each miRNA, the p-values may be too optimistic and, thus, over-estimate the actual discriminatory power. Hence, the p-values are corrected for multiple testing by the Benjamini Hochberg approach.

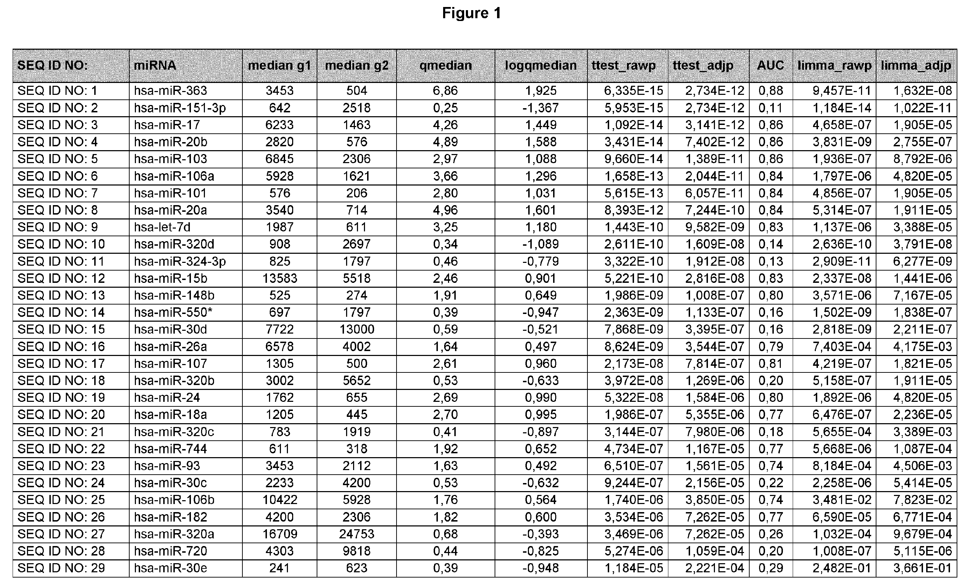

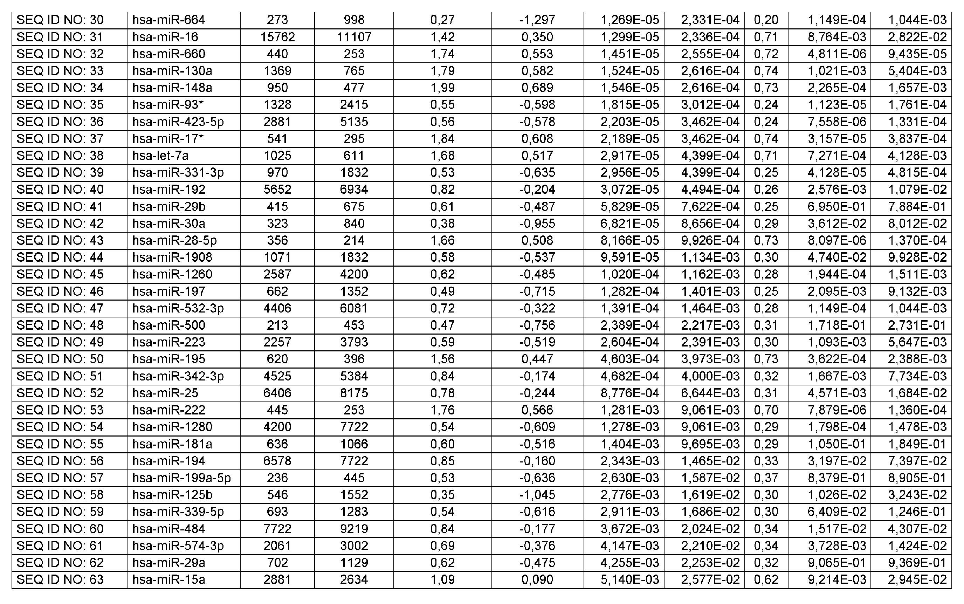

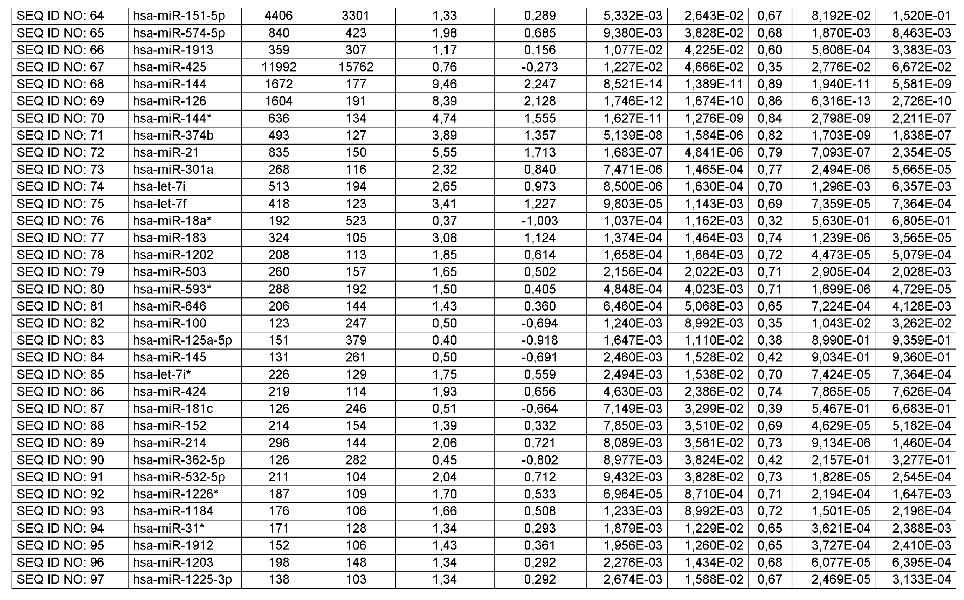

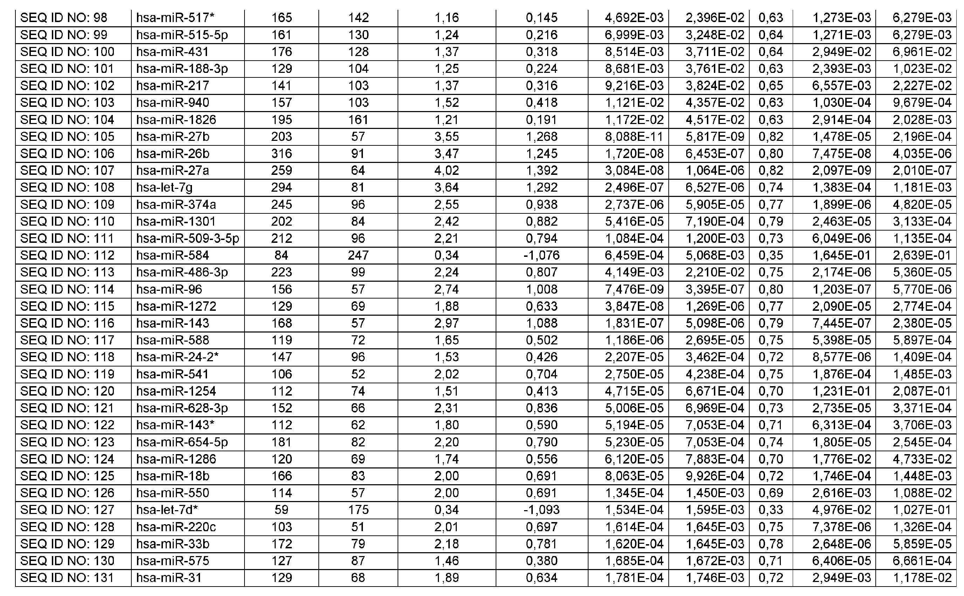

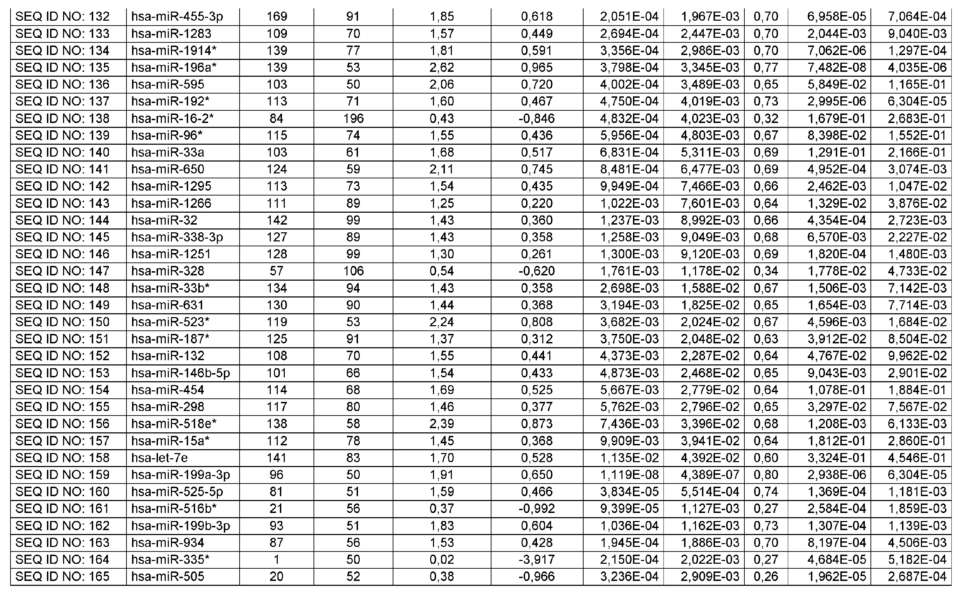

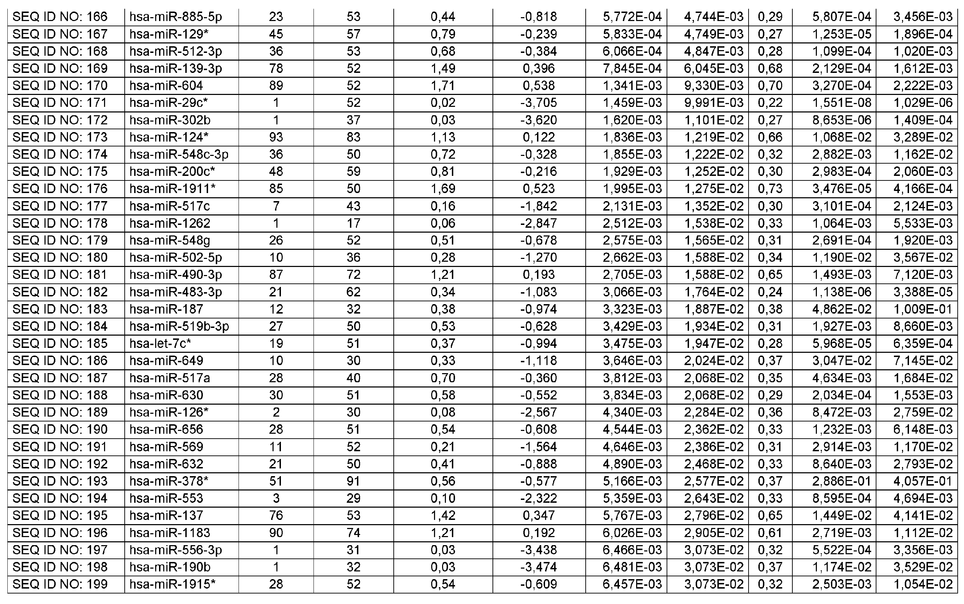

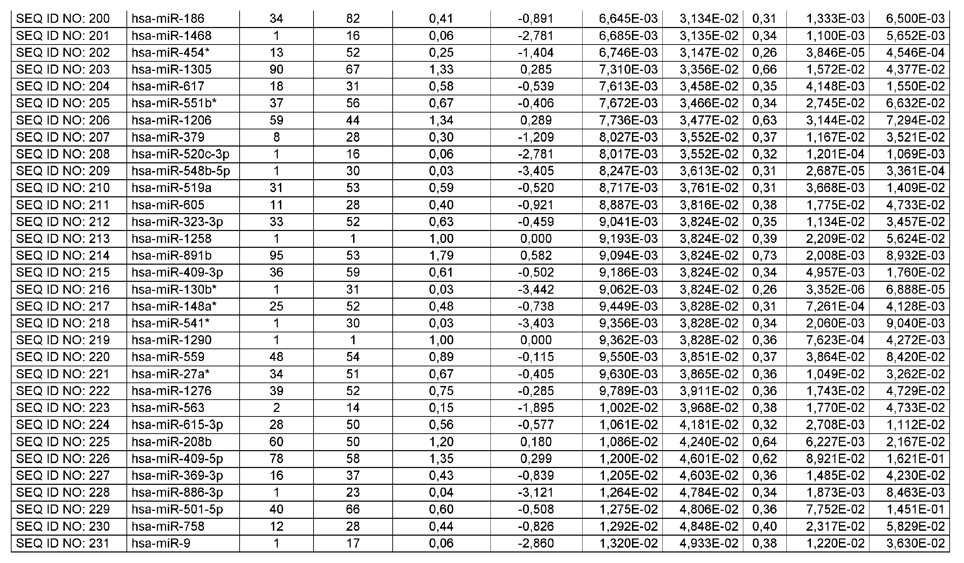

An overview of the 231 miRNAs that are found to be significantly dysregulated in blood samples of subjects having sarcoidosis compared to a cohort of controls (healthy subjects) and that performed best according to t-test, limma-test or AUC is provided in **Figure 1** (Experimental details: SEQ ID NO: sequence identification number, miRNA: identifier of the miRNA according to miRBase, median g1: median intensity obtained from microarray analysis for healthy controls, median g2: median intensity obtained from microarray analysis for subjects having sarcoidosis, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.). The group of miRNAs comprises miRNAs according to SEQ ID NO: 1 to SEQ ID NO: 231. The miRNAs comprised in this group are sorted in order of their t-test significance (see ttest_adjp= adjusted p-value calculated according to ttest) and intensity as described in more detail in the experimental section. Thus, the most predictive miRNAs are listed first. It should be noted that the lower the ttest_adjp value of a single miRNA, the higher is the diagnostic power of said miRNA. The median intensity obtained from the microarray represents a measure for the abundance of a miRNA in a sample and represents the expression level of this miRNA. The higher the intensity of a single miRNA, the higher the abundance/expression of said miRNA.

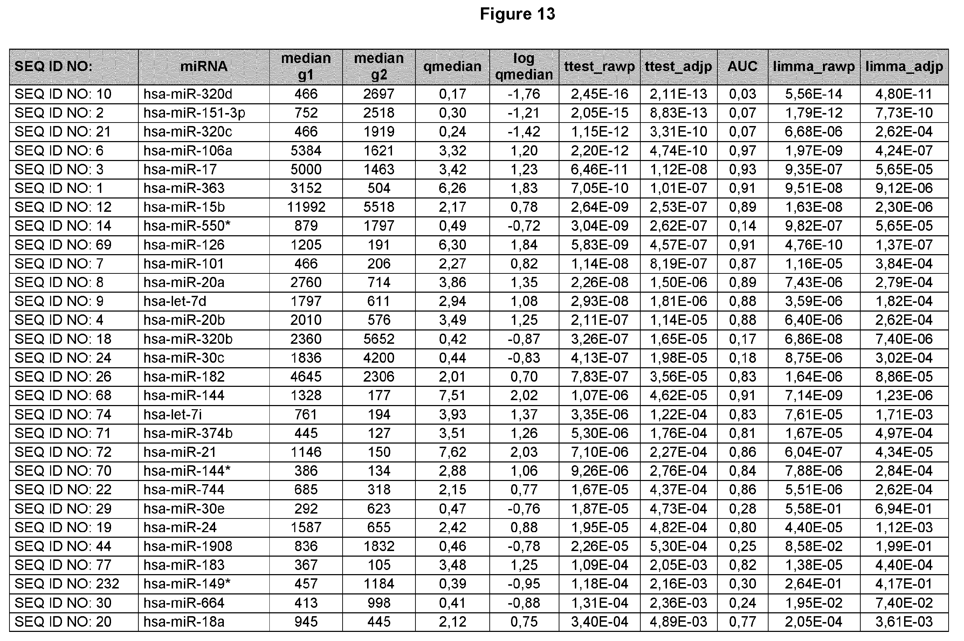

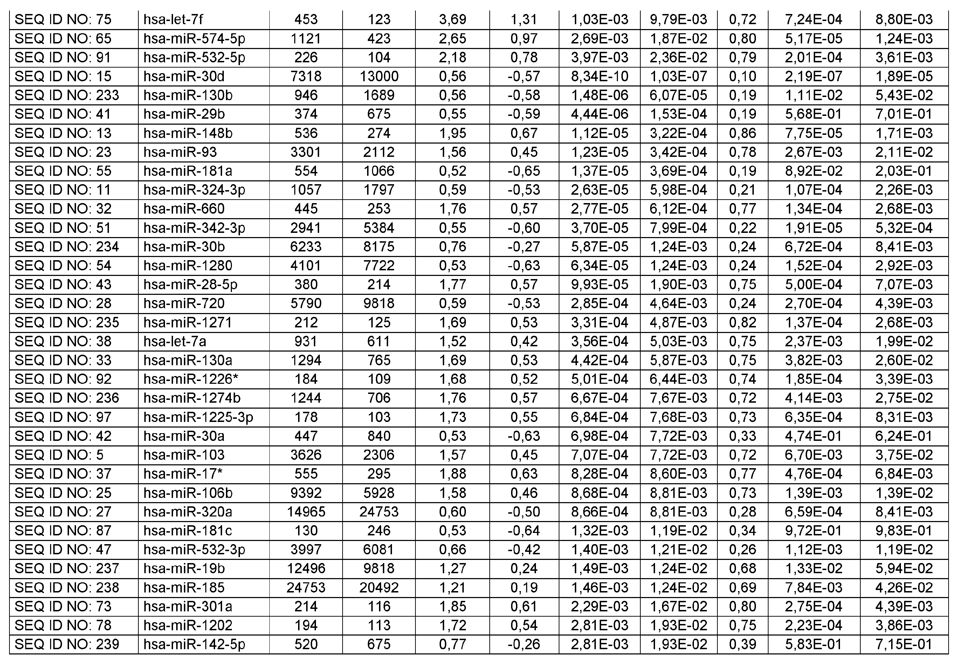

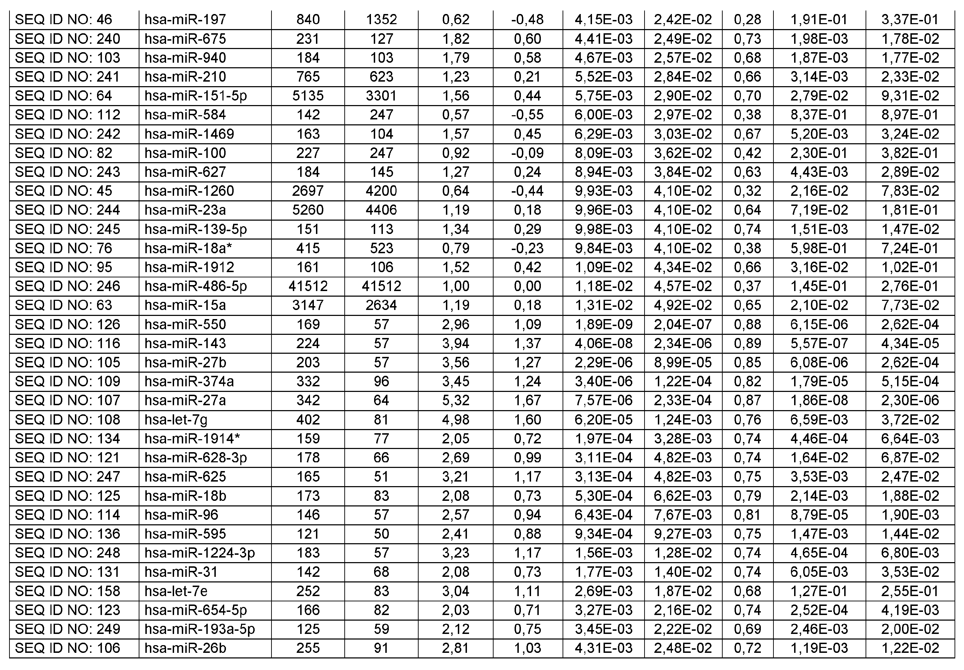

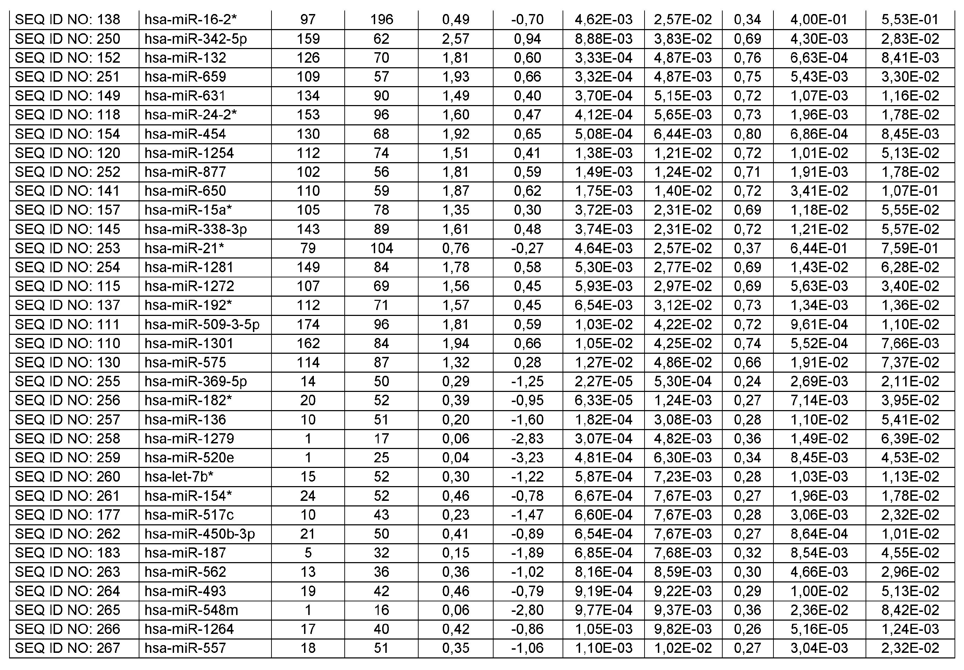

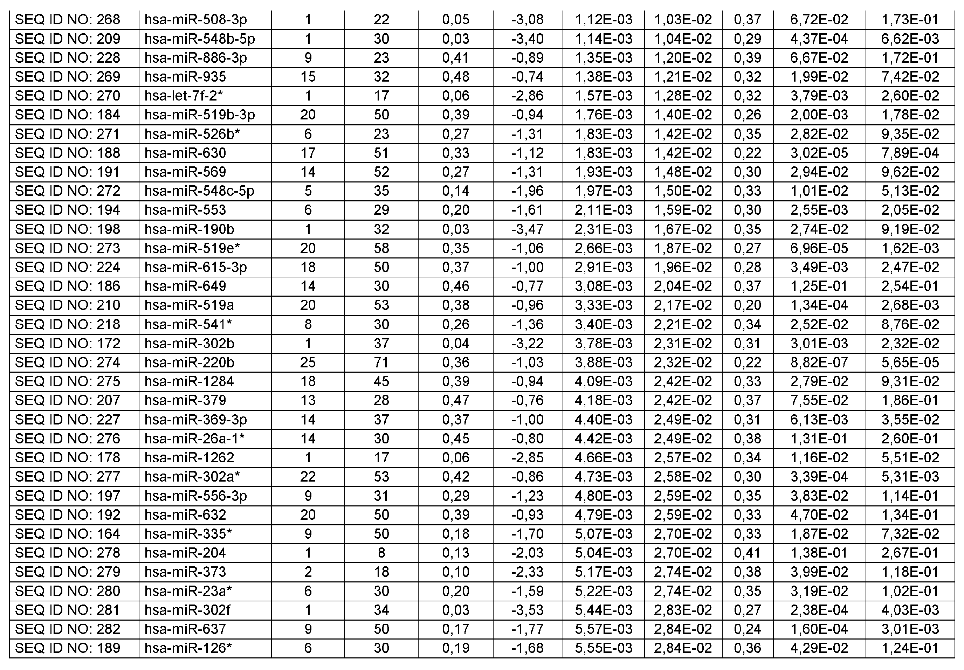

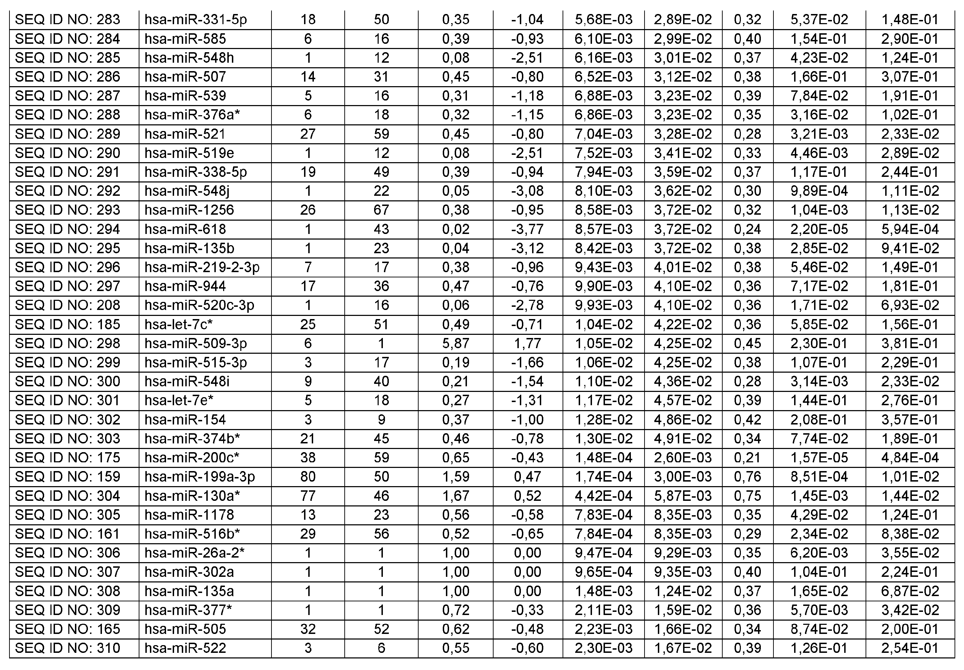

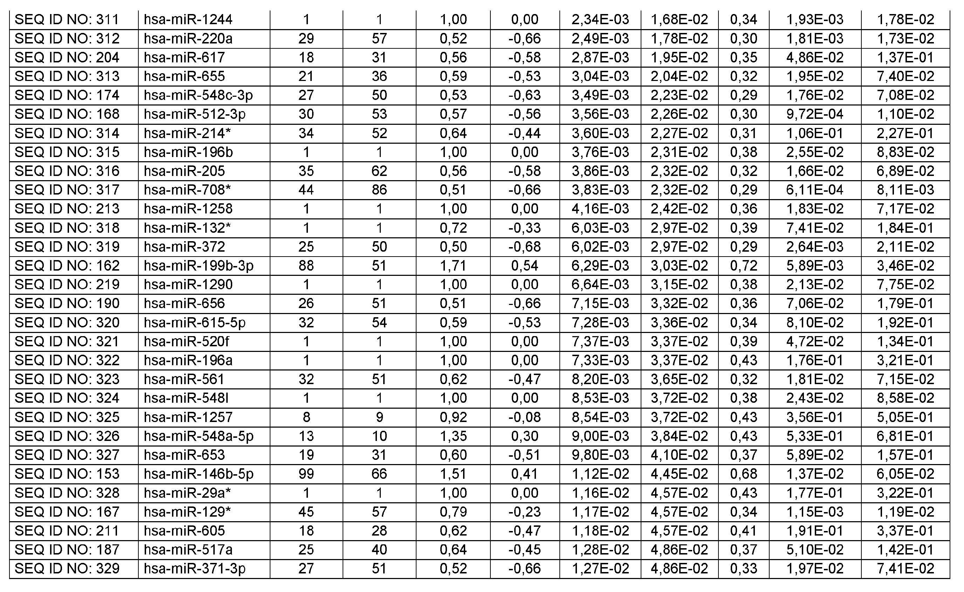

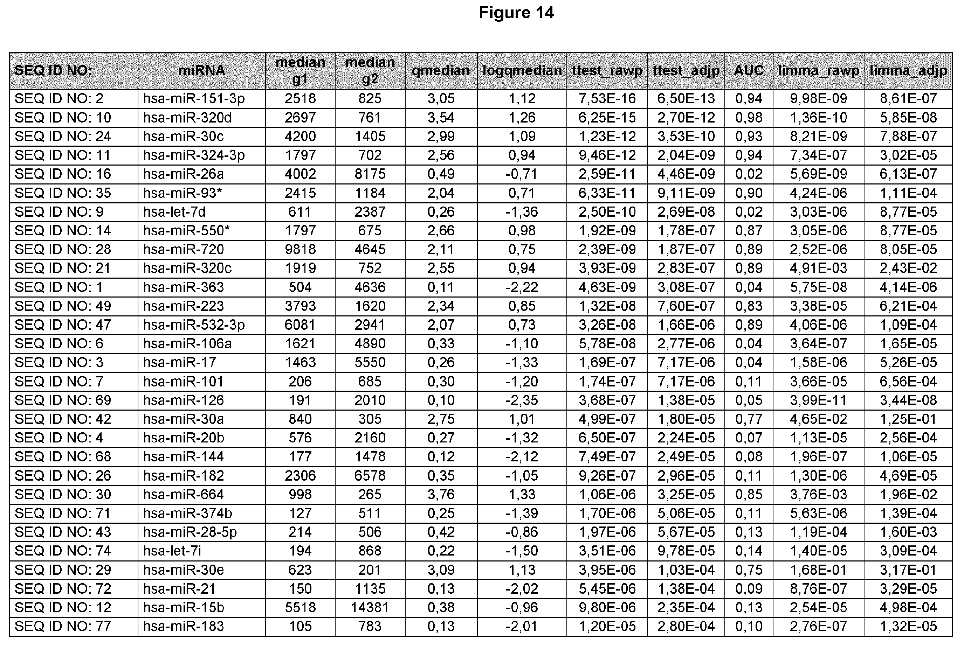

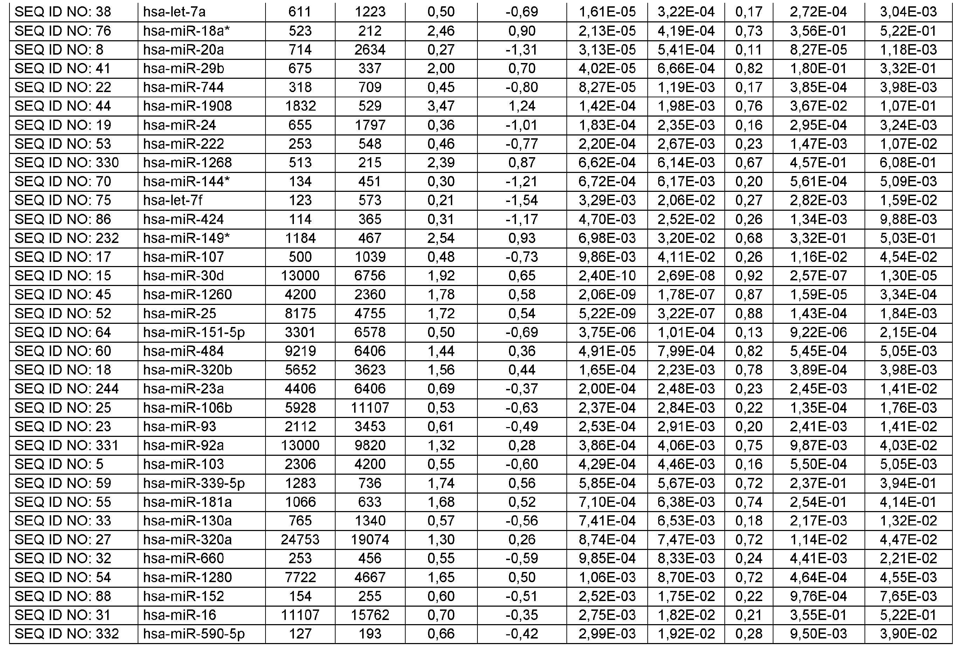

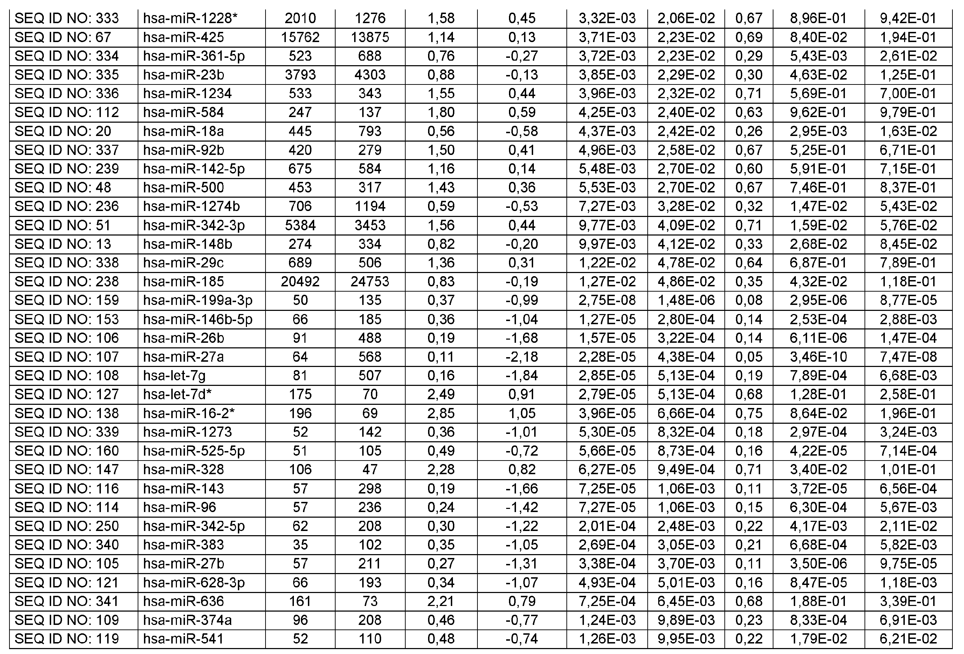

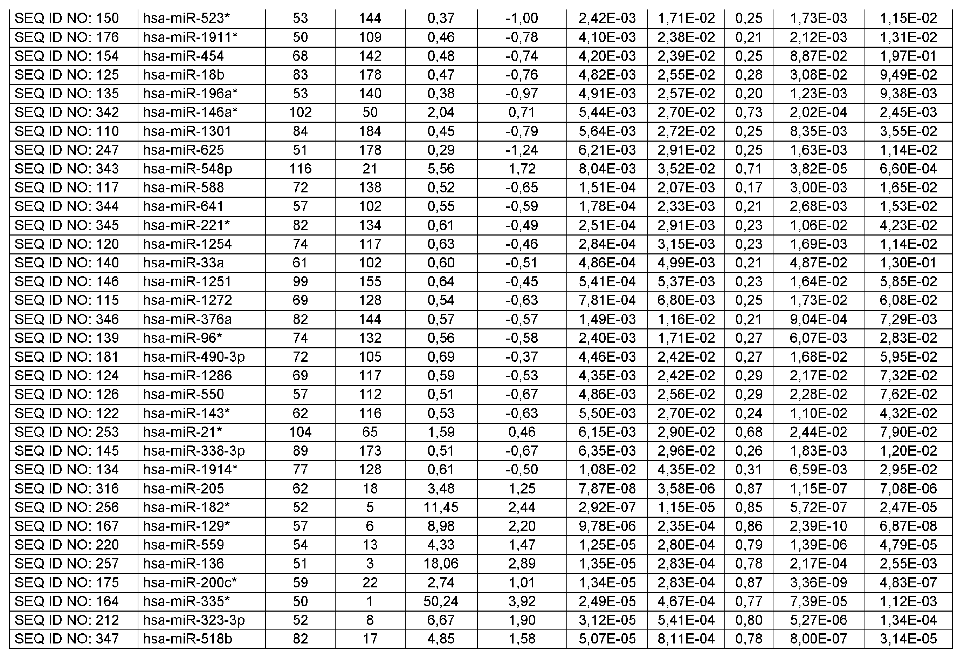

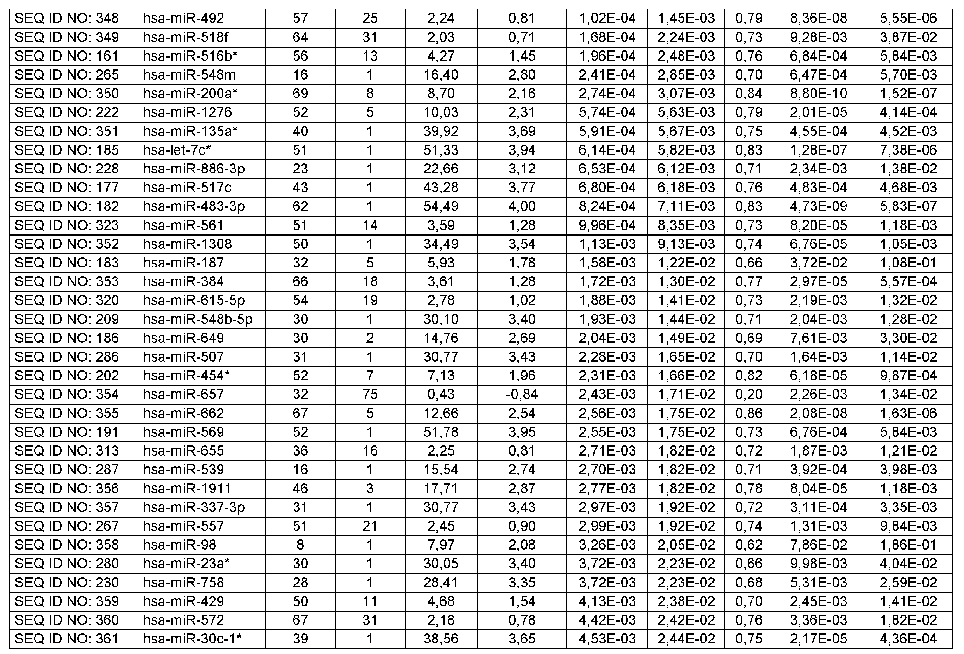

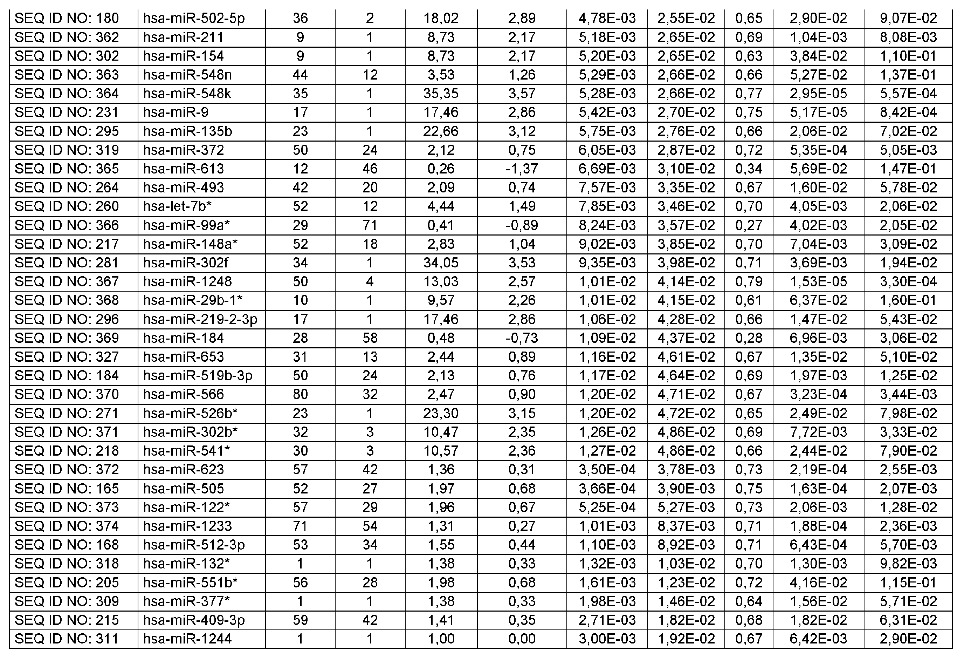

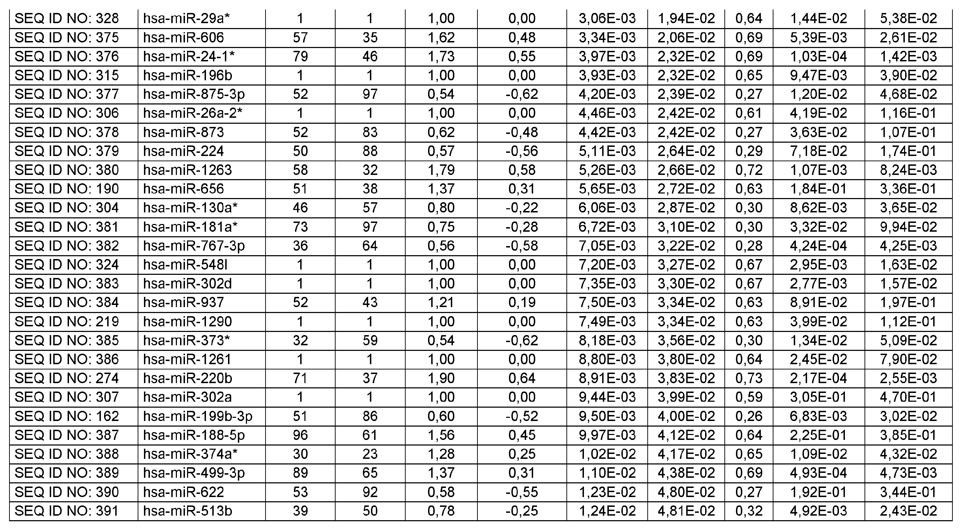

Further, an overview of the 229 miRNAs that are found to be significantly dysregulated in blood samples of subjects having sarcoidosis compared to subjects having lung cancer and that performed best according to t-test, limma-test or AUC is provided in **Figure 13** and an overview of the 226 miRNAs that are found to be significantly dysregulated in blood samples of subjects having sarcoidosis compared to subjects having COPD and that performed best according to t-test, limma-test or AUC is provided in **Figure 14**. Again, the miRNAs comprised in the group listed in **Figure 13** and the miRNAs comprised in the group listed in **Figure 14** are sorted in order of their t-test significance (see ttest_adjp= adjusted p-value calculated according to ttest) and intensity.

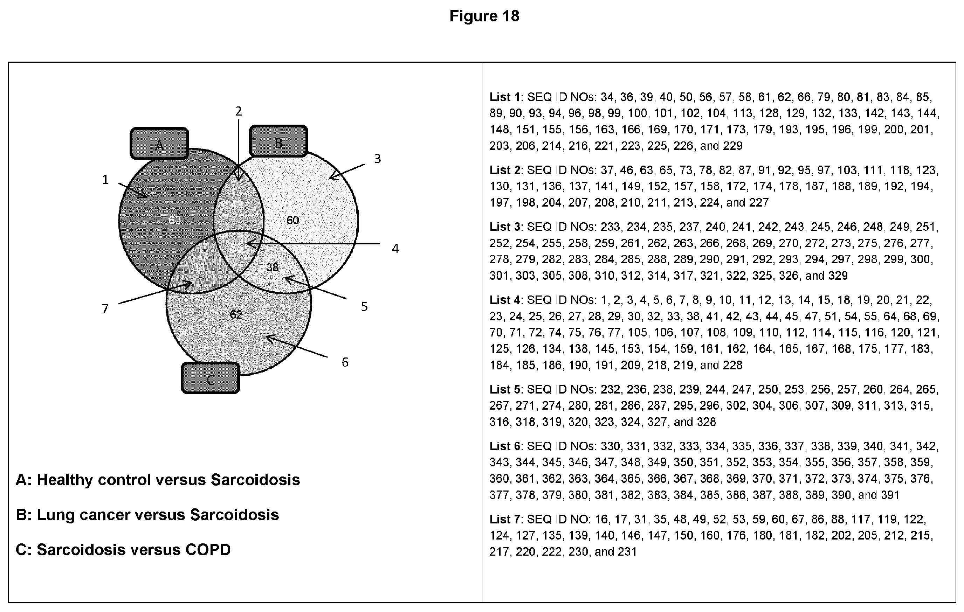

**Figure 18** shows in circle diagrams all 391 miRNAs which have been found to be suitable for the diagnosis and/or prognosis of a lung disease (e.g. sarcoidosis, lung cancer, and/or COPD).

In detail, circle A consisting of areas 1, 2, 4, and 7 represents the 231 miRNAs found to be differentially regulated in blood samples of subjects having sarcoidosis compared to a cohort of controls (healthy subjects). The inventors of the present invention found that these miRNAs, particularly the 62 miRNAs in area 1, are suitable for the diagnosis and/or prognosis of sarcoidosis in a biological sample from a patient. Further, circle B consisting of areas 3, 2, 4, and 5 represents the 229 miRNAs found to be differentially regulated in blood samples of subjects having sarcoidosis compared to subjects having lung cancer. The inventors of the present invention found that these miRNAs, particularly the 60 miRNAs in area 3, are suitable for the diagnosis and/or prognosis of lung cancer in a biological sample from a patient. Furthermore, circle C consisting of areas 6, 7, 4, and 5 represents the 226 miRNAs found to be differentially regulated in blood samples of subjects having sarcoidosis compared to subjects having COPD. The inventors of the present invention found that these miRNAs, particularly the 62 miRNAs in area 6, are suitable for the diagnosis and/or prognosis of COPD in a biological sample from a patient. In this respect, it should be noted that the detection of at least one miRNA comprised in area 1, at least one miRNA comprised in area 3 and/or at least one miRNA comprised in area 6 may allow the diagnosis and/or prognosis of sarcoidosis, lung cancer, and/or COPD, e.g. the diagnosis and/or prognosis of sarcoidosis, lung cancer, COPD, sarcoidosis and lung cancer, sarcoidosis and COPD, lung cancer and COPD, or sarcoidosis, lung cancer and COPD.

In addition, area 2 shared by circles A and B further represents the 43 miRNAs found to be differentially regulated in blood samples of subjects having sarcoidosis compared to a cohort of controls (healthy subjects) and subjects having sarcoidosis compared to subjects having lung cancer; area 7 shared by circles A and C represents the 38 miRNAs found to be differentially regulated in blood samples of subjects having sarcoidosis compared to a cohort of controls (healthy subjects) and subjects having sarcoidosis compared to subjects having COPD; area 5 shared by circles B and C represents the 38 miRNAs found to be differentially regulated in blood samples of subjects having sarcoidosis compared to subjects having lung cancer and subjects having sarcoidosis compared to subjects having COPD; and area 4 shared by circles A, B and C represents the 88 miRNAs found to be differentially regulated in blood samples of subjects having sarcoidosis compared to a cohort of controls (healthy subjects), subjects having sarcoidosis compared to subjects having lung cancer and subjects having sarcoidosis compared to subjects having COPD. The inventors of the present invention also found that these miRNAs that are shared by circles A, B and/or C further allow the differential diagnosis with respect to sarcoidosis, lung cancer, and/or COPD in a biological sample from a patient. This means that (i) the miRNAs that are shared by circles A and B (i.e. miRNAs comprised in area 2) allow to distinguish/differentiate between the lung diseases sarcoidosis and lung cancer, (ii) the miRNAs that are shared by circles A and C (i.e. miRNAs comprised in area 7) allow to distinguish/differentiate between the lung diseases sarcoidosis and COPD, (iii) that the miRNAs that are shared by circles B and C (i.e. miRNAs comprised in area 5) allow to distinguish/differentiate between the lung diseases lung cancer and COPD, and (iv) that the miRNAs that are shared by circles A, B and C (i.e. miRNAs comprised in area 4) allow to distinguish/differentiate between the lung diseases sarcoidosis, lung cancer and COPD.

More specifically, the miRNAs comprised in area 2, for example, allow to diagnose and/or prognose sarcoidosis in a patient and to differentiate said disease with respect to lung cancer, the miRNAs comprised in area 7, for example, allow to diagnose and/or prognose sarcoidosis in a patient and to differentiate said disease with respect to COPD, the miRNAs comprised in area 5, for example, allow to diagnose and/or prognose lung cancer in a patient and to differentiate said disease with respect to COPD, and the miRNAs comprised in area 4, for example, allow to diagnose and/or prognose sarcoidosis in a patient and to differentiate said disease with respect to COPD and lung cancer.

The miRNAs comprised in areas 1, 3 and 6 are valuable as they allow to decide whether (i) the patient is healthy or suffers from sarcoidosis, (ii) the patient is healthy or suffers from lung cancer, or (iii) the patient is healthy or suffers from COPD, respectively. Thus, said miRNAs allow the differentiation between 2 conditions.

The miRNAs comprised in areas 2, 7, and 5, however, are very valuable as they allow the differentiation between 3 conditions. In detail, (i) the miRNAs comprised in area 2 allow the differentiation between the conditions healthy, suffering from sarcoidosis and suffering from lung cancer, (ii) the miRNAs comprised in area 7 allow the differentiation between the conditions healthy, suffering from sarcoidosis and suffering from COPD, and (iii) the miRNAs comprised in area 5 allow the differentiation between the conditions suffering from sarcoidosis, suffering from COPD and suffering from lung cancer. It should be noted that the result of the differential diagnosis in item (i) above may be that the patient is healthy, suffers from sarcoidosis, suffers from lung cancer, or suffers from a combination thereof; that the result of the differential diagnosis in item (ii) above may be that the patient is healthy, suffers from sarcoidosis, suffers from COPD, or suffers from a combination thereof; or that the result of the differential diagnosis in item (iii) above may be that the patient suffers from sarcoidosis, suffers from lung cancer, suffers from COPD, or suffers from a combination thereof. This is caused by the fact that sometimes no differentiation between the different diseases/conditions (e.g. the conditions COPD and lung cancer) is possible as the patient tested also suffers from said disease(s)/condition(s) (e.g. the conditions COPD and lung cancer).

The miRNAs comprised in area 4 are particularly valuable as they allow the differentiation between 4 conditions. In detail, area 4 allows the differentiation between the conditions healthy, suffering from sarcoidosis, suffering from lung cancer, and suffering from COPD. It should be noted that the result of this differential diagnosis may be that the patient is healthy, suffers from sarcoidosis, suffers from lung cancer, suffers from COPD, suffers from sarcoidosis and lung cancer, suffers from sarcoidosis and COPD, suffers from lung cancer and COPD, or suffers from sarcoidosis, lung cancer, and COPD.

Usually the diagnostic power of a single miRNA biomarker is not sufficient to reach high accuracy, specificity and sensitivity, for example, for the discrimination between two clinical conditions, e.g. healthy, i.e. absence of a lung disease, and diseased, i.e. presence of a lung disease. However, the inventors of the present invention surprisingly found that also a single miRNA, wherein the nucleotide sequence of said miRNA is selected from the group consisting of SEQ ID NO: 1 to SEQ ID NO: 391, can predict or determine with high diagnostic accuracy, specificity and sensitivity a lung disease (e.g. sarcoidosis, lung cancer, and/or COPD) in a patient, e.g. a human.

One or more miRNA biomarkers were employed i.e. sets (signatures) of miRNA biomarkers, to further increase and/or improve the performance for diagnosing and/or prognosing of patients, e.g. humans, having a lung disease (e.g. sarcoidosis, lung cancer, and/or COPD).

In order to be able to better discriminate, for example, between two or more clinical conditions, e.g. the presence of a lung disease such as sarcoidosis, lung cancer, and/or COPD and the absence of a lung disease such as sarcoidosis, lung cancer, and/or COPD, for a defined set (signature) of miRNA biomarkers, the inventors of the present invention applied a machine learning approach (e.g. t-test, AUC, support vector machine, hierarchical clustering, or k-means) which leads to an algorithm or a mathematical function that is trained by reference data (i.e. data of reference miRNA expression profiles from the two or more clinical conditions, e.g. the presence of a lung disease such as sarcoidosis, lung cancer, and/or COPD and the absence of a lung disease such as sarcoidosis, lung cancer, and/or COPD, for the defined set (signature) of miRNA markers) to discriminate between the two or more statistical classes (i.e. two or more clinical conditions), e.g. the presence of a lung disease such as sarcoidosis, lung cancer, and/or COPD and the absence of a lung disease such as sarcoidosis, lung cancer, and/or COPD.

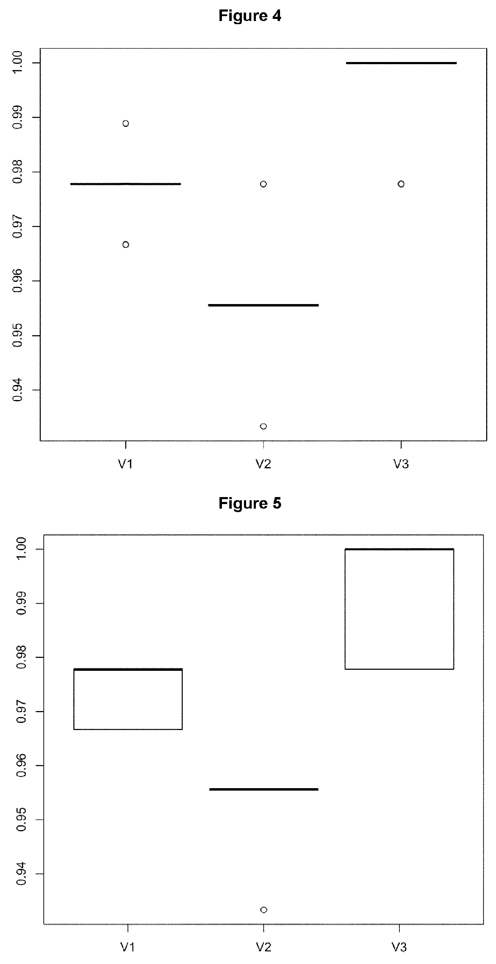

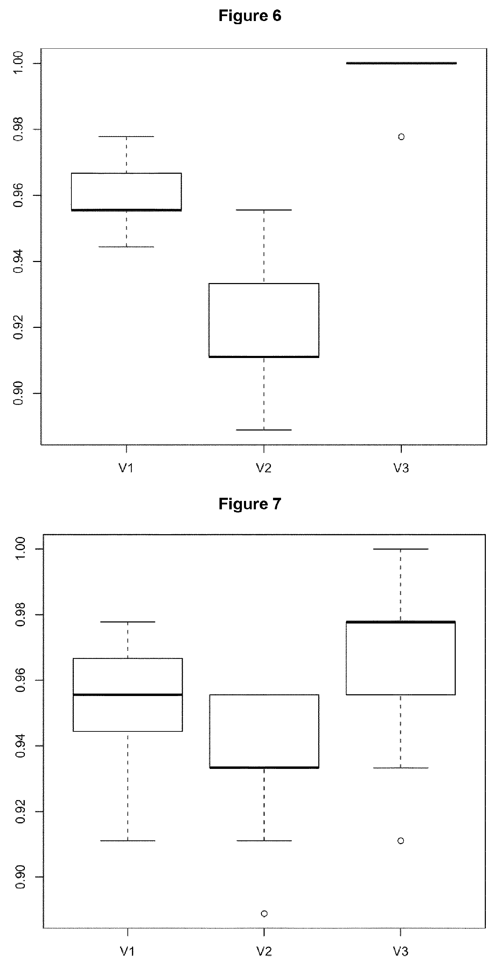

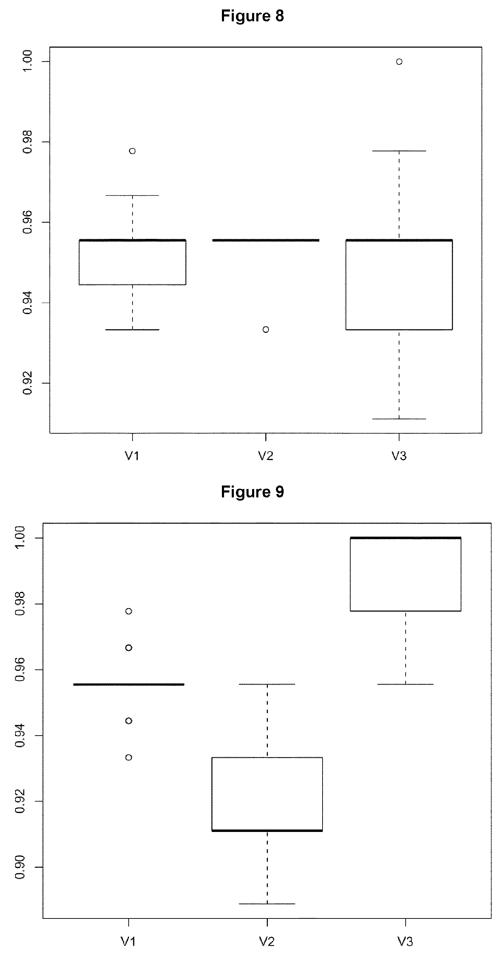

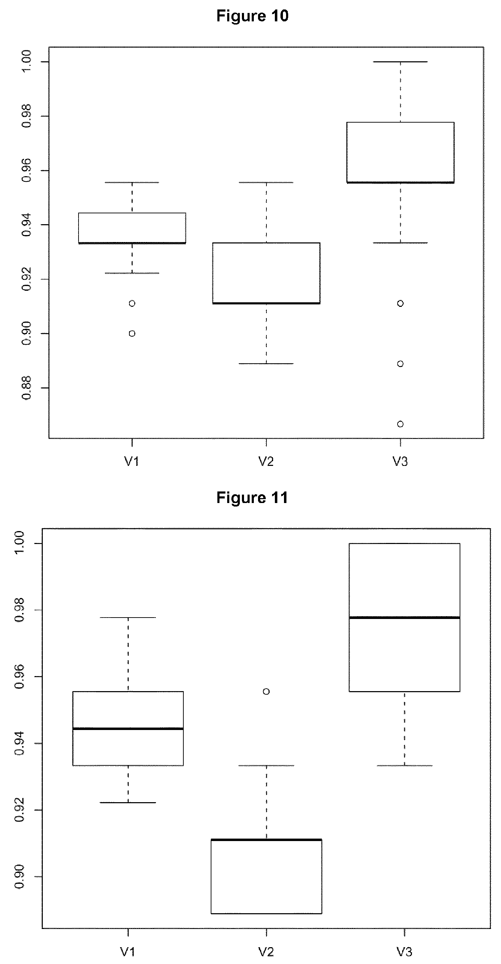

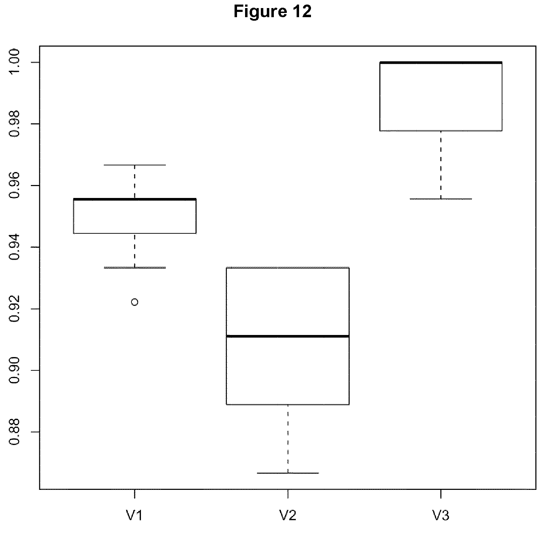

It was found that this approach yields miRNA sets (signatures) that can predict or determine with very high diagnostic accuracy, specificity and sensitivity a lung disease, e.g. sarcoidosis, lung cancer, and/or COPD, in a patient, e.g. a human or an animal. Said miRNA sets (signatures) comprise at least two miRNAs, wherein the nucleotide sequences of said miRNAs are selected from the group consisting of SEQ ID NO: 1 to SEQ ID NO: 391 (see for example **Figures 4 to 12** and **Figures 15 to 17**).

An exemplarily approach to arrive at miRNA sets/signatures that correlate with the lung disease sarcoidosis is summarized below:

Step 1: Total RNA (or subfractions thereof) is extracted from a blood (including plasma, serum, PBMC or other blood fractions) sample of a subject(s) with sarcoidosis using suitable kits and/or purification methods.

Step 2: From the respective sample, the quantity (expression level) of one miRNA or sets of at least two miRNAs selected from the group consisting of SEQ ID NO: 1 to SEQ ID NO: 231 is measured using experimental techniques. These techniques include, but are not restricted to, array based approaches, amplification methods (PCR, RT-PCR, or qPCR), sequencing, next generation sequencing, and/or mass spectroscopy.

Step 3: In order to gather information on the diagnostic/prognostic value and the redundancy of each of the single miRNA biomarkers, mathematical methods are applied. These methods include, but are not restricted to, basic mathematic approaches (e.g. Fold Quotients, Signal-to-Noise ratios, Correlation), statistical methods as hypothesis tests (e.g. t-test, Wilcoxon-Mann-Whitney test), the Area under the Receiver operator Characteristics Curve, information theory approaches, (e.g. the Mutual Information, Cross-entropy), probability theory (e.g. joint and conditional probabilities) or combinations and modifications of the previously mentioned methods.

Step 4: The information gathered in step 3) is used to estimate for each miRNA biomarker the diagnostic content or value. Usually, however, this diagnostic value is too small to get a highly accurate diagnosis with accuracy rates, specificities and sensitivities beyond the 80% barrier. The diagnostic content of the miRNAs suitable for diagnosing/prognosing sarcoidosis is listed in **Figure 1** (Experimental details: SEQ ID NO: sequence identification number, miRNA: identifier of the miRNA according to miRBase, median g1: median intensity obtained from microarray analysis for healthy controls, median g2: median intensity obtained from microarray analysis for individuals with sarcoidosis, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment.). This Figure includes the miRNAs according to SEQ ID NO: 1 to SEQ ID NO: 231.

Step 5: In order to increase the performance for diagnosing/prognosing of individuals suffering from sarcoidosis, more than one miRNA biomarker needs to be employed. Thus statistical learning / machine learning / bioinformatics / computational approaches are applied for set selection in order to select/define sets of miRNA biomarkers (comprising miRNAs SEQ ID NO: 1 to SEQ ID NO: 231) that are tailored for the detection of sarcoidosis. These techniques include, but are not restricted to, Wrapper subset selection techniques (e.g. forward step-wise, backward step-wise, combinatorial approaches, optimization approaches), filter subset selection methods (e.g. the methods mentioned in Step 3), principal component analysis, or combinations and modifications of such methods (e.g. hybrid approaches).

Step 6: The subsets, selected/defined in Step 5, which may range from only a small number (at least two for the set) to all measured biomarkers, are then used to carry out a diagnosis/prognosis of sarcoidosis. To this end, statistical learning / machine learning / bioinformatics / computational approaches are applied that include but are not restricted to any type of supervised or unsupervised analysis: classification techniques (e.g. naive Bayes, Linear Discriminant Analysis, Quadratic Discriminant Analysis Neural Nets, Tree based approaches, Support Vector Machines, Nearest Neighbour Approaches), Regression techniques (e.g. linear Regression, Multiple Regression, logistic regression, probit regression, ordinal logistic regression ordinal Probit-Regression, Poisson Regression, negative binomial Regression, multinomial logistic Regression, truncated regression), Clustering techniques (e.g. k-means clustering, hierarchical clustering, PCA), Adaptations, extensions, and combinations of the previously mentioned approaches.

Step 7: By combination of subset selection (Step 5) and machine learning approaches (Step 6) an algorithm or a mathematical function for diagnosing/prognosing sarcoidosis is obtained. This algorithm or mathematical function is applied to a miRNA expression profile (miRNA expression profile data) of an individual (patient) to be diagnosed for sarcoidosis.

Thus, described herein but not encompassed by the present invention is (are) (the use of) one or more polynucleotide(s) for detecting one or more miRNA(s) for diagnosing and/or prognosing of a lung disease in a biological sample from a patient, wherein the nucleotide sequence of said miRNA or the nucleotide sequences of said miRNAs is (are) selected from the group consisting of SEQ ID NOs: 1 to 391. Preferably the lung disease is sarcoidosis, lung cancer, and/or chronic obstructive pulmonary disease (COPD), e.g. (i), sarcoidosis, (ii) lung cancer, (iii) COPD, (iv) sarcoidosis and lung cancer, (v) sarcoidosis and COPD, (vi) lung cancer and COPD, or (vii) sarcoidosis, lung cancer, and COPD.

In a first aspect, the present invention relates to the use of polynucleotides for detecting miRNAs for diagnosing sarcoidosis in a blood sample from a patient, wherein the nucleotide sequences of said miRNAs comprise the nucleotide sequences according to Set No. 1 to 42 of Figure 15.

The terms "one polynucleotide" and "a single polynucleotide" or "one miRNA" and "a single miRNA" can interchangeable be used herein. Further, "more than one polynucleotides" (i.e. at least two polynucleotides) or "more than one miRNAs" (i.e. at least two miRNAs) may be comprised in a set (e.g. a set of polynucleotides such as a set comprising at least two polynucleotides or a set of miRNAs such as a set comprising at least two miRNAs). The miRNAs which are comprised in a set may represent miRNA signatures. The term "biological sample", as used herein, refers to any biological sample containing miRNA(s). Said biological sample may be a body fluid sample, a tissue sample, or a cell sample. For example, biological samples are tissue (e.g. section or explant) samples, single cell samples, cell colony samples, cell culture samples, blood (e.g. whole blood or a blood fraction such as serum or plasma) samples, urine samples, or samples from other peripheral sources. Said biological samples may be mixed or pooled, e.g. a biological sample may be a mixture of blood and urine samples. A "biological sample" may be provided by removing cell(s), cell colonies, an explant, or a section from a patient or control subject, but may also be provided by using a previously isolated sample. For example, a tissue sample may be removed from a patient or control subject by conventional biopsy techniques or a blood sample may be taken from a patient or control subject by conventional blood collection techniques. The biological sample, e.g. tissue or blood sample, may be obtained from a patient prior to initiation of the therapeutic treatment, during the therapeutic treatment, and/or after the therapeutic treatment. In one embodiment, the therapeutical treatment is monitored on the basis of the detection of the miRNA(s) by the polynucleotide(s) described herein. It is also preferred that total RNA or a subtraction thereof, isolated (e.g. extracted) from a biological sample of a patient (e.g. human or animal), is used for detecting the miRNA(s) by the polynucleotide(s) described herein.

It is preferred that the biological sample is a body fluid sample, a tissue sample, a cell colony sample, a single cell sample or a cell culture sample. Said biological samples may be mixed or pooled, e.g. the biological sample may be a mixture of a body fluid sample and a tissue sample or a mixture of a body fluid sample and a cell culture sample.

More preferably, the tissue sample from a patient (e.g. human or animal) is a section or an explant sample.

It is preferred that the tissue sample from a patient (e.g. human or animal) has a weight of between 0.1 and 500 mg, more preferably of between 0.5 and 250 mg, and most preferably of between 1 and 50 mg, i.e. 0.1, 0.2, 0.3, 0.4, 0.5, 0.6, 0.7, 0.8, 0.9, 1, 2, 2.5, 3, 4, 5, 6, 7, 8, 9, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, or 500 mg.

It is also preferred that the cell sample (e.g. cell colony sample or cell culture sample) from a patient (e.g. human or animal) consists of between $10^2$ and $10^{10}$ cells, more preferably of between $10^3$ and $10^7$ cells, and most preferably of between $10^4$ and $10^6$ cells, i.e. $10^2$, $10^3$, $10^4$, $10^5$, $10^6$, $10^7$, $10^8$, $10^9$, or $10^{10}$ cells.

The term "body fluid sample", as used herein, refers to a liquid sample derived from the body of a subject, e.g. human or animal. Said body fluid sample may be a blood, urine, sputum, breast milk, cerebrospinal fluid, cerumen (earwax), endolymph, perilymph, gastric juice, mucus, peritoneal fluid, pleural fluid, saliva, sebum (skin oil), semen, sweat, tears, vaginal secretion, or vomit sample including components or fractions thereof. Said body fluid samples may be mixed or pooled. Thus, a body fluid sample may be a mixture of blood and urine samples or a mixture of blood and cerebrospinal fluid samples. A "body fluid sample" may be provided by removing a body liquid from a subject, but may also be provided by using previously isolated body fluid sample material. Said "body fluid sample" allows for a non-invasive diagnosis/and or prognosis of a subject, e.g. human or animal.

It is preferred that the body fluid sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

More preferably, the body fluid sample is a blood sample or an urine sample or mixtures thereof. In the context of the present invention, the body fluid sample is a blood sample.

Most preferably, the blood sample from a patient (e.g. human or animal) is whole blood or a blood fraction such as serum or plasma. It is also particularly preferred to use blood cells also known as hemopoietic cells. The term "hemopoietic cells" refers to mature cell types and their immature precursors that are identifiable either by morphology or, mostly, by a distinct pattern of cell surface markers. Said term is used to distinguish these cells from other cell types found in the body and also includes T-cells and distinctive subsets, which are the only hematopoietic cells that are not generated in the bone marrow. Said blood cells may be erythrocytes, leukocytes and/or thrombocytes. Peripheral blood mononuclear cells (PBMCs) such as lymphocytes, monocytes or macrophages may also be used. In the context of the present invention, it is preferred that the blood sample is a blood cell sample.

It is preferred that the blood sample from a patient (e.g. human or animal) has a volume of between 0.1 and 20 ml, more preferably of between 0.5 and 10 ml, and most preferably of between 1 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml. It is further preferred that the blood sample is collected by a blood collection tube. Preferably, the blood collection tube includes means for stabilizing the RNA-fraction, especially the small RNA fraction within the blood sample. Not limiting examples for blood collection tubes already including a RNA-stabilization agent are PAXgene tubes (www.Preanalytix.com), or Tempus Blood RNA Tubes (Ambion, Applied Biosystems). Conventionally blood collection tubes, to which optionally a RNA-stabilizing agent like RNAlater (Ambion) can be added, are EDTA-, Heparin-, or Serum-tubes.

It is also preferred that the urine sample from a patient (e.g. human or animal) has a volume of between 0.1 and 20 ml, more preferably of between 0.5 and 15 ml, and most preferably of between 3 and 12 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

It is preferred that the polynucleotides described herein, preferably comprised in a set, are for detecting 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, or 391 miRNAs, preferably comprised in a set, for diagnosing and/or prognosing of a lung disease, preferably sarcoidosis, lung cancer, and/or chronic obstructive pulmonary disease (COPD), in a biological sample from a patient, wherein the nucleotide sequence of said miRNA or the nucleotide sequences of said miRNAs is (are) selected from the group consisting of SEQ ID NOs: 1 to 391.

Preferably, the polynucleotide(s) is (are) for detecting one or more miRNA(s) for diagnosing and/or prognosing of sarcoidosis, wherein the nucleotide sequence of said miRNA or the nucleotide sequences of said miRNAs is (are) selected from the group consisting of SEQ ID NOs: 1 to 231 (see lists 1, 2, 4 and 7 of **Figure 18**). In this respect, it is particularly preferred that the polynucleotide(s) is (are) for detecting one or more miRNA(s) for diagnosing and/or prognosing of sarcoidosis, wherein the nucleotide sequence of said miRNA or the nucleotide sequences of said miRNAs is (are) selected from the group consisting of SEQ ID NOs: 34, 36, 39, 40, 50, 56, 57, 58, 61, 62, 66, 79, 80, 81, 83, 84, 85, 89, 90, 93, 94, 96, 98, 99, 100, 101, 102, 104, 113, 128, 129, 132, 133, 142, 143, 144, 148, 151, 155, 156, 163, 166, 169, 170, 171, 173, 179, 193, 195, 196, 199, 200, 201, 203, 206, 214, 216, 221, 223, 225, 226, and 229 (see list 1 of **Figure 18**). It is further particularly preferred that the polynucleotides are for detecting 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 miRNAs for diagnosing and/or prognosing of sarcoidosis, wherein the nucleotide sequences of said miRNAs are selected from the group consisting of SEQ ID NOs: 34, 36, 39, 40, 50, 56, 57, 58, 61, 62, 66, 79, 80, 81, 83, 84, 85, 89, 90, 93, 94, 96, 98, 99, 100, 101, 102, 104, 113, 128, 129, 132, 133, 142, 143, 144, 148, 151, 155, 156, 163, 166, 169, 170, 171, 173, 179, 193, 195, 196, 199, 200, 201, 203, 206, 214, 216, 221, 223, 225, 226, and 229 (see list 1 of **Figure 18**).

More preferably, said polynucleotide(s) is (are) further for detecting one or more miRNA(s) for differential diagnosis (of sarcoidosis) with respect to lung cancer and/or COPD, wherein the nucleotide sequence of said miRNA or the nucleotide sequences of said miRNAs is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, 227 (see list 2 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231 (see list 7 of **Figure 18**).

Most preferably,

(i) said polynucleotide(s) is (are) for detecting one or more miRNA(s) for differential diagnosis (of sarcoidosis) with respect to lung cancer, wherein the nucleotide sequence of said miRNA or the nucleotide sequences of said miRNAs is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, and 227 (see list 2 of **Figure 18**),

(ii) said polynucleotide(s) is (are) for detecting one or more miRNA(s) for differential diagnosis (of sarcoidosis) with respect to lung cancer and COPD, wherein the nucleotide sequence of said miRNA or the nucleotide sequences of said miRNAs is (are) selected from the group consisting of SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228 (see list 4 of **Figure 18**), or

(iii) said polynucleotide(s) is (are) for detecting one or more miRNA(s) for differential diagnosis (of sarcoidosis) with respect to COPD, wherein the nucleotide sequence of said miRNA or the nucleotide sequences of said miRNAs is (are) selected from the group consisting of SEQ ID NOs: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231 (see list 7 of **Figure 18**).

Preferably, the polynucleotide(s) is (are) for detecting one or more miRNA(s) for diagnosing and/or prognosing of lung cancer, wherein the nucleotide sequence of said miRNA or the nucleotide sequences of said miRNAs is (are) selected from the group consisting of SEQ ID NOs: 233, 234, 235, 237, 240, 241, 242, 243, 245, 246, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, 329 (see list 3 of **Figure 18**), 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, 227 (see list 2 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**). In this respect, it is particularly preferred that the polynucleotide(s) is (are) for detecting one or more miRNA(s) for diagnosing and/or prognosing of lung cancer, wherein the nucleotide sequence of said miRNA or the nucleotide sequences of said miRNAs is (are) selected from the group consisting of SEQ ID NOs: 233, 234, 235, 237, 240, 241, 242, 243, 245, 246, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, and 329 (list 3 of **Figure 18**). It is further particularly preferred that the polynucleotides are for detecting 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 miRNAs for diagnosing and/or prognosing of lung cancer, wherein the nucleotide sequences of said miRNAs are selected from the group consisting of SEQ ID NOs: 233, 234, 235, 237, 240, 241, 242, 243, 245, 246, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, and 329 (list 3 of **Figure 18**).

More preferably, said polynucleotide(s) is (are) further for detecting one or more miRNA(s) for differential diagnosis (of lung cancer) with respect to sarcoidosis and/or COPD, wherein the nucleotide sequence of said miRNA or the nucleotide sequences of said miRNAs is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, 227 (see list 2 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**).

Most preferably,

(i) said polynucleotide(s) is (are) for detecting one or more miRNA(s) for differential diagnosis (of lung cancer) with respect to sarcoidosis, wherein the nucleotide sequence of said miRNA or the nucleotide sequences of said miRNAs is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, and 227 (see list 2 of **Figure 18**),

(ii) said polynucleotide(s) is (are) for detecting one or more miRNA(s) for differential diagnosis (of lung cancer) with respect to sarcoidosis and COPD, and wherein the nucleotide sequence of said miRNA or the nucleotide sequences of said miRNAs is (are) selected from the group consisting of SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228 (see list 4 of **Figure 18**), or

(iii) said polynucleotide(s) is (are) for detecting one or more miRNA(s) for differential diagnosis (of lung cancer) with respect to COPD, and wherein the nucleotide sequence of said miRNA or the nucleotide sequences of said miRNAs is (are) selected from the group consisting of SEQ ID NOs: 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**).

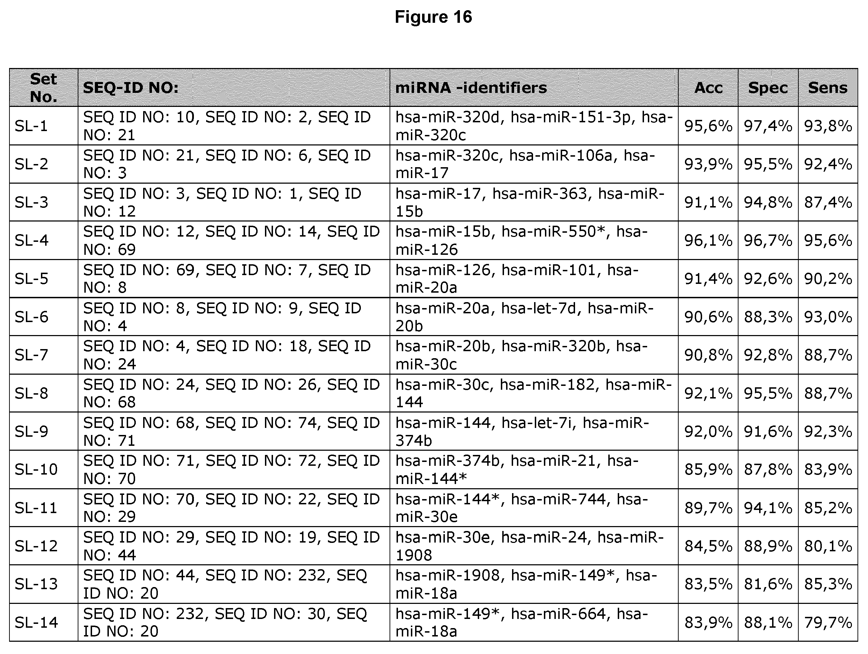

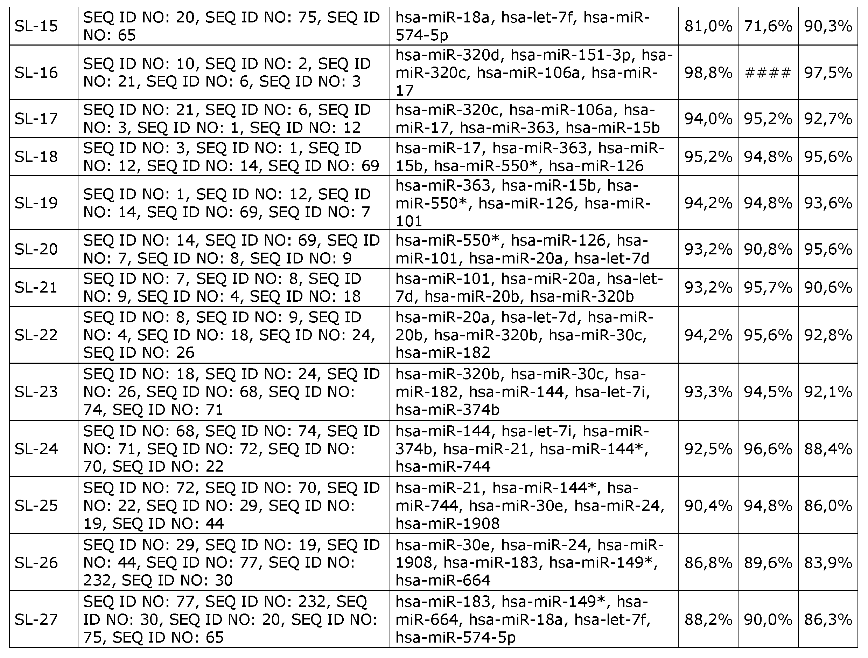

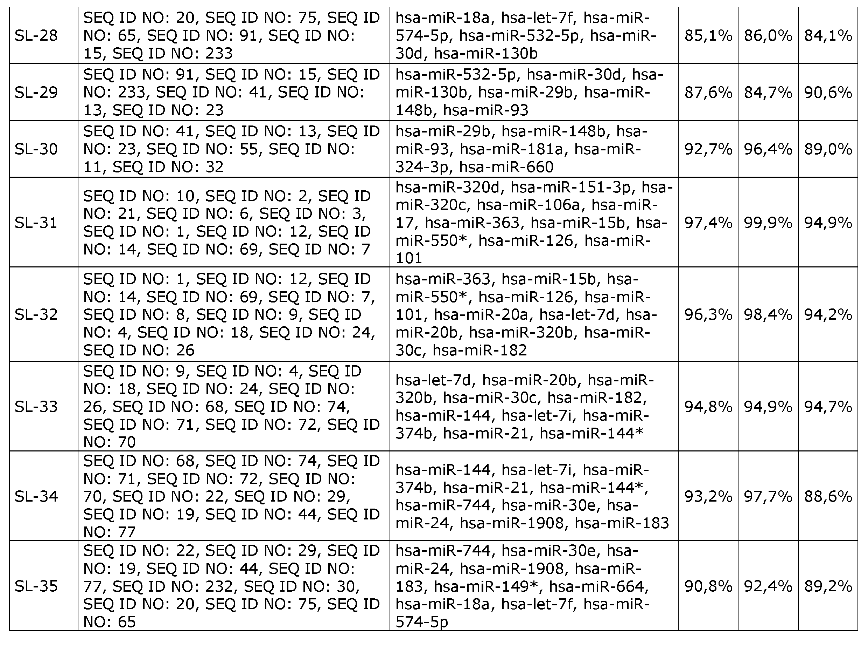

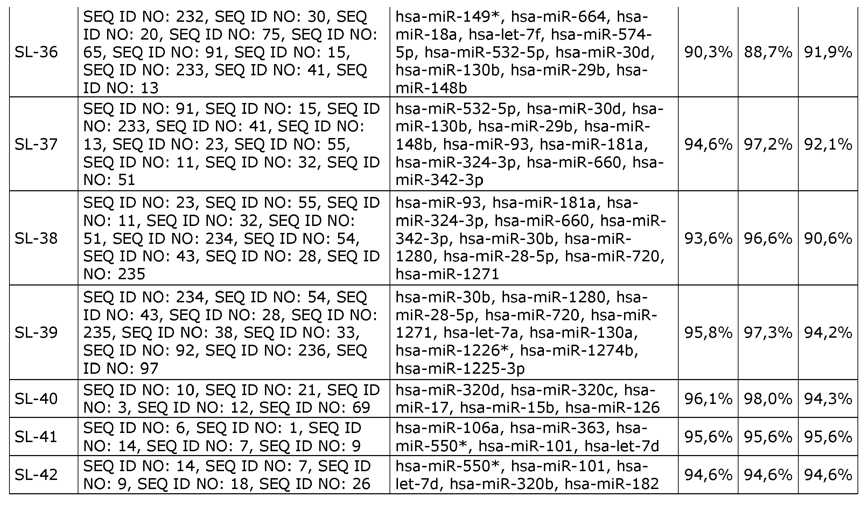

In preferred embodiments, the nucleotide sequences of said miRNAs are the nucleotide sequences according to Set No. 1 to 42 (i.e. SL-1 to SL-42) of **Figure 16.**

Preferably, the polynucleotide(s) is (are) for detecting one or more miRNA(s) for diagnosing and/or prognosing of COPD, wherein the nucleotide sequence of said miRNA or the nucleotide sequences of said miRNAs is (are) selected from the group consisting of SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391 (see list 6 of **Figure 18**), 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, 231 (see list 7 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**). In this respect, it is particularly preferred that the polynucleotide(s) is (are) for detecting one or more miRNA(s) for diagnosing and/or prognosing of COPD, wherein the nucleotide sequence of said miRNA or the nucleotide sequences of said miRNAs is (are) selected from the group consisting of SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, and 391 (see list 6 of **Figure 18**). It is further particularly preferred that the polynucleotides are for detecting 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 miRNAs for diagnosing and/or prognosing of COPD, wherein the nucleotide sequences of said miRNAs are selected from the group consisting of SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, and 391 (see list 6 of **Figure 18**).

More preferably, said polynucleotide(s) is (are) further for detecting one or more miRNA(s) for differential diagnosis (of COPD) with respect to sarcoidosis and/or lung cancer, and wherein the nucleotide sequence of said miRNA or the nucleotide sequences of said miRNAs is (are) selected from the group consisting of SEQ ID NOs: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, 231 (see list 7 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**).

Most preferably,

(i) said polynucleotide(s) is (are) for detecting one or more miRNA(s) for differential diagnosis (of COPD) with respect to sarcoidosis, wherein the nucleotide sequence of said miRNA or the nucleotide sequences of said miRNAs is (are) selected from SEQ ID NOs: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231 (see list 7 of **Figure 18**),

(ii) said polynucleotide(s) is (are) for detecting one or more miRNA(s) for differential diagnosis (of COPD) with respect to sarcoidosis and lung cancer, wherein the nucleotide sequence of said miRNA or the nucleotide sequences of said miRNAs is (are) selected from SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228 (see list 4 of **Figure 18**), or

(iii) said polynucleotide(s) is (are) for detecting one or more miRNA(s) for differential diagnosis (of COPD) with respect to lung cancer, wherein the nucleotide sequence of said miRNA or the nucleotide sequences of said miRNAs is (are) selected from SEQ ID NOs: 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**).

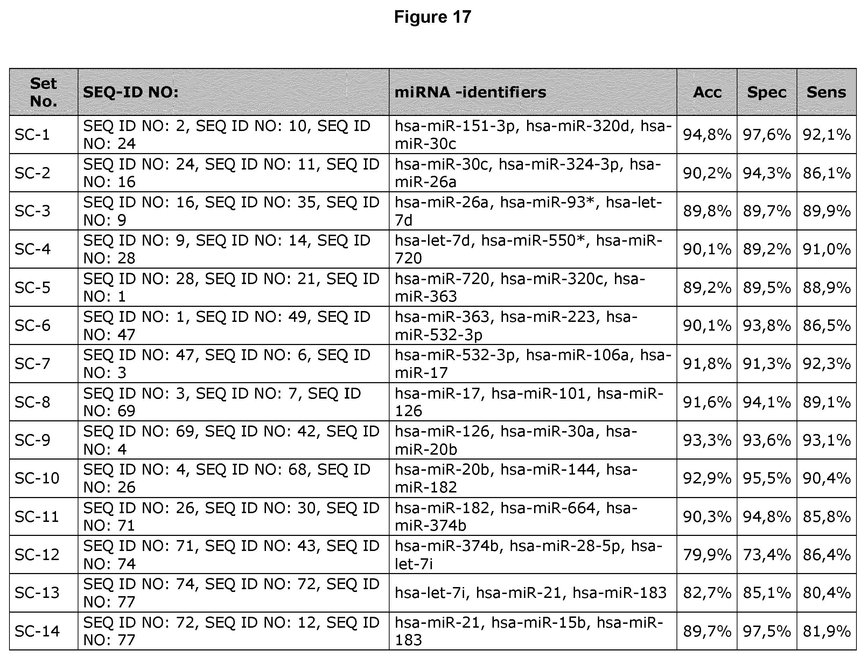

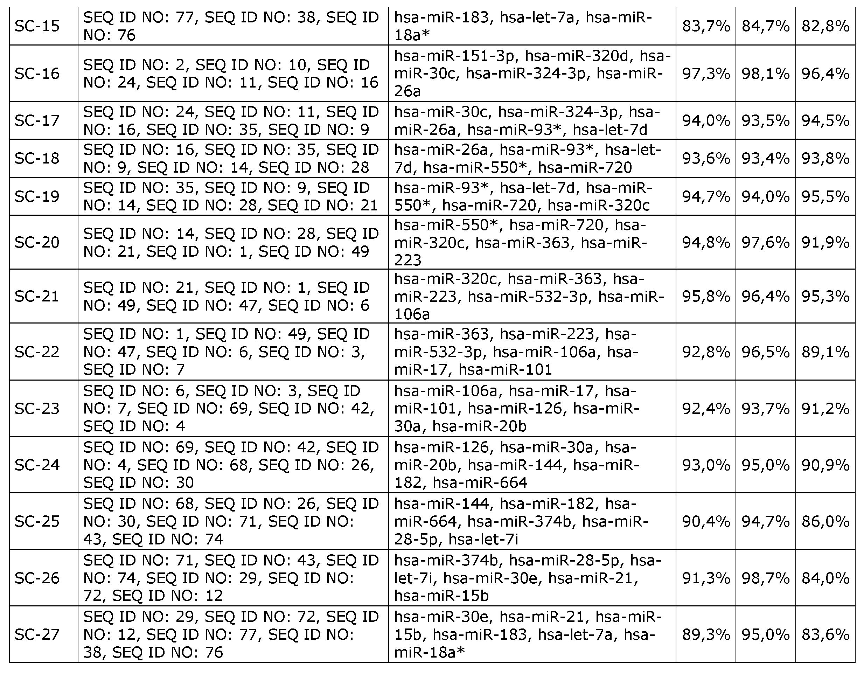

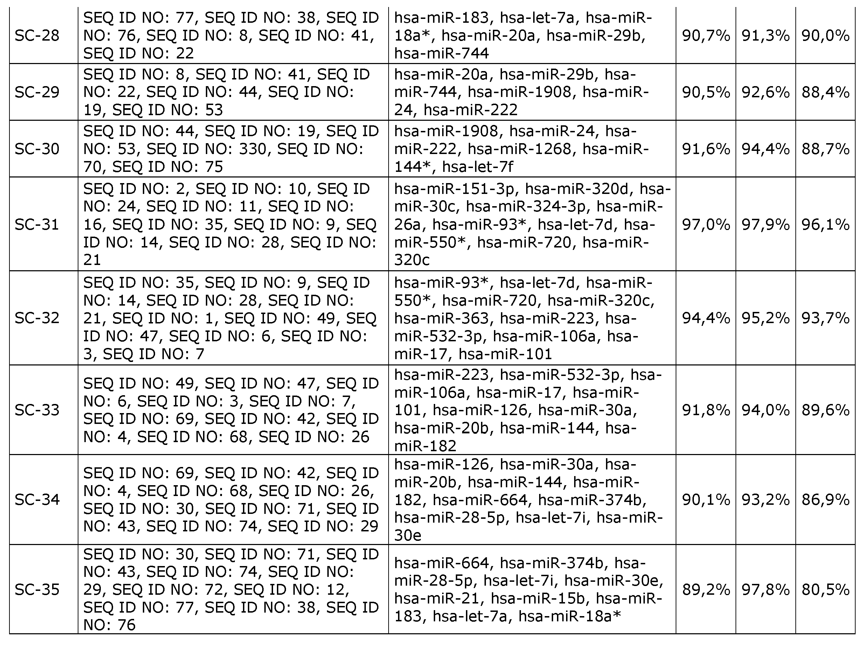

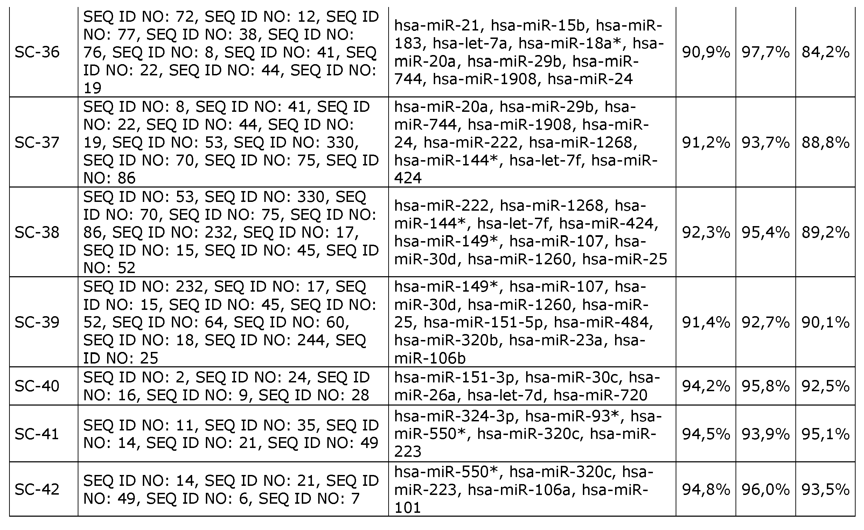

In preferred embodiments, the nucleotide sequences of said miRNAs are the nucleotide sequences according to Set No. 1 to 42 (i.e. SC-1 to SC-42) of **Figure 17.**

It is preferred that the nucleotide sequences of the miRNAs have (i) SEQ ID NO: 2 and SEQ ID NO: 3, (ii) SEQ ID NO: 3 and SEQ ID NO: 4, (iii) SEQ ID NO: 5, SEQ ID NO: 6, and SEQ ID NO: 7, (iv) SEQ ID NO: 8, SEQ ID NO: 9, and SEQ ID NO: 10, (v) SEQ ID NO: 11, SEQ ID NO: 12, and SEQ ID NO: 13, (vi) SEQ ID NO: 14, SEQ ID NO: 15, and SEQ ID NO: 16, (vii) SEQ ID NO: 17, SEQ ID NO: 18, SEQ ID NO: 19, and SEQ ID NO: 20, (viii) SEQ ID NO: 21, SEQ ID NO: 22, SEQ ID NO: 23, and SEQ ID NO: 24, (ix) SEQ ID NO: 25, SEQ ID NO: 26, SEQ ID NO: 27, SEQ ID NO: 28, SEQ ID NO: 29, and SEQ ID NO: 30, (x) SEQ ID NO: 31, SEQ ID NO: 32, SEQ ID NO: 33, SEQ ID NO: 34, SEQ ID NO: 35, and SEQ ID NO: 36, or (xi) SEQ ID NO: 37, SEQ ID NO: 38, SEQ ID NO: 39, SEQ ID NO: 40. Said miRNAs (miRNA combinations) may also be further combined with each other, e.g. (i) with (ii), (ii) with (iii), (iii) with (iv), (iv) with (v), (v) with (vi), (vi) with (vii), or any other possible combination.

It is also preferred that the nucleotide sequences of the miRNAs have (i) SEQ ID NO: 1 and SEQ ID NO: 2, (ii) SEQ ID NO: 1 and SEQ ID NO: 3, (iii) SEQ ID NO: 1 and SEQ ID NO: 4, (iv) SEQ ID NO: 4 and SEQ ID NO: 5, (v) SEQ ID NO: 5 and SEQ ID NO: 6, (vi) SEQ ID NO: 6 and SEQ ID NO: 7, (vii) SEQ ID NO: 7 and SEQ ID NO: 8, (viii) SEQ ID NO: 8 and SEQ ID NO: 9, (ix) SEQ ID NO: 9 and SEQ ID NO. 10, (x) SEQ ID NO: 10 and SEQ ID NO: 11, or (xi) SEQ ID NO: 11 and SEQ ID NO: 12. Said miRNAs (miRNA combinations) may also be further combined with each other, e.g. (i) with (ii), (ii) with (iii), (iii) with (iv), (iv) with (v), (v) with (vi), (vi) with (vii), or any other possible combination. It is also preferred that the nucleotide sequences of the miRNAs have (i) SEQ ID NO: 1 to SEQ ID NO: 3, (ii) SEQ ID NO: 2 to SEQ ID NO: 4, (iii) SEQ ID NO: 3 to SEQ ID NO: 5, (iv) SEQ ID NO: 4 to SEQ ID NO: 6, (v) SEQ ID NO: 5 to SEQ ID NO: 7, (vi) SEQ ID NO: 6 to SEQ ID NO: 8, (vii) SEQ ID NO: 7 to SEQ ID NO: 9, (viii) SEQ ID NO: 8 to SEQ ID NO: 10, (ix) SEQ ID NO: 9 to SEQ ID NO: 11, (x) SEQ ID NO: 10 to SEQ ID NO: 12, or (xi) SEQ ID NO: 11 to SEQ ID NO: 13. Said miRNAs (miRNA combinations) may also be further combined with each other, e.g. (i) with (ii), (ii) with (iii), (iii) with (iv), (iv) with (v), (v) with (vi), (vi) with (vii), or any other possible combination.

It is particularly preferred that the nucleotide sequences of the miRNAs have (i) SEQ ID NO: 1, SEQ ID NO: 2, SEQ ID NO: 3, SEQ ID NO: 4, SEQ ID NO: 68, SEQ ID NO: 5, SEQ ID NO: 6, SEQ ID NO: 7, SEQ ID NO: 69, and SEQ ID NO: 8, (ii) SEQ ID NO: 68, SEQ ID NO: 5, SEQ ID NO: 6, SEQ ID NO: 7, SEQ ID NO: 69, SEQ ID NO: 8, SEQ ID NO: 70, SEQ ID NO: 105, SEQ ID NO: 9, and SEQ ID NO: 10, (iii) SEQ ID NO: 8, SEQ ID NO: 70, SEQ ID NO: 105, SEQ ID NO: 9, SEQ ID NO: 10, SEQ ID NO: 11, SEQ ID NO: 12, SEQ ID NO: 13, SEQ ID NO: 14, and SEQ ID NO: 114, (iv) SEQ ID NO: 68, SEQ ID NO: 1, SEQ ID NO: 4, SEQ ID NO: 69, SEQ ID NO: 3, SEQ ID NO: 5, SEQ ID NO: 8, SEQ ID NO: 70, SEQ ID NO: 7, and SEQ ID NO: 6, (v) SEQ ID NO: 3, SEQ ID NO: 5, SEQ ID NO: 8, SEQ ID NO: 70, SEQ ID NO: 7, SEQ ID NO: 6, SEQ ID NO: 12, SEQ ID NO: 9, SEQ ID NO: 105, and SEQ ID NO: 107, or (vi) SEQ ID NO: 6, SEQ ID NO: 12, SEQ ID NO: 9, SEQ ID NO: 105, SEQ ID NO: 107, SEQ ID NO: 71, SEQ ID NO: 17, SEQ ID NO: 159, SEQ ID NO: 19, and SEQ ID NO: 106. Said miRNAs (miRNA combinations) may also be further combined with each other, e.g. (i) with (ii), (ii) with (iii), (iii) with (iv), (iv) with (v), (v) with (vi), (vi) with (vii), or any other possible combination.

In another preferred embodiment, the nucleotide sequence of the (single) miRNA is selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10, or is selected from the group consisting of SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, and SEQ ID NO: 20. It is particularly preferred that the nucleotide sequence of the (single) miRNA is selected from the group consisting of SEQ ID NO: 1, SEQ ID NO: 2, SEQ ID NO: 3, SEQ ID NO: 4, and SEQ ID NO: 5.

It should be noted that the above mentioned polynucleotides (at least two polynucleotides) described herein may be comprised in a set in order to detect miRNAs (at least two miRNAs) which may be also comprised in a set for diagnosing and/or prognosing of a lung disease such as sarcoidosis, lung cancer, and/or COPD, wherein the nucleotide sequences of said miRNAs are selected from the group consisting of SEQ ID NOs: 1 to 391. For example, said set of polynucleotides may comprise, essentially consist of, or consist of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, or 391 polynucleotides for detecting a set of miRNAs which may comprise, essentially consist, or consist of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, or 391 miRNAs for diagnosing and/or prognosing of a lung disease such as sarcoidosis, lung cancer, and/or COPD in a biological sample from a patient, wherein the nucleotide sequences of said miRNAs are selected from the group consisting of SEQ ID NOs: 1 to 391.

It is preferred that

(i) the polynucleotide described herein is complementary to the miRNA, wherein the nucleotide sequence of said miRNA is selected from the group consisting of SEQ ID NOs: 1 to 391, or the polynucleotides described herein are complementary to the miRNAs, wherein the nucleotide sequences of said miRNAs are selected from the group consisting of SEQ ID NOs: 1 to 391,
(ii) the polynucleotide is a fragment of the polynucleotide according to (i), preferably the polynucleotide is a fragment which is between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the polynucleotide according to (i), or the polynucleotides are fragments of the polynucleotides according to (i), preferably the polynucleotides are fragments which are between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the polynucleotides according to (i), or
(iii) the polynucleotide has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the polynucleotide sequence of the polynucleotide according to (i) or polynucleotide fragment according to (ii), or the polynucleotides have at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the polynucleotide sequences of the polynucleotides according to (i) or polynucleotide fragments according to (ii).

In the context of the present invention, it is preferred that the polynucleotides are complementary to the miRNAs, wherein the nucleotide sequences of said miRNAs comprise the nucleotide sequences according to Set. 1 to 42 of Figure 15. It is particularly preferred that the polynucleotide as defined in (iii) has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity over a continuous stretch of at least 10, 11, 12, 13, 14, 15, 16, 17, 18, 19, 20 or more nucleotides, preferably over the whole length, to the polynucleotide sequence of the polynucleotide according to (i) or polynucleotide fragment according to (ii), or that the polynucleotides as defined in (iii) have at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity over a continuous stretch of at least 10, 11, 12, 13, 14, 15, 16, 17, 18, 19, 20 or more nucleotides, preferably over the whole length, to the polynucleotide sequences of the polynucleotides according to (i) or polynucleotide fragments according to (ii).

In addition, the polynucleotide or polynucleotides as defined in (ii) (i.e. polynucleotide fragment(s)) or (iii) (i.e. polynucleotide variant(s) or polynucleotide fragment variant(s)) is (are) only regarded as a polynucleotide or polynucleotides as defined in (ii) (i.e. polynucleotide fragment(s)) or (iii) (i.e. polynucleotide variant(s) or polynucleotide fragment variant(s)) within the context described herein, if it is or they are still capable of binding to, hybridizing with, or detecting a target miRNA of complementary sequence or target miRNAs of complementary sequences, e.g. the respective target miRNA(s) according to SEQ ID NO: 1 to SEQ ID NO: 391, through one or more types of chemical bonds, usually through complementary base pairing, usually through hydrogen bond formation under stringent hybridization conditions. The skilled person can readily assess whether a polynucleotide or polynucleotides as defined in (ii) (i.e. polynucleotide fragment(s)) or (iii) (i.e. polynucleotide variant(s) or polynucleotide fragment variant(s)) is (are) still capable of binding to, hybridizing with, recognizing or detecting a target miRNA of complementary sequence or target miRNAs of complementary sequences, e.g. the respective target miRNA(s) according to SEQ ID NO: 1 to SEQ ID NO: 391. Suitable assays to determine whether hybridization under stringent conditions still occurs are well known in the art. However, as an example, a suitable assay to determine whether hybridization still occurs comprises the steps of: (a) incubating the polynucleotide or polynucleotides as defined in (ii) or (iii) attached onto a biochip with the miRNA(s) of complementary sequence(s), e.g. the respective target miRNA(s) according to SEQ ID NO: 1 to SEQ ID NO: 391, labeled with biotin under stringent hybridization conditions, (b) washing the biochip to remove unspecific bindings, (c) subjecting the biochip to a detection system, and (c) analyzing whether the polynucleotide(s) can still hybridize with the target miRNA(s) of complementary sequence(s), e.g. the respective target miRNA(s) according to SEQ ID NO: 1 to SEQ ID NO: 391. As a positive control, the respective non-mutated and not fragmented polynucleotide as defined in (i) may be used. Preferably stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %,Tween 20, 0.1 x TE buffer, 0.5 mg/ml BSA, 0.1 mg/ml herring sperm DNA, incubating at 42°C with wash in 0.5x SSPE and 6x SSPE at 45°C.

Described herein but not encompassed by the present invention is a method for diagnosing and/or prognosing a lung disease comprising the steps of: (i) determining an expression profile of one or more miRNA(s) in a biological sample from a patient, wherein the nucleotide sequence of said miRNA or the nucleotide sequences of said miRNAs is (are) selected from the group consisting of SEQ ID NOs: 1 to 391, a fragment thereof, and a sequence having at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity thereto, and (ii) comparing said expression profile (data) to a reference, wherein the comparison of said expression profile (data) to said reference allows for the diagnosis and/or prognosis of a lung disease, and/or applying an algorithm or a mathematical function to said expression profile, wherein the application of said algorithm or mathematical function to said expression profile allows for the diagnosis and/or prognosis of a lung disease. Preferably the lung disease is sarcoidosis, lung cancer, and/or chronic obstructive pulmonary disease (COPD), e.g. (i), sarcoidosis, (ii) lung cancer, (iii) COPD, (iv) sarcoidosis and lung cancer, (v) sarcoidosis and COPD, (vi) lung cancer and COPD, or (vii) sarcoidosis, lung cancer, and COPD.

In a second aspect, the present invention relates to a method for diagnosing sarcoidosis comprising the steps of: (i) determining an expression profile of miRNAs in a blood sample from a patient, wherein the nucleotide sequences of said miRNAs comprise the nucleotide sequences according to Set No. 1 to 42 of Figure 15, and (ii) comparing said expression profile to a reference, wherein the comparison of said expression profile to said reference allows for the diagnosis of sarcoidosis. It is preferred that the patient is a mammal including both a human and another mammal, e.g. an animal such as a mouse, a rat, a rabbit, or a monkey. It is particularly preferred that the patient is a human. The term "miRNA expression profile", as used herein, represents the expression level of a single (1) miRNA or a collection of expression levels of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 25.9, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368,369,370,371,372,373,374,375,376,377, 378, 379, 380, 381, 382, 383, 384, 385, 386, 387, 388, 389, or 390 miRNAs, or of 391 miRNAs, preferably comprised in a set, wherein the nucleotide sequence of said miRNA or the nucleotide sequences of said miRNAs is (are) selected from the group consisting of SEQ ID NOs: 1 to 391, a fragment thereof, and a sequence having at least 80% sequence identity thereto. In the context of the present invention, an expression profile of miRNAs is determined, wherein the nucleotide sequences of said miRNAs comprise the nucleotide sequences according to Set No. 1 to 42 of Figure 15. It is preferred that the biological sample is a body fluid sample, a tissue sample, a cell colony sample, a single cell sample or a cell culture sample. Said biological samples may be mixed or pooled, e.g. the biological sample may be a mixture of a body fluid sample and a tissue sample or a mixture of a body fluid sample and a cell culture sample. More preferably, the tissue sample from a patient (e.g. human or animal) is a section or an explant sample. It is preferred that the tissue sample from a patient (e.g. human or animal) has a weight of between 0.1 and 500 mg, more preferably of between 0.5 and 250 mg, and most preferably of between 1 and 50 mg, i.e. 0.1, 0.2, 0.3, 0.4, 0.5, 0.6, 0.7, 0.8, 0.9, 1, 2, 2.5, 3, 4, 5, 6, 7, 8, 9, 10, 15, 20, 25, 30, 35, 40, 45, 50, 55, 60, 65, 70, 75, 80, 85, 90, 95, 100, 125, 150, 175, 200, 225, 250, 275, 300, 325, 350, 375, 400, 425, 450, 475, or 500 mg. It is also preferred that the cell sample (e.g. cell colony sample or cell culture sample) from a patient (e.g. human or animal) consists of between $10^2$ and $10^{10}$ cells, more preferably of between $10^3$ and $10^7$ cells, and most preferably of between $10^4$ and $10^6$ cells, i.e. $10^2$, $10^3$, $10^4$, $10^5$, $10^6$, $10^7$, $10^8$, $10^9$, or $10^{10}$ cells. More preferably, the body fluid sample is a blood, urine, sputum, breast milk, cerebrospinal fluid, cerumen (earwax), endolymph, perilymph, gastric juice, mucus, peritoneal fluid, pleural fluid, saliva, sebum (skin oil), semen, sweat, tears, vaginal secretion, or vomit sample including components or fractions thereof. It is preferred that the body fluid sample from a subject (e.g. human or animal) has a volume of between 0.1 and 20 ml, more preferably of between 0.5 and 10 ml, more preferably between 1 and 8 ml and most preferably between 2 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml. In the context of the present invention, the body fluid sample is a blood sample. Most preferably, the blood sample from a patient (e.g. human or animal) is whole blood or a blood fraction such as serum or plasma. It is also particularly preferred to use blood cells also known as hemopoietic cells. The term "hemopoietic cells" refers to mature cell types and their immature precursors that are identifiable either by morphology or, mostly, by a distinct pattern of cell surface markers. Said term is used to distinguish these cells from other cell types found in the body and also includes T-cells and distinctive subsets, which are the only hematopoietic cells that are not generated in the bone marrow. Said blood cells may be erythrocytes, leukocytes and/or thrombocytes. Peripheral blood mononuclear cells (PBMCs) such as lymphocytes, monocytes or macrophages may also be used. In the context of the present invention, it is preferred that the blood sample is a blood cell sample.

It is preferred that the blood sample from a patient (e.g. human or animal) has a volume of between 0.1 and 20 ml, more preferably of between 0.5 and 10 ml, and most preferably of between 1 and 5 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

The inventors of the present invention surprisingly found that miRNAs are not only present in a blood sample but also that miRNAs remain stable and that, thus, blood miRNAs can be used as biomarkers for detecting and/or prognosis of a lung disease (e.g. sarcoidosis, lung cancer, and/or COPD) in patients (e.g. humans or animals). Furthermore, the use of blood samples for detection and/or prognosis of a lung disease has a number of advantage, for example, blood miRNAs have a high sensitivity, blood is relatively easy to obtain and even can be collected via routine physical examination, the costs for detection are low, and the samples can easily be preserved (e.g. at - 20°C). Further, blood circulates to all tissues in the body and, therefore, blood is able to reflect the physiological pathology of the whole organism. Accordingly, the detection of blood miRNAs is an indicator of individual's health and, thus, is an indication whether an individual suffers from a lung disease. Furthermore, this method can widely be used in general screening for a lung disease.

It should be noted that the determination of an expression profile of one or more miRNA(s) in a blood sample from a patient (e.g. human or animal) is possible, (i) as the miRNAs, preferably the miRNAs disclosed herein, are expressed within and/or among cells or tissues and are subsequently released/transferred into circulating blood and/or (ii) as the miRNAs, preferably the miRNAs disclosed herein, are directly expressed in hemopoietic cells, also known as blood cells, e.g. erythrocytes, leukocytes and/or thrombocytes. Thus, the term "determining an expression profile of miRNAs in a blood sample" does not solely mean that the miRNAs are actually expressed in blood. Said miRNAs may be expressed within and/or among surrounding cells or tissues and may be subsequently released/transferred into circulating blood and/or said miRNAs may be directly expressed in blood, namely in blood cells, e.g. peripheral blood mononuclear cells (PBMCs). Accordingly, the expression levels of miRNAs which are preferably determined in a blood sample indirectly represent the expression levels of said miRNAs in the cells or tissues from which they originate and/or directly represent the expression levels of miRNAs in blood cells.

It is also preferred that the urine sample from a patient (e.g. human or animal) has a volume of between 0.1 and 20 ml, more preferably of between 0.5 and 15 ml, and most preferably of between 3 and 12 ml, i.e. 0.1, 0.2, 0.3, 0.4, 0.5, 0.6, 0.7, 0.8, 0.9, 1, 2, 2.5, 3, 4, 5, 6, 7, 8, 9, 10, 11, 12, 13, 14, 15, 16, 17, 18, 19, or 20 ml.

The miRNA expression profiles may be generated by any convenient means for determining miRNA expression levels (see below) and allow the analysis of differential miRNA expression levels between samples, for example, between a sample of a patient and (a) sample(s) of (a) control subject(s), e.g. between a sample of a patient and a sample of a subject known not to suffer form a lung disease such as sarcoidosis, lung cancer, and/or COPD (i.e. being healthy), or between a sample of a patient and a sample of a subject known to suffer from a lung disease such as sarcoidosis, lung cancer, and/or COPD (i.e. being diseased). Thereby, generally, each miRNA is represented by a numerical value. The higher the value of an individual miRNA, the higher is the expression level of said miRNA, or the lower the value of an individual miRNA, the lower is the expression level of said miRNA. The miRNA expression profile may include expression data for 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, or 391 miRNAs, wherein the nucleotide sequence of said miRNA or the nucleotide sequences of said miRNAs may be selected from the group consisting of SEQ ID NOs: 1 to 391. In the context of the present invention, an expression profile of miRNAs is determined, wherein the nucleotide sequences of said miRNAs comprise the nucleotide sequences according to Set No. 1 to 42 of Figure 15.

The term "differential expression" of miRNAs as used herein, means qualitative and/or quantitative differences in the temporal and/or local miRNA expression patterns, e.g. within and/or among cells, tissues, or within blood. Thus, a differentially expressed miRNA may qualitatively have its expression altered, including an activation or inactivation in, for example, normal tissue versus diseased tissue. The difference in miRNA expression may also be quantitative, e.g. in that expression is modulated, i.e. either up-regulated, resulting in an increased amount of miRNA, or down-regulated, resulting in a decreased amount of miRNA. The degree to which miRNA expression differs need only be large enough to be quantified via standard characterization techniques, e.g. quantitative hybridization of miRNA, labeled miRNA, or amplified miRNA, quantitative PCR (qPCR) such as real time quantitative PCR (qRT-PCR), ELISA for quantitation, next generation sequencing and the like.

The one or more miRNAs having a nucleic acid sequence selected from the group consisting of SEQ ID NOs: 1 to 391 are known to be differentially expressed between subject being healthy and subject having a lung disease (e.g. sarcoidosis, lung cancer, and/or COPD) as well as between subjects having a lung disease (e.g. sarcoidosis, lung cancer and/or COPD) (see **Figure 18**). For example, an increase in expression of one or more miRNAs having a nucleic acid sequence selected from the group consisting of SEQ ID NOs: 1 to 391 and/or a decrease in expression of one or more miRNAs having a nucleic acid sequence selected from the group consisting of SEQ ID NOs: 1 to 391 may be indicative for healthiness, sarcoidosis, lung cancer, and/or COPD (see for example median intensity of the miRNAs (g1 and g2) obtained from microarray analysis in **Figures 1, 13** and **14**). In this respect, it should be noted that the same miRNA may be up-regulated in subjects known to suffer from sarcoidosis and down-regulated in subjects known to suffer from lung cancer compared to subjects known to be healthy. It is also possible that the same miRNA may be up-regulated in subjects known to suffer from COPD and in subject known to suffer from lung cancer compared to subjects known to be healthy, or down-regulated in subject known to suffer from COPD and in subjects known to suffer from lung cancer compared to subjects known to be healthy, but that the grade of up-regulation or the grade of down-regulation is different.

As mentioned above, an expression profile of one or more miRNAs, e.g. 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, or 391 miRNAs, in a biological sample from a patient is determined in the step (i) of the method described herein, wherein the nucleotide sequence of said miRNA or the nucleotide sequences of said miRNAs is (are) selected from the group consisting of SEQ ID NOs: 1 to 391, a fragment thereof, and a sequence having at least 80% sequence identity thereto.

Preferably, the nucleotide sequence of said miRNA or the nucleotide sequences of said miRNAs in step (i) of the method described herein is (are) selected from the group consisting of SEQ ID NOs: 1 to 231 (see lists 1, 2, 4 and 7 of **Figure 18**), wherein the comparison and/or application in step (ii) of the method described herein allow(s) for the diagnosis and/or prognosis of sarcoidosis.

In this respect it is particularly preferred that the nucleotide sequence of said miRNA or the nucleotide sequences of said miRNAs in step (i) of the method described herein is (are) selected from the group consisting of SEQ ID NOs: 34, 36, 39, 40, 50, 56, 57, 58, 61, 62, 66, 79, 80, 81, 83, 84, 85, 89, 90, 93, 94, 96, 98, 99, 100, 101, 102, 104, 113, 128, 129, 132, 133, 142, 143, 144, 148, 151, 155, 156, 163, 166, 169, 170, 171, 173, 179, 193, 195, 196, 199, 200, 201, 203, 206, 214, 216, 221, 223, 225, 226, and 229 (see list 1 of **Figure 18**), wherein the comparison and/or application in step (ii) allow(s) for the diagnosis and/or prognosis of sarcoidosis. It is further particularly preferred that the nucleotide sequences of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 miRNAs in step (i) of the method described herein are selected from the group consisting of SEQ ID NOs: 34, 36, 39, 40, 50, 56, 57, 58, 61, 62, 66, 79, 80, 81, 83, 84, 85, 89, 90, 93, 94, 96, 98, 99, 100, 101, 102, 104, 113, 128, 129, 132, 133, 142, 143, 144, 148, 151, 155, 156, 163, 166, 169, 170, 171, 173, 179, 193, 195, 196, 199, 200, 201, 203, 206, 214, 216, 221, 223, 225, 226, and 229 (see list 1 of **Figure 18**), wherein the comparison and/or application in step (ii) allow(s) for the diagnosis and/or prognosis of sarcoidosis.

More preferably, the nucleotide sequence of said miRNA or the nucleotide sequences of said miRNAs in step (i) is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, 227 (see list 2 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231 (see list 7 of **Figure 18**), wherein the comparison and/or application in step (ii) allow(s) for differential diagnosis (of sarcoidosis) with respect to lung cancer and/or COPD.

Most preferably,

(i) the nucleotide sequence of said miRNA or the nucleotide sequences of said miRNAs in step (i) is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, and 227 (see list 2 of **Figure 18**), wherein the comparison and/or application in step (ii) allow(s) for differential diagnosis (of sarcoidosis) with respect to lung cancer,

(ii) the nucleotide sequence of said miRNA or the nucleotide sequences of said miRNAs in step (i) is (are) selected from the group consisting of SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228 (see list 4 of **Figure 18**), wherein the comparison and/or application in step (ii) allow(s) for differential diagnosis (of sarcoidosis) with respect to lung cancer and COPD, or

(iii) the nucleotide sequence of said miRNA or the nucleotide sequences of said miRNAs in step (i) is (are) selected from the group consisting of SEQ ID NOs: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231 (see list 7 of **Figure 18**), wherein the comparison and/or application in step (ii) allow(s) for differential diagnosis (of sarcoidosis) with respect to COPD.

Preferably, the nucleotide sequence of said miRNA or the nucleotide sequences of said miRNAs in step (i) is (are) selected from the group consisting of SEQ ID NOs: 233, 234, 235, 237, 240, 241, 242, 243, 245, 246, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, 329 (see list 3 of **Figure 18**), 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, 227 (see list 2 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**), wherein the comparison and/or application in step (ii) allow(s) for the diagnosis and/or prognosis of lung cancer.

In this respect it is particularly preferred that the nucleotide sequence of said miRNA or the nucleotide sequences of said miRNAs is (are) selected from the group consisting of SEQ ID NOs: 233, 234, 235, 237, 240, 241, 242, 243, 245, 24b, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, and 329 (see list 3 of **Figure 18**), wherein the comparison and/or application in step (ii) allow(s) for the diagnosis and/or prognosis of lung cancer. It is further particularly preferred that the nucleotide sequences of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 miRNAs in step (i) of the method described herein are selected from the group consisting of SEQ ID NOs: 233, 234, 235, 237, 240, 241, 242, 243, 245, 246, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, and 329 (see list 3 of **Figure 18**), wherein the comparison and/or application in step (ii) allow(s) for the diagnosis and/or prognosis of lung cancer.

More preferably, the nucleotide sequence of said miRNA or the nucleotide sequences of said miRNAs in step (i) is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, 227 (see list 2 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**), wherein the comparison and/or application in step (ii) allow(s) for differential diagnosis (of lung cancer) with respect to sarcoidosis and/or COPD.

Most preferably,

(i) the nucleotide sequence of said miRNA or the nucleotide sequences of said miRNAs in step (i) is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, and 227 (see list 2 of **Figure 18**), wherein the comparison and/or application in step (ii) allow(s) for differential diagnosis (of lung cancer) with respect to sarcoidosis,

(ii) the nucleotide sequence of said miRNA or the nucleotide sequences of said miRNAs in step (i) is (are) selected from the group consisting of SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228 (see list 4 of **Figure 18**), wherein the comparison and/or application in step (ii) allow(s) for differential diagnosis (of lung cancer) with respect to sarcoidosis and COPD, or

(iii) the nucleotide sequence of said miRNA or the nucleotide sequences of said miRNAs is (are) selected from the group consisting of SEQ ID NOs: 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**), wherein the comparison and/or application in step (ii) allow(s) for differential diagnosis (of lung cancer) with respect to COPD.

In preferred embodiments, the nucleotide sequences of said miRNAs are the nucleotide sequences according to Set No. 1 to 42 (i.e. SL-1 to SL-42) of **Figure 16.**

Preferably, the nucleotide sequence of said miRNA or the nucleotide sequences of said miRNAs in step (i) is (are) selected from the group consisting of SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391 (see list 6 of **Figure 18**), 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, 231 (see list 7 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**), wherein the comparison and/or application in step (ii) allow(s) for the diagnosis and/or prognosis of COPD.

In this respect it is particularly preferred that the nucleotide sequence of said miRNA or the nucleotide sequences of said miRNAs in step (i) is (are) selected from the group consisting of SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, and 391 (see list 6 of **Figure 18**), wherein the comparison and/or application in step (ii) allow(s) for the diagnosis and/or prognosis of COPD. It is further particularly preferred that that the nucleotide sequences of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 miRNAs in step (i) of the method described herein are selected from the group consisting of SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, and 391 (see list 6 of **Figure 18**), wherein the comparison and/or application in step (ii) allow(s) for the diagnosis and/or prognosis of COPD.

More preferably, the nucleotide sequence of said miRNA or the nucleotide sequences of said miRNAs in step (i) is (are) selected from the group consisting of SEQ ID NOs: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, 231 (see list 7 of **Figure 18**), 1,2,3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**), wherein the comparison and/or application in step (ii) allow(s) for differential diagnosis (of COPD) with respect to sarcoidosis and/or lung cancer.

Most preferably,

(i) the nucleotide sequence of said miRNA or the nucleotide sequences of said miRNAs in step (i) is (are) selected from SEQ ID NOs: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231 (see list 7 of **Figure 18**), wherein the comparison and/or application in step (ii) allow(s) for differential diagnosis (of COPD) with respect to sarcoidosis,
(ii) the nucleotide sequence of said miRNAs or the nucleotide sequences of said miRNAs is (are) selected from SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228 (see list 4 of **Figure 18**) ), wherein the comparison and/or application in step (ii) allow(s) for differential diagnosis (of COPD) with respect to sarcoidosis and lung cancer, or
(iii) the nucleotide sequence of said miRNA or the nucleotide sequences of said miRNAs is (are) selected from SEQ ID NOs: 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**), wherein the comparison and/or application in step (ii) allow(s) for differential diagnosis (of COPD) with respect to lung cancer.

In preferred embodiments, the nucleotide sequences of said miRNAs are the nucleotide sequences according to Set No. 1 to 42 (i.e. SC-1 to SC-42) of **Figure 17.**

It is preferred that the nucleotide sequences of the miRNAs have (i) SEQ ID NO: 2 and SEQ ID NO: 3, (ii) SEQ ID NO: 3 and SEQ ID NO: 4, (iii) SEQ ID NO: 5, SEQ ID NO: 6, and SEQ ID NO: 7, (iv) SEQ ID NO: 8, SEQ ID NO: 9, and SEQ ID NO: 10, (v) SEQ ID NO: 11, SEQ ID NO: 12, and SEQ ID NO: 13, (vi) SEQ ID NO: 14, SEQ ID NO: 15, and SEQ ID NO: 16, (vii) SEQ ID NO: 17, SEQ ID NO: 18, SEQ ID NO: 19, and SEQ ID NO: 20, (viii) SEQ ID NO: 21, SEQ ID NO: 22, SEQ ID NO: 23, and SEQ ID NO: 24, (ix) SEQ ID NO: 25, SEQ ID NO: 26, SEQ ID NO: 27, SEQ ID NO: 28, SEQ ID NO: 29, and SEQ ID NO: 30, (x) SEQ ID NO: 31, SEQ ID NO: 32, SEQ ID NO: 33, SEQ ID NO: 34, SEQ ID NO: 35, and SEQ ID NO: 36, or (xi) SEQ ID NO: 37, SEQ ID NO: 38, SEQ ID NO: 39, SEQ ID NO: 40. Said miRNAs (miRNA combinations) may also be further combined with each other, e.g. (i) with (ii), (ii) with (iii), (iii) with (iv), (iv) with (v), (v) with (vi), (vi) with (vii), or any other possible combination.

It is also preferred that the nucleotide sequences of the miRNAs have (i) SEQ ID NO: 1 and SEQ ID NO: 2, (ii) SEQ ID NO: 1 and SEQ ID NO: 3, (iii) SEQ ID NO: 1 and SEQ ID NO: 4, (iv) SEQ ID NO: 4 and SEQ ID NO: 5, (v) SEQ ID NO: 5 and SEQ ID NO: 6, (vi) SEQ ID NO: 6 and SEQ ID NO: 7, (vii) SEQ ID NO: 7 and SEQ ID NO: 8, (viii) SEQ ID NO: 8 and SEQ ID NO: 9, (ix) SEQ ID NO: 9 and SEQ ID NO. 10, (x) SEQ ID NO: 10 and SEQ ID NO: 11, or (xi) SEQ ID NO: 11 and SEQ ID NO: 12. Said miRNAs (miRNA combinations) may also be further combined with each other, e.g. (i) with (ii), (ii) with (iii), (iii) with (iv), (iv) with (v), (v) with (vi), (vi) with (vii), or any other possible combination. It is also preferred that the nucleotide sequences of the miRNAs have (i) SEQ ID NO: 1 to SEQ ID NO: 3, (ii) SEQ ID NO: 2 to SEQ ID NO: 4, (iii) SEQ ID NO: 3 to SEQ ID NO: 5, (iv) SEQ ID NO: 4 to SEQ ID NO: 6, (v) SEQ ID NO: 5 to SEQ ID NO: 7, (vi) SEQ ID NO: 6 to SEQ ID NO: 8, (vii) SEQ ID NO: 7 to SEQ ID NO: 9, (viii) SEQ ID NO: 8 to SEQ ID NO: 10, (ix) SEQ ID NO: 9 to SEQ ID NO: 11, (x) SEQ ID NO: 10 to SEQ ID NO: 12, or (xi) SEQ ID NO: 11 to SEQ ID NO: 13. Said miRNAs (miRNA combinations) may also be further combined with each other, e.g. (i) with (ii), (ii) with (iii), (iii) with (iv), (iv) with (v), (v) with (vi), (vi) with (vii), or any other possible combination.

It is particularly preferred that the nucleotide sequences of the miRNAs have (i) SEQ ID NO: 1, SEQ ID NO: 2, SEQ ID NO: 3, SEQ ID NO: 4, SEQ ID NO: 68, SEQ ID NO: 5, SEQ ID NO: 6, SEQ ID NO: 7, SEQ ID NO: 69, and SEQ ID NO: 8, (ii) SEQ ID NO: 68, SEQ ID NO: 5, SEQ ID NO: 6, SEQ ID NO: 7, SEQ ID NO: 69, SEQ ID NO: 8, SEQ ID NO: 70, SEQ ID NO: 105, SEQ ID NO: 9, and SEQ ID NO: 10, (iii) SEQ ID NO: 8, SEQ ID NO: 70, SEQ ID NO: 105, SEQ ID NO: 9, SEQ ID NO: 10, SEQ ID NO: 11, SEQ ID NO: 12, SEQ ID NO: 13, SEQ ID NO: 14, and SEQ ID NO: 114, (iv) SEQ ID NO: 68, SEQ ID NO: 1, SEQ ID NO: 4, SEQ ID NO: 69, SEQ ID NO: 3, SEQ ID NO: 5, SEQ ID NO: 8, SEQ ID NO: 70, SEQ ID NO: 7, and SEQ ID NO: 6, (v) SEQ ID NO: 3, SEQ ID NO: 5, SEQ ID NO: 8, SEQ ID NO: 70, SEQ ID NO: 7, SEQ ID NO: 6, SEQ ID NO: 12, SEQ ID NO: 9, SEQ ID NO: 105, and SEQ ID NO: 107, or (vi) SEQ ID NO: 6, SEQ ID NO: 12, SEQ ID NO: 9, SEQ ID NO: 105, SEQ ID NO: 107, SEQ ID NO: 71, SEQ ID NO: 17, SEQ ID NO: 159, SEQ ID NO: 19, and SEQ ID NO: 106. Said miRNAs (miRNA combinations) may also be further combined with each other, e.g. (i) with (ii), (ii) with (iii), (iii) with (iv), (iv) with (v), (v) with (vi), (vi) with (vii), or any other possible combination.

In another preferred embodiment, the nucleotide sequence of the (single) miRNA is selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10, or is selected from the group consisting of SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, and SEQ ID NO: 20. It is particularly preferred that the nucleotide sequence of the (single) miRNA is selected from the group consisting of SEQ ID NO: 1, SEQ ID NO: 2, SEQ ID NO: 3, SEQ ID NO: 4, and SEQ ID NO: 5.

It should be noted that the miRNAs disclosed herein and mentioned above (at least two miRNAs) may be comprised in a set. For example, said set may comprise, essentially consist of, or consist of at least 2, 3, 4, 5, 6, 7, 8, 9, 10, 11, 12, 13, 14, 15, 16, 17, 18, 19, 20, 21, 22, 23, 24, 25, 26, 27, 28, 29, 0, 31, 32, 33, 4, 5, 6, 7, 8, 9, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175. 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, or 391 miRNAs, wherein the nucleotide sequences of said miRNAs are selected from the group consisting of SEQ ID NOs: 1 to 391.

As mentioned above, an expression profile of one or more miRNA(s) in a biological sample from a patient is determined in the first step of the method described herein, wherein the nucleotide sequence of said miRNA or the nucleotide sequences of said miRNAs is (are) selected from the group consisting of

(i) a nucleotide sequence according to SEQ ID NOs: 1 to 391,

(ii) a nucleotide sequence that is a fragment of the nucleotide sequence according to (i), preferably, a nucleotide sequence that is a fragment which is between 1 and 12, more preferably between 1 and 8, and most preferably between 1 and 5 or 1 and 3, i.e. 1, 2, 3, 4, 5, 6, 7, 8, 9, 10, 11, or 12, nucleotides shorter than the nucleotide sequence according to (i), and

(iii) a nucleotide sequence that has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity to the nucleotide sequence according to (i) or nucleotide sequence fragment according to (ii).

It is particularly preferred that the nucleotide sequence as defined in (iii) has at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity over a continuous stretch of at least 10, 11, 12, 13, 14, 15, 16, 17, 18, 19, 20 or more nucleotides, preferably over the whole length, to the nucleotide sequence of the nucleotide according to (i) or nucleotide fragment according to (ii).

In addition, the nucleotide sequence as defined in (ii) (i.e. nucleotide sequence fragment) or (iii) (i.e. nucleotide sequence variant or nucleotide sequence fragment variant) is only regarded as a nucleotide sequence as defined in (ii) (i.e. nucleotide sequence fragment) or (iii) (i.e. nucleotide sequence variant or nucleotide sequence fragment variant) within the context described herein, if it can still be bound, hybridized, recognized, or detected by a polynucleotide (probe) of complementary sequence, e.g. a polynucleotide (probe) which is complementary to the respective nucleotide sequence as defined in (i), through one or more types of chemical bonds, usually through complementary base pairing, usually through hydrogen bond formation under stringent hybridization conditions. The skilled person can readily assess whether a nucleotide sequence as defined in (ii) (i.e. nucleotide sequence fragment) or (iii) (i.e. nucleotide sequence variant or nucleotide sequence fragment variant) can still be bound, hybridized, recognized, or detected by a polynucleotide (probe) of complementary sequence, e.g. a polynucleotide (probe) which is complementary to the respective nucleotide sequence as defined in (i). Suitable assays to determine whether hybridization under stringent conditions still occurs are well known in the art. However, as an example, a suitable assay to determine whether hybridization still occurs comprises the steps of: (a) incubating a nucleotide sequence as defined in (ii) or (iii) labelled with biotin with a polynucleotide (probe) of complementary sequence, e.g. a polynucleotide (probe) which is complementary to the respective nucleotide sequence as defined in (i), wherein the polynucleotide (probe) is attached onto a biochip, under stringent hybridization conditions, (b) washing the biochip to remove unspecific bindings, (c) subjecting the biochip to a detection system, and (c) analyzing whether the nucleotide sequence can still be hybridized or detected by a polynucleotide (probe) of complementary sequence, e.g. a polynucleotide (probe) which is complementary to the respective nucleotide sequence as defined in (i). As a positive control, the respective miRNA as defined in (i) may be used. Preferably stringent hybridization conditions include the following: 50% formamide, 5x SSC, and 1% SDS, incubating at 42°C, or, 5x SSC, 1% SDS, incubating at 65°C, with wash in 0.2x SSC, and 0.1% SDS at 65°C; or 6x SSPE, 10 % formamide, 0.01 %,Tween 20, 0.1 x TE buffer, 0.5 mg/ml BSA, 0.1 mg/ml herring sperm DNA, incubating at 42°C with wash in 0.5x SSPE and 6x SSPE at 45°C.

It is preferred that in step (i) of the method according to the second aspect of the present invention, polynucleotides according to the first aspect of the present invention are used for determining an expression profile of miRNAs in a blood sample from a patient. Thus, for example, in step (i) of the method of the second aspect of the present invention, polynucleotides are used for determining an expression profile of miRNAs in a blood sample from a patient, e.g. human or animal, wherein the nucleotide sequences of said miRNAs comprise the nucleotide sequences according to Set No. 1 to 42 of Figure 15.

As mentioned above, in step (i) of the method of the second aspect of the present invention, an expression profile of miRNAs is determined in a blood sample from a patient, e.g. a human or an animal. The determination may be carried out by any convenient means for determining a RNA expression level or RNA expression levels. For expression profiling, qualitative, semi-quantitative and/or quantitative detection methods may be used. A variety of techniques are well known to the person skilled in the art. It is preferred that the expression profile of the miRNAs is determined by nucleic acid hybridization, nucleic acid amplification, polymerase extension, sequencing, mass spectroscopy, or any combination thereof.

Nucleic acid amplification may be performed using real time polymerase chain reaction (RT-PCR) such as real time quantitative polymerase chain reaction (RT qPCR). The real time polymerase chain reaction (RT-PCR) is preferred for the analysis of a single miRNA or a low number of miRNAs (e.g. at least 2 to 50 miRNAs such as 2, 5, 10, 20, 30, 40, or 50 miRNAs). It is particularly suitable for detecting low abundance miRNAs. The real time quantitative polymerase chain reaction (RT qPCR), however, allows the analysis of a single miRNA as well as a high number of miRNAs (e.g. at least 2 to 391 miRNAs such as 2, 10, 20, 30, 40, 50, 60, 70, 80, 90, 100, 200, 300, or 391 miRNAs) comprised in a biological sample from a patient.

The aforesaid real time polymerase chain reaction (RT-PCR) may include the following steps: (i) extracting the total RNA from a biological sample such as tissue, urine or blood (e.g. whole blood, serum, or plasma) sample of a patient such as a human or an animal, and obtaining cDNA samples by RNA reverse transcription (RT) reaction using miRNA-specific primers, or collecting a biological sample such as a urine or blood (e.g. whole blood, serum, or plasma) sample of a patient such as a human or an animal, and conducting reverse transcriptase reaction using miRNA-specific primers with the biological sample such as the urine or blood (e.g. whole blood, serum, or plasma) sample being a buffer so as to prepare cDNA samples, (ii) designing miRNA-specific cDNA forward primers and providing universal reverse primers to amplify the cDNA via polymerase chain reaction (PCR), (iii) adding a fluorescent probe to conduct PCR, and (iv) detecting the miRNA(s) level in the biological sample such as the tissue, urine or blood (e.g. whole blood, serum, or plasma) sample.

A variety of kits and protocols to determine an expression profile by real time polymerase chain reaction (RT-PCR) such as real time quantitative polymerase chain reaction (qRT-PCR) are available. For example, reverse transcription of miRNAs may be performed using the TaqMan MicroRNA Reverse Transcription Kit (Applied Biosystems) according to manufacturer's recommendations. Briefly, miRNA may be combined with dNTPs, MultiScribe reverse transcriptase and the primer specific for the target miRNA. The resulting cDNA may be diluted and may be used for PCR reaction. The PCR may be performed according to the manufacturer's recommendation (Applied Biosystems). Briefly, cDNA may be combined with the TaqMan assay specific for the target miRNA and PCR reaction may be performed using ABI 7300.

Nucleic acid hybridization may be performed using a microarray/biochip or *in situ* hybridization. *In situ* hybridization is preferred for the analysis of a single miRNA or a low number of miRNAs (e.g. at least 2 to 50 miRNAs such as 2, 5, 10, 20, 30, 40, or 50 miRNAs). The microarray/biochip, however, allows the analysis of a single miRNA as well as a high number of miRNAs (e.g. at least 2 to 391 miRNAs such as at least such as 2, 10, 20, 30, 40, 50, 60, 70, 80, 90, 100, 200, 300, or 391 miRNAs) comprised in a biological sample such as blood or tissue sample from a patient such as a human or an animal.

For nucleic acid hybridization, for example, the polynucleotides (probes) described herein with complementarity to the corresponding miRNAs to be detected are attached to a solid phase to generate a microarray/biochip (e.g. 391 polynucleotides (probes) which are complementary to the 391 miRNAs having SEQ ID NOs: 1 to 391). Said microarray/biochip is then incubated with miRNAs, isolated (e.g. extracted) from the biological sample such as blood or tissue sample from a patient such as a human or an animal, which may be labelled, e.g. fluorescently labelled, or unlabelled. Upon hybridization of the labelled miRNAs to the complementary polynucleotide sequences on the microarray/biochip, the success of hybridisation may be controlled and the intensity of hybridization may be determined via the hybridisation signal of the label in order to determine the expression level of each tested miRNA in said biological sample, e.g. blood or tissue sample.

Preferably, (i) the nucleic acid hybridization is performed using a microarray/biochip, or using *in situ* hybridization, and/or (ii) the nucleic acid amplification is performed using real-time PCR.

Subsequent to the determination of an expression profile (data) of one or more miRNAs as defined above in a biological sample (e.g. blood or tissue sample) from a patient (e.g. human or animal) in step (i) of the method described herein, said expression profile (data) is compared to a reference in step (ii) of the method described herein, wherein the comparison of said expression profile (data) to said reference allows for the diagnosis and/or prognosis of a lung disease such as sarcoidosis, lung cancer, and/or COPD, and/or an algorithm or a mathematical function is applied to said expression profile (data) in step (ii) of the method described herein, wherein the application of said algorithm or mathematical function to said expression profile (data) allows for the diagnosis and/or prognosis of a lung disease such as sarcoidosis, lung cancer, and/or COPD. The reference may be any reference which allows for the diagnosis and/or prognosis of a lung disease such as sarcoidosis, lung cancer, and/or COPD, e.g. an indicated value or values, and/or the algorithm or mathematical function may be any algorithm or mathematical function which allows for the diagnosis and/or prognosis of a lung disease such as sarcoidosis, lung cancer, and/or COPD. In the method according to the second aspect of the present invention, the expression profile of the miRNAs determined in a blood sample from a patient in step (i) is compared to a reference in step (ii), wherein the comparison of said expression profile to said reference allows for the diagnosis of sarcoidosis. The term "known (clinical) condition" (biological state or health state), as used herein, means a known status of a subject that can be described by physical, mental or social criteria. It includes so-called "healthy" and "diseased" conditions. For the definition of "healthy" and "diseased" conditions it is referred to the international classification of diseases (ICD) of the WHO (http://www.int/classifications/icd/en/index.html). When the expression profile (data) determined in a patient is compared to a reference of a (at least one) known (clinical) condition or when an algorithm or a mathematical function obtained from a reference of a (at least one) known (clinical) condition is applied to the expression profile (data) determined in a patient, said known (clinical) condition may be healthiness or a lung disease such as sarcoidosis, lung cancer or COPD.

Further, for example, when the expression profile (data) determined in a patient is compared to a reference of at least two known (clinical) conditions or when an algorithm or a mathematical function obtained from a reference of at least two known (clinical) conditions is applied to the expression profile (data) determined in a patient, said at least two known (clinical) conditions may be (i) healthiness and sarcoidosis, (ii) healthiness and lung cancer, (iii) healthiness and COPD, (iv) sarcoidosis and lung cancer, (v) sarcoidosis and COPD, or (vi) lung cancer and COPD.

Furthermore, for example, when the expression profile (data) determined in a patient is compared to a reference of at least three known (clinical) conditions or when an algorithm or a mathematical function obtained from a reference of at least three known (clinical) conditions is applied to the expression profile (data) determined in a patient, said at least three known (clinical) conditions may be (i) healthiness, sarcoidosis and lung cancer, (ii) healthiness, lung cancer and COPD, (iii) healthiness, sarcoidosis and COPD, or (iv) sarcoidosis, lung cancer and COPD.

In addition, for example, when the expression profile (data) determined in a patient is compared to a reference of at least four known (clinical) conditions or when an algorithm or a mathematical function obtained from a reference of at least four known (clinical) conditions is applied to the expression profile (data) determined in a patient, said at least four known (clinical) conditions may be healthiness, sarcoidosis, lung cancer and COPD.

It should be noted that the term "reference expression profile (data)", as used herein, means an expression profile (data) of a (one) miRNA that has a nucleotide sequence that essentially corresponds (is essentially identical), preferably that corresponds (is identical), to the nucleotide sequence of the miRNA of step (i), or an expression profile (data) of (more than one) miRNAs that have nucleotide sequences that essentially correspond (are essentially identical), preferably that correspond (are identical), to the nucleotide sequences of the miRNAs of step (i), but means an expression profile which is obtained from a (control) subject(s) with a known (clinical) condition, e.g. healthiness or a lung disease such as sarcoidosis, lung cancer or COPD.

The terms "essentially correspond(s)" or "essentially identical", as used herein, mean that the miRNA(s) of step (i) and the miRNA(s) of the reference expression profile may slightly differ in their nucleotide sequence, whereas said difference is so marginal that it may still allow for the diagnosis and/or prognosis of a lung disease such as sarcoidosis, lung cancer, and/or COPD. Preferably, the nucleotide sequence(s) of the miRNA(s) of step (i) and the nucleotide sequence(s) of the miRNA(s) of the reference expression profile differ in 1 to 5, more preferably in 1 to 3, and most preferably in 1 to 2 nucleotides, i.e. in 1, 2, 3, 4, or 5 nucleotides. It is preferred that said difference resides in 1 to 5, more preferably 1 to 3, and most preferably 1 to 2 nucleotide mutations, i.e. 1, 2, 3, 4, or 5 nucleotide mutations (e.g. substitutions, additions, insertions, and/or deletions). This is caused by the fact that the miRNAs between the species (e.g. humans and animals) and within the species (e.g. humans or animals) may differ. For example, the miRNA(s) of the reference expression profile may be from the same species (e.g. human) as the miRNA(s) of (i) (e.g. human) or may be from a different species (e.g. human) as the miRNA(s) of (i) (e.g. animal), particularly under the condition that the nucleotide sequences of said miRNAs only differ in 1 to 5 nucleotides (e.g. 1, 2, 3, 4, or 5 nucleotides) (see above). It is preferred that the miRNA(s) of step (i) and the miRNA(s) of the reference expression profile do not differ in their nucleotide sequence, i.e. are identical, and/or that the miRNA(s) of step (i) and the miRNA(s) of the reference expression profile are from the same species (e.g. human or animal). It is particularly preferred that the miRNA(s) of step (i) and the miRNA(s) of the reference expression profile are derived from (control) subjects/patients of the same gender and/or similar age or phase of life.

Preferably, both the reference expression profile and the expression profile of step (i) are determined in the same biological sample, e.g. tissue, urine, or blood sample including a blood serum sample, blood plasma sample, or a blood cell sample (e.g. erythrocytes, leukocytes and/or thrombocytes). It is understood that the reference expression profile is not necessarily obtained from a single (control) subject, e.g. a subject known to be affected by a lung disease such as sarcoidosis, lung cancer or COPD, or a subject known to be not affected by a lung disease such as sarcoidosis, lung cancer or COPD (i.e. healthy), but may be an average reference expression profile of a plurality of (control) subjects, e.g. subjects known to be affected by a lung disease such as sarcoidosis, lung cancer or COPD, or subjects known to be not affected by a lung disease such as sarcoidosis, lung cancer or COPD (i.e. healthy), e.g. at least 2 to 40 subjects, more preferably at least 10 to 25 subjects, and most preferably at least 15 to 20 subjects, i.e. at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects. The reference expression profile and the expression profile of step (i) may be obtained from a (control) subject/patient of the same species (e.g. human or animal), or may be obtained from a (control) subject/patient of a different species (e.g. human or animal). Preferably, both the reference expression profile and the expression profile of step (i) are obtained from a (control) subject/patient of the same species (e.g. human or animal), of the same gender (e.g. female or male) and/or of a similar age or phase of life (e.g. infant, young child, juvenile, adult).

Thus, for example, the reference is a reference expression profile (data) of at least one subject, preferably the reference is an average expression profile (data) of at least 2 to 40 subjects, more preferably of at least 10 to 25 subjects, and most preferably of at least 15 to 20 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with a (at least one) known clinical condition, wherein the reference expression profile is the profile of a (one) miRNA that has a nucleotide sequence that essentially corresponds (is essentially identical), preferably that corresponds (is identical), to the nucleotide sequence of the miRNA of step (i) or is the profile of (more than one) miRNAs that have nucleotide sequences that essentially correspond (are essentially identical), preferably that correspond (are identical), to the nucleotide sequences of the miRNAs of step (i). More preferably, the known clinical condition is healthiness or a lung disease (e.g. sarcoidosis, lung cancer or COPD). Most preferably, the known clinical condition is selected from the group consisting of healthiness, sarcoidosis, lung cancer, and COPD.

For example, said reference may be a reference expression profile of at least one subject known to be not affected by a lung disease such as sarcoidosis, lung cancer or COPD (i.e. healthy) or known to be affected by a lung disease such as sarcoidosis, lung cancer or COPD (i.e. diseased), wherein the reference expression profile is the profile of a miRNA that has a nucleotide sequence that essentially corresponds (is essentially identical), preferably that corresponds (is identical), to the nucleotide sequence of the miRNA of step (i) or is the profile of miRNAs that have nucleotide sequences that essentially correspond (are essentially identical), preferably that correspond (are identical), to the nucleotide sequences of the miRNAs of step (i).

The comparison of the expression profile of the patient to be diagnosed and/or prognosed to the (average) reference expression profile (data), may then allow for the diagnosis and/or prognosis of a lung disease such as sarcoidosis, lung cancer or COPD (step (ii)).

Considering the above, diagnosing preferably means comparing the expression profile (data) of a patient determined in step (i) to the (average) reference expression profile (data) as mentioned above to decide, if the known clinical condition, which is a lung disease such as sarcoidosis, lung cancer or COPD, or which is no lung disease such as sarcoidosis, lung cancer or COPD (i.e. healthy), is present in said patient. Prognosing preferably means comparing the expression profile (data) of a patient determined in step (i) to the (average) reference expression profile (data) as mentioned above to decide, if the known clinical condition, which is a lung disease such as sarcoidosis, lung cancer or COPD, or which is no lung disease such as sarcoidosis, lung cancer or COPD (i.e. healthy), will likely be present in said patient.

For example, the patient may be diagnosed as not suffering from a lung disease such as sarcoidosis, lung cancer or COPD (i.e. being healthy), or as suffering from a lung disease such as sarcoidosis, lung cancer or COPD (i.e. being diseased). Further, for example, the patient may be prognosed as not developing a lung disease such as sarcoidosis, lung cancer or COPD (i.e. staying healthy), or as developing a lung disease such as sarcoidosis, lung cancer or COPD (i.e. getting diseased).

Diagnosing/prognosing of a lung disease such as sarcoidosis, lung cancer or COPD based on (a) reference expression profile (data) as reference may take place as follows: For instance, (i) if the miRNA(s) of step (i) (e.g. a single miRNA or at least 2 miRNAs) in the expression profile of a patient to be diagnosed for a lung disease such as sarcoidosis, lung cancer or COPD is (are), for example, at least 2 fold higher (up-regulated) compared to said miRNA(s) (e.g. a single miRNA or at least 2 miRNAs) in the reference expression profile of a subject known not to suffer from a lung disease such as sarcoidosis, lung cancer or COPD (i.e. being healthy), the patient tested is diagnosed as suffering from a lung disease such as sarcoidosis, lung cancer or COPD (i.e. being diseased) or prognosed as likely developing a lung disease such as sarcoidosis, lung cancer or COPD (i.e. getting diseased), or (ii) if the miRNA(s) of step (i) (e.g. a single miRNA or at least 2 miRNAs) in the expression profile of a patient to be diagnosed for a lung disease such as sarcoidosis, lung cancer or COPD is (are), for example, at least 2 fold lower (down-regulated) compared to said miRNA(s) (e.g. a single miRNA or a set of at least 2 miRNAs) in the reference expression profile of a subject known not to suffer from a lung disease such as sarcoidosis, lung cancer or COPD (i.e. being healthy), the patient tested is diagnosed as suffering from a lung disease such as sarcoidosis, lung cancer or COPD (i.e. being diseased) or prognosed as likely developing a lung disease such as sarcoidosis, lung cancer or COPD (i.e. getting diseased).

In the converse case, (i) if the miRNA(s) of step (i) (e.g. a single miRNA or at least 2 miRNAs) in the expression profile of a patient to be diagnosed for a lung disease such as sarcoidosis, lung cancer or COPD is (are), for example, not at least 2 fold higher (up-regulated) compared to said miRNA(s) (e.g. a single miRNA or at least 2 miRNAs) in the reference expression profile of a subject known not to suffer from a lung disease such as sarcoidosis, lung cancer or COPD (i.e. being healthy), the patient tested is diagnosed as not suffering from a lung disease such as sarcoidosis, lung cancer or COPD (i.e. being healthy) or prognosed as likely not developing a lung disease such as sarcoidosis, lung cancer or COPD (i.e. staying healthy), or (ii) if the miRNA(s) of step (i) (e.g. a single miRNA or at least 2 miRNAs) in the expression profile of a patient to be diagnosed for a lung disease such as sarcoidosis, lung cancer or COPD is (are), for example, not at least 2 fold lower (down-regulated) compared to said miRNA(s) (e.g. a single miRNA or at least 2 miRNAs) in the reference expression profile of subject known not to suffer from a lung disease such as sarcoidosis, lung cancer or COPD (i.e. being healthy), the patient tested (e.g. human or animal) is diagnosed as not suffering from a lung disease such as sarcoidosis, lung cancer or COPD (i.e. being healthy) or prognosed as likely not developing a lung disease such as sarcoidosis, lung cancer or COPD (i.e. staying healthy).

It should be noted that a patient that is diagnosed as being healthy, i.e. not suffering from a lung disease, may possibly suffer from another disease not tested/known, or a patient that is prognosed as staying healthy, i.e. likely not developing a lung disease, may possibly developing another disease not tested/known (see also definition of the term "patient" above).

Preferably, the algorithm or mathematical function is obtained from a reference expression profile of at least one subject, preferably of at least 2 to 40 subjects, more preferably of at least 10 to 25 subjects, and most preferably of at least 15 to 20 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with a (at least one) known clinical condition, wherein the reference expression profile is the profile of a (one) miRNA that has a nucleotide sequence that essentially corresponds (is essentially identical), preferably that corresponds (is identical), to the nucleotide sequence of the miRNA of step (i), or is the profile of (more than one) miRNAs that have nucleotide sequences that essentially correspond (are essentially identical), preferably that correspond (are identical), to the nucleotide sequences of the miRNAs of step (i). More preferably, the known clinical condition is healthiness or a lung disease (e.g. sarcoidosis, lung cancer or COPD). Most preferably, the known clinical condition is selected from the group consisting of healthiness, sarcoidosis, lung cancer, and COPD.

For example, said algorithm or mathematical function may be obtained from a reference expression profile of at least one subject known to be not affected by a lung disease such as sarcoidosis, lung cancer or COPD (i.e. healthy), or known to be affected by a lung disease such as sarcoidosis, lung cancer or COPD (i.e. diseased), wherein the reference expression profile is the profile of a miRNA that has a nucleotide sequence that essentially corresponds (is essentially identical), preferably that corresponds (is identical), to the nucleotide sequence of the miRNA of step (i) or is the profile of miRNAs that have nucleotide sequences that essentially correspond (are essentially identical), preferably that correspond (are identical), to the nucleotide sequences of the miRNAs of step (i).

It is particularly preferred that the algorithm or mathematical function is obtained from a reference expression profile of at least one subject, preferably of at least 2 to 40 subjects, more preferably of at least 10 to 25 subjects, and most preferably of at least 15 to 20 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with at least one known clinical condition, wherein the reference expression profile is the profile of a miRNA that has a nucleotide sequence that essentially corresponds (is essentially identical), preferably that corresponds (is identical), to the nucleotide sequence of the miRNA of step (i) or is the profile of miRNAs that have nucleotide sequences that essentially correspond (are essentially identical), preferably that correspond (are identical), to the nucleotide sequences of the miRNAs of step (i). More preferably, the at least one known clinical condition is healthiness or a lung disease (e.g. sarcoidosis, lung cancer or COPD). Most preferably, the at least one known clinical condition is selected from the group consisting of healthiness, sarcoidosis, lung cancer, and COPD.

It is also particularly preferred that the algorithm or mathematical function is obtained from reference expression profiles of at least two subjects, preferably of at least 3 to 40 subjects, more preferably of at least 10 to 25 subjects, and most preferably of at least 15 to 20 subjects, i.e. of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with at least two known clinical conditions, preferably 2 to 4 (e.g. 2, 3, or 4) known clinical conditions, wherein the reference expression profiles are the profiles of a miRNA that has a nucleotide sequence that essentially corresponds (is essentially identical), preferably that corresponds (is identical) to the nucleotide sequence of the miRNA of step (i), or are the profiles of miRNAs that have nucleotide sequences that essentially correspond (are essentially identical), preferably that correspond (are identical), to the nucleotide sequences of the miRNAs of step (i). Preferably, the at least two known clinical conditions are selected from the group consisting of healthiness, sarcoidosis, lung cancer, and COPD.

More preferably, the algorithm or mathematical function is obtained from (a) reference expression profile(s) of

(i) at least one subject with the known clinical condition of sarcoidosis,
(ii) at least one subject with the known clinical condition of healthiness,
(iii) at least one subject with the known clinical condition of lung cancer, and/or
(iv) at least one subject with the known clinical condition of COPD, wherein the reference expression profile(s) is (are) the profile(s) of a miRNA that has a nucleotide sequence that essentially corresponds (is essentially identical), preferably that corresponds (is identical), to the nucleotide sequence of the miRNA of step (i), or is (are) the profile(s) of miRNAs that have nucleotide sequences that essentially correspond (are essentially identical), preferably that correspond (are identical), to the nucleotide sequences of the miRNAs of step (i).

For example, the algorithm or mathematical function may be obtained from a reference expression profile of

(i) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, healthiness, lung cancer, or COPD,
(ii) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, lung cancer, or COPD,
(iii) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer or COPD,
(iv) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD,
(v) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer,
(vi) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD,

(vii) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD,

(viii) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD, or

(ix) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD,

wherein the reference expression profile(s) is (are) the profile(s) of a miRNA that has a nucleotide sequence that essentially corresponds (is essentially identical), preferably that corresponds (is identical), to the nucleotide sequence of the miRNA of step (i), or is (are) the profile(s) of miRNAs that have nucleotide sequences that essentially correspond (are essentially identical), preferably that correspond (are identical), to the nucleotide sequences of the miRNAs of step (i).

Preferably, the above mentioned algorithm or mathematical function is obtained from reference expression profiles of the same number of (control) subjects (e.g. subjects known to be healthy or diseased). For example, the algorithm or mathematical function may be obtained from reference expression profiles of 10 subjects known to suffer from a lung disease such as sarcoidosis, lung cancer or COPD (positive control) and 10 subjects known not to suffer from a lung disease such as sarcoidosis, lung cancer or COPD (negative control). The algorithm or mathematical function may also be obtained from reference expression profiles of 20 subjects known to suffer from a lung disease such as sarcoidosis, lung cancer or COPD (positive control) and 20 subjects known not to suffer from a lung disease such as sarcoidosis, lung cancer or COPD (negative control).

It is preferred that the algorithm or mathematical function is obtained using a machine learning approach. Machine learning approaches may include but are not limited to supervised or unsupervised analysis: classification techniques (e.g. naive Bayes, Linear Discriminant Analysis, Quadratic Discriminant Analysis Neural Nets, Tree based approaches, Support Vector Machines, Nearest Neighbour Approaches), Regression techniques (e.g. linear Regression, Multiple Regression, logistic regression, probit regression, ordinal logistic regression ordinal probit regression, Poisson Regression, negative binomial Regression, multinomial logistic Regression, truncated regression), Clustering techniques (e.g. k-means clustering, hierarchical clustering, PCA), Adaptations, extensions, and combinations of the previously mentioned approaches.

The inventors of the present invention surprisingly found that the application of a machine learning approach (e.g. t-test, AUC, support vector machine, hierarchical clustering, or k-means) leads to the obtainment of an algorithm or a mathematical function that is trained by the reference expression profile(s) (data) mentioned above and that this allows (i) a better discrimination between the known clinical conditions (statistical classes) (e.g. 2, 3 or 4 known clinical conditions or 2, 3 or 4 known statistical classes) or (ii) a better decision, whether a (at least one) known clinical condition (statistical class) (e.g. 1, 2, 3 or 4 known clinical condition(s) or 1, 2, 3 or 4 known statistical classe(s)) is present. In this way, the performance for diagnosing/prognosing of patients suffering from a lung disease such as sarcoidosis, lung cancer and/or COPD can be increased (see also experimental section for details).

Preferably, the machine learning approach involves the following steps:

(i) inputting the reference expression profile of at least one subject, preferably of at least 2 to 40 subjects, more preferably of at least 10 to 25 subjects, and most preferably of at least 15 to 20 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with a (at least one) known clinical condition, and
(ii) computing an algorithm or a mathematical function based on said reference expression profile that is suitable to decide if the known clinical condition is present or will likely be present in said patient.

More preferably, the known clinical condition may be healthiness or a lung disease (e.g. sarcoidosis, lung cancer or COPD). Most preferably, the known clinical condition is selected from the group consisting of healthiness, sarcoidosis, lung cancer, and COPD.

It is particularly preferred that the machine learning approach involves the following steps:

(i) inputting the reference expression profile of at least one subject, preferably of at least 2 to 40 subjects, more preferably of at least 10 to 25 subjects, and most preferably of at least 15 to 20 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with at least one known clinical condition, and
(ii) computing an algorithm or a mathematical function based on said reference expression profile that is suitable to decide if the at least one known clinical condition is present or will likely be present in said patient.

More preferably, the at least one known clinical condition is healthiness or a lung disease (e.g. sarcoidosis, lung cancer or COPD). Most preferably, the at least one known clinical condition is selected from the group consisting of healthiness, sarcoidosis, lung cancer, and COPD.

It is also particularly preferred that the machine learning approach involves the following steps:

(i) inputting the reference expression profile of at least two subjects, preferably of at least 3 to 40 subjects, more preferably of at least 10 to 25 subjects, and most preferably of at least 15 to 20 subjects, i.e. of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with at least two known clinical conditions, preferably 2 to 4 (e.g. 2, 3, or 4) known clinical conditions, and
(ii) computing an algorithm or a mathematical function based on said reference expression profile that is suitable to decide if the at least two known clinical conditions are present or will likely be present in said patient.

Preferably, the at least two known clinical conditions are selected from the group consisting of healthiness, sarcoidosis, lung cancer, and COPD.

More preferably, the machine learning approach involves the following steps:

(i) inputting the reference expression profile(s) of

(ia) at least one subject with the known clinical condition of sarcoidosis,
(ib) at least one subject with the known clinical condition of healthiness,
(ic) at least one subject with the known clinical condition of lung cancer, and/or
(id) at least one subject with the known clinical condition of COPD, and

(ii) computing an algorithm or a mathematical function based on said reference expression profile(s) that is suitable to decide if the known clinical condition(s) selected from the group consisting of sarcoidosis, healthiness, lung cancer and COPD is (are) present or will likely be present in said patient.

For example, the machine learning approach involves the following steps:

(i) inputting the reference expression profile(s) of

(ia) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, healthiness, lung cancer, or COPD,

(ib) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, lung cancer, or COPD,

(ic) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer or COPD,

(id) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD,

(ie) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer,

(if) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD,

(ig) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD,

(ih) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD, or

(ij) at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of healthiness, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of sarcoidosis, at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of lung cancer, and at least one subject, preferably of at least 2 to 40 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with the known clinical condition of COPD, and
(ii) computing an algorithm or a mathematical function based on said reference expression profile(s) that is suitable to decide if the known clinical condition(s) selected from the group consisting of sarcoidosis, healthiness, lung cancer and COPD is (are) present or will likely be present in said patient.

It is, for example, possible that the known clinical condition sarcoidosis, healthiness, lung cancer or COPD is present or will likely be present in said patient. It is, for example, also possible that the known clinical conditions sarcoidosis and lung cancer, sarcoidosis and COPD, lung cancer and COPD, or sarcoidosis, lung cancer and COPD are present or will likely be present in said patient.

The application of the algorithm or mathematical function as mentioned above to the expression profile of the patient to be diagnosed and/or prognosed may then allow for diagnosing and/or prognosing of a lung disease such as sarcoidosis, lung cancer, and/or COPD. It may also allow (i) for differentially diagnosing sarcoidosis with respect to lung cancer and/or COPD, (ii) for differentially diagnosing lung cancer with respect to sarcoidosis and/or COPD, or (iii) for differentially diagnosing COPD with respect to sarcoidosis and/or lung cancer (see above and Figure 18). In this respect it should be noted that the differential diagnosis may lead to the result that no differentiation between the different diseases/conditions is possible as the patient tested also suffers from said disease/condition or said diseases/conditions. This applies, where a differential diagnosis is carried out. Thus, for example, (i) the differential diagnosis of sarcoidosis with respect to lung cancer and/or COPD may lead to the result that the patient also suffers from (ia) lung cancer, (ib) COPD, or (ic) lung cancer and COPD, (ii) the differential diagnosis of lung cancer with respect to sarcoidosis and/or COPD may lead to the result that the patient also suffers from (iia) sarcoidosis, (iib) COPD, or (iic) sarcoidosis and COPD, or (iii) the differential diagnosis of COPD with respect to sarcoidosis and/or lung cancer may lead to the result that the patient also suffers from (iiia) sarcoidosis, (iiib) lung cancer, or (iiic) sarcoidosis and lung cancer.

Considering the above, diagnosing preferably means applying the algorithm or mathematical function as mentioned above to the expression profile of a patient to decide, if the known clinical condition, which is a lung disease such as sarcoidosis, lung cancer or COPD (i.e. diseased condition), or which is no lung disease such as sarcoidosis, lung cancer or COPD (i.e. healthy condition), is present in said patient. More preferably, the known clinical condition is selected from the group consisting of healthiness, sarcoidosis, lung cancer and COPD. Prognosing preferably means applying the algorithm or mathematical function as mentioned above to the expression profile of a patient to decide, if the known clinical condition, which is a lung disease such as sarcoidosis, lung cancer or COPD (i.e. diseased condition), or which is no lung disease such as sarcoidosis, lung cancer or COPD (i.e. healthy condition), will likely be present in said patient. More preferably, the known clinical condition is selected from the group consisting of healthiness, sarcoidosis, lung cancer and COPD.

For example, the patient may be diagnosed as not suffering from a lung disease such as sarcoidosis, lung cancer or COPD (i.e. being healthy), or as suffering from a lung disease such as sarcoidosis, lung cancer or COPD (i.e. being diseased). Further, for example, the patient may be prognosed as not developing a lung disease such as sarcoidosis, lung cancer or COPD (i.e. staying healthy), or as developing a lung disease such as sarcoidosis, lung cancer or COPD (i.e. getting diseased).

Further, considering the above, diagnosing preferably means applying the algorithm or mathematical function as mentioned above to the expression profile of a patient to decide, if the at least one known clinical condition, which is a lung disease such as sarcoidosis, lung cancer or COPD (i.e. diseased condition), or which is no lung disease such as sarcoidosis, lung cancer or COPD (i.e. healthy condition), is present in said patient. More preferably, the at least one known clinical condition is selected from the group consisting of healthiness, sarcoidosis, lung cancer and COPD. Prognosing preferably means applying the algorithm or mathematical function as mentioned above to the expression profile of a patient to decide, if the at least one known clinical condition, which is a lung disease such as sarcoidosis, lung cancer or COPD (i.e. diseased condition), or which is no lung disease such as sarcoidosis, lung cancer or COPD (i.e. healthy condition), will likely be present in said patient. More preferably, the at least one known clinical condition is selected from the group consisting of healthiness, sarcoidosis, lung cancer and COPD.

Furthermore, considering the above, diagnosing preferably means applying the algorithm or mathematical function as mentioned above to the expression profile of a patient to decide/differentiate which of the at least two known clinical conditions (e.g. 2, 3, or 4 known clinical conditions) selected from the group consisting of healthiness, sarcoidosis, lung cancer and COPD are present in said patient. Prognosing preferably means applying the algorithm or mathematical function as mentioned above to the expression profile of a patient to decide/differentiate which of the at least two known clinical conditions (e.g. 2, 3, or 4 known clinical conditions) selected from the group consisting of healthiness, sarcoidosis, lung cancer and COPD will likely be present in said patient.

For example, if the at least two known clinical conditions are sarcoidosis, lung cancer or COPD (i.e. diseased condition) and no lung disease such as sarcoidosis, lung cancer or COPD (i.e. healthy condition), the patient may be diagnosed as suffering from a lung disease such as sarcoidosis, lung cancer or COPD (i.e. being diseased), or as not suffering from a lung disease such as sarcoidosis, lung cancer or COPD (i.e. being healthy). If the at least two known clinical conditions are a lung disease such as sarcoidosis, lung cancer or COPD (i.e. diseased condition) and no lung disease such as sarcoidosis, lung cancer or COPD (i.e. healthy condition), the patient may be prognosed as developing a lung disease such as sarcoidosis, lung cancer or COPD (i.e. getting diseased), or as not developing a lung disease such as sarcoidosis, lung cancer or COPD (i.e. staying healthy).

Diagnosing/prognosing of a lung disease such as sarcoidosis, lung cancer or COPD based on an algorithm or a mathematical function may take place as follows: For instance, if the algorithm or mathematical function, which is obtained from a reference expression profile of at least one subject with the known clinical condition of a lung disease such as sarcoidosis, lung cancer or COPD, is applied to the expression profile (data) of a patient, the patient is classified as suffering from a lung disease such as sarcoidosis, lung cancer or COPD (i.e. being diseased), if the resulting score is below a specified threshold, or if the algorithm or mathematical function, which is obtained from a reference expression profile of at least one subject with the known clinical condition of no lung disease such as sarcoidosis, lung cancer or COPD, is applied to the expression profile (data) of the patient, the patient is classified as not suffering from a lung disease such as sarcoidosis, lung cancer or COPD (i.e. being healthy), if the resulting score is above a specified threshold.

Further, if the algorithm or mathematical function, which is obtained from a reference expression profile of at least one subject with the known clinical condition of a lung disease such as sarcoidosis, lung cancer or COPD and from a reference expression profile of at least one subject with the known clinical condition of no lung disease such as sarcoidosis, lung cancer or COPD, is applied to the expression profile (data) of a patient, the patient is classified as suffering from a lung disease such as sarcoidosis, lung cancer or COPD (i.e. being diseased), if the resulting score is below a specified threshold, and the patient is classified as not suffering from a lung disease such as sarcoidosis, lung cancer or COPD (i.e. being healthy), if the resulting score is above a specified threshold.

Preferably, machine learning approaches are employed to develop/obtain algorithms or mathematical functions for diagnosing and/or prognosing of clinical conditions such as lung diseases, e.g. sarcoidosis, lung cancer, and/or COPD. Support vector machines (SVMs) are a set of related supervised learning methods which are preferably used for classification and regression. For example, given a set of training examples (e.g. reference expression profile(s) of (a) subject(s) with the known clinical condition(s) of a lung disease such as sarcoidosis, lung cancer or COPD, and/or no lung disease such as sarcoidosis, lung cancer or COPD), each marked as belonging to one of one category (e.g. condition of a lung disease such as sarcoidosis, lung cancer or COPD, or no lung disease such as sarcoidosis, lung cancer or COPD) or to one of two categories (e.g. condition of a lung disease such as sarcoidosis, lung cancer or COPD, and no lung disease such as sarcoidosis, lung cancer o COPD), an SVM algorithm builds a model that predicts whether a new example (e.g. sample from a patient to be tested) falls into one category or the other (e.g. condition of a lung disease such as sarcoidosis, lung cancer or COPD, or no lung disease such as sarcoidosis, lung cancer or COPD). A SVM model is a representation of the training examples (e.g. reference expression profile(s) of (a) subject(s) with the known clinical condition of a lung disease such as sarcoidosis, lung cancer or COPD, and/or no lung disease such as sarcoidosis, lung cancer or COPD) as points in space, mapped so that the training examples of the separate categories (e.g. condition of a lung disease such as sarcoidosis, lung cancer or COPD, or no lung disease such as sarcoidosis, lung cancer or COPD) are divided by a clear gap that is as wide as possible. New examples (e.g. sample from a patient to be tested) are then mapped into that same space and predicted to belong to a category based on which side of the gap they fall on (e.g. lung disease such as sarcoidosis, lung cancer or COPD, or no lung disease such as sarcoidosis, lung cancer or COPD). More formally, a support vector machine constructs a hyperplane or set of hyperplanes in a high or infinite dimensional space, which can be used for classification, regression or other tasks. A good separation is achieved by the hyperplane that has the largest distance to the nearest training data points of any class (so-called functional margin), since in general the larger the margin the lower the generalization error of the classifier.

Classifying data is a preferred task in machine learning. For example, considering some given data points each belong to one (e.g. a lung disease such as sarcoidosis, lung cancer or COPD, or no lung disease such as sarcoidosis, lung cancer or COPD) of two classes (e.g. a lung disease such as sarcoidosis, lung cancer or COPD, and no lung disease such as sarcoidosis, lung cancer or COPD), the goal is to decide which class a new data point (e.g. achieved from a patient) will be in. In the preferred case of support vector machines, a data point is viewed as a p-dimensional vector (a list of p numbers), and the question is, whether it is possible to separate such points with a p-1-dimensional hyperplane. This is called a linear classifier. There are many hyperplanes that might classify the data. One reasonable choice as the best hyperplane is the one that represents the largest separation, or margin, between the two classes (e.g. condition of a lung disease such as sarcoidosis, lung cancer or COPD, or no lung disease such as sarcoidosis, lung cancer or COPD). Thus, the hyperplane should be chosen so that the distance from it to the nearest data point on each side is maximized. If such a hyperplane exists, it is known as the maximum-margin hyperplane and the linear classifier it defines is known as a maximum margin classifier.

The formalization of the support vector machine is exemplary summarised below. For example, given are training data $\mathcal{D}$ (e.g. reference expression profile(s) of (a) subject(s) with the known clinical condition of a lung disease such as sarcoidosis, lung cancer or COPD, and/or no lung disease such as sarcoidosis, lung cancer or COPD), a set of *n* subject samples (e.g. subjects known to suffer from a lung disease such as sarcoidosis, lung cancer or COPD, and/or no lung disease such as sarcoidosis, lung cancer or COPD) of the form

$$\mathcal{D} = \{(\mathbf{x}_i, c_i) \mid \mathbf{x}_i \in \mathbb{R}^p,\, c_i \in \{-1, 1\}\}_{i=1}^{n}$$

where the $c_i$ is either 1 or -1, indicating the class labels (e.g. a lung disease such as sarcoidosis, lung cancer or COPD, and/or no lung disease such as sarcoidosis, lung cancer or COPD) to which the miRNA biomarker intensities $\mathbf{X}_i$ belongs. Each $\mathbf{X}_i$ is a *p*-dimensional real vector (with p being the number of miRNA biomarkers). The task is know to find the maximum-margin hyperplane that divides the points having $c_i$ = 1 from those having $c_i$ = - 1. Any hyperplane can be written as the set of points **X** satisfying

$$\mathbf{w} \cdot \mathbf{x} - b = 0,$$

where "." denotes the dot product. The vector **W** is a normal vector: it is perpendicular to the hyperplane. The parameter $\frac{b}{\|\mathbf{w}\|}$ determines the offset of the hyperplane from the origin along the normal vector **W**.

**W** and *b* should be chosen to maximize the margin, or distance between the parallel hyperplanes that are as far apart as possible while still separating the data. These hyperplanes can be described by the equations

$$\mathbf{w} \cdot \mathbf{x} - b = 1$$

and

$$\mathbf{w} \cdot \mathbf{x} - b = -1.$$

Note that if the training data are linearly separable, the two hyperplanes of the margin can be selected in a way that there are no points between them and then by trying to maximize their distance. For example, by using geometry, the distance between these two hyperplanes is $\frac{2}{\|\mathbf{w}\|}$ so ||**W**|| should be minimized. To also prevent data points falling into the margin, the following constraint should be added: for each *i* either

$$\mathbf{w} \cdot \mathbf{x}_i - b \geq 1 \qquad \text{for } \mathbf{x}_i \text{ of the first class (e.g. a lung disease)}$$

or

$$\mathbf{w} \cdot \mathbf{x}_i - b \leq -1 \qquad \text{for } \mathbf{x}_i \text{ of the second class (no lung disease).}$$

This can be rewritten as:

$$c_i(\mathbf{w} \cdot \mathbf{x}_i - b) \geq 1, \quad \text{for all } 1 \leq i \leq n. \qquad (1)$$

This can be put together to get the optimization problem:

Minimize (in **w**, *b*)

$$\|\mathbf{w}\|$$

subject to (for any $i$ = 1, ..., $n$)

$$c_i(\mathbf{w}\cdot\mathbf{x_i}-b)\geq 1.$$

The optimization problem presented in the preceding section is difficult to solve because it depends on ||**w**||, the norm of **w**, which involves a square root. It is, however, possible to alter the equation by substituting ||**w**|| with $\frac{1}{2}\|\mathbf{w}\|^2$ (the factor of 1/2 being used for mathematical convenience) without changing the solution (the minimum of the original and the modified equation have the same **w** and b). This is a quadratic programming (QP) optimization problem. More clearly:

Minimize (in **w**, $b$)

$$\frac{1}{2}\|\mathbf{w}\|^2$$

subject to (for any $i$ = 1,...,$n$)

$$c_i(\mathbf{w}\cdot\mathbf{x_i}-b)\geq 1.$$

The previous problem may be expressed by means of non-negative Lagrange multipliers $\alpha_i$ as

$$\min_{\mathbf{w},b,\boldsymbol{\alpha}}\{\frac{1}{2}\|\mathbf{w}\|^2-\sum_{i=1}^{n}\alpha_i[c_i(\mathbf{w}\cdot\mathbf{x_i}-b)-1]\}$$

but this would led to an incorrect result. The reason is the following: suppose that a family of hyperplanes which divide the points can be found; then all $c_i$ (**w** · $\mathbf{x_i}$ - $b$) - 1 ≥ 0. Hence the minimum by sending all $\alpha_i$ to +∞ could be found, and this minimum would be reached for all the members of the family, not only for the best one which can be chosen solving the original problem.

Nevertheless the previous constrained problem can be expressed as

$$\min_{\mathbf{w},b}\max_{\boldsymbol{\alpha}}\{\frac{1}{2}\|\mathbf{w}\|^2-\sum_{i=1}^{n}\alpha_i[c_i(\mathbf{w}\cdot\mathbf{x_i}-b)-1]\}$$

looking for a saddle point. In doing so all the points which can be separated as $c_i$(**w** · $\mathbf{x_i}$ - $b$) - 1 > 0 do not matter since the corresponding $\alpha_i$ must be set to zero.

This problem can be solved by standard quadratic programming techniques and programs. The solution can be expressed by terms of linear combination of the training vectors as

$$\mathbf{w}=\sum_{i=1}^{n}\alpha_i c_i\mathbf{x_i}$$

Only a few $\alpha_i$ will be greater than zero. The corresponding $\mathbf{x_i}$ are exactly the *support vectors*, which lie on the margin and satisfy $c_i$(**w** · $\mathbf{x_i}$ - $b$) = **1**. From this it can be derived that the support vectors also satisfy

$$\mathbf{w} \cdot \mathbf{x_i} - b = 1/c_i = c_i \iff b = \mathbf{w} \cdot \mathbf{x_i} - c_i$$

which allows one to define the offset *b*. In practice, it is more robust to average over all $N_{SV}$ support vectors:

$$b = \frac{1}{N_{SV}} \sum_{i=1}^{N_{SV}} (\mathbf{w} \cdot \mathbf{x_i} - c_i)$$

Writing the classification rule in its unconstrained dual form reveals that the maximum margin hyperplane and therefore the classification task is only a function of the *support vectors,* the training data that lie on the margin.

Using the fact, that $||\mathbf{w}||^2 = w \cdot w$ and substituting $\mathbf{w} = \sum_{i=0}^{n} \alpha_i c_i \mathbf{x_i}$, it can show shown that the dual of the SVM reduces to the following optimization problem:

Maximize (in $\alpha_i$)

$$\tilde{L}(\alpha) = \sum_{i=1}^{n} \alpha_i - \frac{1}{2} \sum_{i,j} \alpha_i \alpha_j c_i c_j \mathbf{x}_i^T \mathbf{x}_j = \sum_{i=1}^{n} \alpha_i - \frac{1}{2} \sum_{i,j} \alpha_i \alpha_j c_i c_j k(\mathbf{x}_i, \mathbf{x}_j)$$

subject to (for any $i = 1,...,n$)

$$\alpha_i \geq 0,$$

and to the constraint from the minimization in *b*

$$\sum_{i=1}^{n} \alpha_i c_i = 0.$$

Here the kernel is defined by $k(x_i, x_j) = x_i \cdot x_j$.

The $\alpha$ terms constitute a dual representation for the weight vector in terms of the training set:

$$\mathbf{w} = \sum_i \alpha_i c_i \mathbf{x}_i.$$

For simplicity reasons, sometimes it is required that the hyperplane passes through the origin of the coordinate system. Such hyperplanes are called *unbiased*, whereas general hyperplanes not necessarily passing through the origin are called *biased.* An unbiased hyperplane can be enforced by setting $b = 0$ in the primal optimization problem. The corresponding dual is identical to the dual given above without the equality constraint

$$\sum_{i=1}^{n} \alpha_i c_i = 0.$$

Transductive support vector machines extend SVMs in that they could also treat partially labeled data in semi-supervised learning. Here, in addition to the training set $\mathcal{D}$, the learner is also given a set

$$\mathcal{D}^\star = \{\mathbf{x}_i^\star | \mathbf{x}_i^\star \in \mathbb{R}^p\}_{i=1}^k$$

of test examples to be classified. Formally, a transductive support vector machine is defined by the following primal optimization problem:

Minimize (in **w**, *b*, c*)

$$\frac{1}{2}\|\mathbf{w}\|^2$$

subject to (for any *i* = 1,...,*n* and any *j* = 1,...,*k*)

$$c_i(\mathbf{w} \cdot \mathbf{x_i} - b) \geq 1,$$
$$c_j^\star(\mathbf{w} \cdot \mathbf{x_j^\star} - b) \geq 1,$$

and

$$c_j^\star \in \{-1, 1\}.$$

Transductive support vector machines were introduced by Vladimir Vapnik in 1998.

SVMs belong to a family of generalized linear classifiers. They can also be considered a special case of Tikhonov regularization. A special property is that they simultaneously minimize the empirical *classification error* and maximize the *geometric margin*; hence they are also known as maximum margin classifiers.

In 1995, Corinna Cortes and Vladimir Vapnik suggested a modified maximum margin idea that allows for mislabeled examples. If there exists no hyperplane that can split the "yes" and "no" examples, the *Soft Margin* method will choose a hyperplane that splits the examples as cleanly as possible, while still maximizing the distance to the nearest cleanly split examples. The method introduces slack variables, $\xi_i$, which measure the degree of misclassification of the datum $x_i$

$$c_i(\mathbf{w} \cdot \mathbf{x_i} - b) \geq 1 - \xi_i \quad 1 \leq i \leq n. \qquad (2)$$

The objective function is then increased by a function which penalizes non-zero $\xi_i$, and the optimization becomes a trade off between a large margin, and a small error penalty. If the penalty function is linear, the optimization problem becomes:

$$\min_{\mathbf{w},\xi} \left\{ \frac{1}{2}\|\mathbf{w}\|^2 + C\sum_{i=1}^{n} \xi_i \right\}$$

subject to (for any *i* = 1,...*n*)

$$c_i(\mathbf{w} \cdot \mathbf{x_i} - b) \geq 1 - \xi_i, \quad \xi_i \geq 0.$$

This constraint in (2) along with the objective of minimizing ||**w**|| can be solved using Lagrange multipliers as done above. One has then to solve the following problem

$$\min_{\mathbf{w},\xi,b}\max_{\boldsymbol{\alpha},\boldsymbol{\beta}}\left\{\frac{1}{2}\|\mathbf{w}\|^2+C\sum_{i=1}^{n}\xi_i-\sum_{i=1}^{n}\alpha_i[c_i(\mathbf{w}\cdot\mathbf{x_i}-b)-1+\xi_i]-\sum_{i=1}^{n}\beta_i\xi_i\right\}$$

with $\alpha_i,\beta_i \geq 0$.

The key advantage of a linear penalty function is that the slack variables vanish from the dual problem, with the constant C appearing only as an additional constraint on the Lagrange multipliers. For the above formulation and its huge impact in practice, Cortes and Vapnik received the 2008 ACM Paris Kanellakis Award. Non-linear penalty functions have been used, particularly to reduce the effect of outliers on the classifier, but unless care is taken, the problem becomes non-convex, and thus it is considerably more difficult to find a global solution.

The original optimal hyperplane algorithm proposed by Vladimir Vapnik in 1963 was a linear classifier. However, in 1992, Bernhard Boser, Isabelle Guyon and Vapnik suggested a way to create non-linear classifiers by applying the kernel trick (originally proposed by Aizerman et al.[4]) to maximum-margin hyperplanes. The resulting algorithm is formally similar, except that every dot product is replaced by a non-linear kernel function. This allows the algorithm to fit the maximum-margin hyperplane in a transformed feature space. The transformation may be non-linear and the transformed space high dimensional; thus though the classifier is a hyperplane in the high-dimensional feature space, it may be non-linear in the original input space.

If the kernel used is a Gaussian radial basis function, the corresponding feature space is a Hilbert space of infinite dimension. Maximum margin classifiers are well regularized, so the infinite dimension does not spoil the results. Some common kernels include,

- Polynomial (homogeneous): $k(\mathbf{x_i}, \mathbf{x_j}) = (\mathbf{x_i} \cdot \mathbf{x_j})^d$
- Polynomial (inhomogeneous): $k(\mathbf{x_i}, \mathbf{x_j}) = (\mathbf{x_i} \cdot \mathbf{x_j} + \mathbf{1})^d$
- Radial Basis Function: $k(\mathbf{x_i}, \mathbf{x_j}) = \exp(-\gamma\|\mathbf{x_i} - \mathbf{x_j}\|^2)$, for $\gamma > 0$
- Gaussian Radial basis function: $k(\mathbf{x_i}, \mathbf{x_j}) = \exp\left(-\frac{\|\mathbf{x_i} - \mathbf{x_j}\|^2}{2\sigma^2}\right)$
- Hyperbolic tangent: $\boldsymbol{k}(\mathbf{x_i}, \mathbf{x_j}) = \mathbf{tanh}(\kappa\mathbf{x_i} \cdot \mathbf{x_j} + \boldsymbol{c})$, for some (not every) $\kappa > 0$ and $c < 0$

The kernel is related to the transform $\varphi(\mathbf{x_i})$ by the equation $\boldsymbol{k}(\mathbf{x_i}, \mathbf{x_j}) = \varphi(\mathbf{x_i}) \cdot \varphi(\mathbf{x_j})$. The value **w** is also in the transformed space, with $\mathbf{w} = \sum_i \alpha_i \boldsymbol{c_i} \varphi(\mathbf{x_i})$ ·Dot products with **w** for classification can again be computed by the kernel trick, i.e. $\mathbf{w} \cdot \varphi(x) = \sum_i \alpha_i \boldsymbol{c_i} \boldsymbol{k}(\mathbf{x_i}, \mathbf{x})$.

However, there does not in general exist a value **w'** such that $\mathbf{w} \cdot \varphi(\mathbf{x}) = \boldsymbol{k}(\mathbf{w'}, \mathbf{x})$.

After the **w** and *b* are determined employing the above mentioned methods these can be used for classifying new datasets (e.g. a expression profile of a patient with *p* miRNA biomarkers and corresponding $\mathbf{x_i}$ intensity values).

As mentioned above, the method described herein is for diagnosing a lung disease (e.g. sarcoidosis, lung cancer and/or COPD) in a patient (e.g. human or animal). Preferably, the diagnosis comprises (i) determining the occurrence/presence of a lung disease (e.g. sarcoidosis, lung cancer and/or COPD), (ii) monitoring the course of a lung disease (e.g. sarcoidosis, lung cancer and/or COPD), (iii) staging of a lung disease (e.g. sarcoidosis, lung cancer and/or COPD), (iv) measuring the response of a patient with a lung disease (e.g. sarcoidosis, lung cancer and/or COPD) to therapeutic intervention, and/or (v) segmentation of a patient suffering from a lung disease (e.g. sarcoidosis, lung cancer and/or COPD). Further, the method described herein is for prognosis of a lung disease (e.g. sarcoidosis, lung cancer and/or COPD) in a patient (e.g. human or animal). Preferably, the prognosis comprises (i) identifying of a patient who has a risk to develop a lung disease (e.g. sarcoidosis, lung cancer and/or COPD), (ii) predicting/estimating the occurrence, preferably the severity of occurrence, of a lung disease (e.g. sarcoidosis, lung cancer and/or COPD), and/or (iii) predicting the response of a patient with a lung disease (e.g. sarcoidosis, lung cancer and/or COPD) to therapeutic intervention. In the context of the present invention, the method is for diagnosing sarcoidosis in a patient.

Like highlighted above, the reference miRNA expression profiles may be classified using machine learning approaches in order to compute accuracy, specificity, and sensitivity for the diagnosis and/or prognosis of a lung disease (e.g. sarcoidosis, lung cancer and/or COPD) (see experimental section for more details). Examples of miRNA sets (signatures) that performed best for the diagnosis of a lung disease (e.g. sarcoidosis, lung cancer and/or COPD) according to their accuracy, specificity, and sensitivity are sets of miRNAs shown in **Figure 15, Figure 16** and **Figure 17.** Further examples of miRNAs sets (signatures) that performed best for the diagnosis of a lung disease (e.g. sarcoidosis, lung cancer and/or COPD) according to their accuracy, specificity, and sensitivity are sets of miRNAs shown in **Figures 4 to 12.**

Preferably, the reference (e.g. (average) reference expression profile (data)) and/or the algorithm or mathematical function is (are) stored in a database, e.g. an internet database, a centralized, or a decentralized database. It is also preferred that the reference (e.g. (average) reference expression profile (data)) and/or the algorithm or mathematical function is (are) stored on a data carrier, e.g. electronically data carrier. It is further preferred that the reference (e.g. (average) reference expression profile (data)) and/or the algorithm or mathematical function is (are) comprised in a computer program stored on an electronically data carrier.

Described herein but not encompassed by the present invention are means for diagnosing and/or prognosing a lung disease, preferably sarcoidosis, lung cancer, and/or chronic obstructive pulmonary disease (COPD), comprising one or more polynucleotide(s) (probe(s)) described herein. It is particularly preferred that the above mentioned means are (i) for differential diagnosis of sarcoidosis with respect to lung cancer and/or COPD, (ii) for differential diagnosis of lung cancer with respect to sarcoidosis and/or COPD, or (iii) for differential diagnosis of COPD with respect to sarcoidosis and/or lung cancer and comprise one or more polynucleotide(s) (probe(s)) described herein (see above). More preferably, the means for diagnosing and/or prognosing a lung disease, preferably sarcoidosis, lung cancer, and/or chronic obstructive pulmonary disease (COPD), comprise, essentially consist of, or consist of a solid support, substrate, surface, platform or matrix comprising one or more polynucleotide(s) (probe(s)) described herein. It is particularly preferred that the above mentioned means are (i) for differential diagnosis of sarcoidosis with respect to lung cancer and/or COPD, (ii) for differential diagnosis of lung cancer with respect to sarcoidosis and/or COPD, or (iii) for differential diagnosis of COPD with respect to sarcoidosis and/or lung cancer and comprise one or more polynucleotide(s) (probe(s)) described herein.

In a third aspect, the present invention relates to the use of means for diagnosing sarcoidosis, wherein said means comprise a solid support, substrate, surface, platform or matrix and polynucleotides according to the first aspect of the present invention, wherein said polynucleotides are attached to the solid support, substrate, surface, platform or matrix. Most preferably, the means used for diagnosing sarcoidosis comprise, essentially consist of, or consist of a microarray/biochip comprising polynucleotides (probes) according to the first aspect of the present invention. It is particularly preferred that the above mentioned means are used (i) for differential diagnosis of sarcoidosis with respect to lung cancer and/or COPD and comprise polynucleotides (probes) according to the first aspect of the present invention.

The polynucleotides (probes) may also be comprised as polynucleotide fragments, polynucleotide variants, or polynucleotide fragment variants in the means for diagnosing sarcoidosis. For example, polynucleotide fragments, polynucleotide variants, or polynucleotide fragment variants may be comprised in the solid support, substrate, surface, platform or matrix, preferably microarray/biochip. Said polynucleotide fragments, polynucleotide variants, or polynucleotide fragment variants may be attached or linked to the solid support, substrate, surface, platform or matrix, preferably microarray/biochip. As to the definition of said polynucleotide fragments, polynucleotide variants, or polynucleotide fragment variants and as to the preferred polynucleotides (probes), it is referred to the first aspect of the present invention.

The terms "biochip" or "microarray", as used herein, refer to a solid phase comprising an attached or immobilized polynucleotide described herein as probe or a set (plurality) of polynucleotides described herein attached or immobilized as probes. The polynucleotide probes may be capable of hybridizing to a target sequence, such as a complementary miRNA or miRNA* sequence, under stringent hybridization conditions. The polynucleotide probes may be attached or immobilized at spatially defined locations on the solid phase. One or more than one nucleotide (probe) per target sequence may be used. The polynucleotide probes may either be synthesized first, with subsequent attachment to the biochip, or may be directly synthesized on the biochip. The solid phase may be a material that may be modified to contain discrete individual sites appropriate for the attachment or association of the polynucleotide probes and is amenable to at least one detection method. Representative examples of solid phase materials include glass and modified or functionalized glass, plastics (including acrylics, polystyrene and copolymers of styrene and other materials, polypropylene, polyethylene, polybutylene, polyurethanes, Teflon, etc.), polysaccharides, nylon or nitrocellulose, resins, silica or silica-based materials including silicon and modified silicon, carbon, metals, inorganic glasses and plastics. The solid phase may allow optical detection without appreciably fluorescing. The solid phase may be planar, although other configurations of solid phase may be used as well. For example, polynucleotide probes may be placed on the inside surface of a tube, for flow- through sample analysis to minimize sample volume. Similarly, the solid phase may be flexible, such as flexible foam, including closed cell foams made of particular plastics. The solid phase of the biochip and the probe may be modified with chemical functional groups for subsequent attachment of the two. For example, the biochip may be modified with a chemical functional group including, but not limited to, amino groups, carboxyl groups, oxo groups or thiol groups. Using these functional groups, the probes may be attached using functional groups on the probes either directly or indirectly using a linker. The polynucleotide probes may be attached to the solid support by either the 5' terminus, 3' terminus, or via an internal nucleotide. The polynucleotide probe may also be attached to the solid support non-covalently. For example, biotinylated polynucleotides can be made, which may bind to surfaces covalently coated with streptavidin, resulting in attachment. Alternatively, polynucleotide probes may be synthesized on the surface using techniques such as photopolymerization and photolithography. In the context of the present invention, the terms "biochip" and "microarray" are interchangeable used.

The terms "attached" or "immobilized", as used herein, refer to the binding between the polynucleotide and the solid support/phase and may mean that the binding between the polynucleotide probe and the solid support is sufficient to be stable under conditions of binding, washing, analysis and removal. The binding may be covalent or non-covalent. Covalent bonds may be formed directly between the polynucleotide and the solid support or may be formed by a cross linker or by inclusion of specific reactive groups on either the solid support or the polynucleotide, or both. Non-covalent binding may be electrostatic, hydrophilic and hydrophobic interactions or combinations thereof. Immobilization or attachment may also involve a combination of covalent and non-covalent interactions.

Said means for diagnosing and/or prognosing a lung disease, preferably sarcoidosis, lung cancer, and/or chronic obstructive pulmonary disease (COPD), may comprise, essentially consist of, or consist of a set of beads or a set of microspheres comprising one or more polynucleotide(s) (probe(s)) described herein. It is particularly preferred that the above mentioned means are (i) for differential diagnosis of sarcoidosis with respect to lung cancer and/or COPD, (ii) for differential diagnosis of lung cancer with respect to sarcoidosis and/or COPD, or (iii) for differential diagnosis of COPD with respect to sarcoidosis and/or lung cancer and comprise one or more polynucleotide(s) (probe(s)) described herein.

Preferably, the above mentioned polynucleotide(s) is (are) attached to or immobilized on the beads or microspheres, e.g. via a covalent or non-covalent linkage (see above). Preferably, said beads or microspheres are made of a synthetic material, e.g. polystyrene, polyethylene or polypropylene. It is preferred that said beads or microspheres have a mean diameter of between 2 to 20 microns, preferably 4 to 10 microns, most preferably 5 to 7 microns, i.e. 2, 3, 4, 5, 6, 7, 8, 9, 10, 11, 12, 13, 14, 15, 16, 17, 18, 19, or 20 microns. It is also preferred that said beads or microspheres are internally dyed, preferably with red and infrared fluorophores. For example, the bead or microsphere setting from Luminex may be used.

Further described herein but not encompassed by the present invention is a kit for diagnosing and/or prognosing a lung disease, preferably sarcoidosis, lung cancer, and/or chronic obstructive pulmonary disease (COPD), comprising

(i) means for determining an expression profile of one or more miRNA(s) in a biological sample from a patient, wherein the nucleotide sequence of said miRNA or the nucleotide sequences of said miRNAs is (are) selected from the group consisting of SEQ ID NOs: 1 to 391, a fragment thereof, and a sequence having at least 80%, preferably at least 85%, more preferably at least 90%, and most preferably at least 95% or 99%, i.e. 80, 81, 82, 83, 84, 85, 86, 87, 88, 89, 90, 91, 92, 93, 94, 95, 96, 97, 98, or 99%, sequence identity thereto, and

(ii) optionally at least one reference and/or algorithm or mathematical function comprised on at least one data carrier.

Preferably, means for determining an expression profile of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, or 391 miRNAs in a biological sample (e.g. body fluid sample such as blood or urine sample or tissue sample) from a patient (e.g. human or animal) are comprised in the kit described herein, wherein the nucleotide sequence of said miRNA or the nucleotide sequences of said miRNAs is (are) selected from the group consisting of SEQ ID NOs: 1 to 391, a fragment thereof, and a sequence having at least 80% sequence identity thereto.

Said means may be one or more polynucleotide(s) (probe(s)) described herein (see above); means used according to the third aspect of the present invention (see above); primer suitable to perform reverse transcriptase reaction and/or real time polymerase chain reaction such as quantitative polymerase chain reaction; and/or means for conducting next generation sequencing.

It is preferred that said kit comprises means used according to the third aspect of the present invention (see above). Thus, said kit preferably comprises means used for diagnosing sarcoidosis comprising polynucleotides (probes) according to the first aspect of the present invention, or more preferably comprises means used for diagnosing sarcoidosis, wherein said means comprise a microarray/biochip comprising polynucleotides (probes) according to the first aspect of the present invention.

It is particularly preferred that said kit comprises

(ia) one or more polynucleotide(s) (probe(s)) described herein, and
(ib) optionally means to extract total RNA (or fractions thereof, e.g. miRNA) from a biological sample, e.g. blood or tissue sample, and/or means to immobilize the polynucleotide(s) onto a solid support or matrix, e.g. biochip,

for determining an expression profile of one or more miRNA(s) in a biological sample (e.g. blood or tissue sample) from a patient (e.g. human or animal), wherein the nucleotide sequence of said miRNA or the nucleotide sequences of said miRNAs is (are) selected from the group consisting of SEQ ID NOs: 1 to 391, a fragment thereof, and a sequence having at least 80% sequence identity thereto.

It is more preferred that said kit comprises

(ia) a solid support, substrate, surface, platform or matrix comprising one or more polynucleotide(s) (probe(s)) described herein, and
(ib) optionally at least one of the means selected from the group consisting of: means to extract total RNA (or fractions thereof, e.g. miRNA) from a biological sample (e.g. blood or tissue sample), means for input/injection of a biological sample (e.g. blood sample), means for holding the solid support, substrate, platform or matrix comprising the polynucleotide(s) (probe(s)), means for labelling the isolated miRNA (e.g. NTP/biotin-NTP), means to carry out hybridization, means to carry out enzymatic reactions (e.g. exonuclease I and/or Klenow enzyme), means for washing steps, means for detecting the hybridization signal, and means for analysing the detected hybridization signal,

for determining an expression profile of one or more miRNA(s) in a biological sample (e.g. blood or tissue sample) from a patient (e.g. human or animal), wherein the nucleotide sequence of said miRNA or the nucleotide sequences of said miRNAs is (are) selected from the group consisting of SEQ ID NOs: 1 to 391, a fragment thereof, and a sequence having at least 80% sequence identity thereto.

Preferably, the above mentioned polynucleotide(s) is (are) attached to or immobilized on the solid support, substrate, surface, platform or matrix.

It is most preferred that said kit comprises

(ia) a microarray/biochip comprising one or more polynucleotide(s) (probe(s)) described herein, and
(ib) optionally at least one of the means selected from the group consisting of: means to extract total RNA (or fractions thereof, e.g. miRNA) from a biological sample (e.g. blood or tissue sample), means for input/injection of a biological sample (e.g. blood sample), means for holding the microarray/biochip comprising the polynucleotide(s) (probe(s)), means for labelling the isolated miRNA (e.g. NTP/biotin-NTP), means to carry out hybridization, means to carry out enzymatic reactions (e.g. exonuclease I and/or Klenow enzyme), means for washing steps, means for detecting the hybridization signal, and means for analysing the detected hybridization signal,

for determining an expression profile of one or more miRNA(s) in a biological sample (e.g. blood or tissue sample) from a patient (e.g. human or animal), wherein the nucleotide sequence of said miRNA or the nucleotide sequences of said miRNAs is (are) selected from the group consisting of SEQ ID NOs: 1 to 391, a fragment thereof, and a sequence having at least 80% sequence identity thereto.

Preferably, the above mentioned polynucleotide(s) is (are) attached to or immobilized on a microarray/biochip.

It is also most preferred that said kit comprises

(ia) a set of beads or a set of microspheres comprising one or more polynucleotide(s) (probe(s)) described herein, and
(ib) optionally at least one of the means selected from the group consisting of: means to extract total RNA (or fractions thereof, e.g. miRNA) from a biological sample (e.g. blood or tissue sample), means for input/injection of a biological sample (e.g. blood sample), means for labelling the isolated miRNA (e.g. NTP/biotin-NTP), means to carry out hybridization, means to carry out enzymatic reactions (e.g. exonuclease I and/or Klenow enzyme), means for washing steps, means for detecting the hybridization signal, and means for analysing the detected hybridization signal,
for determining an expression profile of one or more miRNA(s) in a biological sample (e.g. blood or tissue sample) from a patient (e.g. human or animal), wherein the nucleotide sequence of said miRNA or the nucleotide sequences of said miRNAs is (are) selected from the group consisting of SEQ ID NOs: 1 to 391, a fragment thereof, and a sequence having at least 80% sequence identity thereto.

Preferably, the above mentioned polynucleotide(s) is (are) attached to or immobilized on the beads or microspheres.

It is also preferred that said kit comprises means for conducting next generation sequencing in order to determine an expression profile of one or more miRNA(s) in a biological sample (e.g. blood or tissue sample) from a patient (e.g. human or animal), wherein the nucleotide sequence of said miRNA or the nucleotide sequences of said miRNAs is (are) selected from the group consisting of SEQ ID NOs: 1 to 391, a fragment thereof, and a sequence having at least 80% sequence identity thereto. Said kit optionally comprises means selected from the group consisting of: means to collect a biological sample (e.g. blood or tissue sample) from a patient (e.g. a human or an animal), means for conserving the RNA-fraction within the biological sample (e.g. blood or tissue sample), and means to extract total RNA (or fractions thereof, e.g. miRNA) from a biological sample (e.g. blood or tissue sample).

It is further preferred that said kit comprises means (e.g. a setting or settings) for conducting a real time polymerase chain reaction (RT-PCR), preferably a real time quantitative polymerase chain reaction (RT-qPCR), in order to determine an expression profile of one or more miRNA(s) in a biological sample (e.g. blood or tissue sample) from a patient (e.g. a human or an animal), wherein the nucleotide sequence of said miRNA or the nucleotide sequences of said miRNAs is (are) selected from the group consisting of SEQ ID NOs: 1 to 391, a fragment thereof, and a sequence having at least 80% sequence identity thereto. The setting for conduction a RT-PCR reaction, preferably a RT-qPCR reaction, may be combined with a setting for conducting a reverse transcription reaction, or may be two separate settings, meaning that both reactions, namely the reverse transcription reaction and RT-PCR reaction, preferably the RT-qPCR reaction, can be run together in one setting or separately in two settings.

As to the definition of the miRNA fragment, miRNA variant, or miRNA fragment variant mentioned above and as to the preferred miRNA(s) determined by the means of (i), it is referred to the first and second aspect of the present invention.

Preferably, the nucleotide sequence of said miRNA or the nucleotide sequences of said miRNAs determined in (i) is (are) selected from the group consisting of SEQ ID NOs: 1 to 231 (see lists 1, 2, 4 and 7 of **Figure 18**). It is preferred that the kit comprising means for the determination of an expression profile of the above mentioned miRNA(s) is for diagnosing and/or prognosing of sarcoidosis.

In this respect it is particularly preferred that the nucleotide sequence of said miRNA or the nucleotide sequences of said miRNAs determined in (i) is (are) selected from the group consisting of SEQ ID NOs: 34, 36, 39, 40, 50, 56, 57, 58, 61, 62, 66, 79, 80, 81, 83, 84, 85, 89, 90, 93, 94, 96, 98, 99, 100, 101, 102, 104, 113, 128, 129, 132, 133, 142, 143, 144, 148, 151, 155, 156, 163, 166, 169, 170, 171, 173, 179, 193, 195, 196, 199, 200, 201, 203, 206, 214, 216, 221, 223, 225, 226, and 229 (see list 1 of **Figure 18**). It is further particularly preferred that the nucleotide sequences of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 miRNAs determined in (i) are selected from the group consisting of SEQ ID NOs: 34, 36, 39, 40, 50, 56, 57, 58, 61, 62, 66, 79, 80, 81, 83, 84, 85, 89, 90, 93, 94, 96, 98, 99, 100, 101, 102, 104, 113, 128, 129, 132, 133, 142, 143, 144, 148, 151, 155, 156, 163, 166, 169, 170, 171, 173, 179, 193, 195, 196, 199, 200, 201, 203, 206, 214, 216, 221, 223, 225, 226, and 229 (see list 1 of **Figure 18**). Preferably, the kit comprising means for the determination of an expression profile of the above mentioned miRNA(s) is for diagnosing and/or prognosing of sarcoidosis.

More preferably, the nucleotide sequence of said miRNA or the nucleotide sequences of said miRNAs determined in (i) is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, 227 (see list 2 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231 (see list 7 of **Figure 18**). It is preferred that the kit comprising means for the determination of an expression profile of the above mentioned miRNA(s) is for differential diagnosis (of sarcoidosis) with respect to lung cancer and/or COPD.

Most preferably, the nucleotide sequence of said miRNA or the nucleotide sequences of said miRNAs determined in (i) is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, and 227 (see list 2 of **Figure 18**). It is preferred that the kit comprising means for the determination of an expression profile of the above mentioned miRNA(s) is for differential diagnosis (of sarcoidosis) with respect to lung cancer. Further, most preferably, the nucleotide sequence of said miRNA or the nucleotide sequences of said miRNAs determined in (i) is (are) selected from the group consisting of SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228 (see list 4 of **Figure 18**). It is preferred that the kit comprising means for the determination of an expression profile of the above mentioned miRNA(s) is for differential diagnosis (of sarcoidosis) with respect to lung cancer and COPD. Furthermore, most preferably, the nucleotide sequence of said miRNA or the nucleotide sequences of said miRNAs determined in (i) is (are) selected from the group consisting of SEQ ID NOs: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231 (see list 7 of **Figure 18**). It is preferred that the kit comprising means for the determination of an expression profile of the above mentioned miRNA(s) is for differential diagnosis (of sarcoidosis) with respect to COPD.

In preferred embodiments, the nucleotide sequences of said miRNAs are the nucleotide sequences according to Set No. 1 to 42 (i.e. SH-1 to SH-42) of **Figure 15**.

Preferably, the nucleotide sequence of said miRNA or the nucleotide sequences of said miRNAs determined in (i) is (are) selected from the group consisting of SEQ ID NOs: 233, 234, 235, 237, 240, 241, 242, 243, 245, 246, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, 329 (see list 3 of **Figure 18**), 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, 227 (see list 2 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**). It is preferred that the kit comprising means for the determination of an expression profile of the above mentioned miRNA(s) is for diagnosing and/or prognosing of lung cancer.

In this respect it is particularly preferred that the nucleotide sequence of said miRNA or the nucleotide sequences of said miRNAs determined in (i) is (are) selected from the group consisting of SEQ ID NOs: 233, 234, 235, 237, 240, 241, 242, 243, 245, 246, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, and 329 (see list 3 of **Figure 18**). It is further particularly preferred that the nucleotide sequences of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, or 60 miRNAs determined in (i) are selected from the group consisting of SEQ ID NOs: 233, 234, 235, 237, 240, 241, 242, 243, 245, 246, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, and 329 (see list 3 of **Figure 18**). Preferably, the kit comprising means for the determination of an expression profile of the above mentioned miRNA(s) is for diagnosing and/or prognosing of lung cancer.

More preferably, the nucleotide sequence of said miRNA or the nucleotide sequences of said miRNAs determined in (i) is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, 227 (see list 2 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**). It is preferred that the kit comprising means for the determination of an expression profile of the above mentioned miRNA(s) is for differential diagnosis (of lung cancer) with respect to sarcoidosis and/or COPD.

Most preferably, the nucleotide sequence of said miRNA or the nucleotide sequences of said miRNAs determined in (i) is (are) selected from the group consisting of SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, and 227 (see list 2 of **Figure 18**). It is preferred that the kit comprising means for the determination of an expression profile of the above mentioned miRNA(s) is for differential diagnosis (of lung cancer) with respect to sarcoidosis. Further, most preferably, the nucleotide sequence of said miRNA or the nucleotide sequences of said miRNAs determined in (i) is (are) selected from the group consisting of SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228 (see list 4 of **Figure 18**). It is preferred that the kit comprising means for the determination of an expression profile of the above mentioned miRNA(s) is for differential diagnosis (of lung cancer) with respect to sarcoidosis and COPD. Furthermore, most preferably, the nucleotide sequence of said miRNA or the nucleotide sequences of said miRNAs determined in (i) is (are) selected from the group consisting of SEQ ID NOs: 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**). It is preferred that the kit comprising means for the determination of an expression profile of the above mentioned miRNA(s) is for differential diagnosis (of lung cancer) with respect to COPD.

In preferred embodiments, the nucleotide sequences of said miRNAs are the nucleotide sequences according to Set No. 1 to 42 of **Figure 16**.

Preferably, the nucleotide sequence of said miRNA or the nucleotide sequences of said miRNAs determined in (i) is (are) selected from the group consisting of SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391 (see list 6 of **Figure 18**), 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, 231 (see list 7 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**). It is preferred that the kit comprising means for the determination of an expression profile of the above mentioned miRNA(s) is for diagnosing and/or prognosing of COPD.

In this respect it is particularly preferred that the nucleotide sequence of said miRNA or the nucleotide sequences of said miRNAs determined in (i) is (are) selected from the group consisting of SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, and 391 (see list 6 of **Figure 18**). It is further particularly preferred that the nucleotide sequences of 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, or 62 miRNAs determined in (i) are selected from the group consisting of SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, and 391 (see list 6 of **Figure 18**). Preferably, the kit comprising means for the determination of an expression profile of the above mentioned miRNA(s) is for diagnosing and/or prognosing of COPD.

More preferably, the nucleotide sequence of said miRNA or the nucleotide sequences of said miRNAs determined in (i) is (are) selected from the group consisting of SEQ ID NOs: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, 231 (see list 7 of **Figure 18**), 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, 228 (see list 4 of **Figure 18**), 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**). It is preferred that the kit comprising means for the determination of an expression profile of the above mentioned miRNA(s) is for differential diagnosis (of COPD) with respect to sarcoidosis and/or lung cancer.

Most preferably, the nucleotide sequence of said miRNA or the nucleotide sequences of said miRNAs determined in (i) is (are) selected from SEQ ID NOs: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231 (see list 7 of **Figure 18**). It is preferred that the kit comprising means for the determination of an expression profile of the above mentioned miRNA(s) is for differential diagnosis (of COPD) with respect to sarcoidosis. Further, most preferably, the nucleotide sequence of said miRNA or the nucleotide sequences of said miRNAs determined in (i) is (are) selected from SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228 (see list 4 of **Figure 18**). It is preferred that the kit comprising means for the determination of an expression profile of the above mentioned miRNA(s) is for differential diagnosis (of COPD) with respect to sarcoidosis and lung cancer. Furthermore, most preferably, the nucleotide sequence of said miRNA or the nucleotide sequences of said miRNAs determined in (i) is (are) selected from SEQ ID NOs: 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328 (see list 5 of **Figure 18**). It is preferred that the kit comprising means for the determination of an expression profile of the above mentioned miRNA(s) is for differential diagnosis (of COPD) with respect to lung cancer.

In preferred embodiments, the nucleotide sequences of said miRNAs are the nucleotide sequences according to Set No. 1 to 42 of **Figure 17**.

As mentioned above, the kit may further optionally comprise at least one reference and/or algorithm or mathematical function comprised on at least one data carrier (see item (ii)).

The at least one reference may be any reference which allows for the diagnosis and/or prognosis of a lung disease such as sarcoidosis, lung cancer and/or COPD, e.g. an indicated value or values, and/or the at least one algorithm or mathematical function may be any algorithm or mathematical function which allows for the diagnosis and/or prognosis of a lung disease such as sarcoidosis, lung cancer and/or COPD.

It is preferred that said reference is a reference expression profile (data) of at least one subject, preferably the reference is an average expression profile (data) of at least 2 to 40 subjects, more preferably at least 10 to 25 subjects, and most preferably at least 15 to 20 subjects, with a (at least one) known clinical condition, wherein the reference expression profile is the profile of a (one) miRNA that has a nucleotide sequence that essentially corresponds (is essentially identical), preferably that corresponds (is identical), to the nucleotide sequence of the miRNA which expression profile is determinable by the means of (i) or is the profile of (more than one) miRNAs that have nucleotide sequences that essentially correspond (are essentially identical), preferably that correspond (are identical), to the nucleotide sequences of the miRNAs which expression profile is determinable by the means of (i). More preferably, the known clinical condition is healthiness or a lung disease (e.g. sarcoidosis, lung cancer or COPD). Most preferably, the known clinical condition is selected from the group consisting of healthiness, sarcoidosis, lung cancer, and COPD.

The term "essentially corresponds (essentially identical)", as herein, means that the miRNA(s) which expression profile is determinable by the means of (i) and the miRNA(s) of the reference expression profile may slightly differ in their nucleotide sequence, whereas said difference is so marginal that it may still allow for the diagnosis and/or prognosis of a lung disease such as sarcoidosis, lung cancer and/or COPD. Preferably, the nucleotide sequence(s) of the miRNA(s) which expression profile is determinable by the means of (i) and the nucleotide sequence(s) of the miRNA(s) of the reference expression profile differ in 1 to 5, more preferably in 1 to 3, and most preferably in 1 to 2 nucleotides, i.e. in 1, 2, 3, 4, or 5 nucleotides. It is preferred that said difference resides in 1 to 5, more preferably 1 to 3, and most preferably 1 to 2 nucleotide mutations, i.e. 1, 2, 3, 4, or 5 nucleotide mutations (e.g. substitutions, additions, insertions, and/or deletions).

Preferably, it is indicated in which type of biological sample (e.g. tissue sample or body fluid sample such as blood and/or urine sample) and/or from which (control) subject(s) (e.g. species, gender and/or age or stage of life), the (average) reference expression profile (data), which is (are) provided with the kit, has (have) been determined. It is preferred that the expression profile (data) of the patient will be determined in the same type of biological sample (e.g. tissue sample or body fluid sample such as blood and/or urine sample) and/or will be obtained from a patient of the same species, gender and/or similar age or stage of life.

Preferably, the algorithm or mathematical function is obtained from a reference expression profile of at least one subject, preferably of at least 2 to 40 subjects, more preferably of at least 10 to 25 subjects, and most preferably of at least 15 to 20 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with a (at least one) known clinical condition, wherein the reference expression profile is the profile of a (one) miRNA that has a nucleotide sequence that essentially corresponds (is essentially identical), preferably that corresponds (is identical), to the nucleotide sequence of the miRNA which expression profile is determinable by the means of (i), or is the profile of (more than one) miRNAs that have nucleotide sequences that essentially correspond (are essentially identical), preferably that correspond (are identical), to the nucleotide sequences of the miRNAs which expression profile is determinable by the means of (i). More preferably, the known clinical condition is healthiness or a lung disease (e.g. sarcoidosis, lung cancer or COPD). Most preferably, the known clinical condition is selected from the group consisting of healthiness, sarcoidosis, lung cancer, and COPD.

It is particularly preferred that the algorithm or mathematical function is obtained from a reference expression profile of at least one subject, preferably of at least 2 to 40 subjects, more preferably of at least 10 to 25 subjects, and most preferably of at least 15 to 20 subjects, i.e. of at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with at least one known clinical condition, wherein the reference expression profile is the profile of a miRNA that has a nucleotide sequence that essentially corresponds (is essentially identical), preferably that corresponds (is identical), to the nucleotide sequence of the miRNA which expression profile is determinable by the means of (i) or is the profile of miRNAs that have nucleotide sequences that essentially correspond (are essentially identical), preferably that correspond (are identical), to the nucleotide sequences of the miRNAs which expression profile is determinable by the means of (i). More preferably, the at least one known clinical condition is healthiness or a lung disease (e.g. sarcoidosis, lung cancer or COPD). Most preferably, the at least one known clinical condition is selected from the group consisting of healthiness, sarcoidosis, lung cancer, and COPD.

It is also particularly preferred that the algorithm or mathematical function is obtained from reference expression profiles of at least two subjects, preferably of at least 3 to 40 subjects, more preferably of at least 10 to 25 subjects, and most preferably of at least 15 to 20 subjects, i.e. of at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 subjects, with at least two known clinical conditions, preferably 2 to 4 (e.g. 2, 3, or 4) known clinical conditions, wherein the reference expression profiles are the profiles of a miRNA that has a nucleotide sequence that essentially corresponds (is essentially identical), preferably that corresponds (is identical) to the nucleotide sequence of the miRNA which expression profile is determinable by the means of (i), or are the profiles of miRNAs that have nucleotide sequences that essentially correspond (are essentially identical), preferably that correspond (are identical), to the nucleotide sequences of the miRNAs which expression profile is determinable by the means of (i). Preferably, the at least two known clinical conditions are selected from the group consisting of healthiness, sarcoidosis, lung cancer, and COPD.

More preferably, the algorithm or mathematical function is obtained from (a) reference expression profile(s) of

(i) at least one subject with the known clinical condition of sarcoidosis,
(ii) at least one subject with the known clinical condition of healthiness,
(iii) at least one subject with the known clinical condition of lung cancer, and/or
(iv) at least one subject with the known clinical condition of COPD,

wherein the reference expression profile(s) is (are) the profile(s) of a miRNA that has a nucleotide sequence that essentially corresponds (is essentially identical), preferably that corresponds (is identical), to the nucleotide sequence of the miRNA which expression profile is determinable by the means of (i), or is (are) the profile(s) of miRNAs that have nucleotide sequences that essentially correspond (are essentially identical), preferably that correspond (are identical), to the nucleotide sequences of the miRNAs which expression profile is determinable by the means of (i).

It is further preferred that the algorithm or mathematical function is obtained using a machine learning approach. The inventors of the present invention surprisingly found that the application of a machine learning approach (e.g. t-test, AUC, support vector machine, hierarchical clustering, or k-means) leads to the obtainment of an algorithm or a mathematical function that is trained by the reference expression profile(s) (data) mentioned above.

Said data carrier may be a graphically data carrier such as an information leaflet or an information sheet (e.g. for comparing tested single reference miRNA biomarkers with the expression profile data of a patient to be diagnosed and/or prognosed) or an electronically data carrier such as a floppy disk, a compact disk (CD), or a digital versatile disk (DVD) (e.g. for comparing tested sets of miRNA biomarkers with the expression profile data of a patient to be diagnosed and/or prognosed). Said reference, preferably said (average) reference expression profile (data), and/or said algorithm or mathematical function may further be comprised in a computer program which is saved on an electronically data carrier.

The kit may alternatively comprise an access code which allows the access to a database, e.g. an internet database, a centralized, or a decentralized database, where (i) the reference, preferably the (average) reference expression profile (data), and/or the algorithm or mathematical function is (are) comprised, or (ii) where a computer program comprising the reference, preferably the (average) reference expression profile (data), and/or the algorithm or mathematical function can be downloaded.

More than one reference and/or more than one algorithm or mathematical function, e.g. 2, 3, 4, 5, or more references and/or algorithms or mathematical functions, may be comprised on one or more data carrier. For example, the kit may comprise (i) references (data), preferably (average) reference expression profile(s) (data), which may be comprised on an information leaflet and/or on a compact disk (CD), e.g. two expression profiles (data), for example, one from a subject(s) known to be healthy and one from a subject(s) known to have a lung disease such as sarcoidosis, lung cancer or COPD, and/or (ii) algorithms or mathematical functions, which may be comprised on a compact disc (CD) and/or on a digital versatile disk (DVD), e.g. two algorithms or mathematical functions, for example, one obtained from a reference expression profile of a subject(s) known to be healthy and one obtained from a reference expression profile of a subject(s) known to have a lung disease such as sarcoidosis, lung cancer or COPD.

Said two or more references (e.g. reference expression profiles) and/or algorithms or mathematical functions may be accessible by on or more access codes as mentioned above which may alternatively be comprised in the kit.

As to the reference or preferred embodiments of the reference (e.g. reference expression profile(s) (data)), it is also referred to the second aspect of the present invention (see above).

BRIEF DESCRIPTION OF THE DRAWINGS

**Figure 1:** MiRNAs for diagnosing and/or prognosing of sarcoidosis. Experimental data obtained from analysis of miRNAs according to SEQ ID NOs: 1 to 231 differentially regulated between subjects being healthy (controls) (g1) and subjects having sarcoidosis (g2). Experimental details: SEQ ID NO: sequence identification number, miRNA: identifier of the miRNA according to miRBase, median g1: median intensity obtained from microarray analysis for healthy controls, median g2: median intensity obtained from microarray analysis for subjects having sarcoidosis, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment. The inventors of the present invention found that these miRNAs allow the diagnosis and/or prognosis of sarcoidosis in a biological sample from a patient.

**Figure 2:** Listing of miRNAs for diagnosing and/or prognosing of a lung disease such as sarcoidosis, lung cancer, and/or chronic obstructive pulmonary disease (COPD). Said list of miRNAs includes sequences according to SEQ ID NOs: 1 to 391 found to be relevant for diagnosis and/or prognosis of a lung disease such as sarcoidosis, lung cancer, and/or COPD. The miRNAs sequences are based on miRBase version 12.0 to 14.0.

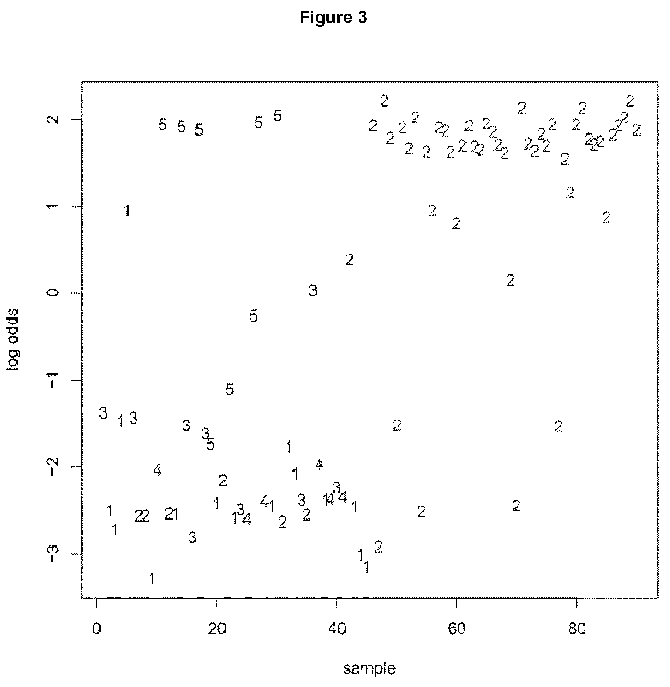

**Figure 3:** Classification plot: separating healthy controls ("0") from individuals suffering from sarcoidosis ("1").

**Figure 4:** Diagnostic miRNA-signature employing SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, and SEQ ID NO: 10; with V1 = accuracy (97.7%), V2 = specificity (95.6%), V3 = sensitivity (100%).

**Figure 5:** Diagnostic miRNA-signature employing SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14; with V1 = accuracy (97.8%), V2 = specificity (95.5%), V3 = sensitivity (100%).

**Figure 6:** Diagnostic miRNA-signature employing SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19; with V1 = accuracy (95.5%), V2 = specificity (91.1%), V3 = sensitivity (100%).

**Figure 7:** Diagnostic miRNA-signature employing SEQ ID NO: 1, SEQ ID NO: 2, SEQ ID NO: 3, SEQ ID NO: 4, SEQ ID NO: 68, SEQ ID NO: 5, SEQ ID NO: 6, SEQ ID NO: 7, SEQ ID NO: 69, SEQ ID NO: 8; with V1 = accuracy (95.5%), V2 = specificity (93.3%), V3 = sensitivity (97.8%).

**Figure 8:** Diagnostic miRNA-signature employing SEQ ID NO: 68, SEQ ID NO: 5, SEQ ID NO: 6, SEQ ID NO: 7, SEQ ID NO: 69, SEQ ID NO: 8, SEQ ID NO: 70, SEQ ID NO: 105, SEQ ID NO: 9, SEQ ID NO: 10; with V1 = accuracy (95.5%), V2 = specificity (95.5%), V3 = sensitivity (95.5%).

**Figure 9:** Diagnostic miRNA-signature employing SEQ ID NO: 8, SEQ ID NO: 70, SEQ ID NO: 105, SEQ ID NO: 9, SEQ ID NO: 10, SEQ ID NO: 11, SEQ ID NO: 12, SEQ ID NO: 13, SEQ ID NO: 14, SEQ ID NO: 114; with V1 = accuracy (95.5%), V2 = specificity (91.1%), V3 = sensitivity (100 %).

**Figure 10:** Diagnostic miRNA-signature employing SEQ ID NO: 68, SEQ ID NO: 1, SEQ ID NO: 4, SEQ ID NO: 69, SEQ ID NO: 3, SEQ ID NO: 5, SEQ ID NO: 8, SEQ ID NO: 70, SEQ ID NO: 7, SEQ ID NO: 6; with V1 = accuracy (93.3%), V2 = specificity (91.0%), V3 = sensitivity (95.5%).

**Figure 11:** Diagnostic miRNA-signature employing SEQ ID NO: 3, SEQ ID NO: 5, SEQ ID NO: 8, SEQ ID NO: 70, SEQ ID NO: 7, SEQ ID NO: 6, SEQ ID NO: 12, SEQ ID NO: 9, SEQ ID NO: 105, SEQ ID NO: 107; with V1 = accuracy (94.4%), V2 = specificity (91.1%), V3 = sensitivity (97.8%).

**Figure 12:** Diagnostic miRNA-signature employing SEQ ID NO: 6, SEQ ID NO: 12, SEQ ID NO: 9, SEQ ID NO: 105, SEQ ID NO: 107, SEQ ID NO: 71, SEQ ID NO: 17, SEQ ID NO: 159, SEQ ID NO: 19, SEQ ID NO: 106; with V1 = accuracy (95.5%), V2 = specificity (91.0%), V3 = sensitivity (100%).

**Figure 13:** MiRNAs for diagnosing and/or prognosing of lung cancer. Experimental data obtained from analysis of miRNAs differentially regulated between subjects having lung cancer (g1) and subjects having sarcoidosis (g2). Experimental details: SEQ ID NO: sequence identification number, miRNA: identifier of the miRNA according to miRBase, median g1: median intensity obtained from microarray analysis for subjects having lung cancer, median g2: median intensity obtained from microarray analysis for subjects having sarcoidosis, qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment. The inventors of the present invention found that these miRNAs allow the diagnosis and/or prognosis of lung cancer in a biological sample from a patient.

**Figure 14:** MiRNAs for diagnosing and/or prognosing of chronic obstructive pulmonary disease (COPD). Experimental data obtained from analysis of miRNAs differentially regulated between subjects having sarcoidosis (g1) and subjects having COPD (g2). Experimental details: SEQ ID NO: sequence identification number, miRNA: identifier of the miRNA according to miRBase, median g1: median intensity obtained from microarray analysis for subjects having sarcoidosis, median g2: median intensity obtained from microarray analysis for subjects having COPD qmedian: ratio of median g1/median g2, logqmedian: log of qmedian, ttest_rawp: p-value obtained when applying t-test, ttest_adjp: adjusted p-value in order to reduce false discovery rate by Benjamini-Hochberg adjustment, AUC: Area under the curve, limma_rawp: p-value obtained when applying limma-test, limma_adjp: adjusted p-value in order to reduce false discovery rate by Benjamini-Hochberg adjustment. The inventors of the present invention found that these miRNAs allow the diagnosis and/or prognosis of COPD in a biological sample from a patient.

**Figure 15:** Diagnostic sets of miRNAs (miRNA-signatures) for diagnosing and/or prognosing of sarcoidosis and for differential diagnosis with respect to lung cancer and/or COPD. Abbreviations: Set-No.: number of the set of miRNAs (miRNA signature); SEQ ID NO: sequence identification number, miRNA-identifier: identifier of the miRNA according to miRBase; acc = accuracy, spec = specificity, sens = sensitivity.

**Figure 16:** Diagnostic sets of miRNAs (miRNA-signatures) for diagnosing and/or prognosing of lung cancer and for differential diagnosis with respect to sarcoidosis and/or COPD. Abbreviations: Set-No.: number of the set of miRNAs (miRNA signature); SEQ ID NO: sequence identification number, miRNA-identifier: identifier of the miRNA according to miRBase; acc = accuracy, spec = specificity, sens = sensitivity.

**Figure 17:** Diagnostic sets of miRNAs (miRNA-signatures) for diagnosing and/or prognosing of COPD and for differential diagnosis with respect to lung cancer and/or sarcoidosis. Abbreviations: Set-No.: number of the set of miRNAs (miRNA signature); SEQ ID NO: sequence identification number, miRNA-identifier: identifier of the miRNA according to miRBase; acc = accuracy, spec = specificity, sens = sensitivity.

**Figure 18:** It shows in circle diagrams all 391 miRNAs which have been found to be suitable for the diagnosis and/or prognosis of a lung disease (e.g. sarcoidosis, lung cancer, and/or COPD). In detail, circle A consisting of areas 1, 2, 4, and 7 represents the 231 miRNAs found to be differentially regulated in blood samples of subjects having sarcoidosis compared to a cohort of controls (healthy subjects). The inventors of the present invention found that these miRNAs, particularly the 62 miRNAs in area 1, are suitable for the diagnosis and/or prognosis of sarcoidosis in a biological sample from a patient. Further, circle B consisting of areas 3, 2, 4, and 5 represents the 229 miRNAs found to be differentially regulated in blood samples of subjects having sarcoidosis compared to subjects having lung cancer. The inventors of the present invention found that these miRNAs, particularly the 60 miRNAs in area 3, are suitable for the diagnosis and/or prognosis of lung cancer in a biological sample from a patient. Furthermore, circle C consisting of areas 6, 7, 4, and 5 represents the 226 miRNAs found to be differentially regulated in blood samples of subjects having sarcoidosis compared to subjects having COPD. The inventors of the present invention found that these miRNAs, particularly the 62 miRNAs in area 6, are suitable for the diagnosis and/or prognosis of COPD in a biological sample from a patient. In this respect, it should be noted that the detection of at least one miRNA comprised in area 1, at least one miRNA comprised in area 3 and/or at least one miRNA comprised in area 6 may allow the diagnosis and/or prognosis of sarcoidosis, lung cancer, and/or COPD, e.g. the diagnosis and/or prognosis of sarcoidosis, lung cancer, COPD, sarcoidosis and lung cancer, sarcoidosis and COPD, lung cancer and COPD, or sarcoidosis, lung cancer and COPD. In addition, area 2 shared by circles A and B further represents the 43 miRNAs found to be differentially regulated in blood samples of subjects having sarcoidosis compared to a cohort of controls (healthy subjects) and subjects having sarcoidosis compared to subjects having lung cancer; area 7 shared by circles A and C represents the 38 miRNAs found to be differentially regulated in blood samples of subjects having sarcoidosis compared to a cohort of controls (healthy subjects) and subjects having sarcoidosis compared to subjects having COPD; area 5 shared by circles B and C represents the 38 miRNAs found to be differentially regulated in blood samples of subjects having sarcoidosis compared to subjects having lung cancer and subjects having sarcoidosis compared to subjects having COPD; and area 4 shared by circles A, B and C represents the 88 miRNAs found to be differentially regulated in blood samples of subjects having sarcoidosis compared to a cohort of controls (healthy subjects), subjects having sarcoidosis compared to subjects having lung cancer and subjects having sarcoidosis compared to subjects having COPD. The inventors of the present invention also found that these miRNAs that are shared by circles A, B and/or C further allow the differential diagnosis with respect to sarcoidosis, lung cancer, and/or COPD in a biological sample from a patient. This means that (i) the miRNAs that are shared by circles A and B (i.e. miRNAs comprised in area 2) allow to distinguish/differentiate between the lung diseases sarcoidosis and lung cancer, (ii) the miRNAs that are shared by circles A and C (i.e. miRNAs comprised in area 7) allow to distinguish/differentiate between the lung diseases sarcoidosis and COPD, (iii) that the miRNAs that are shared by circles B and C (i.e. miRNAs comprised in area 5) allow to distinguish/differentiate between the lung diseases lung cancer and COPD, and (iv) that the miRNAs that are shared by circles A, B and C (i.e. miRNAs comprised in area 4) allow to distinguish/differentiate between the lung diseases sarcoidosis, lung cancer and COPD.

EXAMPLES

The Examples are designed in order to further illustrate the present invention and serve a better understanding. They are not to be construed as limiting the scope of the invention in any way.

**Materials and Methods**

Samples

All blood donors participating in this study have given their written informed consent. The patient samples have been prepared at Heidelberg University. Besides the samples of diseased patients, also control samples were provided.

MiRNA extraction and microarray screening

Blood of patients has been extracted as previously described [1]. In brief, 2.5 to 5 ml blood was extracted in PAXgene Blood RNA tubes (BD, Franklin Lakes, New Jersey USA) and centrifuged at 5000 x g for 10 min at room temperature. The miRNeasy kit (Qiagen GmbH, Hilden) was used to isolate total RNA including miRNA from the resuspended pellet according to manufacturer's instructions. The eluted RNA was stored at -70°C.

All samples were analyzed with the Geniom RT Analyzer (febit biomed GmbH, Heidelberg, Germany) at the in-house genomic service department using the Geniom Biochip miRNA Homo sapiens. Each array contains 7 replicates of about 863 miRNAs and miRNA star sequences as annotated in the Sanger miRBase releases 12.0 to 14. On-chip sample labeling with biotin was carried out by microfluidic-based primer extension labeling of miRNAs (MPEA [2]). Following hybridization for 16 hours at 42°C, the biochip was washed and a program for signal enhancement was carried out. All steps from sample loading to miRNA detection were processed without any manual intervention and inside the machine. The detection pictures were evaluated using the Geniom Wizard Software. For each feature, the median signal intensity was calculated. Following a background correction step, the median of the 7 replicates of each miRNA was computed. To normalize the data across different arrays, quantile normalization [3] was applied and all further analyses were carried out using the normalized and background subtracted intensity values. Since the miRBase has been upgraded twice in the past year from version 12.0 to 14, we used for the final data analysis the 863 miRNAs that were consistently present in all three versions.

Statistical analysis

To estimate the value of single miRNAs, t-tests (unpaired, two-tailed) were carried out. The resulting p-values have been adjusted for multiple testing by Benjamini-Hochberg adjustment [4, 5].

In addition to this single biomarker analysis, we performed supervised classification of samples by using Support Vector Machines (SVM [6]) as implemented in the R e1071 package [7]. As parameters, we evaluated different kernel methods including linear, polynomial (degree 2 to 5), sigmoid and radial basis function kernels. The cost parameter was sampled from 0.01 to 10 in decimal powers. As subset selection technique, a filter approach based on t-test was carried out. In each iteration, the *s* miRNAs with lowest p-values were computed on the training set in each fold of a standard 10-fold cross validation, where *s* was sampled in regular intervals between 2 and 300. The respective subset was used to train the SVM and to carry out the prediction of the test samples in the cross validation. To compute probabilities for classes instead of class labels, a regression approach based on the output of the support vectors has been applied. To test for overtraining, non-parametric permutation tests have been applied. All computations were carried out using R [7], a freely available language for statistical tasks.

For all analysis, i.e. healthy versus sarcoidosis, lung cancer versus sarcoidosis, and sarcoidosis versus COPD, the same protocol (as described above) has been used (see also Figure 18 and legend to Figure 18).

The results are shown in the Figures.

REFERENCES

1. Keller A, Leidinger P, Borries A, Wendschlag A, Wucherpfennig F, Scheffler M, Huwer H, Lenhof HP, Meese E: miRNAs in lung cancer - studying complex fingerprints in patient's blood cells by microarray experiments. BMC Cancer 2009, 9:353.
2. Vorwerk S, Ganter K, Cheng Y, Hoheisel J, Stahler PF, Beier M: Microfluidic-based enzymatic on-chip labeling of miRNAs. N Biotechnol 2008, 25(2-3):142-149.
3. Bolstad BM, Irizarry RA, Astrand M, Speed TP: A comparison of normalization methods for high density oligonucleotide array data based on variance and bias. Bioinformatics 2003, 19(2):185-193.
4. Benjamini Y, Drai D, Elmer G, Kafkafi N, Golani I: Controlling the false discovery rate in behavior genetics research. Behav Brain Res 2001, 125(1-2):279-284.
5. Hochberg Y: A sharper bonferroni procedure for multiple tests of significance. Biometrica 1988, 75:185-193.
6. Vapnik V: The nature of statistical learning theory., 2nd edition edn. New York: Spinger; 2000.
7. Team R: R: A Language and Environment for Statistical Computing. In. Vienna: R Foundation for Statistical Computing; 2008.


## Claims

1. Use of polynucleotides for detecting miRNAs for diagnosing sarcoidosis in a blood sample from a patient, wherein the nucleotide sequences of said miRNAs comprise the nucleotide sequences according to Set No. 1 to 42 of Figure 15.

2. The use of claim 1, wherein the polynucleotides are complementary to the miRNAs according to claim 1.

3. A method for diagnosing sarcoidosis comprising the steps of:

   (i) determining an expression profile of miRNAs in a blood sample from a patient, wherein the nucleotide sequences of said miRNAs comprise the nucleotide sequences according to Set No. 1 to 42 of Figure 15, and
   (ii) comparing said expression profile to a reference, wherein the comparison of said expression profile to said reference allows for the diagnosis of sarcoidosis.

4. Use of means for diagnosing sarcoidosis, wherein said means comprise a solid support, substrate, surface, platform or matrix and polynucleotides according to claim 1, wherein said polynucleotides are attached to the solid support, substrate, surface, platform or matrix.

5. Use of claims 1 or 2, or method of claim 3, wherein the blood sample is a blood cell sample.

## Patentansprüche

1. Verwendung von Polynukleotiden zum Detektieren von miRNAs zum Diagnostizieren von Sarkoidose in einer Blutprobe eines Patienten, wobei die Nukleotidsequenzen dieser miRNAs die Nukleotidsequenzen gemäß Set Nr.1 bis 42 von Figur 15 umfassen.

**2.** Verwendung nach Anspruch 1, wobei die Polynukleotide komplementär zu den miRNAs gemäß Anspruch 1 sind.

**3.** Verfahren zum Diagnostizieren von Sarkoidose, umfassend die Schritte:

(i) Bestimmen eines Expressionsprofils von miRNAs in einer Blutprobe eines Patienten, wobei die Nukleotidsequenzen dieser miRNAs die Nukleotidsequenzen gemäß Set Nr.1 bis 42 von Figur 15 umfassen, und
(ii) Vergleichen dieses Expressionsprofils mit einer Referenz, wobei der Vergleich dieses Expressionsprofils mit dieser Referenz die Diagnose von Sarkoidose erlaubt.

**4.** Verwendung von Mitteln zum Diagnostizieren von Sarkoidose, wobei diese Mittel einen festen Träger, ein Substrat, eine Oberfläche, eine Plattform oder eine Matrix und Polynukleotide gemäß Anspruch 1 umfassen, wobei die Polynukleotide auf dem festen Träger, dem Substrat, der Oberfläche, der Plattform oder der Matrix aufgebracht sind.

**5.** Verwendung nach Anspruch 1 oder 2, oder Verfahren nach Anspruch 3, wobei die Blutprobe eine Blutzellprobe ist.

## Revendications

**1.** Utilisation de polynucléotides pour la détection de miARNs pour le diagnostic de la sarcoïdose dans un échantillon de sang provenant d'un patient, dans laquelle les séquences nucléotidiques desdits miARNs comprennent les séquences nucléotidiques selon l'ensemble No. 1 à 42 de la Figure 15.

**2.** Utilisation selon la revendication 1, dans laquelle les polynucléotides sont complémentaires des miARNs selon la revendication 1.

**3.** Procédé pour le diagnostic de la sarcoïdose comprenant les étapes de:

(i) détermination d'un profil d'expression de miARNs dans un échantillon de sang provenant d'un patient, tandis que les séquences nucléotidiques desdits miARNs comprennent les séquences nucléotidiques selon l'ensemble No. 1 à 42 de la Figure 15, et
(ii) comparaison dudit profil d'expression avec une référence, tandis que la comparaison dudit profil d'expression avec ladite référence permet le diagnostic de la sarcoïdose.

**4.** Utilisation de moyens pour le diagnostic de la sarcoïdose, dans laquelle lesdits moyens comprennent un support, substrat, surface, plate-forme ou matrice solides et des polynucléotides selon la revendication 1, lesdits polynucléotides étant fixés au support, substrat, surface, plate-forme ou matrice solides.

**5.** Utilisation selon les revendications 1 ou 2, ou procédé selon la revendication 3, dans laquelle ou lequel l'échantillon de sang est un échantillon de cellules sanguines.

# Figure 1

| SEQ ID NO: | miRNA | median g1 | median g2 | qmedian | logqmedian | ttest_rawp | ttest_adjp | AUC | limma_rawp | limma_adjp |
|---|---|---|---|---|---|---|---|---|---|---|
| SEQ ID NO: 1 | hsa-miR-363 | 3453 | 504 | 6,86 | 1,925 | 6,335E-15 | 2,734E-12 | 0,88 | 9,457E-11 | 1,632E-08 |
| SEQ ID NO: 2 | hsa-miR-151-3p | 642 | 2518 | 0,25 | -1,367 | 5,953E-15 | 2,734E-12 | 0,11 | 1,184E-14 | 1,022E-11 |
| SEQ ID NO: 3 | hsa-miR-17 | 6233 | 1463 | 4,26 | 1,449 | 1,092E-14 | 3,141E-12 | 0,86 | 4,658E-07 | 1,905E-05 |
| SEQ ID NO: 4 | hsa-miR-20b | 2820 | 576 | 4,89 | 1,588 | 3,431E-14 | 7,402E-12 | 0,86 | 3,831E-09 | 2,755E-07 |
| SEQ ID NO: 5 | hsa-miR-103 | 6845 | 2306 | 2,97 | 1,088 | 9,660E-14 | 1,389E-11 | 0,86 | 1,936E-07 | 8,792E-06 |
| SEQ ID NO: 6 | hsa-miR-106a | 5928 | 1621 | 3,66 | 1,296 | 1,658E-13 | 2,044E-11 | 0,84 | 1,797E-06 | 4,820E-05 |
| SEQ ID NO: 7 | hsa-miR-101 | 576 | 206 | 2,80 | 1,031 | 5,615E-13 | 6,057E-11 | 0,84 | 4,856E-07 | 1,905E-05 |
| SEQ ID NO: 8 | hsa-miR-20a | 3540 | 714 | 4,96 | 1,601 | 8,393E-12 | 7,244E-10 | 0,84 | 5,314E-07 | 1,911E-05 |
| SEQ ID NO: 9 | hsa-let-7d | 1987 | 611 | 3,25 | 1,180 | 1,443E-10 | 9,582E-09 | 0,83 | 1,137E-06 | 3,388E-05 |
| SEQ ID NO: 10 | hsa-miR-320d | 908 | 2697 | 0,34 | -1,089 | 2,611E-10 | 1,609E-08 | 0,14 | 2,636E-10 | 3,791E-08 |
| SEQ ID NO: 11 | hsa-miR-324-3p | 825 | 1797 | 0,46 | -0,779 | 3,322E-10 | 1,912E-08 | 0,13 | 2,909E-11 | 6,277E-09 |
| SEQ ID NO: 12 | hsa-miR-15b | 13583 | 5518 | 2,46 | 0,901 | 5,221E-10 | 2,816E-08 | 0,83 | 2,337E-08 | 1,441E-06 |
| SEQ ID NO: 13 | hsa-miR-148b | 525 | 274 | 1,91 | 0,649 | 1,986E-09 | 1,008E-07 | 0,80 | 3,571E-06 | 7,167E-05 |
| SEQ ID NO: 14 | hsa-miR-550* | 697 | 1797 | 0,39 | -0,947 | 2,363E-09 | 1,133E-07 | 0,16 | 1,502E-09 | 1,838E-07 |
| SEQ ID NO: 15 | hsa-miR-30d | 7722 | 13000 | 0,59 | -0,521 | 7,868E-09 | 3,395E-07 | 0,16 | 2,818E-09 | 2,211E-07 |
| SEQ ID NO: 16 | hsa-miR-26a | 6578 | 4002 | 1,64 | 0,497 | 8,624E-09 | 3,544E-07 | 0,79 | 7,403E-04 | 4,175E-03 |
| SEQ ID NO: 17 | hsa-miR-107 | 1305 | 500 | 2,61 | 0,960 | 2,173E-08 | 7,814E-07 | 0,81 | 4,219E-07 | 1,821E-05 |
| SEQ ID NO: 18 | hsa-miR-320b | 3002 | 5652 | 0,53 | -0,633 | 3,972E-08 | 1,269E-06 | 0,20 | 5,158E-07 | 1,911E-05 |
| SEQ ID NO: 19 | hsa-miR-24 | 1762 | 655 | 2,69 | 0,990 | 5,322E-08 | 1,584E-06 | 0,80 | 1,892E-06 | 4,820E-05 |
| SEQ ID NO: 20 | hsa-miR-18a | 1205 | 445 | 2,70 | 0,995 | 1,986E-07 | 5,355E-06 | 0,77 | 6,476E-07 | 2,236E-05 |
| SEQ ID NO: 21 | hsa-miR-320c | 783 | 1919 | 0,41 | -0,897 | 3,144E-07 | 7,980E-06 | 0,18 | 5,655E-04 | 3,389E-03 |
| SEQ ID NO: 22 | hsa-miR-744 | 611 | 318 | 1,92 | 0,652 | 4,734E-07 | 1,167E-05 | 0,77 | 5,668E-06 | 1,087E-04 |
| SEQ ID NO: 23 | hsa-miR-93 | 3453 | 2112 | 1,63 | 0,492 | 6,510E-07 | 1,561E-05 | 0,74 | 8,184E-04 | 4,506E-03 |
| SEQ ID NO: 24 | hsa-miR-30c | 2233 | 4200 | 0,53 | -0,632 | 9,244E-07 | 2,156E-05 | 0,22 | 2,258E-06 | 5,414E-05 |
| SEQ ID NO: 25 | hsa-miR-106b | 10422 | 5928 | 1,76 | 0,564 | 1,740E-06 | 3,850E-05 | 0,74 | 3,481E-02 | 7,823E-02 |
| SEQ ID NO: 26 | hsa-miR-182 | 4200 | 2306 | 1,82 | 0,600 | 3,534E-06 | 7,262E-05 | 0,77 | 6,590E-05 | 6,771E-04 |
| SEQ ID NO: 27 | hsa-miR-320a | 16709 | 24753 | 0,68 | -0,393 | 3,469E-06 | 7,262E-05 | 0,26 | 1,032E-04 | 9,679E-04 |
| SEQ ID NO: 28 | hsa-miR-720 | 4303 | 9818 | 0,44 | -0,825 | 5,274E-06 | 1,059E-04 | 0,20 | 1,008E-07 | 5,115E-06 |
| SEQ ID NO: 29 | hsa-miR-30e | 241 | 623 | 0,39 | -0,948 | 1,184E-05 | 2,221E-04 | 0,29 | 2,482E-01 | 3,661E-01 |

| | | | | | | | | | | |
|---|---|---|---|---|---|---|---|---|---|---|
| SEQ ID NO: 30 | hsa-miR-664 | 273 | 998 | 0,27 | -1,297 | 1,269E-05 | 2,331E-04 | 0,20 | 1,149E-04 | 1,044E-03 |
| SEQ ID NO: 31 | hsa-miR-16 | 15762 | 11107 | 1,42 | 0,350 | 1,299E-05 | 2,336E-04 | 0,71 | 8,764E-03 | 2,822E-02 |
| SEQ ID NO: 32 | hsa-miR-660 | 440 | 253 | 1,74 | 0,553 | 1,451E-05 | 2,555E-04 | 0,72 | 4,811E-06 | 9,435E-05 |
| SEQ ID NO: 33 | hsa-miR-130a | 1369 | 765 | 1,79 | 0,582 | 1,524E-05 | 2,616E-04 | 0,74 | 1,021E-03 | 5,404E-03 |
| SEQ ID NO: 34 | hsa-miR-148a | 950 | 477 | 1,99 | 0,689 | 1,546E-05 | 2,616E-04 | 0,73 | 2,265E-04 | 1,657E-03 |
| SEQ ID NO: 35 | hsa-miR-93* | 1328 | 2415 | 0,55 | -0,598 | 1,815E-05 | 3,012E-04 | 0,24 | 1,123E-05 | 1,761E-04 |
| SEQ ID NO: 36 | hsa-miR-423-5p | 2881 | 5135 | 0,56 | -0,578 | 2,203E-05 | 3,462E-04 | 0,24 | 7,558E-06 | 1,331E-04 |
| SEQ ID NO: 37 | hsa-miR-17* | 541 | 295 | 1,84 | 0,608 | 2,189E-05 | 3,462E-04 | 0,74 | 3,157E-05 | 3,837E-04 |
| SEQ ID NO: 38 | hsa-let-7a | 1025 | 611 | 1,68 | 0,517 | 2,917E-05 | 4,399E-04 | 0,71 | 7,271E-04 | 4,128E-03 |
| SEQ ID NO: 39 | hsa-miR-331-3p | 970 | 1832 | 0,53 | -0,635 | 2,956E-05 | 4,399E-04 | 0,25 | 4,128E-05 | 4,815E-04 |
| SEQ ID NO: 40 | hsa-miR-192 | 5652 | 6934 | 0,82 | -0,204 | 3,072E-05 | 4,494E-04 | 0,26 | 2,576E-03 | 1,079E-02 |
| SEQ ID NO: 41 | hsa-miR-29b | 415 | 675 | 0,61 | -0,487 | 5,829E-05 | 7,622E-04 | 0,25 | 6,950E-01 | 7,884E-01 |
| SEQ ID NO: 42 | hsa-miR-30a | 323 | 840 | 0,38 | -0,955 | 6,821E-05 | 8,656E-04 | 0,29 | 3,612E-02 | 8,012E-02 |
| SEQ ID NO: 43 | hsa-miR-28-5p | 356 | 214 | 1,66 | 0,508 | 8,166E-05 | 9,926E-04 | 0,73 | 8,097E-06 | 1,370E-04 |
| SEQ ID NO: 44 | hsa-miR-1908 | 1071 | 1832 | 0,58 | -0,537 | 9,591E-05 | 1,134E-03 | 0,30 | 4,740E-02 | 9,928E-02 |
| SEQ ID NO: 45 | hsa-miR-1260 | 2587 | 4200 | 0,62 | -0,485 | 1,020E-04 | 1,162E-03 | 0,28 | 1,944E-04 | 1,511E-03 |
| SEQ ID NO: 46 | hsa-miR-197 | 662 | 1352 | 0,49 | -0,715 | 1,282E-04 | 1,401E-03 | 0,25 | 2,095E-03 | 9,132E-03 |
| SEQ ID NO: 47 | hsa-miR-532-3p | 4406 | 6081 | 0,72 | -0,322 | 1,391E-04 | 1,464E-03 | 0,28 | 1,149E-04 | 1,044E-03 |
| SEQ ID NO: 48 | hsa-miR-500 | 213 | 453 | 0,47 | -0,756 | 2,389E-04 | 2,217E-03 | 0,31 | 1,718E-01 | 2,731E-01 |
| SEQ ID NO: 49 | hsa-miR-223 | 2257 | 3793 | 0,59 | -0,519 | 2,604E-04 | 2,391E-03 | 0,30 | 1,093E-03 | 5,647E-03 |
| SEQ ID NO: 50 | hsa-miR-195 | 620 | 396 | 1,56 | 0,447 | 4,603E-04 | 3,973E-03 | 0,73 | 3,622E-04 | 2,388E-03 |
| SEQ ID NO: 51 | hsa-miR-342-3p | 4525 | 5384 | 0,84 | -0,174 | 4,682E-04 | 4,000E-03 | 0,32 | 1,667E-03 | 7,734E-03 |
| SEQ ID NO: 52 | hsa-miR-25 | 6406 | 8175 | 0,78 | -0,244 | 8,776E-04 | 6,644E-03 | 0,31 | 4,571E-03 | 1,684E-02 |
| SEQ ID NO: 53 | hsa-miR-222 | 445 | 253 | 1,76 | 0,566 | 1,281E-03 | 9,061E-03 | 0,70 | 7,879E-06 | 1,360E-04 |
| SEQ ID NO: 54 | hsa-miR-1280 | 4200 | 7722 | 0,54 | -0,609 | 1,278E-03 | 9,061E-03 | 0,29 | 1,798E-04 | 1,478E-03 |
| SEQ ID NO: 55 | hsa-miR-181a | 636 | 1066 | 0,60 | -0,516 | 1,404E-03 | 9,695E-03 | 0,29 | 1,050E-01 | 1,849E-01 |
| SEQ ID NO: 56 | hsa-miR-194 | 6578 | 7722 | 0,85 | -0,160 | 2,343E-03 | 1,465E-02 | 0,33 | 3,197E-02 | 7,397E-02 |
| SEQ ID NO: 57 | hsa-miR-199a-5p | 236 | 445 | 0,53 | -0,636 | 2,630E-03 | 1,587E-02 | 0,37 | 8,379E-01 | 8,905E-01 |
| SEQ ID NO: 58 | hsa-miR-125b | 546 | 1552 | 0,35 | -1,045 | 2,776E-03 | 1,619E-02 | 0,30 | 1,026E-02 | 3,243E-02 |
| SEQ ID NO: 59 | hsa-miR-339-5p | 693 | 1283 | 0,54 | -0,616 | 2,911E-03 | 1,686E-02 | 0,30 | 6,409E-02 | 1,246E-01 |
| SEQ ID NO: 60 | hsa-miR-484 | 7722 | 9219 | 0,84 | -0,177 | 3,672E-03 | 2,024E-02 | 0,34 | 1,517E-02 | 4,307E-02 |
| SEQ ID NO: 61 | hsa-miR-574-3p | 2061 | 3002 | 0,69 | -0,376 | 4,147E-03 | 2,210E-02 | 0,34 | 3,728E-03 | 1,424E-02 |
| SEQ ID NO: 62 | hsa-miR-29a | 702 | 1129 | 0,62 | -0,475 | 4,255E-03 | 2,253E-02 | 0,32 | 9,065E-01 | 9,369E-01 |
| SEQ ID NO: 63 | hsa-miR-15a | 2881 | 2634 | 1,09 | 0,090 | 5,140E-03 | 2,577E-02 | 0,62 | 9,214E-03 | 2,945E-02 |

| | | | | | | | | | | |
|---|---|---|---|---|---|---|---|---|---|---|
| SEQ ID NO: 64 | hsa-miR-151-5p | 4406 | 3301 | 1,33 | 0,289 | 5,332E-03 | 2,643E-02 | 0,67 | 8,192E-02 | 1,520E-01 |
| SEQ ID NO: 65 | hsa-miR-574-5p | 840 | 423 | 1,98 | 0,685 | 9,380E-03 | 3,828E-02 | 0,68 | 1,870E-03 | 8,463E-03 |
| SEQ ID NO: 66 | hsa-miR-1913 | 359 | 307 | 1,17 | 0,156 | 1,077E-02 | 4,225E-02 | 0,60 | 5,606E-04 | 3,383E-03 |
| SEQ ID NO: 67 | hsa-miR-425 | 11992 | 15762 | 0,76 | -0,273 | 1,227E-02 | 4,666E-02 | 0,35 | 2,776E-02 | 6,672E-02 |
| SEQ ID NO: 68 | hsa-miR-144 | 1672 | 177 | 9,46 | 2,247 | 8,521E-14 | 1,389E-11 | 0,89 | 1,940E-11 | 5,581E-09 |
| SEQ ID NO: 69 | hsa-miR-126 | 1604 | 191 | 8,39 | 2,128 | 1,746E-12 | 1,674E-10 | 0,86 | 6,316E-13 | 2,726E-10 |
| SEQ ID NO: 70 | hsa-miR-144* | 636 | 134 | 4,74 | 1,555 | 1,627E-11 | 1,276E-09 | 0,84 | 2,798E-09 | 2,211E-07 |
| SEQ ID NO: 71 | hsa-miR-374b | 493 | 127 | 3,89 | 1,357 | 5,139E-08 | 1,584E-06 | 0,82 | 1,703E-09 | 1,838E-07 |
| SEQ ID NO: 72 | hsa-miR-21 | 835 | 150 | 5,55 | 1,713 | 1,683E-07 | 4,841E-06 | 0,79 | 7,093E-07 | 2,354E-05 |
| SEQ ID NO: 73 | hsa-miR-301a | 268 | 116 | 2,32 | 0,840 | 7,471E-06 | 1,465E-04 | 0,77 | 2,494E-06 | 5,665E-05 |
| SEQ ID NO: 74 | hsa-let-7i | 513 | 194 | 2,65 | 0,973 | 8,500E-06 | 1,630E-04 | 0,70 | 1,296E-03 | 6,357E-03 |
| SEQ ID NO: 75 | hsa-let-7f | 418 | 123 | 3,41 | 1,227 | 9,803E-05 | 1,143E-03 | 0,69 | 7,359E-05 | 7,364E-04 |
| SEQ ID NO: 76 | hsa-miR-18a* | 192 | 523 | 0,37 | -1,003 | 1,037E-04 | 1,162E-03 | 0,32 | 5,630E-01 | 6,805E-01 |
| SEQ ID NO: 77 | hsa-miR-183 | 324 | 105 | 3,08 | 1,124 | 1,374E-04 | 1,464E-03 | 0,74 | 1,239E-06 | 3,565E-05 |
| SEQ ID NO: 78 | hsa-miR-1202 | 208 | 113 | 1,85 | 0,614 | 1,658E-04 | 1,664E-03 | 0,72 | 4,473E-05 | 5,079E-04 |
| SEQ ID NO: 79 | hsa-miR-503 | 260 | 157 | 1,65 | 0,502 | 2,156E-04 | 2,022E-03 | 0,71 | 2,905E-04 | 2,028E-03 |
| SEQ ID NO: 80 | hsa-miR-593* | 288 | 192 | 1,50 | 0,405 | 4,848E-04 | 4,023E-03 | 0,71 | 1,699E-06 | 4,729E-05 |
| SEQ ID NO: 81 | hsa-miR-646 | 206 | 144 | 1,43 | 0,360 | 6,460E-04 | 5,068E-03 | 0,65 | 7,224E-04 | 4,128E-03 |
| SEQ ID NO: 82 | hsa-miR-100 | 123 | 247 | 0,50 | -0,694 | 1,240E-03 | 8,992E-03 | 0,35 | 1,043E-02 | 3,262E-02 |
| SEQ ID NO: 83 | hsa-miR-125a-5p | 151 | 379 | 0,40 | -0,918 | 1,647E-03 | 1,110E-02 | 0,38 | 8,990E-01 | 9,359E-01 |
| SEQ ID NO: 84 | hsa-miR-145 | 131 | 261 | 0,50 | -0,691 | 2,460E-03 | 1,528E-02 | 0,42 | 9,034E-01 | 9,360E-01 |
| SEQ ID NO: 85 | hsa-let-7i* | 226 | 129 | 1,75 | 0,559 | 2,494E-03 | 1,538E-02 | 0,70 | 7,424E-05 | 7,364E-04 |
| SEQ ID NO: 86 | hsa-miR-424 | 219 | 114 | 1,93 | 0,656 | 4,630E-03 | 2,386E-02 | 0,74 | 7,865E-05 | 7,626E-04 |
| SEQ ID NO: 87 | hsa-miR-181c | 126 | 246 | 0,51 | -0,664 | 7,149E-03 | 3,299E-02 | 0,39 | 5,467E-01 | 6,683E-01 |
| SEQ ID NO: 88 | hsa-miR-152 | 214 | 154 | 1,39 | 0,332 | 7,850E-03 | 3,510E-02 | 0,69 | 4,629E-05 | 5,182E-04 |
| SEQ ID NO: 89 | hsa-miR-214 | 296 | 144 | 2,06 | 0,721 | 8,089E-03 | 3,561E-02 | 0,73 | 9,134E-06 | 1,460E-04 |
| SEQ ID NO: 90 | hsa-miR-362-5p | 126 | 282 | 0,45 | -0,802 | 8,977E-03 | 3,824E-02 | 0,42 | 2,157E-01 | 3,277E-01 |
| SEQ ID NO: 91 | hsa-miR-532-5p | 211 | 104 | 2,04 | 0,712 | 9,432E-03 | 3,828E-02 | 0,73 | 1,828E-05 | 2,545E-04 |
| SEQ ID NO: 92 | hsa-miR-1226* | 187 | 109 | 1,70 | 0,533 | 6,964E-05 | 8,710E-04 | 0,71 | 2,194E-04 | 1,647E-03 |
| SEQ ID NO: 93 | hsa-miR-1184 | 176 | 106 | 1,66 | 0,508 | 1,233E-03 | 8,992E-03 | 0,72 | 1,501E-05 | 2,196E-04 |
| SEQ ID NO: 94 | hsa-miR-31* | 171 | 128 | 1,34 | 0,293 | 1,879E-03 | 1,229E-02 | 0,65 | 3,621E-04 | 2,388E-03 |
| SEQ ID NO: 95 | hsa-miR-1912 | 152 | 106 | 1,43 | 0,361 | 1,956E-03 | 1,260E-02 | 0,65 | 3,727E-04 | 2,410E-03 |
| SEQ ID NO: 96 | hsa-miR-1203 | 198 | 148 | 1,34 | 0,292 | 2,276E-03 | 1,434E-02 | 0,68 | 6,077E-05 | 6,395E-04 |
| SEQ ID NO: 97 | hsa-miR-1225-3p | 138 | 103 | 1,34 | 0,292 | 2,674E-03 | 1,588E-02 | 0,67 | 2,469E-05 | 3,133E-04 |

| | | | | | | | | | | |
|---|---|---|---|---|---|---|---|---|---|---|
| SEQ ID NO: 98 | hsa-miR-517* | 165 | 142 | 1,16 | 0,145 | 4,692E-03 | 2,396E-02 | 0,63 | 1,273E-03 | 6,279E-03 |
| SEQ ID NO: 99 | hsa-miR-515-5p | 161 | 130 | 1,24 | 0,216 | 6,999E-03 | 3,248E-02 | 0,64 | 1,271E-03 | 6,279E-03 |
| SEQ ID NO: 100 | hsa-miR-431 | 176 | 128 | 1,37 | 0,318 | 8,514E-03 | 3,711E-02 | 0,64 | 2,949E-02 | 6,961E-02 |
| SEQ ID NO: 101 | hsa-miR-188-3p | 129 | 104 | 1,25 | 0,224 | 8,681E-03 | 3,761E-02 | 0,63 | 2,393E-03 | 1,023E-02 |
| SEQ ID NO: 102 | hsa-miR-217 | 141 | 103 | 1,37 | 0,316 | 9,216E-03 | 3,824E-02 | 0,65 | 6,557E-03 | 2,227E-02 |
| SEQ ID NO: 103 | hsa-miR-940 | 157 | 103 | 1,52 | 0,418 | 1,121E-02 | 4,357E-02 | 0,63 | 1,030E-04 | 9,679E-04 |
| SEQ ID NO: 104 | hsa-miR-1826 | 195 | 161 | 1,21 | 0,191 | 1,172E-02 | 4,517E-02 | 0,63 | 2,914E-04 | 2,028E-03 |
| SEQ ID NO: 105 | hsa-miR-27b | 203 | 57 | 3,55 | 1,268 | 8,088E-11 | 5,817E-09 | 0,82 | 1,478E-05 | 2,196E-04 |
| SEQ ID NO: 106 | hsa-miR-26b | 316 | 91 | 3,47 | 1,245 | 1,720E-08 | 6,453E-07 | 0,80 | 7,475E-08 | 4,035E-06 |
| SEQ ID NO: 107 | hsa-miR-27a | 259 | 64 | 4,02 | 1,392 | 3,084E-08 | 1,064E-06 | 0,82 | 2,097E-09 | 2,010E-07 |
| SEQ ID NO: 108 | hsa-let-7g | 294 | 81 | 3,64 | 1,292 | 2,496E-07 | 6,527E-06 | 0,74 | 1,383E-04 | 1,181E-03 |
| SEQ ID NO: 109 | hsa-miR-374a | 245 | 96 | 2,55 | 0,938 | 2,737E-06 | 5,905E-05 | 0,77 | 1,899E-06 | 4,820E-05 |
| SEQ ID NO: 110 | hsa-miR-1301 | 202 | 84 | 2,42 | 0,882 | 5,416E-05 | 7,190E-04 | 0,79 | 2,463E-05 | 3,133E-04 |
| SEQ ID NO: 111 | hsa-miR-509-3-5p | 212 | 96 | 2,21 | 0,794 | 1,084E-04 | 1,200E-03 | 0,73 | 6,049E-06 | 1,135E-04 |
| SEQ ID NO: 112 | hsa-miR-584 | 84 | 247 | 0,34 | -1,076 | 6,459E-04 | 5,068E-03 | 0,35 | 1,645E-01 | 2,639E-01 |
| SEQ ID NO: 113 | hsa-miR-486-3p | 223 | 99 | 2,24 | 0,807 | 4,149E-03 | 2,210E-02 | 0,75 | 2,174E-06 | 5,360E-05 |
| SEQ ID NO: 114 | hsa-miR-96 | 156 | 57 | 2,74 | 1,008 | 7,476E-09 | 3,395E-07 | 0,80 | 1,203E-07 | 5,770E-06 |
| SEQ ID NO: 115 | hsa-miR-1272 | 129 | 69 | 1,88 | 0,633 | 3,847E-08 | 1,269E-06 | 0,77 | 2,090E-05 | 2,774E-04 |
| SEQ ID NO: 116 | hsa-miR-143 | 168 | 57 | 2,97 | 1,088 | 1,831E-07 | 5,098E-06 | 0,79 | 7,445E-07 | 2,380E-05 |
| SEQ ID NO: 117 | hsa-miR-588 | 119 | 72 | 1,65 | 0,502 | 1,186E-06 | 2,695E-05 | 0,75 | 5,398E-05 | 5,897E-04 |
| SEQ ID NO: 118 | hsa-miR-24-2* | 147 | 96 | 1,53 | 0,426 | 2,207E-05 | 3,462E-04 | 0,72 | 8,577E-06 | 1,409E-04 |
| SEQ ID NO: 119 | hsa-miR-541 | 106 | 52 | 2,02 | 0,704 | 2,750E-05 | 4,238E-04 | 0,75 | 1,876E-04 | 1,485E-03 |
| SEQ ID NO: 120 | hsa-miR-1254 | 112 | 74 | 1,51 | 0,413 | 4,715E-05 | 6,671E-04 | 0,70 | 1,231E-01 | 2,087E-01 |
| SEQ ID NO: 121 | hsa-miR-628-3p | 152 | 66 | 2,31 | 0,836 | 5,006E-05 | 6,969E-04 | 0,73 | 2,735E-05 | 3,371E-04 |
| SEQ ID NO: 122 | hsa-miR-143* | 112 | 62 | 1,80 | 0,590 | 5,194E-05 | 7,053E-04 | 0,71 | 6,313E-04 | 3,706E-03 |
| SEQ ID NO: 123 | hsa-miR-654-5p | 181 | 82 | 2,20 | 0,790 | 5,230E-05 | 7,053E-04 | 0,74 | 1,805E-05 | 2,545E-04 |
| SEQ ID NO: 124 | hsa-miR-1286 | 120 | 69 | 1,74 | 0,556 | 6,120E-05 | 7,883E-04 | 0,70 | 1,776E-02 | 4,733E-02 |
| SEQ ID NO: 125 | hsa-miR-18b | 166 | 83 | 2,00 | 0,691 | 8,063E-05 | 9,926E-04 | 0,72 | 1,746E-04 | 1,448E-03 |
| SEQ ID NO: 126 | hsa-miR-550 | 114 | 57 | 2,00 | 0,691 | 1,345E-04 | 1,450E-03 | 0,69 | 2,616E-03 | 1,088E-02 |
| SEQ ID NO: 127 | hsa-let-7d* | 59 | 175 | 0,34 | -1,093 | 1,534E-04 | 1,595E-03 | 0,33 | 4,976E-02 | 1,027E-01 |
| SEQ ID NO: 128 | hsa-miR-220c | 103 | 51 | 2,01 | 0,697 | 1,614E-04 | 1,645E-03 | 0,75 | 7,378E-06 | 1,326E-04 |
| SEQ ID NO: 129 | hsa-miR-33b | 172 | 79 | 2,18 | 0,781 | 1,620E-04 | 1,645E-03 | 0,78 | 2,648E-06 | 5,859E-05 |
| SEQ ID NO: 130 | hsa-miR-575 | 127 | 87 | 1,46 | 0,380 | 1,685E-04 | 1,672E-03 | 0,71 | 6,406E-05 | 6,661E-04 |
| SEQ ID NO: 131 | hsa-miR-31 | 129 | 68 | 1,89 | 0,634 | 1,781E-04 | 1,746E-03 | 0,72 | 2,949E-03 | 1,178E-02 |

| | | | | | | | | | | |
|---|---|---|---|---|---|---|---|---|---|---|
| SEQ ID NO: 132 | hsa-miR-455-3p | 169 | 91 | 1,85 | 0,618 | 2,051E-04 | 1,967E-03 | 0,70 | 6,958E-05 | 7,064E-04 |
| SEQ ID NO: 133 | hsa-miR-1283 | 109 | 70 | 1,57 | 0,449 | 2,694E-04 | 2,447E-03 | 0,70 | 2,044E-03 | 9,040E-03 |
| SEQ ID NO: 134 | hsa-miR-1914* | 139 | 77 | 1,81 | 0,591 | 3,356E-04 | 2,986E-03 | 0,70 | 7,062E-06 | 1,297E-04 |
| SEQ ID NO: 135 | hsa-miR-196a* | 139 | 53 | 2,62 | 0,965 | 3,798E-04 | 3,345E-03 | 0,77 | 7,482E-08 | 4,035E-06 |
| SEQ ID NO: 136 | hsa-miR-595 | 103 | 50 | 2,06 | 0,720 | 4,002E-04 | 3,489E-03 | 0,65 | 5,849E-02 | 1,165E-01 |
| SEQ ID NO: 137 | hsa-miR-192* | 113 | 71 | 1,60 | 0,467 | 4,750E-04 | 4,019E-03 | 0,73 | 2,995E-06 | 6,304E-05 |
| SEQ ID NO: 138 | hsa-miR-16-2* | 84 | 196 | 0,43 | -0,846 | 4,832E-04 | 4,023E-03 | 0,32 | 1,679E-01 | 2,683E-01 |
| SEQ ID NO: 139 | hsa-miR-96* | 115 | 74 | 1,55 | 0,436 | 5,956E-04 | 4,803E-03 | 0,67 | 8,398E-02 | 1,552E-01 |
| SEQ ID NO: 140 | hsa-miR-33a | 103 | 61 | 1,68 | 0,517 | 6,831E-04 | 5,311E-03 | 0,69 | 1,291E-01 | 2,166E-01 |
| SEQ ID NO: 141 | hsa-miR-650 | 124 | 59 | 2,11 | 0,745 | 8,481E-04 | 6,477E-03 | 0,69 | 4,952E-04 | 3,074E-03 |
| SEQ ID NO: 142 | hsa-miR-1295 | 113 | 73 | 1,54 | 0,435 | 9,949E-04 | 7,466E-03 | 0,66 | 2,462E-03 | 1,047E-02 |
| SEQ ID NO: 143 | hsa-miR-1266 | 111 | 89 | 1,25 | 0,220 | 1,022E-03 | 7,601E-03 | 0,64 | 1,329E-02 | 3,876E-02 |
| SEQ ID NO: 144 | hsa-miR-32 | 142 | 99 | 1,43 | 0,360 | 1,237E-03 | 8,992E-03 | 0,66 | 4,354E-04 | 2,723E-03 |
| SEQ ID NO: 145 | hsa-miR-338-3p | 127 | 89 | 1,43 | 0,358 | 1,258E-03 | 9,049E-03 | 0,68 | 6,570E-03 | 2,227E-02 |
| SEQ ID NO: 146 | hsa-miR-1251 | 128 | 99 | 1,30 | 0,261 | 1,300E-03 | 9,120E-03 | 0,69 | 1,820E-04 | 1,480E-03 |
| SEQ ID NO: 147 | hsa-miR-328 | 57 | 106 | 0,54 | -0,620 | 1,761E-03 | 1,178E-02 | 0,34 | 1,778E-02 | 4,733E-02 |
| SEQ ID NO: 148 | hsa-miR-33b* | 134 | 94 | 1,43 | 0,358 | 2,698E-03 | 1,588E-02 | 0,67 | 1,506E-03 | 7,142E-03 |
| SEQ ID NO: 149 | hsa-miR-631 | 130 | 90 | 1,44 | 0,368 | 3,194E-03 | 1,825E-02 | 0,65 | 1,654E-03 | 7,714E-03 |
| SEQ ID NO: 150 | hsa-miR-523* | 119 | 53 | 2,24 | 0,808 | 3,682E-03 | 2,024E-02 | 0,67 | 4,596E-03 | 1,684E-02 |
| SEQ ID NO: 151 | hsa-miR-187* | 125 | 91 | 1,37 | 0,312 | 3,750E-03 | 2,048E-02 | 0,63 | 3,912E-02 | 8,504E-02 |
| SEQ ID NO: 152 | hsa-miR-132 | 108 | 70 | 1,55 | 0,441 | 4,373E-03 | 2,287E-02 | 0,64 | 4,767E-02 | 9,962E-02 |
| SEQ ID NO: 153 | hsa-miR-146b-5p | 101 | 66 | 1,54 | 0,433 | 4,873E-03 | 2,468E-02 | 0,65 | 9,043E-03 | 2,901E-02 |
| SEQ ID NO: 154 | hsa-miR-454 | 114 | 68 | 1,69 | 0,525 | 5,667E-03 | 2,779E-02 | 0,64 | 1,078E-01 | 1,884E-01 |
| SEQ ID NO: 155 | hsa-miR-298 | 117 | 80 | 1,46 | 0,377 | 5,762E-03 | 2,796E-02 | 0,65 | 3,297E-02 | 7,567E-02 |
| SEQ ID NO: 156 | hsa-miR-518e* | 138 | 58 | 2,39 | 0,873 | 7,436E-03 | 3,396E-02 | 0,68 | 1,208E-03 | 6,133E-03 |
| SEQ ID NO: 157 | hsa-miR-15a* | 112 | 78 | 1,45 | 0,368 | 9,909E-03 | 3,941E-02 | 0,64 | 1,812E-01 | 2,860E-01 |
| SEQ ID NO: 158 | hsa-let-7e | 141 | 83 | 1,70 | 0,528 | 1,135E-02 | 4,392E-02 | 0,60 | 3,324E-01 | 4,546E-01 |
| SEQ ID NO: 159 | hsa-miR-199a-3p | 96 | 50 | 1,91 | 0,650 | 1,119E-08 | 4,389E-07 | 0,80 | 2,938E-06 | 6,304E-05 |
| SEQ ID NO: 160 | hsa-miR-525-5p | 81 | 51 | 1,59 | 0,466 | 3,834E-05 | 5,514E-04 | 0,74 | 1,369E-04 | 1,181E-03 |
| SEQ ID NO: 161 | hsa-miR-516b* | 21 | 56 | 0,37 | -0,992 | 9,399E-05 | 1,127E-03 | 0,27 | 2,584E-04 | 1,859E-03 |
| SEQ ID NO: 162 | hsa-miR-199b-3p | 93 | 51 | 1,83 | 0,604 | 1,036E-04 | 1,162E-03 | 0,73 | 1,307E-04 | 1,139E-03 |
| SEQ ID NO: 163 | hsa-miR-934 | 87 | 56 | 1,53 | 0,428 | 1,945E-04 | 1,886E-03 | 0,70 | 8,197E-04 | 4,506E-03 |
| SEQ ID NO: 164 | hsa-miR-335* | 1 | 50 | 0,02 | -3,917 | 2,150E-04 | 2,022E-03 | 0,27 | 4,684E-05 | 5,182E-04 |
| SEQ ID NO: 165 | hsa-miR-505 | 20 | 52 | 0,38 | -0,966 | 3,236E-04 | 2,909E-03 | 0,26 | 1,962E-05 | 2,687E-04 |

| | | | | | | | | | | |
|---|---|---|---|---|---|---|---|---|---|---|
| SEQ ID NO: 166 | hsa-miR-885-5p | 23 | 53 | 0,44 | -0,818 | 5,772E-04 | 4,744E-03 | 0,29 | 5,807E-04 | 3,456E-03 |
| SEQ ID NO: 167 | hsa-miR-129* | 45 | 57 | 0,79 | -0,239 | 5,833E-04 | 4,749E-03 | 0,27 | 1,253E-05 | 1,896E-04 |
| SEQ ID NO: 168 | hsa-miR-512-3p | 36 | 53 | 0,68 | -0,384 | 6,066E-04 | 4,847E-03 | 0,28 | 1,099E-04 | 1,020E-03 |
| SEQ ID NO: 169 | hsa-miR-139-3p | 78 | 52 | 1,49 | 0,396 | 7,845E-04 | 6,045E-03 | 0,68 | 2,129E-04 | 1,612E-03 |
| SEQ ID NO: 170 | hsa-miR-604 | 89 | 52 | 1,71 | 0,538 | 1,341E-03 | 9,330E-03 | 0,70 | 3,270E-04 | 2,222E-03 |
| SEQ ID NO: 171 | hsa-miR-29c* | 1 | 52 | 0,02 | -3,705 | 1,459E-03 | 9,991E-03 | 0,22 | 1,551E-08 | 1,029E-06 |
| SEQ ID NO: 172 | hsa-miR-302b | 1 | 37 | 0,03 | -3,620 | 1,620E-03 | 1,101E-02 | 0,27 | 8,653E-06 | 1,409E-04 |
| SEQ ID NO: 173 | hsa-miR-124* | 93 | 83 | 1,13 | 0,122 | 1,836E-03 | 1,219E-02 | 0,66 | 1,068E-02 | 3,289E-02 |
| SEQ ID NO: 174 | hsa-miR-548c-3p | 36 | 50 | 0,72 | -0,328 | 1,855E-03 | 1,222E-02 | 0,32 | 2,882E-03 | 1,162E-02 |
| SEQ ID NO: 175 | hsa-miR-200c* | 48 | 59 | 0,81 | -0,216 | 1,929E-03 | 1,252E-02 | 0,30 | 2,983E-04 | 2,060E-03 |
| SEQ ID NO: 176 | hsa-miR-1911* | 85 | 50 | 1,69 | 0,523 | 1,995E-03 | 1,275E-02 | 0,73 | 3,476E-05 | 4,166E-04 |
| SEQ ID NO: 177 | hsa-miR-517c | 7 | 43 | 0,16 | -1,842 | 2,131E-03 | 1,352E-02 | 0,30 | 3,101E-04 | 2,124E-03 |
| SEQ ID NO: 178 | hsa-miR-1262 | 1 | 17 | 0,06 | -2,847 | 2,512E-03 | 1,538E-02 | 0,33 | 1,064E-03 | 5,533E-03 |
| SEQ ID NO: 179 | hsa-miR-548g | 26 | 52 | 0,51 | -0,678 | 2,575E-03 | 1,565E-02 | 0,31 | 2,691E-04 | 1,920E-03 |
| SEQ ID NO: 180 | hsa-miR-502-5p | 10 | 36 | 0,28 | -1,270 | 2,662E-03 | 1,588E-02 | 0,34 | 1,190E-02 | 3,567E-02 |
| SEQ ID NO: 181 | hsa-miR-490-3p | 87 | 72 | 1,21 | 0,193 | 2,705E-03 | 1,588E-02 | 0,65 | 1,493E-03 | 7,120E-03 |
| SEQ ID NO: 182 | hsa-miR-483-3p | 21 | 62 | 0,34 | -1,083 | 3,066E-03 | 1,764E-02 | 0,24 | 1,138E-06 | 3,388E-05 |
| SEQ ID NO: 183 | hsa-miR-187 | 12 | 32 | 0,38 | -0,974 | 3,323E-03 | 1,887E-02 | 0,38 | 4,862E-02 | 1,009E-01 |
| SEQ ID NO: 184 | hsa-miR-519b-3p | 27 | 50 | 0,53 | -0,628 | 3,429E-03 | 1,934E-02 | 0,31 | 1,927E-03 | 8,660E-03 |
| SEQ ID NO: 185 | hsa-let-7c* | 19 | 51 | 0,37 | -0,994 | 3,475E-03 | 1,947E-02 | 0,28 | 5,968E-05 | 6,359E-04 |
| SEQ ID NO: 186 | hsa-miR-649 | 10 | 30 | 0,33 | -1,118 | 3,646E-03 | 2,024E-02 | 0,37 | 3,047E-02 | 7,145E-02 |
| SEQ ID NO: 187 | hsa-miR-517a | 28 | 40 | 0,70 | -0,360 | 3,812E-03 | 2,068E-02 | 0,35 | 4,634E-03 | 1,684E-02 |
| SEQ ID NO: 188 | hsa-miR-630 | 30 | 51 | 0,58 | -0,552 | 3,834E-03 | 2,068E-02 | 0,29 | 2,034E-04 | 1,553E-03 |
| SEQ ID NO: 189 | hsa-miR-126* | 2 | 30 | 0,08 | -2,567 | 4,340E-03 | 2,284E-02 | 0,36 | 8,472E-03 | 2,759E-02 |
| SEQ ID NO: 190 | hsa-miR-656 | 28 | 51 | 0,54 | -0,608 | 4,544E-03 | 2,362E-02 | 0,33 | 1,232E-03 | 6,148E-03 |
| SEQ ID NO: 191 | hsa-miR-569 | 11 | 52 | 0,21 | -1,564 | 4,646E-03 | 2,386E-02 | 0,31 | 2,914E-03 | 1,170E-02 |
| SEQ ID NO: 192 | hsa-miR-632 | 21 | 50 | 0,41 | -0,888 | 4,890E-03 | 2,468E-02 | 0,33 | 8,640E-03 | 2,793E-02 |
| SEQ ID NO: 193 | hsa-miR-378* | 51 | 91 | 0,56 | -0,577 | 5,166E-03 | 2,577E-02 | 0,37 | 2,886E-01 | 4,057E-01 |
| SEQ ID NO: 194 | hsa-miR-553 | 3 | 29 | 0,10 | -2,322 | 5,359E-03 | 2,643E-02 | 0,33 | 8,595E-04 | 4,694E-03 |
| SEQ ID NO: 195 | hsa-miR-137 | 76 | 53 | 1,42 | 0,347 | 5,767E-03 | 2,796E-02 | 0,65 | 1,449E-02 | 4,141E-02 |
| SEQ ID NO: 196 | hsa-miR-1183 | 90 | 74 | 1,21 | 0,192 | 6,026E-03 | 2,905E-02 | 0,61 | 2,719E-03 | 1,112E-02 |
| SEQ ID NO: 197 | hsa-miR-556-3p | 1 | 31 | 0,03 | -3,438 | 6,466E-03 | 3,073E-02 | 0,32 | 5,522E-04 | 3,356E-03 |
| SEQ ID NO: 198 | hsa-miR-190b | 1 | 32 | 0,03 | -3,474 | 6,481E-03 | 3,073E-02 | 0,37 | 1,174E-02 | 3,529E-02 |
| SEQ ID NO: 199 | hsa-miR-1915* | 28 | 52 | 0,54 | -0,609 | 6,457E-03 | 3,073E-02 | 0,32 | 2,503E-03 | 1,054E-02 |

| | | | | | | | | | | |
|---|---|---|---|---|---|---|---|---|---|---|
| SEQ ID NO: 200 | hsa-miR-186 | 34 | 82 | 0,41 | -0,891 | 6,645E-03 | 3,134E-02 | 0,31 | 1,333E-03 | 6,500E-03 |
| SEQ ID NO: 201 | hsa-miR-1468 | 1 | 16 | 0,06 | -2,781 | 6,685E-03 | 3,135E-02 | 0,34 | 1,100E-03 | 5,652E-03 |
| SEQ ID NO: 202 | hsa-miR-454* | 13 | 52 | 0,25 | -1,404 | 6,746E-03 | 3,147E-02 | 0,26 | 3,846E-05 | 4,546E-04 |
| SEQ ID NO: 203 | hsa-miR-1305 | 90 | 67 | 1,33 | 0,285 | 7,310E-03 | 3,356E-02 | 0,66 | 1,572E-02 | 4,377E-02 |
| SEQ ID NO: 204 | hsa-miR-617 | 18 | 31 | 0,58 | -0,539 | 7,613E-03 | 3,458E-02 | 0,35 | 4,148E-03 | 1,550E-02 |
| SEQ ID NO: 205 | hsa-miR-551b* | 37 | 56 | 0,67 | -0,406 | 7,672E-03 | 3,466E-02 | 0,34 | 2,745E-02 | 6,632E-02 |
| SEQ ID NO: 206 | hsa-miR-1206 | 59 | 44 | 1,34 | 0,289 | 7,736E-03 | 3,477E-02 | 0,63 | 3,144E-02 | 7,294E-02 |
| SEQ ID NO: 207 | hsa-miR-379 | 8 | 28 | 0,30 | -1,209 | 8,027E-03 | 3,552E-02 | 0,37 | 1,167E-02 | 3,521E-02 |
| SEQ ID NO: 208 | hsa-miR-520c-3p | 1 | 16 | 0,06 | -2,781 | 8,017E-03 | 3,552E-02 | 0,32 | 1,201E-04 | 1,069E-03 |
| SEQ ID NO: 209 | hsa-miR-548b-5p | 1 | 30 | 0,03 | -3,405 | 8,247E-03 | 3,613E-02 | 0,31 | 2,687E-05 | 3,361E-04 |
| SEQ ID NO: 210 | hsa-miR-519a | 31 | 53 | 0,59 | -0,520 | 8,717E-03 | 3,761E-02 | 0,31 | 3,668E-03 | 1,409E-02 |
| SEQ ID NO: 211 | hsa-miR-605 | 11 | 28 | 0,40 | -0,921 | 8,887E-03 | 3,816E-02 | 0,38 | 1,775E-02 | 4,733E-02 |
| SEQ ID NO: 212 | hsa-miR-323-3p | 33 | 52 | 0,63 | -0,459 | 9,041E-03 | 3,824E-02 | 0,35 | 1,134E-02 | 3,457E-02 |
| SEQ ID NO: 213 | hsa-miR-1258 | 1 | 1 | 1,00 | 0,000 | 9,193E-03 | 3,824E-02 | 0,39 | 2,209E-02 | 5,624E-02 |
| SEQ ID NO: 214 | hsa-miR-891b | 95 | 53 | 1,79 | 0,582 | 9,094E-03 | 3,824E-02 | 0,73 | 2,008E-03 | 8,932E-03 |
| SEQ ID NO: 215 | hsa-miR-409-3p | 36 | 59 | 0,61 | -0,502 | 9,186E-03 | 3,824E-02 | 0,34 | 4,957E-03 | 1,760E-02 |
| SEQ ID NO: 216 | hsa-miR-130b* | 1 | 31 | 0,03 | -3,442 | 9,062E-03 | 3,824E-02 | 0,26 | 3,352E-06 | 6,888E-05 |
| SEQ ID NO: 217 | hsa-miR-148a* | 25 | 52 | 0,48 | -0,738 | 9,449E-03 | 3,828E-02 | 0,31 | 7,261E-04 | 4,128E-03 |
| SEQ ID NO: 218 | hsa-miR-541* | 1 | 30 | 0,03 | -3,403 | 9,356E-03 | 3,828E-02 | 0,34 | 2,060E-03 | 9,040E-03 |
| SEQ ID NO: 219 | hsa-miR-1290 | 1 | 1 | 1,00 | 0,000 | 9,362E-03 | 3,828E-02 | 0,36 | 7,623E-04 | 4,272E-03 |
| SEQ ID NO: 220 | hsa-miR-559 | 48 | 54 | 0,89 | -0,115 | 9,550E-03 | 3,851E-02 | 0,37 | 3,864E-02 | 8,420E-02 |
| SEQ ID NO: 221 | hsa-miR-27a* | 34 | 51 | 0,67 | -0,405 | 9,630E-03 | 3,865E-02 | 0,36 | 1,049E-02 | 3,262E-02 |
| SEQ ID NO: 222 | hsa-miR-1276 | 39 | 52 | 0,75 | -0,285 | 9,789E-03 | 3,911E-02 | 0,36 | 1,743E-02 | 4,729E-02 |
| SEQ ID NO: 223 | hsa-miR-563 | 2 | 14 | 0,15 | -1,895 | 1,002E-02 | 3,968E-02 | 0,38 | 1,770E-02 | 4,733E-02 |
| SEQ ID NO: 224 | hsa-miR-615-3p | 28 | 50 | 0,56 | -0,577 | 1,061E-02 | 4,181E-02 | 0,32 | 2,708E-03 | 1,112E-02 |
| SEQ ID NO: 225 | hsa-miR-208b | 60 | 50 | 1,20 | 0,180 | 1,086E-02 | 4,240E-02 | 0,64 | 6,227E-03 | 2,167E-02 |
| SEQ ID NO: 226 | hsa-miR-409-5p | 78 | 58 | 1,35 | 0,299 | 1,200E-02 | 4,601E-02 | 0,62 | 8,921E-02 | 1,621E-01 |
| SEQ ID NO: 227 | hsa-miR-369-3p | 16 | 37 | 0,43 | -0,839 | 1,205E-02 | 4,603E-02 | 0,36 | 1,485E-02 | 4,230E-02 |
| SEQ ID NO: 228 | hsa-miR-886-3p | 1 | 23 | 0,04 | -3,121 | 1,264E-02 | 4,784E-02 | 0,34 | 1,873E-03 | 8,463E-03 |
| SEQ ID NO: 229 | hsa-miR-501-5p | 40 | 66 | 0,60 | -0,508 | 1,275E-02 | 4,806E-02 | 0,36 | 7,752E-02 | 1,451E-01 |
| SEQ ID NO: 230 | hsa-miR-758 | 12 | 28 | 0,44 | -0,826 | 1,292E-02 | 4,848E-02 | 0,40 | 2,317E-02 | 5,829E-02 |
| SEQ ID NO: 231 | hsa-miR-9 | 1 | 17 | 0,06 | -2,860 | 1,320E-02 | 4,933E-02 | 0,38 | 1,220E-02 | 3,630E-02 |

# Figure 2

| SEQ ID NO: | miRNA | miRNA-sequence |
|---|---|---|
| SEQ ID NO: 1 | hsa-miR-363 | aauugcacgguauccaucugua |
| SEQ ID NO: 2 | hsa-miR-151-3p | cuagacugaagcuccuugagg |
| SEQ ID NO: 3 | hsa-miR-17 | caaagugcuuacagugcagguag |
| SEQ ID NO: 4 | hsa-miR-20b | caaagugcucauagugcagguag |
| SEQ ID NO: 5 | hsa-miR-103 | agcagcauuguacagggcuauga |
| SEQ ID NO: 6 | hsa-miR-106a | aaaagugcuuacagugcagguag |
| SEQ ID NO: 7 | hsa-miR-101 | uacaguacugugauaacugaa |
| SEQ ID NO: 8 | hsa-miR-20a | uaaagugcuuauagugcagguag |
| SEQ ID NO: 9 | hsa-let-7d | agagguaguagguugcauaguu |
| SEQ ID NO: 10 | hsa-miR-320d | aaaagcugggguugagagga |
| SEQ ID NO: 11 | hsa-miR-324-3p | acugccccaggugcugcugg |
| SEQ ID NO: 12 | hsa-miR-15b | uagcagcacaucaugguuuaca |
| SEQ ID NO: 13 | hsa-miR-148b | ucagugcaucacagaacuuugu |
| SEQ ID NO: 14 | hsa-miR-550* | ugucuuacucccucaggcacau |
| SEQ ID NO: 15 | hsa-miR-30d | uguaaacauccccgacuggaag |
| SEQ ID NO: 16 | hsa-miR-26a | uucaaguaauccaggauaggcu |
| SEQ ID NO: 17 | hsa-miR-107 | agcagcauuguacagggcuauca |
| SEQ ID NO: 18 | hsa-miR-320b | aaaagcuggguugagagggcaa |
| SEQ ID NO: 19 | hsa-miR-24 | uggcucaguucagcaggaacag |
| SEQ ID NO: 20 | hsa-miR-18a | uaaggugcaucuagugcagauag |
| SEQ ID NO: 21 | hsa-miR-320c | aaaagcuggguugagagggu |
| SEQ ID NO: 22 | hsa-miR-744 | ugcggggcuagggcuaacagca |
| SEQ ID NO: 23 | hsa-miR-93 | caaagugcuguucgugcagguag |
| SEQ ID NO: 24 | hsa-miR-30c | uguaaacauccuacacucucagc |
| SEQ ID NO: 25 | hsa-miR-106b | uaaagugcugacagugcagau |
| SEQ ID NO: 26 | hsa-miR-182 | uuuggcaaugguagaacucacacu |
| SEQ ID NO: 27 | hsa-miR-320a | aaaagcuggguugagagggcga |
| SEQ ID NO: 28 | hsa-miR-720 | ucucgcuggggccucca |
| SEQ ID NO: 29 | hsa-miR-30e | uguaaacauccuugacuggaag |
| SEQ ID NO: 30 | hsa-miR-664 | uauucauuuauccccagccuaca |
| SEQ ID NO: 31 | hsa-miR-16 | uagcagcacguaaauauuggcg |
| SEQ ID NO: 32 | hsa-miR-660 | uacccauugcauaucggaguug |
| SEQ ID NO: 33 | hsa-miR-130a | cagugcaauguuaaaagggcau |
| SEQ ID NO: 34 | hsa-miR-148a | ucagugcacuacagaacuuugu |
| SEQ ID NO: 35 | hsa-miR-93* | acugcugagcuagcacuucccg |
| SEQ ID NO: 36 | hsa-miR-423-5p | ugaggggcagagagcgagacuuu |
| SEQ ID NO: 37 | hsa-miR-17* | acugcagugaaggcacuuguag |
| SEQ ID NO: 38 | hsa-let-7a | ugagguaguagguuguauaguu |
| SEQ ID NO: 39 | hsa-miR-331-3p | gccccugggccuauccuagaa |
| SEQ ID NO: 40 | hsa-miR-192 | cugaccuaugaauugacagcc |
| SEQ ID NO: 41 | hsa-miR-29b | uagcaccauuugaaaucaguguu |
| SEQ ID NO: 42 | hsa-miR-30a | uguaaacauccucgacuggaag |

| | | |
|---|---|---|
| SEQ ID NO: 43 | hsa-miR-28-5p | aaggagcucacagucuauugag |
| SEQ ID NO: 44 | hsa-miR-1908 | cggcggggacggcgauugguc |
| SEQ ID NO: 45 | hsa-miR-1260 | aucccaccucugccacca |
| SEQ ID NO: 46 | hsa-miR-197 | uucaccaccuucuccacccagc |
| SEQ ID NO: 47 | hsa-miR-532-3p | ccucccacacccaaggcuugca |
| SEQ ID NO: 48 | hsa-miR-500 | uaauccuugcuaccugggugaga |
| SEQ ID NO: 49 | hsa-miR-223 | ugucaguuugucaaauacccca |
| SEQ ID NO: 50 | hsa-miR-195 | uagcagcacagaaauauuggc |
| SEQ ID NO: 51 | hsa-miR-342-3p | ucucacacagaaaucgcacccgu |
| SEQ ID NO: 52 | hsa-miR-25 | cauugcacuugucucggucuga |
| SEQ ID NO: 53 | hsa-miR-222 | agcuacaucuggcuacugggu |
| SEQ ID NO: 54 | hsa-miR-1280 | ucccaccgcugccaccc |
| SEQ ID NO: 55 | hsa-miR-181a | aacauucaacgcugucggugagu |
| SEQ ID NO: 56 | hsa-miR-194 | uguaacagcaacuccaugugga |
| SEQ ID NO: 57 | hsa-miR-199a-5p | cccaguguucagacuaccuguuc |
| SEQ ID NO: 58 | hsa-miR-125b | ucccugagacccuaacuuguga |
| SEQ ID NO: 59 | hsa-miR-339-5p | ucccuguccuccaggagcucacg |
| SEQ ID NO: 60 | hsa-miR-484 | ucaggcucaguccccucccgau |
| SEQ ID NO: 61 | hsa-miR-574-3p | cacgcucaugcacacacccaca |
| SEQ ID NO: 62 | hsa-miR-29a | uagcaccaucugaaaucgguua |
| SEQ ID NO: 63 | hsa-miR-15a | uagcagcacauaaugguuugug |
| SEQ ID NO: 64 | hsa-miR-151-5p | ucgaggagcucacagucuagu |
| SEQ ID NO: 65 | hsa-miR-574-5p | ugagugugugugugugagugugu |
| SEQ ID NO: 66 | hsa-miR-1913 | ucugcccccuccgcugcugcca |
| SEQ ID NO: 67 | hsa-miR-425 | aaugacacgaucacucccguuga |
| SEQ ID NO: 68 | hsa-miR-144 | uacaguauagaugauguacu |
| SEQ ID NO: 69 | hsa-miR-126 | ucguaccgugaguaauaaugcg |
| SEQ ID NO: 70 | hsa-miR-144* | ggauaucaucauauacuguaag |
| SEQ ID NO: 71 | hsa-miR-374b | auauaauacaaccugcuaagug |
| SEQ ID NO: 72 | hsa-miR-21 | uagcuuaucagacugauguuga |
| SEQ ID NO: 73 | hsa-miR-301a | cagugcaauaguauugucaaagc |
| SEQ ID NO: 74 | hsa-let-7i | ugagguaguaguuugugcuguu |
| SEQ ID NO: 75 | hsa-let-7f | ugagguaguagauuguauaguu |
| SEQ ID NO: 76 | hsa-miR-18a* | acugcccuaagugcuccuucugg |
| SEQ ID NO: 77 | hsa-miR-183 | uauggcacugguagaauucacu |
| SEQ ID NO: 78 | hsa-miR-1202 | gugccagcugcagugggggag |
| SEQ ID NO: 79 | hsa-miR-503 | uagcagcgggaacaguucugcag |
| SEQ ID NO: 80 | hsa-miR-593* | aggcaccagccaggcauugcucagc |
| SEQ ID NO: 81 | hsa-miR-646 | aagcagcugccucugaggc |
| SEQ ID NO: 82 | hsa-miR-100 | aacccguagauccgaacuugug |
| SEQ ID NO: 83 | hsa-miR-125a-5p | ucccugagacccuuuaaccuguga |
| SEQ ID NO: 84 | hsa-miR-145 | guccaguuuucccaggaaucccu |
| SEQ ID NO: 85 | hsa-let-7i* | cugcgcaagcuacugccuugcu |
| SEQ ID NO: 86 | hsa-miR-424 | cagcagcaauucauguuuugaa |
| SEQ ID NO: 87 | hsa-miR-181c | aacauucaaccugucggugagu |
| SEQ ID NO: 88 | hsa-miR-152 | ucagugcaugacagaacuugg |

| | | |
|---|---|---|
| SEQ ID NO: 89 | hsa-miR-214 | acagcaggcacagacaggcagu |
| SEQ ID NO: 90 | hsa-miR-362-5p | aauccuuggaaccuaggugugagu |
| SEQ ID NO: 91 | hsa-miR-532-5p | caugccuugaguguaggaccgu |
| SEQ ID NO: 92 | hsa-miR-1226* | gugagggcaugcaggccuggaugggg |
| SEQ ID NO: 93 | hsa-miR-1184 | ccugcagcgacuugauggcuucc |
| SEQ ID NO: 94 | hsa-miR-31* | ugcuaugccaacauauugccau |
| SEQ ID NO: 95 | hsa-miR-1912 | uacccagagcaugcagugugaa |
| SEQ ID NO: 96 | hsa-miR-1203 | cccggagccaggaugcagcuc |
| SEQ ID NO: 97 | hsa-miR-1225-3p | ugagccccugugccgcccccag |
| SEQ ID NO: 98 | hsa-miR-517* | ccucuagauggaagcacugucu |
| SEQ ID NO: 99 | hsa-miR-515-5p | uucuccaaaagaaagcacuuucug |
| SEQ ID NO: 100 | hsa-miR-431 | ugucuugcaggccgucaugca |
| SEQ ID NO: 101 | hsa-miR-188-3p | cucccacaugcaggguuugca |
| SEQ ID NO: 102 | hsa-miR-217 | uacugcaucaggaacugauugga |
| SEQ ID NO: 103 | hsa-miR-940 | aaggcagggcccccgcucccc |
| SEQ ID NO: 104 | hsa-miR-1826 | auugaucaucgacacuucgaacgcaau |
| SEQ ID NO: 105 | hsa-miR-27b | uucacaguggcuaaguucugc |
| SEQ ID NO: 106 | hsa-miR-26b | uucaaguaauucaggauaggu |
| SEQ ID NO: 107 | hsa-miR-27a | uucacaguggcuaaguuccgc |
| SEQ ID NO: 108 | hsa-let-7g | ugagguaguaguuuguacaguu |
| SEQ ID NO: 109 | hsa-miR-374a | uuauaauacaaccugauaagug |
| SEQ ID NO: 110 | hsa-miR-1301 | uugcagcugccugggagugacuuc |
| SEQ ID NO: 111 | hsa-miR-509-3-5p | uacugcagacguggcaaucaug |
| SEQ ID NO: 112 | hsa-miR-584 | uuaugguuugccugggacugag |
| SEQ ID NO: 113 | hsa-miR-486-3p | cggggcagcucaguacaggau |
| SEQ ID NO: 114 | hsa-miR-96 | uuuggcacuagcacauuuuugcu |
| SEQ ID NO: 115 | hsa-miR-1272 | gaugaugauggcagcaaauucugaaa |
| SEQ ID NO: 116 | hsa-miR-143 | ugagaugaagcacuguagcuc |
| SEQ ID NO: 117 | hsa-miR-588 | uuggccacaaugggguuagaac |
| SEQ ID NO: 118 | hsa-miR-24-2* | ugccuacugagcugaaacacag |
| SEQ ID NO: 119 | hsa-miR-541 | uggugggcacagaaucuggacu |
| SEQ ID NO: 120 | hsa-miR-1254 | agccuggaagcuggagccugcagu |
| SEQ ID NO: 121 | hsa-miR-628-3p | ucuaguaagaguggcagucga |
| SEQ ID NO: 122 | hsa-miR-143* | ggugcagugcugcaucucuggu |
| SEQ ID NO: 123 | hsa-miR-654-5p | ugguggccgcagaacaugugc |
| SEQ ID NO: 124 | hsa-miR-1286 | ugcaggaccaagaugagcccu |
| SEQ ID NO: 125 | hsa-miR-18b | uaaggugcaucuagugcaguuag |
| SEQ ID NO: 126 | hsa-miR-550 | agugccugagggaguaagagccc |
| SEQ ID NO: 127 | hsa-let-7d* | cuauacgaccugcugccuuucu |
| SEQ ID NO: 128 | hsa-miR-220c | acacagggcuguugugaagacu |
| SEQ ID NO: 129 | hsa-miR-33b | gugcauugcuguugcauugc |
| SEQ ID NO: 130 | hsa-miR-575 | gagccaguuggacaggagc |
| SEQ ID NO: 131 | hsa-miR-31 | aggcaagaugcuggcauagcu |
| SEQ ID NO: 132 | hsa-miR-455-3p | gcaguccaugggcauauacac |
| SEQ ID NO: 133 | hsa-miR-1283 | ucuacaaaggaaagcgcuuucu |
| SEQ ID NO: 134 | hsa-miR-1914* | ggaggggucccgcacugggagg |

| | | |
|---|---|---|
| SEQ ID NO: 135 | hsa-miR-196a* | cggcaacaagaaacugccugag |
| SEQ ID NO: 136 | hsa-miR-595 | gaagugugccgugguguguc u |
| SEQ ID NO: 137 | hsa-miR-192* | cugccaauuccauaggucacag |
| SEQ ID NO: 138 | hsa-miR-16-2* | ccaauauuacugugcugcuuua |
| SEQ ID NO: 139 | hsa-miR-96* | aaucaugugcagugccaauaug |
| SEQ ID NO: 140 | hsa-miR-33a | gugcauuguaguugcauugca |
| SEQ ID NO: 141 | hsa-miR-650 | aggaggcagcgcucucaggac |
| SEQ ID NO: 142 | hsa-miR-1295 | uuaggccgcagaucugggu ga |
| SEQ ID NO: 143 | hsa-miR-1266 | ccucagggcuguagaacagggcu |
| SEQ ID NO: 144 | hsa-miR-32 | uauugcacauuacuaaguugca |
| SEQ ID NO: 145 | hsa-miR-338-3p | uccagcaucagugauuuuguug |
| SEQ ID NO: 146 | hsa-miR-1251 | acucuagcugccaaaggcgcu |
| SEQ ID NO: 147 | hsa-miR-328 | cuggcccucucugcccuuccgu |
| SEQ ID NO: 148 | hsa-miR-33b* | cagugccucggcagugcagccc |
| SEQ ID NO: 149 | hsa-miR-631 | agaccuggcccagaccucagc |
| SEQ ID NO: 150 | hsa-miR-523* | cucuagagggaagcgcuuucug |
| SEQ ID NO: 151 | hsa-miR-187* | ggcuacaacacaggacccgggc |
| SEQ ID NO: 152 | hsa-miR-132 | uaacagucuacagccauggucg |
| SEQ ID NO: 153 | hsa-miR-146b-5p | ugagaacugaauuccauaggcu |
| SEQ ID NO: 154 | hsa-miR-454 | uagugcaauauugcuuauagggu |
| SEQ ID NO: 155 | hsa-miR-298 | agcagaagcagggagguucuccca |
| SEQ ID NO: 156 | hsa-miR-518e* | cucuagagggaagcgcuuucug |
| SEQ ID NO: 157 | hsa-miR-15a* | caggccauauugugcugccuca |
| SEQ ID NO: 158 | hsa-let-7e | ugagguaggagguuguauaguu |
| SEQ ID NO: 159 | hsa-miR-199a-3p | acaguagucugcacauugguua |
| SEQ ID NO: 160 | hsa-miR-525-5p | cuccagagggaugcacuuucu |
| SEQ ID NO: 161 | hsa-miR-516b* | ugcuuccuuucagagggu |
| SEQ ID NO: 162 | hsa-miR-199b-3p | acaguagucugcacauugguua |
| SEQ ID NO: 163 | hsa-miR-934 | ugucuacuacuggagacacugg |
| SEQ ID NO: 164 | hsa-miR-335* | uuuuucauuauugcuccugacc |
| SEQ ID NO: 165 | hsa-miR-505 | cgucaacacuugcugguuuccu |
| SEQ ID NO: 166 | hsa-miR-885-5p | uccauuacacuacccugccucu |
| SEQ ID NO: 167 | hsa-miR-129* | aagcccuuaccccaaaaaguau |
| SEQ ID NO: 168 | hsa-miR-512-3p | aagugcugucauagcugagguc |
| SEQ ID NO: 169 | hsa-miR-139-3p | ggagacgcggcccuguuggagu |
| SEQ ID NO: 170 | hsa-miR-604 | aggcugcggaauucaggac |
| SEQ ID NO: 171 | hsa-miR-29c* | ugaccgauuucuccugguguuc |
| SEQ ID NO: 172 | hsa-miR-302b | uaagugcuuccauguuuuaguag |
| SEQ ID NO: 173 | hsa-miR-124* | cguguucacagcggaccuugau |
| SEQ ID NO: 174 | hsa-miR-548c-3p | caaaaaucucaauuacuuuugc |
| SEQ ID NO: 175 | hsa-miR-200c* | cgucuuacccagcaguguuugg |
| SEQ ID NO: 176 | hsa-miR-1911* | caccaggcauuguggucucc |
| SEQ ID NO: 177 | hsa-miR-517c | aucgugcauccuuuuagagugu |
| SEQ ID NO: 178 | hsa-miR-1262 | augggugaauuuguagaaggau |
| SEQ ID NO: 179 | hsa-miR-548g | aaaacuguaauuacuuuuguac |
| SEQ ID NO: 180 | hsa-miR-502-5p | auccuugcuaucugggugcua |

| | | |
|---|---|---|
| SEQ ID NO: 181 | hsa-miR-490-3p | caaccuggaggacuccaugcug |
| SEQ ID NO: 182 | hsa-miR-483-3p | ucacuccucuccucccgucuu |
| SEQ ID NO: 183 | hsa-miR-187 | ucgugucuuguguugcagccgg |
| SEQ ID NO: 184 | hsa-miR-519b-3p | aaagugcauccuuuuagagguu |
| SEQ ID NO: 185 | hsa-let-7c* | uagaguuacacccugggaguua |
| SEQ ID NO: 186 | hsa-miR-649 | aaaccuguguuguucaagaguc |
| SEQ ID NO: 187 | hsa-miR-517a | aucgugcaucccuuuagagugu |
| SEQ ID NO: 188 | hsa-miR-630 | aguauucuguaccagggaaggu |
| SEQ ID NO: 189 | hsa-miR-126* | cauuauuacuuuugguacgcg |
| SEQ ID NO: 190 | hsa-miR-656 | aauauuauacagucaaccucu |
| SEQ ID NO: 191 | hsa-miR-569 | aguuaaugaauccuggaaagu |
| SEQ ID NO: 192 | hsa-miR-632 | gugucugcuuccuguggga |
| SEQ ID NO: 193 | hsa-miR-378* | cuccugacuccagguccugugu |
| SEQ ID NO: 194 | hsa-miR-553 | aaaacggugagauuuuguuuu |
| SEQ ID NO: 195 | hsa-miR-137 | uuauugcuuaagaauacgcguag |
| SEQ ID NO: 196 | hsa-miR-1183 | cacuguaggugauggugagagugggca |
| SEQ ID NO: 197 | hsa-miR-556-3p | auauuaccauuagcucaucuuu |
| SEQ ID NO: 198 | hsa-miR-190b | ugauauguuugauauuggguu |
| SEQ ID NO: 199 | hsa-miR-1915* | accuugccuugcugcccgggcc |
| SEQ ID NO: 200 | hsa-miR-186 | caaagaauucuccuuuugggcu |
| SEQ ID NO: 201 | hsa-miR-1468 | cuccguuugccuguuucgcug |
| SEQ ID NO: 202 | hsa-miR-454* | acccuaucaauauugucucugc |
| SEQ ID NO: 203 | hsa-miR-1305 | uuuucaacucuaaugggagaga |
| SEQ ID NO: 204 | hsa-miR-617 | agacuucccauuugaagguggc |
| SEQ ID NO: 205 | hsa-miR-551b* | gaaaucaagcgugggugagacc |
| SEQ ID NO: 206 | hsa-miR-1206 | uguucauguagauguuuaagc |
| SEQ ID NO: 207 | hsa-miR-379 | ugguagacuauggaacguagg |
| SEQ ID NO: 208 | hsa-miR-520c-3p | aaagugcuuccuuuuagagggu |
| SEQ ID NO: 209 | hsa-miR-548b-5p | aaaaguaauugugguuuuggcc |
| SEQ ID NO: 210 | hsa-miR-519a | aaagugcauccuuuuagagugu |
| SEQ ID NO: 211 | hsa-miR-605 | uaaaucccaugguggccuucuccu |
| SEQ ID NO: 212 | hsa-miR-323-3p | cacauuacacggucgaccucu |
| SEQ ID NO: 213 | hsa-miR-1258 | aguuaggauuaggucguggaa |
| SEQ ID NO: 214 | hsa-miR-891b | ugcaacuuaccugagucauuga |
| SEQ ID NO: 215 | hsa-miR-409-3p | gaauguugcucggugaaccccu |
| SEQ ID NO: 216 | hsa-miR-130b* | acucuuucccuguugcacuac |
| SEQ ID NO: 217 | hsa-miR-148a* | aaaguucugagacacuccgacu |
| SEQ ID NO: 218 | hsa-miR-541* | aaaggauucugcugucggucccacu |
| SEQ ID NO: 219 | hsa-miR-1290 | uggauuuuuggaucaggga |
| SEQ ID NO: 220 | hsa-miR-559 | uaaaguaaauaugcaccaaaa |
| SEQ ID NO: 221 | hsa-miR-27a* | agggcuuagcugcuugugagca |
| SEQ ID NO: 222 | hsa-miR-1276 | uaaagagcccuguggagaca |
| SEQ ID NO: 223 | hsa-miR-563 | agguugacauacguuuccc |
| SEQ ID NO: 224 | hsa-miR-615-3p | uccgagccugggucucccucuu |
| SEQ ID NO: 225 | hsa-miR-208b | auaagacgaacaaaagguuugu |
| SEQ ID NO: 226 | hsa-miR-409-5p | agguuacccgagcaacuuugcau |

| | | |
|---|---|---|
| SEQ ID NO: 227 | hsa-miR-369-3p | aauaauacaugguugaucuuu |
| SEQ ID NO: 228 | hsa-miR-886-3p | cgcgggugcuuacugacccuu |
| SEQ ID NO: 229 | hsa-miR-501-5p | aauccuuugucccugggugaga |
| SEQ ID NO: 230 | hsa-miR-758 | uuugugaccugguccacuaacc |
| SEQ ID NO: 231 | hsa-miR-9 | ucuuugguuaucuagcuguauga |
| SEQ ID NO: 232 | hsa-miR-149* | agggagggacgggggcugugc |
| SEQ ID NO: 233 | hsa-miR-130b | cagugcaaugaugaaagggcau |
| SEQ ID NO: 234 | hsa-miR-30b | uguaaacauccuacacucagcu |
| SEQ ID NO: 235 | hsa-miR-1271 | cuuggcaccuagcaagcacuca |
| SEQ ID NO: 236 | hsa-miR-1274b | ucccuguucgggcgcca |
| SEQ ID NO: 237 | hsa-miR-19b | ugugcaaauccaugcaaaacuga |
| SEQ ID NO: 238 | hsa-miR-185 | uggagagaaaggcaguuccuga |
| SEQ ID NO: 239 | hsa-miR-142-5p | cauaaaguagaaagcacuacu |
| SEQ ID NO: 240 | hsa-miR-675 | uggugcggagagggcccacagug |
| SEQ ID NO: 241 | hsa-miR-210 | cugugcgugugacagcggcuga |
| SEQ ID NO: 242 | hsa-miR-1469 | cucggcgcggggcgcgggcucc |
| SEQ ID NO: 243 | hsa-miR-627 | gugagucucuaagaaaagagga |
| SEQ ID NO: 244 | hsa-miR-23a | aucacauugccagggauuucc |
| SEQ ID NO: 245 | hsa-miR-139-5p | ucuacagugcacgugucuccag |
| SEQ ID NO: 246 | hsa-miR-486-5p | uccuguacugagcugccccgag |
| SEQ ID NO: 247 | hsa-miR-625 | agggggaaaguucuauagucc |
| SEQ ID NO: 248 | hsa-miR-1224-3p | ccccaccuccucucucccucag |
| SEQ ID NO: 249 | hsa-miR-193a-5p | ugggucuuugcgggcgagauga |
| SEQ ID NO: 250 | hsa-miR-342-5p | aggggugcuaucugugauuga |
| SEQ ID NO: 251 | hsa-miR-659 | cuugguucagggagggucccca |
| SEQ ID NO: 252 | hsa-miR-877 | guagaggagauggcgcaggg |
| SEQ ID NO: 253 | hsa-miR-21* | caacaccagucgaugggcugu |
| SEQ ID NO: 254 | hsa-miR-1281 | ucgccuccuccucuccc |
| SEQ ID NO: 255 | hsa-miR-369-5p | agaucgaccguguuauauucgc |
| SEQ ID NO: 256 | hsa-miR-182* | ugguucuagacuugccaacua |
| SEQ ID NO: 257 | hsa-miR-136 | acuccauuuguuuugaugaugga |
| SEQ ID NO: 258 | hsa-miR-1279 | ucauauugcuucuuucu |
| SEQ ID NO: 259 | hsa-miR-520e | aaagugcuuccuuuuugaggg |
| SEQ ID NO: 260 | hsa-let-7b* | cuauacaaccuacugccuuccc |
| SEQ ID NO: 261 | hsa-miR-154* | aaucauacacgguugaccuauu |
| SEQ ID NO: 262 | hsa-miR-450b-3p | uugggaucauuuugcauccaua |
| SEQ ID NO: 263 | hsa-miR-562 | aaaguagcuguaccauuugc |
| SEQ ID NO: 264 | hsa-miR-493 | ugaaggucuacugugugccagg |
| SEQ ID NO: 265 | hsa-miR-548m | caaagguauuuguggguuuuug |
| SEQ ID NO: 266 | hsa-miR-1264 | caagucuuauuugagcaccuguu |
| SEQ ID NO: 267 | hsa-miR-557 | guuugcacgggugggccuugucu |
| SEQ ID NO: 268 | hsa-miR-508-3p | ugauuguagccuuuuggaguaga |
| SEQ ID NO: 269 | hsa-miR-935 | ccaguuaccgcuuccgcuaccgc |
| SEQ ID NO: 270 | hsa-let-7f-2* | cuauacagucuacugucuuucc |
| SEQ ID NO: 271 | hsa-miR-526b* | gaaagugcuuccuuuuagaggc |
| SEQ ID NO: 272 | hsa-miR-548c-5p | aaaaguaauugcgguuuuugcc |

| | | |
|---|---|---|
| SEQ ID NO: 273 | hsa-miR-519e* | uucuccaaaagggagcacuuuc |
| SEQ ID NO: 274 | hsa-miR-220b | ccaccaccgugucugacacuu |
| SEQ ID NO: 275 | hsa-miR-1284 | ucuauacagacccuggcuuuuc |
| SEQ ID NO: 276 | hsa-miR-26a-1* | ccuauucuugguuacuugcacg |
| SEQ ID NO: 277 | hsa-miR-302a* | acuuaaacguggauguacuugcu |
| SEQ ID NO: 278 | hsa-miR-204 | uucccuuugucauccuaugccu |
| SEQ ID NO: 279 | hsa-miR-373 | gaagugcuucgauuuuggggugu |
| SEQ ID NO: 280 | hsa-miR-23a* | gggguuccuggggaugggauuu |
| SEQ ID NO: 281 | hsa-miR-302f | uaauugcuuccauguuu |
| SEQ ID NO: 282 | hsa-miR-637 | acugggggcuuucgggcucugcgu |
| SEQ ID NO: 283 | hsa-miR-331-5p | cuagguauggucccagggaucc |
| SEQ ID NO: 284 | hsa-miR-585 | ugggcguaucuguaugcua |
| SEQ ID NO: 285 | hsa-miR-548h | aaaaguaaucgcgguuuuuguc |
| SEQ ID NO: 286 | hsa-miR-507 | uuuugcaccuuuuggagugaa |
| SEQ ID NO: 287 | hsa-miR-539 | ggagaaauuauccuuggugugu |
| SEQ ID NO: 288 | hsa-miR-376a* | guagauucuccuucuaugagua |
| SEQ ID NO: 289 | hsa-miR-521 | aacgcacuucccuuuagagugu |
| SEQ ID NO: 290 | hsa-miR-519e | aagugccuccuuuuagaguguu |
| SEQ ID NO: 291 | hsa-miR-338-5p | aacaauauccuggugcugagug |
| SEQ ID NO: 292 | hsa-miR-548j | aaaaguaauugcggucuuuggu |
| SEQ ID NO: 293 | hsa-miR-1256 | aggcauugacuucucacuagcu |
| SEQ ID NO: 294 | hsa-miR-618 | aaacucuacuuguccuucugagu |
| SEQ ID NO: 295 | hsa-miR-135b | uauggcuuuucauuccuauguga |
| SEQ ID NO: 296 | hsa-miR-219-2-3p | agaauuguggcuggacaucugu |
| SEQ ID NO: 297 | hsa-miR-944 | aaauuauuguacaucggaugag |
| SEQ ID NO: 298 | hsa-miR-509-3p | ugauugguacgucugugggguag |
| SEQ ID NO: 299 | hsa-miR-515-3p | gagugccuucuuuuggagcguu |
| SEQ ID NO: 300 | hsa-miR-548i | aaaaguaauugcggauuuugcc |
| SEQ ID NO: 301 | hsa-let-7e* | cuauacggccuccuagcuuucc |
| SEQ ID NO: 302 | hsa-miR-154 | uagguuauccguguugccuucg |
| SEQ ID NO: 303 | hsa-miR-374b* | cuuagcagguuguauuaucauu |
| SEQ ID NO: 304 | hsa-miR-130a* | uucacauugugcuacugucugc |
| SEQ ID NO: 305 | hsa-miR-1178 | uugcucacuguucuucccuag |
| SEQ ID NO: 306 | hsa-miR-26a-2* | ccuauucuugauuacuuguuuc |
| SEQ ID NO: 307 | hsa-miR-302a | uaagugcuuccauguuuugguga |
| SEQ ID NO: 308 | hsa-miR-135a | uauggcuuuuuauuccuauguga |
| SEQ ID NO: 309 | hsa-miR-377* | agagguugcccuuggugaauuc |
| SEQ ID NO: 310 | hsa-miR-522 | aaaaugguucccuuuagagugu |
| SEQ ID NO: 311 | hsa-miR-1244 | aaguaguugguuuguaugagaugguu |
| SEQ ID NO: 312 | hsa-miR-220a | ccacaccguaucugacacuuu |
| SEQ ID NO: 313 | hsa-miR-655 | auaauacaugguuaaccucuuu |
| SEQ ID NO: 314 | hsa-miR-214* | ugccugucuacacuugcugugc |
| SEQ ID NO: 315 | hsa-miR-196b | uagguaguuuccuguuguuggg |
| SEQ ID NO: 316 | hsa-miR-205 | uccuucauuccaccggagucug |
| SEQ ID NO: 317 | hsa-miR-708* | caacuagacugugagcuucuag |
| SEQ ID NO: 318 | hsa-miR-132* | accguggcuuucgauuguuacu |

| | | |
|---|---|---|
| SEQ ID NO: 319 | hsa-miR-372 | aaagugcugcgacauuugagcgu |
| SEQ ID NO: 320 | hsa-miR-615-5p | gggggucccggugcucggauc |
| SEQ ID NO: 321 | hsa-miR-520f | aagugcuuccuuuuagaggguu |
| SEQ ID NO: 322 | hsa-miR-196a | uagguaguuucauguuguuggg |
| SEQ ID NO: 323 | hsa-miR-561 | caaaguuuaagauccuugaagu |
| SEQ ID NO: 324 | hsa-miR-548l | aaaaguauuugcggguuuuguc |
| SEQ ID NO: 325 | hsa-miR-1257 | agugaaugaugggguucugacc |
| SEQ ID NO: 326 | hsa-miR-548a-5p | aaaaguaauugcgaguuuuacc |
| SEQ ID NO: 327 | hsa-miR-653 | guguugaaacaaucucuacug |
| SEQ ID NO: 328 | hsa-miR-29a* | acugauuucuuuugguguucag |
| SEQ ID NO: 329 | hsa-miR-371-3p | aagugccgccaucuuuugagugu |
| SEQ ID NO: 330 | hsa-miR-1268 | cgggcgugguggugggg |
| SEQ ID NO: 331 | hsa-miR-92a | uauugcacuugucccggccugu |
| SEQ ID NO: 332 | hsa-miR-590-5p | gagcuuauucauaaaagugcag |
| SEQ ID NO: 333 | hsa-miR-1228* | gugggcgggggcaggugugug |
| SEQ ID NO: 334 | hsa-miR-361-5p | uuaucagaaucuccaggggguac |
| SEQ ID NO: 335 | hsa-miR-23b | aucacauugccagggauuacc |
| SEQ ID NO: 336 | hsa-miR-1234 | ucggccugaccacccaccccac |
| SEQ ID NO: 337 | hsa-miR-92b | uauugcacucgucccggccucc |
| SEQ ID NO: 338 | hsa-miR-29c | uagcaccauuugaaaucgguua |
| SEQ ID NO: 339 | hsa-miR-1273 | gggcgacaaagcaagacucuuucuu |
| SEQ ID NO: 340 | hsa-miR-383 | agaucagaaggugauuguggcu |
| SEQ ID NO: 341 | hsa-miR-636 | ugugcuugcucgucccgcccgca |
| SEQ ID NO: 342 | hsa-miR-146a* | ccucugaaauucaguucuucag |
| SEQ ID NO: 343 | hsa-miR-548p | uagcaaaaacugcaguuacuuu |
| SEQ ID NO: 344 | hsa-miR-641 | aaagacauaggauagagucaccuc |
| SEQ ID NO: 345 | hsa-miR-221* | accuggcauacaauguagauuu |
| SEQ ID NO: 346 | hsa-miR-376a | aucauagaggaaaauccacgu |
| SEQ ID NO: 347 | hsa-miR-518b | caaagcgcuccccuuuagaggu |
| SEQ ID NO: 348 | hsa-miR-492 | aggaccugcgggacaagauucuu |
| SEQ ID NO: 349 | hsa-miR-518f | gaaagcgcuucucuuuagagg |
| SEQ ID NO: 350 | hsa-miR-200a* | caucuuaccggacagugcugga |
| SEQ ID NO: 351 | hsa-miR-135a* | uauagggauuggagccguggcg |
| SEQ ID NO: 352 | hsa-miR-1308 | gcauggggugguucagugg |
| SEQ ID NO: 353 | hsa-miR-384 | auuccuagaaauuguucaua |
| SEQ ID NO: 354 | hsa-miR-657 | ggcagguucucacccucucuagg |
| SEQ ID NO: 355 | hsa-miR-662 | ucccacguuguggcccagcag |
| SEQ ID NO: 356 | hsa-miR-1911 | ugaguaccgccaugucuguuggg |
| SEQ ID NO: 357 | hsa-miR-337-3p | cuccuauaugaugccuuucuuc |
| SEQ ID NO: 358 | hsa-miR-98 | ugagguaguaaguuguauuguu |
| SEQ ID NO: 359 | hsa-miR-429 | uaauacugucugguaaaaccgu |
| SEQ ID NO: 360 | hsa-miR-572 | guccgcucggcgguggccca |
| SEQ ID NO: 361 | hsa-miR-30c-1* | cugggagagggguuguuuacucc |
| SEQ ID NO: 362 | hsa-miR-211 | uucccuuugucauccuucgccu |
| SEQ ID NO: 363 | hsa-miR-548n | caaaaguaauuguggauuuugu |
| SEQ ID NO: 364 | hsa-miR-548k | aaaaguacuugcggauuuugcu |

| | | |
|---|---|---|
| SEQ ID NO: 365 | hsa-miR-613 | aggaauguuccuucuuugcc |
| SEQ ID NO: 366 | hsa-miR-99a* | caagcucgcuucuaugggucug |
| SEQ ID NO: 367 | hsa-miR-1248 | accuucuuguauaagcacugugcuaaa |
| SEQ ID NO: 368 | hsa-miR-29b-1* | gcugguuucauauggugguuuaga |
| SEQ ID NO: 369 | hsa-miR-184 | uggacggagaacugauaagggu |
| SEQ ID NO: 370 | hsa-miR-566 | gggcgccugugaucccaac |
| SEQ ID NO: 371 | hsa-miR-302b* | acuuuaacauggaagugcuuuc |
| SEQ ID NO: 372 | hsa-miR-623 | aucccuugcaggggcuguugggu |
| SEQ ID NO: 373 | hsa-miR-122* | aacgccauuaucacacuaaaua |
| SEQ ID NO: 374 | hsa-miR-1233 | ugagcccuguccucccgcag |
| SEQ ID NO: 375 | hsa-miR-606 | aaacuacugaaaaucaaagau |
| SEQ ID NO: 376 | hsa-miR-24-1* | ugccuacugagcugauaucagu |
| SEQ ID NO: 377 | hsa-miR-875-3p | ccuggaaacacugagguugug |
| SEQ ID NO: 378 | hsa-miR-873 | gcaggaacuugugagucuccu |
| SEQ ID NO: 379 | hsa-miR-224 | caagucacuagugguuccguu |
| SEQ ID NO: 380 | hsa-miR-1263 | augguacccuggcauacugagu |
| SEQ ID NO: 381 | hsa-miR-181a* | accaucgaccguugauuguacc |
| SEQ ID NO: 382 | hsa-miR-767-3p | ucugcucauaccccaugguuucu |
| SEQ ID NO: 383 | hsa-miR-302d | uaagugcuuccauguuugagugu |
| SEQ ID NO: 384 | hsa-miR-937 | auccgcgcucugacucucugcc |
| SEQ ID NO: 385 | hsa-miR-373* | acucaaaaugggggcgcuuucc |
| SEQ ID NO: 386 | hsa-miR-1261 | auggauaaggcuuuggcuu |
| SEQ ID NO: 387 | hsa-miR-188-5p | caucccuugcaugguggaggg |
| SEQ ID NO: 388 | hsa-miR-374a* | cuuaucagauuguauuguaauu |
| SEQ ID NO: 389 | hsa-miR-499-3p | aacaucacagcaagucugugcu |
| SEQ ID NO: 390 | hsa-miR-622 | acagucugcugagguuggagc |
| SEQ ID NO: 391 | hsa-miR-513b | uucacaaggaggugucauuuau |

**Figure 3**

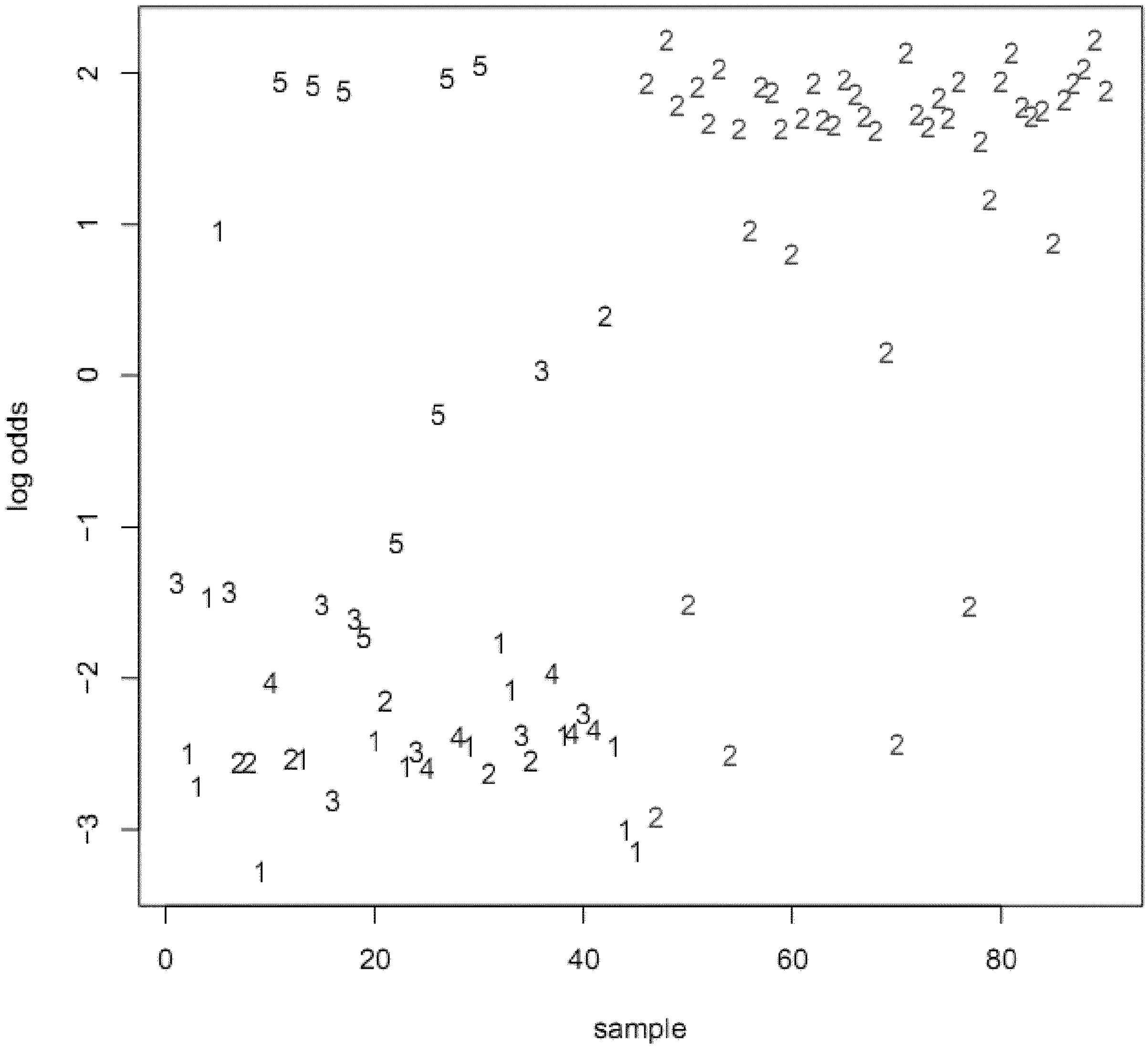

Figure 4

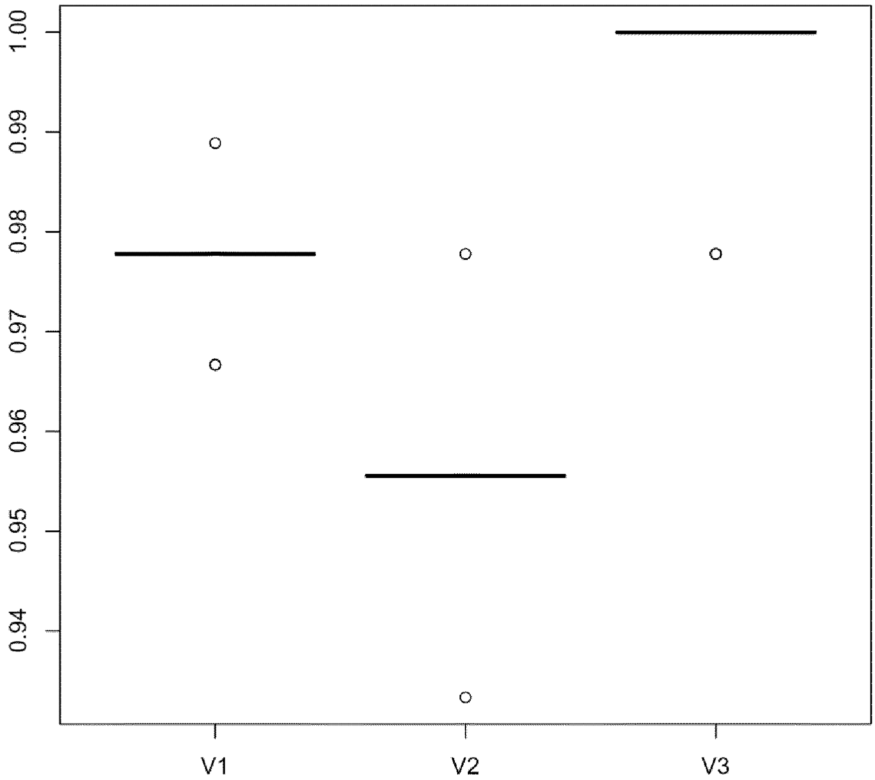


Figure 5

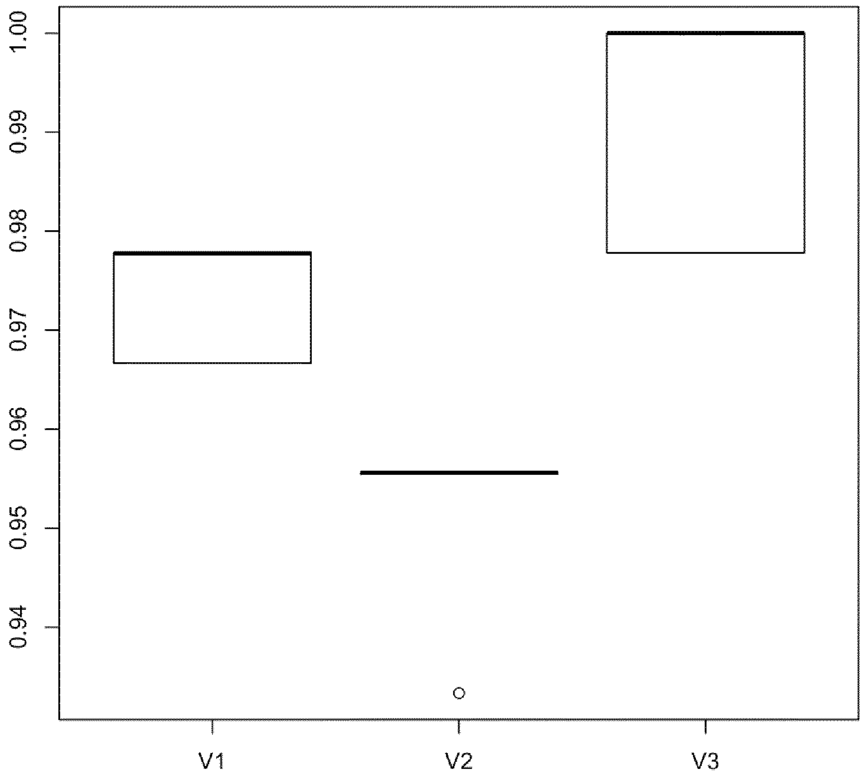

Figure 6

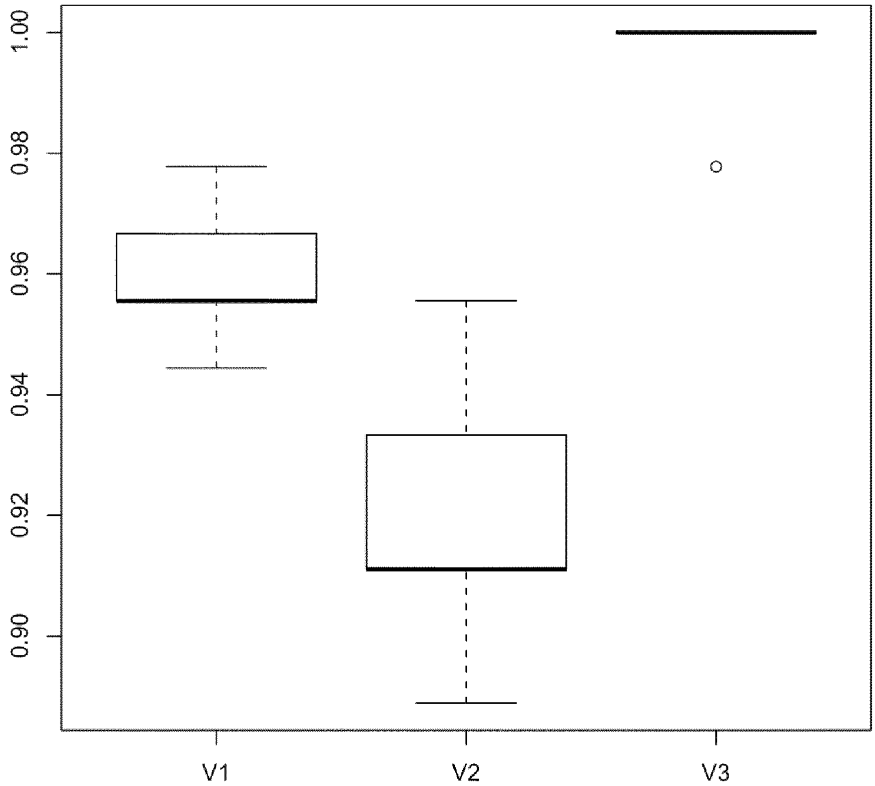


Figure 7

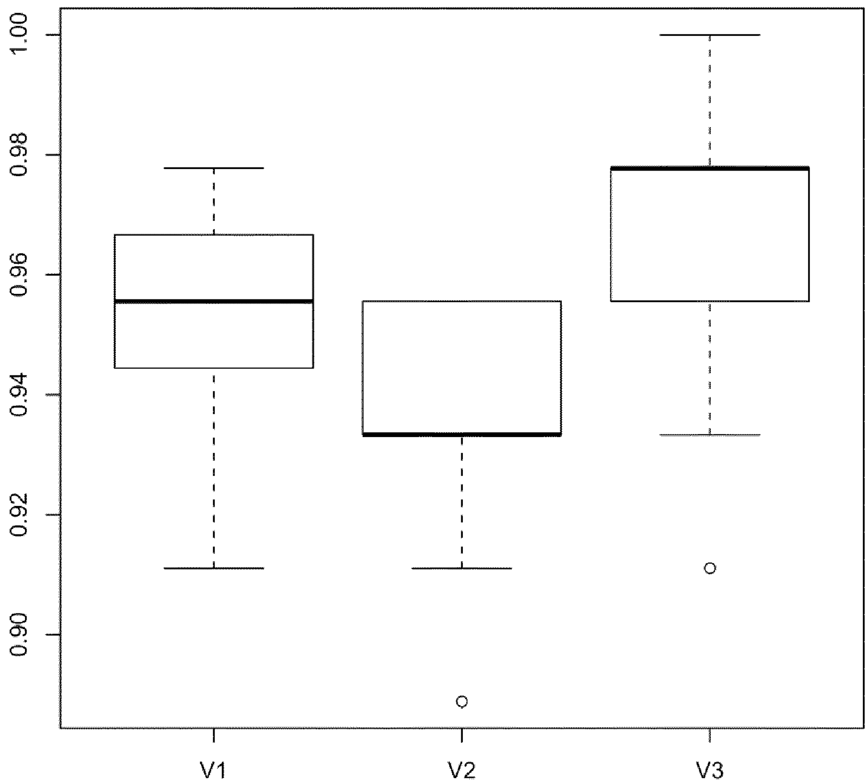

**Figure 8**

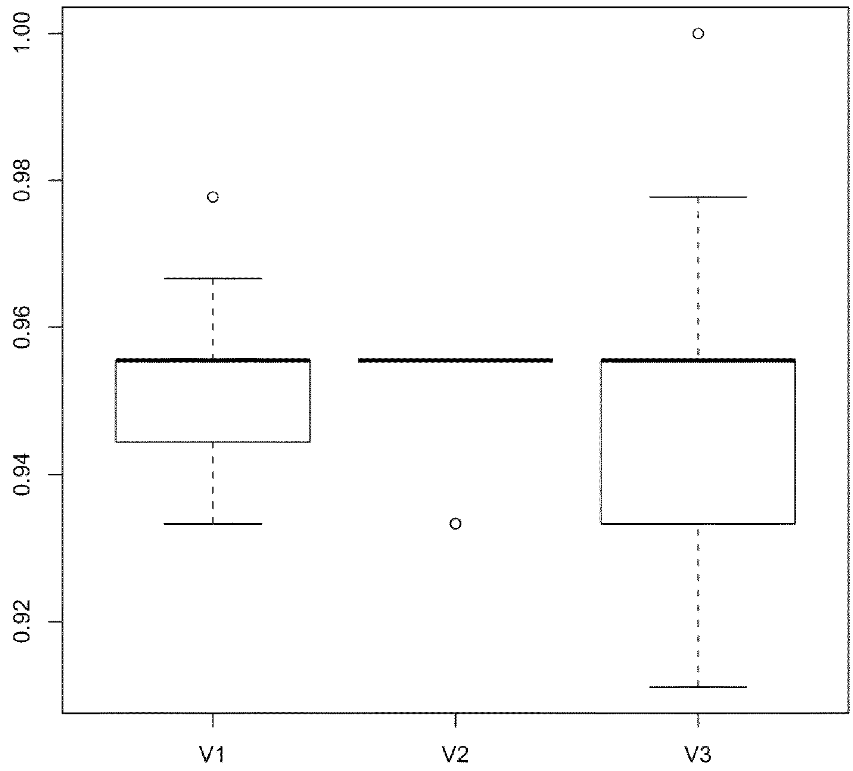


**Figure 9**

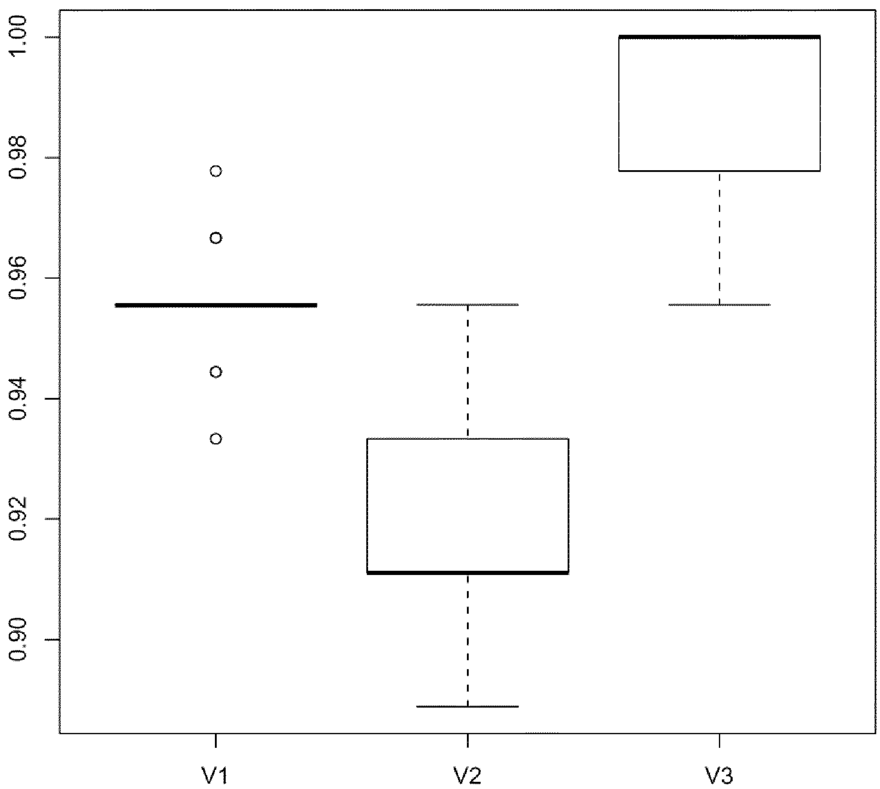

Figure 10

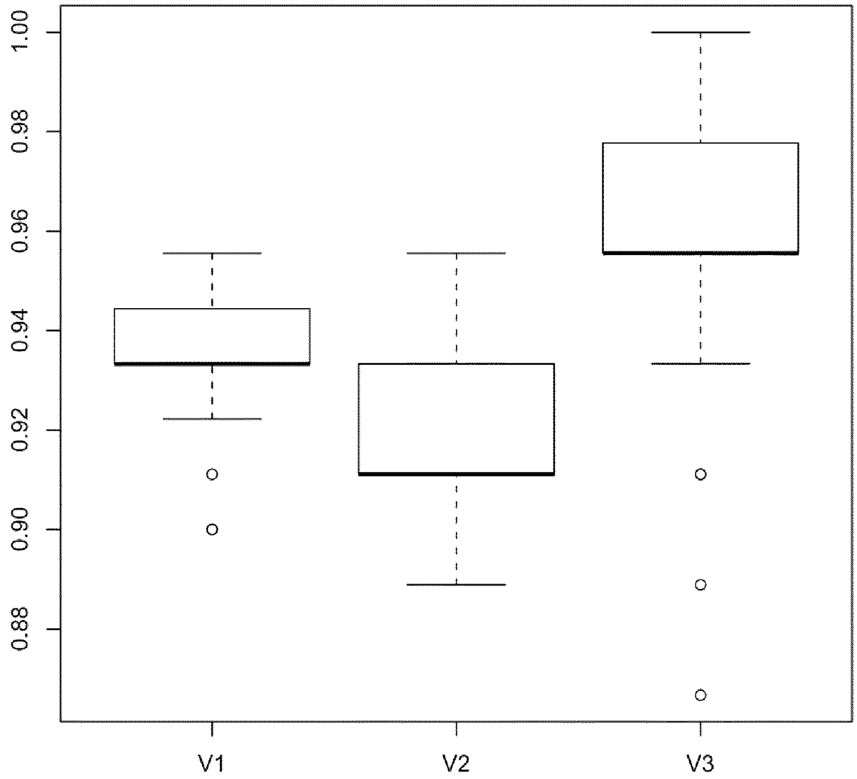


Figure 11

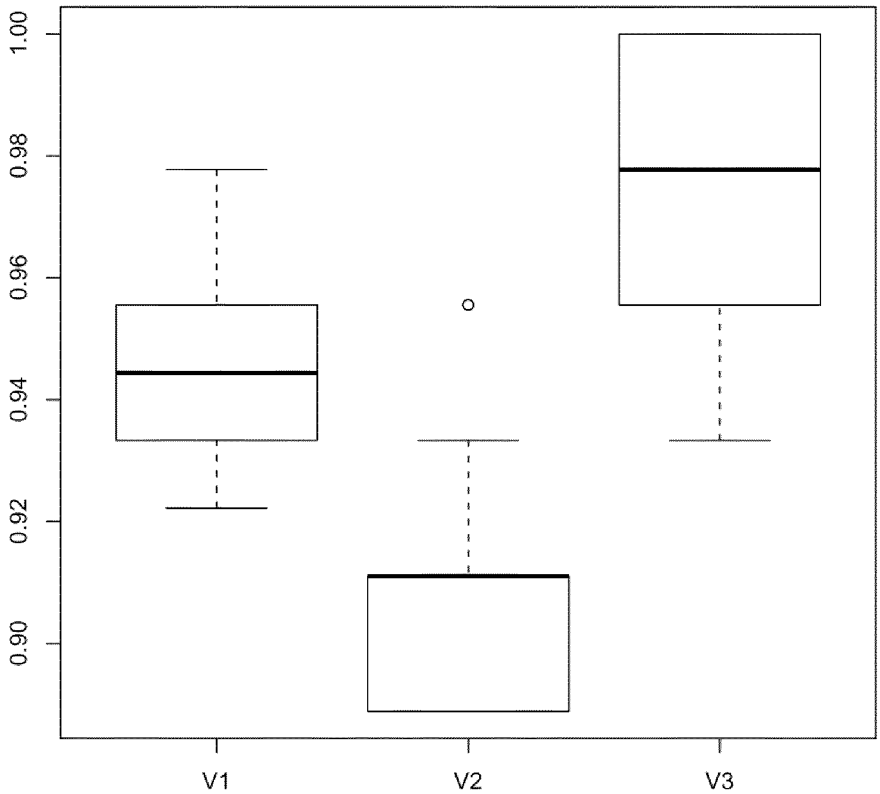

Figure 12

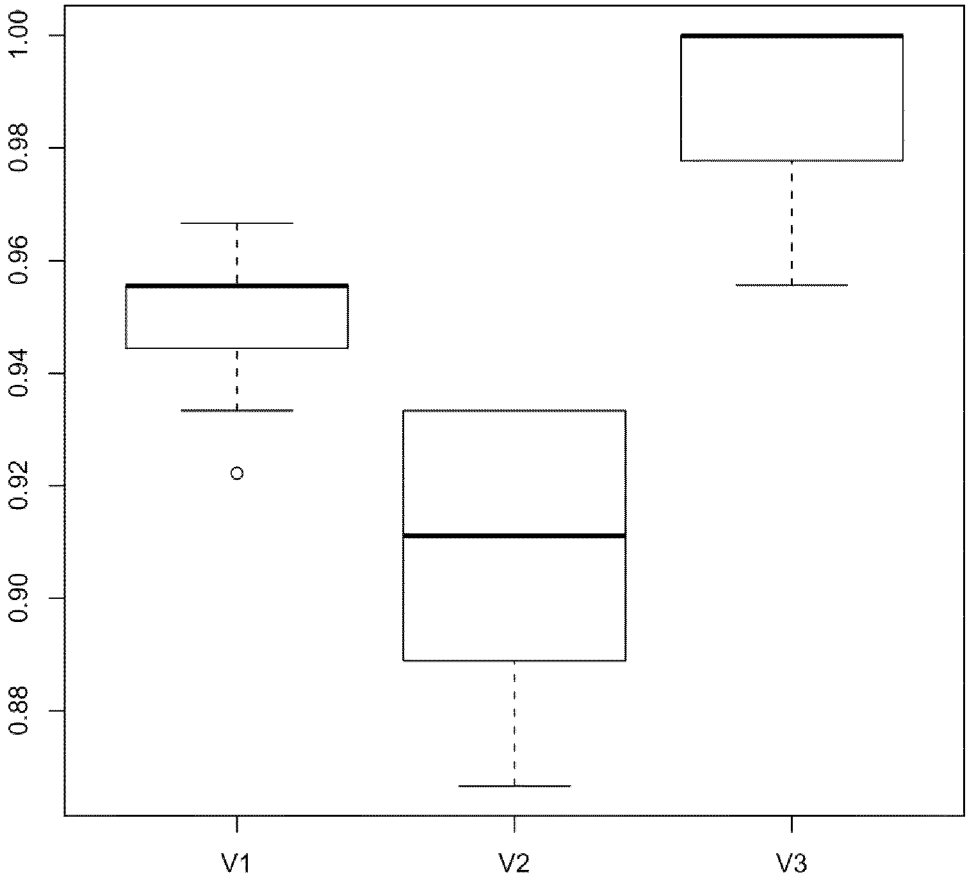

# Figure 13

| SEQ ID NO: | miRNA | median g1 | median g2 | qmedian | log qmedian | ttest_rawp | ttest_adjp | AUC | limma_rawp | limma_adjp |
|---|---|---|---|---|---|---|---|---|---|---|
| SEQ ID NO: 10 | hsa-miR-320d | 466 | 2697 | 0,17 | -1,76 | 2,45E-16 | 2,11E-13 | 0,03 | 5,56E-14 | 4,80E-11 |
| SEQ ID NO: 2 | hsa-miR-151-3p | 752 | 2518 | 0,30 | -1,21 | 2,05E-15 | 8,83E-13 | 0,07 | 1,79E-12 | 7,73E-10 |
| SEQ ID NO: 21 | hsa-miR-320c | 466 | 1919 | 0,24 | -1,42 | 1,15E-12 | 3,31E-10 | 0,07 | 6,68E-06 | 2,62E-04 |
| SEQ ID NO: 6 | hsa-miR-106a | 5384 | 1621 | 3,32 | 1,20 | 2,20E-12 | 4,74E-10 | 0,97 | 1,97E-09 | 4,24E-07 |
| SEQ ID NO: 3 | hsa-miR-17 | 5000 | 1463 | 3,42 | 1,23 | 6,46E-11 | 1,12E-08 | 0,93 | 9,35E-07 | 5,65E-05 |
| SEQ ID NO: 1 | hsa-miR-363 | 3152 | 504 | 6,26 | 1,83 | 7,05E-10 | 1,01E-07 | 0,91 | 9,51E-08 | 9,12E-06 |
| SEQ ID NO: 12 | hsa-miR-15b | 11992 | 5518 | 2,17 | 0,78 | 2,64E-09 | 2,53E-07 | 0,89 | 1,63E-08 | 2,30E-06 |
| SEQ ID NO: 14 | hsa-miR-550* | 879 | 1797 | 0,49 | -0,72 | 3,04E-09 | 2,62E-07 | 0,14 | 9,82E-07 | 5,65E-05 |
| SEQ ID NO: 69 | hsa-miR-126 | 1205 | 191 | 6,30 | 1,84 | 5,83E-09 | 4,57E-07 | 0,91 | 4,76E-10 | 1,37E-07 |
| SEQ ID NO: 7 | hsa-miR-101 | 466 | 206 | 2,27 | 0,82 | 1,14E-08 | 8,19E-07 | 0,87 | 1,16E-05 | 3,84E-04 |
| SEQ ID NO: 8 | hsa-miR-20a | 2760 | 714 | 3,86 | 1,35 | 2,26E-08 | 1,50E-06 | 0,89 | 7,43E-06 | 2,79E-04 |
| SEQ ID NO: 9 | hsa-let-7d | 1797 | 611 | 2,94 | 1,08 | 2,93E-08 | 1,81E-06 | 0,88 | 3,59E-06 | 1,82E-04 |
| SEQ ID NO: 4 | hsa-miR-20b | 2010 | 576 | 3,49 | 1,25 | 2,11E-07 | 1,14E-05 | 0,88 | 6,40E-06 | 2,62E-04 |
| SEQ ID NO: 18 | hsa-miR-320b | 2360 | 5652 | 0,42 | -0,87 | 3,26E-07 | 1,65E-05 | 0,17 | 6,86E-08 | 7,40E-06 |
| SEQ ID NO: 24 | hsa-miR-30c | 1836 | 4200 | 0,44 | -0,83 | 4,13E-07 | 1,98E-05 | 0,18 | 8,75E-06 | 3,02E-04 |
| SEQ ID NO: 26 | hsa-miR-182 | 4645 | 2306 | 2,01 | 0,70 | 7,83E-07 | 3,56E-05 | 0,83 | 1,64E-06 | 8,86E-05 |
| SEQ ID NO: 68 | hsa-miR-144 | 1328 | 177 | 7,51 | 2,02 | 1,07E-06 | 4,62E-05 | 0,91 | 7,14E-09 | 1,23E-06 |
| SEQ ID NO: 74 | hsa-let-7i | 761 | 194 | 3,93 | 1,37 | 3,35E-06 | 1,22E-04 | 0,83 | 7,61E-05 | 1,71E-03 |
| SEQ ID NO: 71 | hsa-miR-374b | 445 | 127 | 3,51 | 1,26 | 5,30E-06 | 1,76E-04 | 0,81 | 1,67E-05 | 4,97E-04 |
| SEQ ID NO: 72 | hsa-miR-21 | 1146 | 150 | 7,62 | 2,03 | 7,10E-06 | 2,27E-04 | 0,86 | 6,04E-07 | 4,34E-05 |
| SEQ ID NO: 70 | hsa-miR-144* | 386 | 134 | 2,88 | 1,06 | 9,26E-06 | 2,76E-04 | 0,84 | 7,88E-06 | 2,84E-04 |
| SEQ ID NO: 22 | hsa-miR-744 | 685 | 318 | 2,15 | 0,77 | 1,67E-05 | 4,37E-04 | 0,86 | 5,51E-06 | 2,62E-04 |
| SEQ ID NO: 29 | hsa-miR-30e | 292 | 623 | 0,47 | -0,76 | 1,87E-05 | 4,73E-04 | 0,28 | 5,58E-01 | 6,94E-01 |
| SEQ ID NO: 19 | hsa-miR-24 | 1587 | 655 | 2,42 | 0,88 | 1,95E-05 | 4,82E-04 | 0,80 | 4,40E-05 | 1,12E-03 |
| SEQ ID NO: 44 | hsa-miR-1908 | 836 | 1832 | 0,46 | -0,78 | 2,26E-05 | 5,30E-04 | 0,25 | 8,58E-02 | 1,99E-01 |
| SEQ ID NO: 77 | hsa-miR-183 | 367 | 105 | 3,48 | 1,25 | 1,09E-04 | 2,05E-03 | 0,82 | 1,38E-05 | 4,40E-04 |
| SEQ ID NO: 232 | hsa-miR-149* | 457 | 1184 | 0,39 | -0,95 | 1,18E-04 | 2,16E-03 | 0,30 | 2,64E-01 | 4,17E-01 |
| SEQ ID NO: 30 | hsa-miR-664 | 413 | 998 | 0,41 | -0,88 | 1,31E-04 | 2,36E-03 | 0,24 | 1,95E-02 | 7,40E-02 |
| SEQ ID NO: 20 | hsa-miR-18a | 945 | 445 | 2,12 | 0,75 | 3,40E-04 | 4,89E-03 | 0,77 | 2,05E-04 | 3,61E-03 |

| | | | | | | | | | | |
|---|---|---|---|---|---|---|---|---|---|---|
| SEQ ID NO: 75 | hsa-let-7f | 453 | 123 | 3,69 | 1,31 | 1,03E-03 | 9,79E-03 | 0,72 | 7,24E-04 | 8,80E-03 |
| SEQ ID NO: 65 | hsa-miR-574-5p | 1121 | 423 | 2,65 | 0,97 | 2,69E-03 | 1,87E-02 | 0,80 | 5,17E-05 | 1,24E-03 |
| SEQ ID NO: 91 | hsa-miR-532-5p | 226 | 104 | 2,18 | 0,78 | 3,97E-03 | 2,36E-02 | 0,79 | 2,01E-04 | 3,61E-03 |
| SEQ ID NO: 15 | hsa-miR-30d | 7318 | 13000 | 0,56 | -0,57 | 8,34E-10 | 1,03E-07 | 0,10 | 2,19E-07 | 1,89E-05 |
| SEQ ID NO: 233 | hsa-miR-130b | 946 | 1689 | 0,56 | -0,58 | 1,48E-06 | 6,07E-05 | 0,19 | 1,11E-02 | 5,43E-02 |
| SEQ ID NO: 41 | hsa-miR-29b | 374 | 675 | 0,55 | -0,59 | 4,44E-06 | 1,53E-04 | 0,19 | 5,68E-01 | 7,01E-01 |
| SEQ ID NO: 13 | hsa-miR-148b | 536 | 274 | 1,95 | 0,67 | 1,12E-05 | 3,22E-04 | 0,86 | 7,75E-05 | 1,71E-03 |
| SEQ ID NO: 23 | hsa-miR-93 | 3301 | 2112 | 1,56 | 0,45 | 1,23E-05 | 3,42E-04 | 0,78 | 2,67E-03 | 2,11E-02 |
| SEQ ID NO: 55 | hsa-miR-181a | 554 | 1066 | 0,52 | -0,65 | 1,37E-05 | 3,69E-04 | 0,19 | 8,92E-02 | 2,03E-01 |
| SEQ ID NO: 11 | hsa-miR-324-3p | 1057 | 1797 | 0,59 | -0,53 | 2,63E-05 | 5,98E-04 | 0,21 | 1,07E-04 | 2,26E-03 |
| SEQ ID NO: 32 | hsa-miR-660 | 445 | 253 | 1,76 | 0,57 | 2,77E-05 | 6,12E-04 | 0,77 | 1,34E-04 | 2,68E-03 |
| SEQ ID NO: 51 | hsa-miR-342-3p | 2941 | 5384 | 0,55 | -0,60 | 3,70E-05 | 7,99E-04 | 0,22 | 1,91E-05 | 5,32E-04 |
| SEQ ID NO: 234 | hsa-miR-30b | 6233 | 8175 | 0,76 | -0,27 | 5,87E-05 | 1,24E-03 | 0,24 | 6,72E-04 | 8,41E-03 |
| SEQ ID NO: 54 | hsa-miR-1280 | 4101 | 7722 | 0,53 | -0,63 | 6,34E-05 | 1,24E-03 | 0,24 | 1,52E-04 | 2,92E-03 |
| SEQ ID NO: 43 | hsa-miR-28-5p | 380 | 214 | 1,77 | 0,57 | 9,93E-05 | 1,90E-03 | 0,75 | 5,00E-04 | 7,07E-03 |
| SEQ ID NO: 28 | hsa-miR-720 | 5790 | 9818 | 0,59 | -0,53 | 2,85E-04 | 4,64E-03 | 0,24 | 2,70E-04 | 4,39E-03 |
| SEQ ID NO: 235 | hsa-miR-1271 | 212 | 125 | 1,69 | 0,53 | 3,31E-04 | 4,87E-03 | 0,82 | 1,37E-04 | 2,68E-03 |
| SEQ ID NO: 38 | hsa-let-7a | 931 | 611 | 1,52 | 0,42 | 3,56E-04 | 5,03E-03 | 0,75 | 2,37E-03 | 1,99E-02 |
| SEQ ID NO: 33 | hsa-miR-130a | 1294 | 765 | 1,69 | 0,53 | 4,42E-04 | 5,87E-03 | 0,75 | 3,82E-03 | 2,60E-02 |
| SEQ ID NO: 92 | hsa-miR-1226* | 184 | 109 | 1,68 | 0,52 | 5,01E-04 | 6,44E-03 | 0,74 | 1,85E-04 | 3,39E-03 |
| SEQ ID NO: 236 | hsa-miR-1274b | 1244 | 706 | 1,76 | 0,57 | 6,67E-04 | 7,67E-03 | 0,72 | 4,14E-03 | 2,75E-02 |
| SEQ ID NO: 97 | hsa-miR-1225-3p | 178 | 103 | 1,73 | 0,55 | 6,84E-04 | 7,68E-03 | 0,73 | 6,35E-04 | 8,31E-03 |
| SEQ ID NO: 42 | hsa-miR-30a | 447 | 840 | 0,53 | -0,63 | 6,98E-04 | 7,72E-03 | 0,33 | 4,74E-01 | 6,24E-01 |
| SEQ ID NO: 5 | hsa-miR-103 | 3626 | 2306 | 1,57 | 0,45 | 7,07E-04 | 7,72E-03 | 0,72 | 6,70E-03 | 3,75E-02 |
| SEQ ID NO: 37 | hsa-miR-17* | 555 | 295 | 1,88 | 0,63 | 8,28E-04 | 8,60E-03 | 0,77 | 4,76E-04 | 6,84E-03 |
| SEQ ID NO: 25 | hsa-miR-106b | 9392 | 5928 | 1,58 | 0,46 | 8,68E-04 | 8,81E-03 | 0,73 | 1,39E-03 | 1,39E-02 |
| SEQ ID NO: 27 | hsa-miR-320a | 14965 | 24753 | 0,60 | -0,50 | 8,66E-04 | 8,81E-03 | 0,28 | 6,59E-04 | 8,41E-03 |
| SEQ ID NO: 87 | hsa-miR-181c | 130 | 246 | 0,53 | -0,64 | 1,32E-03 | 1,19E-02 | 0,34 | 9,72E-01 | 9,83E-01 |
| SEQ ID NO: 47 | hsa-miR-532-3p | 3997 | 6081 | 0,66 | -0,42 | 1,40E-03 | 1,21E-02 | 0,26 | 1,12E-03 | 1,19E-02 |
| SEQ ID NO: 237 | hsa-miR-19b | 12496 | 9818 | 1,27 | 0,24 | 1,49E-03 | 1,24E-02 | 0,68 | 1,33E-02 | 5,94E-02 |
| SEQ ID NO: 238 | hsa-miR-185 | 24753 | 20492 | 1,21 | 0,19 | 1,46E-03 | 1,24E-02 | 0,69 | 7,84E-03 | 4,26E-02 |
| SEQ ID NO: 73 | hsa-miR-301a | 214 | 116 | 1,85 | 0,61 | 2,29E-03 | 1,67E-02 | 0,80 | 2,75E-04 | 4,39E-03 |
| SEQ ID NO: 78 | hsa-miR-1202 | 194 | 113 | 1,72 | 0,54 | 2,81E-03 | 1,93E-02 | 0,75 | 2,23E-04 | 3,86E-03 |
| SEQ ID NO: 239 | hsa-miR-142-5p | 520 | 675 | 0,77 | -0,26 | 2,81E-03 | 1,93E-02 | 0,39 | 5,83E-01 | 7,15E-01 |

| | | | | | | | | | | |
|---|---|---|---|---|---|---|---|---|---|---|
| SEQ ID NO: 46 | hsa-miR-197 | 840 | 1352 | 0,62 | -0,48 | 4,15E-03 | 2,42E-02 | 0,28 | 1,91E-01 | 3,37E-01 |
| SEQ ID NO: 240 | hsa-miR-675 | 231 | 127 | 1,82 | 0,60 | 4,41E-03 | 2,49E-02 | 0,73 | 1,98E-03 | 1,78E-02 |
| SEQ ID NO: 103 | hsa-miR-940 | 184 | 103 | 1,79 | 0,58 | 4,67E-03 | 2,57E-02 | 0,68 | 1,87E-03 | 1,77E-02 |
| SEQ ID NO: 241 | hsa-miR-210 | 765 | 623 | 1,23 | 0,21 | 5,52E-03 | 2,84E-02 | 0,66 | 3,14E-03 | 2,33E-02 |
| SEQ ID NO: 64 | hsa-miR-151-5p | 5135 | 3301 | 1,56 | 0,44 | 5,75E-03 | 2,90E-02 | 0,70 | 2,79E-02 | 9,31E-02 |
| SEQ ID NO: 112 | hsa-miR-584 | 142 | 247 | 0,57 | -0,55 | 6,00E-03 | 2,97E-02 | 0,38 | 8,37E-01 | 8,97E-01 |
| SEQ ID NO: 242 | hsa-miR-1469 | 163 | 104 | 1,57 | 0,45 | 6,29E-03 | 3,03E-02 | 0,67 | 5,20E-03 | 3,24E-02 |
| SEQ ID NO: 82 | hsa-miR-100 | 227 | 247 | 0,92 | -0,09 | 8,09E-03 | 3,62E-02 | 0,42 | 2,30E-01 | 3,82E-01 |
| SEQ ID NO: 243 | hsa-miR-627 | 184 | 145 | 1,27 | 0,24 | 8,94E-03 | 3,84E-02 | 0,63 | 4,43E-03 | 2,89E-02 |
| SEQ ID NO: 45 | hsa-miR-1260 | 2697 | 4200 | 0,64 | -0,44 | 9,93E-03 | 4,10E-02 | 0,32 | 2,16E-02 | 7,83E-02 |
| SEQ ID NO: 244 | hsa-miR-23a | 5260 | 4406 | 1,19 | 0,18 | 9,96E-03 | 4,10E-02 | 0,64 | 7,19E-02 | 1,81E-01 |
| SEQ ID NO: 245 | hsa-miR-139-5p | 151 | 113 | 1,34 | 0,29 | 9,98E-03 | 4,10E-02 | 0,74 | 1,51E-03 | 1,47E-02 |
| SEQ ID NO: 76 | hsa-miR-18a* | 415 | 523 | 0,79 | -0,23 | 9,84E-03 | 4,10E-02 | 0,38 | 5,98E-01 | 7,24E-01 |
| SEQ ID NO: 95 | hsa-miR-1912 | 161 | 106 | 1,52 | 0,42 | 1,09E-02 | 4,34E-02 | 0,66 | 3,16E-02 | 1,02E-01 |
| SEQ ID NO: 246 | hsa-miR-486-5p | 41512 | 41512 | 1,00 | 0,00 | 1,18E-02 | 4,57E-02 | 0,37 | 1,45E-01 | 2,76E-01 |
| SEQ ID NO: 63 | hsa-miR-15a | 3147 | 2634 | 1,19 | 0,18 | 1,31E-02 | 4,92E-02 | 0,65 | 2,10E-02 | 7,73E-02 |
| SEQ ID NO: 126 | hsa-miR-550 | 169 | 57 | 2,96 | 1,09 | 1,89E-09 | 2,04E-07 | 0,88 | 6,15E-06 | 2,62E-04 |
| SEQ ID NO: 116 | hsa-miR-143 | 224 | 57 | 3,94 | 1,37 | 4,06E-08 | 2,34E-06 | 0,89 | 5,57E-07 | 4,34E-05 |
| SEQ ID NO: 105 | hsa-miR-27b | 203 | 57 | 3,56 | 1,27 | 2,29E-06 | 8,99E-05 | 0,85 | 6,08E-06 | 2,62E-04 |
| SEQ ID NO: 109 | hsa-miR-374a | 332 | 96 | 3,45 | 1,24 | 3,40E-06 | 1,22E-04 | 0,82 | 1,79E-05 | 5,15E-04 |
| SEQ ID NO: 107 | hsa-miR-27a | 342 | 64 | 5,32 | 1,67 | 7,57E-06 | 2,33E-04 | 0,87 | 1,86E-08 | 2,30E-06 |
| SEQ ID NO: 108 | hsa-let-7g | 402 | 81 | 4,98 | 1,60 | 6,20E-05 | 1,24E-03 | 0,76 | 6,59E-03 | 3,72E-02 |
| SEQ ID NO: 134 | hsa-miR-1914* | 159 | 77 | 2,05 | 0,72 | 1,97E-04 | 3,28E-03 | 0,74 | 4,46E-04 | 6,64E-03 |
| SEQ ID NO: 121 | hsa-miR-628-3p | 178 | 66 | 2,69 | 0,99 | 3,11E-04 | 4,82E-03 | 0,74 | 1,64E-02 | 6,87E-02 |
| SEQ ID NO: 247 | hsa-miR-625 | 165 | 51 | 3,21 | 1,17 | 3,13E-04 | 4,82E-03 | 0,75 | 3,53E-03 | 2,47E-02 |
| SEQ ID NO: 125 | hsa-miR-18b | 173 | 83 | 2,08 | 0,73 | 5,30E-04 | 6,62E-03 | 0,79 | 2,14E-03 | 1,88E-02 |
| SEQ ID NO: 114 | hsa-miR-96 | 146 | 57 | 2,57 | 0,94 | 6,43E-04 | 7,67E-03 | 0,81 | 8,79E-05 | 1,90E-03 |
| SEQ ID NO: 136 | hsa-miR-595 | 121 | 50 | 2,41 | 0,88 | 9,34E-04 | 9,27E-03 | 0,75 | 1,47E-03 | 1,44E-02 |
| SEQ ID NO: 248 | hsa-miR-1224-3p | 183 | 57 | 3,23 | 1,17 | 1,56E-03 | 1,28E-02 | 0,74 | 4,65E-04 | 6,80E-03 |
| SEQ ID NO: 131 | hsa-miR-31 | 142 | 68 | 2,08 | 0,73 | 1,77E-03 | 1,40E-02 | 0,74 | 6,05E-03 | 3,53E-02 |
| SEQ ID NO: 158 | hsa-let-7e | 252 | 83 | 3,04 | 1,11 | 2,69E-03 | 1,87E-02 | 0,68 | 1,27E-01 | 2,55E-01 |
| SEQ ID NO: 123 | hsa-miR-654-5p | 166 | 82 | 2,03 | 0,71 | 3,27E-03 | 2,16E-02 | 0,74 | 2,52E-04 | 4,19E-03 |
| SEQ ID NO: 249 | hsa-miR-193a-5p | 125 | 59 | 2,12 | 0,75 | 3,45E-03 | 2,22E-02 | 0,69 | 2,46E-03 | 2,00E-02 |
| SEQ ID NO: 106 | hsa-miR-26b | 255 | 91 | 2,81 | 1,03 | 4,31E-03 | 2,48E-02 | 0,72 | 1,19E-03 | 1,22E-02 |

| | | | | | | | | | | |
|---|---|---|---|---|---|---|---|---|---|---|
| SEQ ID NO: 138 | hsa-miR-16-2* | 97 | 196 | 0,49 | -0,70 | 4,62E-03 | 2,57E-02 | 0,34 | 4,00E-01 | 5,53E-01 |
| SEQ ID NO: 250 | hsa-miR-342-5p | 159 | 62 | 2,57 | 0,94 | 8,88E-03 | 3,83E-02 | 0,69 | 4,30E-03 | 2,83E-02 |
| SEQ ID NO: 152 | hsa-miR-132 | 126 | 70 | 1,81 | 0,60 | 3,33E-04 | 4,87E-03 | 0,76 | 6,63E-04 | 8,41E-03 |
| SEQ ID NO: 251 | hsa-miR-659 | 109 | 57 | 1,93 | 0,66 | 3,32E-04 | 4,87E-03 | 0,75 | 5,43E-03 | 3,30E-02 |
| SEQ ID NO: 149 | hsa-miR-631 | 134 | 90 | 1,49 | 0,40 | 3,70E-04 | 5,15E-03 | 0,72 | 1,07E-03 | 1,16E-02 |
| SEQ ID NO: 118 | hsa-miR-24-2* | 153 | 96 | 1,60 | 0,47 | 4,12E-04 | 5,65E-03 | 0,73 | 1,96E-03 | 1,78E-02 |
| SEQ ID NO: 154 | hsa-miR-454 | 130 | 68 | 1,92 | 0,65 | 5,08E-04 | 6,44E-03 | 0,80 | 6,86E-04 | 8,45E-03 |
| SEQ ID NO: 120 | hsa-miR-1254 | 112 | 74 | 1,51 | 0,41 | 1,38E-03 | 1,21E-02 | 0,72 | 1,01E-02 | 5,13E-02 |
| SEQ ID NO: 252 | hsa-miR-877 | 102 | 56 | 1,81 | 0,59 | 1,49E-03 | 1,24E-02 | 0,71 | 1,91E-03 | 1,78E-02 |
| SEQ ID NO: 141 | hsa-miR-650 | 110 | 59 | 1,87 | 0,62 | 1,75E-03 | 1,40E-02 | 0,72 | 3,41E-02 | 1,07E-01 |
| SEQ ID NO: 157 | hsa-miR-15a* | 105 | 78 | 1,35 | 0,30 | 3,72E-03 | 2,31E-02 | 0,69 | 1,18E-02 | 5,55E-02 |
| SEQ ID NO: 145 | hsa-miR-338-3p | 143 | 89 | 1,61 | 0,48 | 3,74E-03 | 2,31E-02 | 0,72 | 1,21E-02 | 5,57E-02 |
| SEQ ID NO: 253 | hsa-miR-21* | 79 | 104 | 0,76 | -0,27 | 4,64E-03 | 2,57E-02 | 0,37 | 6,44E-01 | 7,59E-01 |
| SEQ ID NO: 254 | hsa-miR-1281 | 149 | 84 | 1,78 | 0,58 | 5,30E-03 | 2,77E-02 | 0,69 | 1,43E-02 | 6,28E-02 |
| SEQ ID NO: 115 | hsa-miR-1272 | 107 | 69 | 1,56 | 0,45 | 5,93E-03 | 2,97E-02 | 0,69 | 5,63E-03 | 3,40E-02 |
| SEQ ID NO: 137 | hsa-miR-192* | 112 | 71 | 1,57 | 0,45 | 6,54E-03 | 3,12E-02 | 0,73 | 1,34E-03 | 1,36E-02 |
| SEQ ID NO: 111 | hsa-miR-509-3-5p | 174 | 96 | 1,81 | 0,59 | 1,03E-02 | 4,22E-02 | 0,72 | 9,61E-04 | 1,10E-02 |
| SEQ ID NO: 110 | hsa-miR-1301 | 162 | 84 | 1,94 | 0,66 | 1,05E-02 | 4,25E-02 | 0,74 | 5,52E-04 | 7,66E-03 |
| SEQ ID NO: 130 | hsa-miR-575 | 114 | 87 | 1,32 | 0,28 | 1,27E-02 | 4,86E-02 | 0,66 | 1,91E-02 | 7,37E-02 |
| SEQ ID NO: 255 | hsa-miR-369-5p | 14 | 50 | 0,29 | -1,25 | 2,27E-05 | 5,30E-04 | 0,24 | 2,69E-03 | 2,11E-02 |
| SEQ ID NO: 256 | hsa-miR-182* | 20 | 52 | 0,39 | -0,95 | 6,33E-05 | 1,24E-03 | 0,27 | 7,14E-03 | 3,95E-02 |
| SEQ ID NO: 257 | hsa-miR-136 | 10 | 51 | 0,20 | -1,60 | 1,82E-04 | 3,08E-03 | 0,28 | 1,10E-02 | 5,41E-02 |
| SEQ ID NO: 258 | hsa-miR-1279 | 1 | 17 | 0,06 | -2,83 | 3,07E-04 | 4,82E-03 | 0,36 | 1,49E-02 | 6,39E-02 |
| SEQ ID NO: 259 | hsa-miR-520e | 1 | 25 | 0,04 | -3,23 | 4,81E-04 | 6,30E-03 | 0,34 | 8,45E-03 | 4,53E-02 |
| SEQ ID NO: 260 | hsa-let-7b* | 15 | 52 | 0,30 | -1,22 | 5,87E-04 | 7,23E-03 | 0,28 | 1,03E-03 | 1,13E-02 |
| SEQ ID NO: 261 | hsa-miR-154* | 24 | 52 | 0,46 | -0,78 | 6,67E-04 | 7,67E-03 | 0,27 | 1,96E-03 | 1,78E-02 |
| SEQ ID NO: 177 | hsa-miR-517c | 10 | 43 | 0,23 | -1,47 | 6,60E-04 | 7,67E-03 | 0,28 | 3,06E-03 | 2,32E-02 |
| SEQ ID NO: 262 | hsa-miR-450b-3p | 21 | 50 | 0,41 | -0,89 | 6,54E-04 | 7,67E-03 | 0,27 | 8,64E-04 | 1,01E-02 |
| SEQ ID NO: 183 | hsa-miR-187 | 5 | 32 | 0,15 | -1,89 | 6,85E-04 | 7,68E-03 | 0,32 | 8,54E-03 | 4,55E-02 |
| SEQ ID NO: 263 | hsa-miR-562 | 13 | 36 | 0,36 | -1,02 | 8,16E-04 | 8,59E-03 | 0,30 | 4,66E-03 | 2,96E-02 |
| SEQ ID NO: 264 | hsa-miR-493 | 19 | 42 | 0,46 | -0,79 | 9,19E-04 | 9,22E-03 | 0,29 | 1,00E-02 | 5,13E-02 |
| SEQ ID NO: 265 | hsa-miR-548m | 1 | 16 | 0,06 | -2,80 | 9,77E-04 | 9,37E-03 | 0,36 | 2,36E-02 | 8,42E-02 |
| SEQ ID NO: 266 | hsa-miR-1264 | 17 | 40 | 0,42 | -0,86 | 1,05E-03 | 9,82E-03 | 0,26 | 5,16E-05 | 1,24E-03 |
| SEQ ID NO: 267 | hsa-miR-557 | 18 | 51 | 0,35 | -1,06 | 1,10E-03 | 1,02E-02 | 0,27 | 3,04E-03 | 2,32E-02 |

| | | | | | | | | | | |
|---|---|---|---|---|---|---|---|---|---|---|
| SEQ ID NO: 268 | hsa-miR-508-3p | 1 | 22 | 0,05 | -3,08 | 1,12E-03 | 1,03E-02 | 0,37 | 6,72E-02 | 1,73E-01 |
| SEQ ID NO: 209 | hsa-miR-548b-5p | 1 | 30 | 0,03 | -3,40 | 1,14E-03 | 1,04E-02 | 0,29 | 4,37E-04 | 6,62E-03 |
| SEQ ID NO: 228 | hsa-miR-886-3p | 9 | 23 | 0,41 | -0,89 | 1,35E-03 | 1,20E-02 | 0,39 | 6,67E-02 | 1,72E-01 |
| SEQ ID NO: 269 | hsa-miR-935 | 15 | 32 | 0,48 | -0,74 | 1,38E-03 | 1,21E-02 | 0,32 | 1,99E-02 | 7,42E-02 |
| SEQ ID NO: 270 | hsa-let-7f-2* | 1 | 17 | 0,06 | -2,86 | 1,57E-03 | 1,28E-02 | 0,32 | 3,79E-03 | 2,60E-02 |
| SEQ ID NO: 184 | hsa-miR-519b-3p | 20 | 50 | 0,39 | -0,94 | 1,76E-03 | 1,40E-02 | 0,26 | 2,00E-03 | 1,78E-02 |
| SEQ ID NO: 271 | hsa-miR-526b* | 6 | 23 | 0,27 | -1,31 | 1,83E-03 | 1,42E-02 | 0,35 | 2,82E-02 | 9,35E-02 |
| SEQ ID NO: 188 | hsa-miR-630 | 17 | 51 | 0,33 | -1,12 | 1,83E-03 | 1,42E-02 | 0,22 | 3,02E-05 | 7,89E-04 |
| SEQ ID NO: 191 | hsa-miR-569 | 14 | 52 | 0,27 | -1,31 | 1,93E-03 | 1,48E-02 | 0,30 | 2,94E-02 | 9,62E-02 |
| SEQ ID NO: 272 | hsa-miR-548c-5p | 5 | 35 | 0,14 | -1,96 | 1,97E-03 | 1,50E-02 | 0,33 | 1,01E-02 | 5,13E-02 |
| SEQ ID NO: 194 | hsa-miR-553 | 6 | 29 | 0,20 | -1,61 | 2,11E-03 | 1,59E-02 | 0,30 | 2,55E-03 | 2,05E-02 |
| SEQ ID NO: 198 | hsa-miR-190b | 1 | 32 | 0,03 | -3,47 | 2,31E-03 | 1,67E-02 | 0,35 | 2,74E-02 | 9,19E-02 |
| SEQ ID NO: 273 | hsa-miR-519e* | 20 | 58 | 0,35 | -1,06 | 2,66E-03 | 1,87E-02 | 0,27 | 6,96E-05 | 1,62E-03 |
| SEQ ID NO: 224 | hsa-miR-615-3p | 18 | 50 | 0,37 | -1,00 | 2,91E-03 | 1,96E-02 | 0,28 | 3,49E-03 | 2,47E-02 |
| SEQ ID NO: 186 | hsa-miR-649 | 14 | 30 | 0,46 | -0,77 | 3,08E-03 | 2,04E-02 | 0,37 | 1,25E-01 | 2,54E-01 |
| SEQ ID NO: 210 | hsa-miR-519a | 20 | 53 | 0,38 | -0,96 | 3,33E-03 | 2,17E-02 | 0,20 | 1,34E-04 | 2,68E-03 |
| SEQ ID NO: 218 | hsa-miR-541* | 8 | 30 | 0,26 | -1,36 | 3,40E-03 | 2,21E-02 | 0,34 | 2,52E-02 | 8,76E-02 |
| SEQ ID NO: 172 | hsa-miR-302b | 1 | 37 | 0,04 | -3,22 | 3,78E-03 | 2,31E-02 | 0,31 | 3,01E-03 | 2,32E-02 |
| SEQ ID NO: 274 | hsa-miR-220b | 25 | 71 | 0,36 | -1,03 | 3,88E-03 | 2,32E-02 | 0,22 | 8,82E-07 | 5,65E-05 |
| SEQ ID NO: 275 | hsa-miR-1284 | 18 | 45 | 0,39 | -0,94 | 4,09E-03 | 2,42E-02 | 0,33 | 2,79E-02 | 9,31E-02 |
| SEQ ID NO: 207 | hsa-miR-379 | 13 | 28 | 0,47 | -0,76 | 4,18E-03 | 2,42E-02 | 0,37 | 7,55E-02 | 1,86E-01 |
| SEQ ID NO: 227 | hsa-miR-369-3p | 14 | 37 | 0,37 | -1,00 | 4,40E-03 | 2,49E-02 | 0,31 | 6,13E-03 | 3,55E-02 |
| SEQ ID NO: 276 | hsa-miR-26a-1* | 14 | 30 | 0,45 | -0,80 | 4,42E-03 | 2,49E-02 | 0,38 | 1,31E-01 | 2,60E-01 |
| SEQ ID NO: 178 | hsa-miR-1262 | 1 | 17 | 0,06 | -2,85 | 4,66E-03 | 2,57E-02 | 0,34 | 1,16E-02 | 5,51E-02 |
| SEQ ID NO: 277 | hsa-miR-302a* | 22 | 53 | 0,42 | -0,86 | 4,73E-03 | 2,58E-02 | 0,30 | 3,39E-04 | 5,31E-03 |
| SEQ ID NO: 197 | hsa-miR-556-3p | 9 | 31 | 0,29 | -1,23 | 4,80E-03 | 2,59E-02 | 0,35 | 3,83E-02 | 1,14E-01 |
| SEQ ID NO: 192 | hsa-miR-632 | 20 | 50 | 0,39 | -0,93 | 4,79E-03 | 2,59E-02 | 0,33 | 4,70E-02 | 1,34E-01 |
| SEQ ID NO: 164 | hsa-miR-335* | 9 | 50 | 0,18 | -1,70 | 5,07E-03 | 2,70E-02 | 0,33 | 1,87E-02 | 7,32E-02 |
| SEQ ID NO: 278 | hsa-miR-204 | 1 | 8 | 0,13 | -2,03 | 5,04E-03 | 2,70E-02 | 0,41 | 1,38E-01 | 2,67E-01 |
| SEQ ID NO: 279 | hsa-miR-373 | 2 | 18 | 0,10 | -2,33 | 5,17E-03 | 2,74E-02 | 0,38 | 3,99E-02 | 1,18E-01 |
| SEQ ID NO: 280 | hsa-miR-23a* | 6 | 30 | 0,20 | -1,59 | 5,22E-03 | 2,74E-02 | 0,35 | 3,19E-02 | 1,02E-01 |
| SEQ ID NO: 281 | hsa-miR-302f | 1 | 34 | 0,03 | -3,53 | 5,44E-03 | 2,83E-02 | 0,27 | 2,38E-04 | 4,03E-03 |
| SEQ ID NO: 282 | hsa-miR-637 | 9 | 50 | 0,17 | -1,77 | 5,57E-03 | 2,84E-02 | 0,24 | 1,60E-04 | 3,01E-03 |
| SEQ ID NO: 189 | hsa-miR-126* | 6 | 30 | 0,19 | -1,68 | 5,55E-03 | 2,84E-02 | 0,36 | 4,29E-02 | 1,24E-01 |

| | | | | | | | | | | |
|---|---|---|---|---|---|---|---|---|---|---|
| SEQ ID NO: 283 | hsa-miR-331-5p | 18 | 50 | 0,35 | -1,04 | 5,68E-03 | 2,89E-02 | 0,32 | 5,37E-02 | 1,48E-01 |
| SEQ ID NO: 284 | hsa-miR-585 | 6 | 16 | 0,39 | -0,93 | 6,10E-03 | 2,99E-02 | 0,40 | 1,54E-01 | 2,90E-01 |
| SEQ ID NO: 285 | hsa-miR-548h | 1 | 12 | 0,08 | -2,51 | 6,16E-03 | 3,01E-02 | 0,37 | 4,23E-02 | 1,24E-01 |
| SEQ ID NO: 286 | hsa-miR-507 | 14 | 31 | 0,45 | -0,80 | 6,52E-03 | 3,12E-02 | 0,38 | 1,66E-01 | 3,07E-01 |
| SEQ ID NO: 287 | hsa-miR-539 | 5 | 16 | 0,31 | -1,18 | 6,88E-03 | 3,23E-02 | 0,39 | 7,84E-02 | 1,91E-01 |
| SEQ ID NO: 288 | hsa-miR-376a* | 6 | 18 | 0,32 | -1,15 | 6,86E-03 | 3,23E-02 | 0,35 | 3,16E-02 | 1,02E-01 |
| SEQ ID NO: 289 | hsa-miR-521 | 27 | 59 | 0,45 | -0,80 | 7,04E-03 | 3,28E-02 | 0,28 | 3,21E-03 | 2,33E-02 |
| SEQ ID NO: 290 | hsa-miR-519e | 1 | 12 | 0,08 | -2,51 | 7,52E-03 | 3,41E-02 | 0,33 | 4,46E-03 | 2,89E-02 |
| SEQ ID NO: 291 | hsa-miR-338-5p | 19 | 49 | 0,39 | -0,94 | 7,94E-03 | 3,59E-02 | 0,37 | 1,17E-01 | 2,44E-01 |
| SEQ ID NO: 292 | hsa-miR-548j | 1 | 22 | 0,05 | -3,08 | 8,10E-03 | 3,62E-02 | 0,30 | 9,89E-04 | 1,11E-02 |
| SEQ ID NO: 293 | hsa-miR-1256 | 26 | 67 | 0,38 | -0,95 | 8,58E-03 | 3,72E-02 | 0,32 | 1,04E-03 | 1,13E-02 |
| SEQ ID NO: 294 | hsa-miR-618 | 1 | 43 | 0,02 | -3,77 | 8,57E-03 | 3,72E-02 | 0,24 | 2,20E-05 | 5,94E-04 |
| SEQ ID NO: 295 | hsa-miR-135b | 1 | 23 | 0,04 | -3,12 | 8,42E-03 | 3,72E-02 | 0,38 | 2,85E-02 | 9,41E-02 |
| SEQ ID NO: 296 | hsa-miR-219-2-3p | 7 | 17 | 0,38 | -0,96 | 9,43E-03 | 4,01E-02 | 0,38 | 5,46E-02 | 1,49E-01 |
| SEQ ID NO: 297 | hsa-miR-944 | 17 | 36 | 0,47 | -0,76 | 9,90E-03 | 4,10E-02 | 0,36 | 7,17E-02 | 1,81E-01 |
| SEQ ID NO: 208 | hsa-miR-520c-3p | 1 | 16 | 0,06 | -2,78 | 9,93E-03 | 4,10E-02 | 0,36 | 1,71E-02 | 6,93E-02 |
| SEQ ID NO: 185 | hsa-let-7c* | 25 | 51 | 0,49 | -0,71 | 1,04E-02 | 4,22E-02 | 0,36 | 5,85E-02 | 1,56E-01 |
| SEQ ID NO: 298 | hsa-miR-509-3p | 6 | 1 | 5,87 | 1,77 | 1,05E-02 | 4,25E-02 | 0,45 | 2,30E-01 | 3,81E-01 |
| SEQ ID NO: 299 | hsa-miR-515-3p | 3 | 17 | 0,19 | -1,66 | 1,06E-02 | 4,25E-02 | 0,38 | 1,07E-01 | 2,29E-01 |
| SEQ ID NO: 300 | hsa-miR-548i | 9 | 40 | 0,21 | -1,54 | 1,10E-02 | 4,36E-02 | 0,28 | 3,14E-03 | 2,33E-02 |
| SEQ ID NO: 301 | hsa-let-7e* | 5 | 18 | 0,27 | -1,31 | 1,17E-02 | 4,57E-02 | 0,39 | 1,44E-01 | 2,76E-01 |
| SEQ ID NO: 302 | hsa-miR-154 | 3 | 9 | 0,37 | -1,00 | 1,28E-02 | 4,86E-02 | 0,42 | 2,08E-01 | 3,57E-01 |
| SEQ ID NO: 303 | hsa-miR-374b* | 21 | 45 | 0,46 | -0,78 | 1,30E-02 | 4,91E-02 | 0,34 | 7,74E-02 | 1,89E-01 |
| SEQ ID NO: 175 | hsa-miR-200c* | 38 | 59 | 0,65 | -0,43 | 1,48E-04 | 2,60E-03 | 0,21 | 1,57E-05 | 4,84E-04 |
| SEQ ID NO: 159 | hsa-miR-199a-3p | 80 | 50 | 1,59 | 0,47 | 1,74E-04 | 3,00E-03 | 0,76 | 8,51E-04 | 1,01E-02 |
| SEQ ID NO: 304 | hsa-miR-130a* | 77 | 46 | 1,67 | 0,52 | 4,42E-04 | 5,87E-03 | 0,75 | 1,45E-03 | 1,44E-02 |
| SEQ ID NO: 305 | hsa-miR-1178 | 13 | 23 | 0,56 | -0,58 | 7,83E-04 | 8,35E-03 | 0,35 | 4,29E-02 | 1,24E-01 |
| SEQ ID NO: 161 | hsa-miR-516b* | 29 | 56 | 0,52 | -0,65 | 7,84E-04 | 8,35E-03 | 0,29 | 2,34E-02 | 8,38E-02 |
| SEQ ID NO: 306 | hsa-miR-26a-2* | 1 | 1 | 1,00 | 0,00 | 9,47E-04 | 9,29E-03 | 0,35 | 6,20E-03 | 3,55E-02 |
| SEQ ID NO: 307 | hsa-miR-302a | 1 | 1 | 1,00 | 0,00 | 9,65E-04 | 9,35E-03 | 0,40 | 1,04E-01 | 2,24E-01 |
| SEQ ID NO: 308 | hsa-miR-135a | 1 | 1 | 1,00 | 0,00 | 1,48E-03 | 1,24E-02 | 0,37 | 1,65E-02 | 6,87E-02 |
| SEQ ID NO: 309 | hsa-miR-377* | 1 | 1 | 0,72 | -0,33 | 2,11E-03 | 1,59E-02 | 0,36 | 5,70E-03 | 3,42E-02 |
| SEQ ID NO: 165 | hsa-miR-505 | 32 | 52 | 0,62 | -0,48 | 2,23E-03 | 1,66E-02 | 0,34 | 8,74E-02 | 2,00E-01 |
| SEQ ID NO: 310 | hsa-miR-522 | 3 | 6 | 0,55 | -0,60 | 2,30E-03 | 1,67E-02 | 0,39 | 1,26E-01 | 2,54E-01 |

| | | | | | | | | | | |
|---|---|---|---|---|---|---|---|---|---|---|
| SEQ ID NO: 311 | hsa-miR-1244 | 1 | 1 | 1,00 | 0,00 | 2,34E-03 | 1,68E-02 | 0,34 | 1,93E-03 | 1,78E-02 |
| SEQ ID NO: 312 | hsa-miR-220a | 29 | 57 | 0,52 | -0,66 | 2,49E-03 | 1,78E-02 | 0,30 | 1,81E-03 | 1,73E-02 |
| SEQ ID NO: 204 | hsa-miR-617 | 18 | 31 | 0,56 | -0,58 | 2,87E-03 | 1,95E-02 | 0,35 | 4,86E-02 | 1,37E-01 |
| SEQ ID NO: 313 | hsa-miR-655 | 21 | 36 | 0,59 | -0,53 | 3,04E-03 | 2,04E-02 | 0,32 | 1,95E-02 | 7,40E-02 |
| SEQ ID NO: 174 | hsa-miR-548c-3p | 27 | 50 | 0,53 | -0,63 | 3,49E-03 | 2,23E-02 | 0,29 | 1,76E-02 | 7,08E-02 |
| SEQ ID NO: 168 | hsa-miR-512-3p | 30 | 53 | 0,57 | -0,56 | 3,56E-03 | 2,26E-02 | 0,30 | 9,72E-04 | 1,10E-02 |
| SEQ ID NO: 314 | hsa-miR-214* | 34 | 52 | 0,64 | -0,44 | 3,60E-03 | 2,27E-02 | 0,31 | 1,06E-01 | 2,27E-01 |
| SEQ ID NO: 315 | hsa-miR-196b | 1 | 1 | 1,00 | 0,00 | 3,76E-03 | 2,31E-02 | 0,38 | 2,55E-02 | 8,83E-02 |
| SEQ ID NO: 316 | hsa-miR-205 | 35 | 62 | 0,56 | -0,58 | 3,86E-03 | 2,32E-02 | 0,32 | 1,66E-02 | 6,89E-02 |
| SEQ ID NO: 317 | hsa-miR-708* | 44 | 86 | 0,51 | -0,66 | 3,83E-03 | 2,32E-02 | 0,29 | 6,11E-04 | 8,11E-03 |
| SEQ ID NO: 213 | hsa-miR-1258 | 1 | 1 | 1,00 | 0,00 | 4,16E-03 | 2,42E-02 | 0,36 | 1,83E-02 | 7,17E-02 |
| SEQ ID NO: 318 | hsa-miR-132* | 1 | 1 | 0,72 | -0,33 | 6,03E-03 | 2,97E-02 | 0,39 | 7,41E-02 | 1,84E-01 |
| SEQ ID NO: 319 | hsa-miR-372 | 25 | 50 | 0,50 | -0,68 | 6,02E-03 | 2,97E-02 | 0,29 | 2,64E-03 | 2,11E-02 |
| SEQ ID NO: 162 | hsa-miR-199b-3p | 88 | 51 | 1,71 | 0,54 | 6,29E-03 | 3,03E-02 | 0,72 | 5,89E-03 | 3,46E-02 |
| SEQ ID NO: 219 | hsa-miR-1290 | 1 | 1 | 1,00 | 0,00 | 6,64E-03 | 3,15E-02 | 0,38 | 2,13E-02 | 7,75E-02 |
| SEQ ID NO: 190 | hsa-miR-656 | 26 | 51 | 0,51 | -0,66 | 7,15E-03 | 3,32E-02 | 0,36 | 7,06E-02 | 1,79E-01 |
| SEQ ID NO: 320 | hsa-miR-615-5p | 32 | 54 | 0,59 | -0,53 | 7,28E-03 | 3,36E-02 | 0,34 | 8,10E-02 | 1,92E-01 |
| SEQ ID NO: 321 | hsa-miR-520f | 1 | 1 | 1,00 | 0,00 | 7,37E-03 | 3,37E-02 | 0,39 | 4,72E-02 | 1,34E-01 |
| SEQ ID NO: 322 | hsa-miR-196a | 1 | 1 | 1,00 | 0,00 | 7,33E-03 | 3,37E-02 | 0,43 | 1,76E-01 | 3,21E-01 |
| SEQ ID NO: 323 | hsa-miR-561 | 32 | 51 | 0,62 | -0,47 | 8,20E-03 | 3,65E-02 | 0,32 | 1,81E-02 | 7,15E-02 |
| SEQ ID NO: 324 | hsa-miR-548l | 1 | 1 | 1,00 | 0,00 | 8,53E-03 | 3,72E-02 | 0,38 | 2,43E-02 | 8,58E-02 |
| SEQ ID NO: 325 | hsa-miR-1257 | 8 | 9 | 0,92 | -0,08 | 8,54E-03 | 3,72E-02 | 0,43 | 3,56E-01 | 5,05E-01 |
| SEQ ID NO: 326 | hsa-miR-548a-5p | 13 | 10 | 1,35 | 0,30 | 9,00E-03 | 3,84E-02 | 0,43 | 5,33E-01 | 6,81E-01 |
| SEQ ID NO: 327 | hsa-miR-653 | 19 | 31 | 0,60 | -0,51 | 9,80E-03 | 4,10E-02 | 0,37 | 5,89E-02 | 1,57E-01 |
| SEQ ID NO: 153 | hsa-miR-146b-5p | 99 | 66 | 1,51 | 0,41 | 1,12E-02 | 4,45E-02 | 0,68 | 1,37E-02 | 6,05E-02 |
| SEQ ID NO: 328 | hsa-miR-29a* | 1 | 1 | 1,00 | 0,00 | 1,16E-02 | 4,57E-02 | 0,43 | 1,77E-01 | 3,22E-01 |
| SEQ ID NO: 167 | hsa-miR-129* | 45 | 57 | 0,79 | -0,23 | 1,17E-02 | 4,57E-02 | 0,34 | 1,15E-03 | 1,19E-02 |
| SEQ ID NO: 211 | hsa-miR-605 | 18 | 28 | 0,62 | -0,47 | 1,18E-02 | 4,57E-02 | 0,41 | 1,91E-01 | 3,37E-01 |
| SEQ ID NO: 187 | hsa-miR-517a | 25 | 40 | 0,64 | -0,45 | 1,28E-02 | 4,86E-02 | 0,37 | 5,10E-02 | 1,42E-01 |
| SEQ ID NO: 329 | hsa-miR-371-3p | 27 | 51 | 0,52 | -0,66 | 1,27E-02 | 4,86E-02 | 0,33 | 1,97E-02 | 7,41E-02 |

# Figure 14

| SEQ ID NO: | miRNA | median g1 | median g2 | qmedian | logqmedian | ttest_rawp | ttest_adjp | AUC | limma_rawp | limma_adjp |
|---|---|---|---|---|---|---|---|---|---|---|
| SEQ ID NO: 2 | hsa-miR-151-3p | 2518 | 825 | 3,05 | 1,12 | 7,53E-16 | 6,50E-13 | 0,94 | 9,98E-09 | 8,61E-07 |
| SEQ ID NO: 10 | hsa-miR-320d | 2697 | 761 | 3,54 | 1,26 | 6,25E-15 | 2,70E-12 | 0,98 | 1,36E-10 | 5,85E-08 |
| SEQ ID NO: 24 | hsa-miR-30c | 4200 | 1405 | 2,99 | 1,09 | 1,23E-12 | 3,53E-10 | 0,93 | 8,21E-09 | 7,88E-07 |
| SEQ ID NO: 11 | hsa-miR-324-3p | 1797 | 702 | 2,56 | 0,94 | 9,46E-12 | 2,04E-09 | 0,94 | 7,34E-07 | 3,02E-05 |
| SEQ ID NO: 16 | hsa-miR-26a | 4002 | 8175 | 0,49 | -0,71 | 2,59E-11 | 4,46E-09 | 0,02 | 5,69E-09 | 6,13E-07 |
| SEQ ID NO: 35 | hsa-miR-93* | 2415 | 1184 | 2,04 | 0,71 | 6,33E-11 | 9,11E-09 | 0,90 | 4,24E-06 | 1,11E-04 |
| SEQ ID NO: 9 | hsa-let-7d | 611 | 2387 | 0,26 | -1,36 | 2,50E-10 | 2,69E-08 | 0,02 | 3,03E-06 | 8,77E-05 |
| SEQ ID NO: 14 | hsa-miR-550* | 1797 | 675 | 2,66 | 0,98 | 1,92E-09 | 1,78E-07 | 0,87 | 3,05E-06 | 8,77E-05 |
| SEQ ID NO: 28 | hsa-miR-720 | 9818 | 4645 | 2,11 | 0,75 | 2,39E-09 | 1,87E-07 | 0,89 | 2,52E-06 | 8,05E-05 |
| SEQ ID NO: 21 | hsa-miR-320c | 1919 | 752 | 2,55 | 0,94 | 3,93E-09 | 2,83E-07 | 0,89 | 4,91E-03 | 2,43E-02 |
| SEQ ID NO: 1 | hsa-miR-363 | 504 | 4636 | 0,11 | -2,22 | 4,63E-09 | 3,08E-07 | 0,04 | 5,75E-08 | 4,14E-06 |
| SEQ ID NO: 49 | hsa-miR-223 | 3793 | 1620 | 2,34 | 0,85 | 1,32E-08 | 7,60E-07 | 0,83 | 3,38E-05 | 6,21E-04 |
| SEQ ID NO: 47 | hsa-miR-532-3p | 6081 | 2941 | 2,07 | 0,73 | 3,26E-08 | 1,66E-06 | 0,89 | 4,06E-06 | 1,09E-04 |
| SEQ ID NO: 6 | hsa-miR-106a | 1621 | 4890 | 0,33 | -1,10 | 5,78E-08 | 2,77E-06 | 0,04 | 3,64E-07 | 1,65E-05 |
| SEQ ID NO: 3 | hsa-miR-17 | 1463 | 5550 | 0,26 | -1,33 | 1,69E-07 | 7,17E-06 | 0,04 | 1,58E-06 | 5,26E-05 |
| SEQ ID NO: 7 | hsa-miR-101 | 206 | 685 | 0,30 | -1,20 | 1,74E-07 | 7,17E-06 | 0,11 | 3,66E-05 | 6,56E-04 |
| SEQ ID NO: 69 | hsa-miR-126 | 191 | 2010 | 0,10 | -2,35 | 3,68E-07 | 1,38E-05 | 0,05 | 3,99E-11 | 3,44E-08 |
| SEQ ID NO: 42 | hsa-miR-30a | 840 | 305 | 2,75 | 1,01 | 4,99E-07 | 1,80E-05 | 0,77 | 4,65E-02 | 1,25E-01 |
| SEQ ID NO: 4 | hsa-miR-20b | 576 | 2160 | 0,27 | -1,32 | 6,50E-07 | 2,24E-05 | 0,07 | 1,13E-05 | 2,56E-04 |
| SEQ ID NO: 68 | hsa-miR-144 | 177 | 1478 | 0,12 | -2,12 | 7,49E-07 | 2,49E-05 | 0,08 | 1,96E-07 | 1,06E-05 |
| SEQ ID NO: 26 | hsa-miR-182 | 2306 | 6578 | 0,35 | -1,05 | 9,26E-07 | 2,96E-05 | 0,11 | 1,30E-06 | 4,69E-05 |
| SEQ ID NO: 30 | hsa-miR-664 | 998 | 265 | 3,76 | 1,33 | 1,06E-06 | 3,25E-05 | 0,85 | 3,76E-03 | 1,96E-02 |
| SEQ ID NO: 71 | hsa-miR-374b | 127 | 511 | 0,25 | -1,39 | 1,70E-06 | 5,06E-05 | 0,11 | 5,63E-06 | 1,39E-04 |
| SEQ ID NO: 43 | hsa-miR-28-5p | 214 | 506 | 0,42 | -0,86 | 1,97E-06 | 5,67E-05 | 0,13 | 1,19E-04 | 1,60E-03 |
| SEQ ID NO: 74 | hsa-let-7i | 194 | 868 | 0,22 | -1,50 | 3,51E-06 | 9,78E-05 | 0,14 | 1,40E-05 | 3,09E-04 |
| SEQ ID NO: 29 | hsa-miR-30e | 623 | 201 | 3,09 | 1,13 | 3,95E-06 | 1,03E-04 | 0,75 | 1,68E-01 | 3,17E-01 |
| SEQ ID NO: 72 | hsa-miR-21 | 150 | 1135 | 0,13 | -2,02 | 5,45E-06 | 1,38E-04 | 0,09 | 8,76E-07 | 3,29E-05 |
| SEQ ID NO: 12 | hsa-miR-15b | 5518 | 14381 | 0,38 | -0,96 | 9,80E-06 | 2,35E-04 | 0,13 | 2,54E-05 | 4,98E-04 |
| SEQ ID NO: 77 | hsa-miR-183 | 105 | 783 | 0,13 | -2,01 | 1,20E-05 | 2,80E-04 | 0,10 | 2,76E-07 | 1,32E-05 |

| | | | | | | | | | | |
|---|---|---|---|---|---|---|---|---|---|---|
| SEQ ID NO: 38 | hsa-let-7a | 611 | 1223 | 0,50 | -0,69 | 1,61E-05 | 3,22E-04 | 0,17 | 2,72E-04 | 3,04E-03 |
| SEQ ID NO: 76 | hsa-miR-18a* | 523 | 212 | 2,46 | 0,90 | 2,13E-05 | 4,19E-04 | 0,73 | 3,56E-01 | 5,22E-01 |
| SEQ ID NO: 8 | hsa-miR-20a | 714 | 2634 | 0,27 | -1,31 | 3,13E-05 | 5,41E-04 | 0,11 | 8,27E-05 | 1,18E-03 |
| SEQ ID NO: 41 | hsa-miR-29b | 675 | 337 | 2,00 | 0,70 | 4,02E-05 | 6,66E-04 | 0,82 | 1,80E-01 | 3,32E-01 |
| SEQ ID NO: 22 | hsa-miR-744 | 318 | 709 | 0,45 | -0,80 | 8,27E-05 | 1,19E-03 | 0,17 | 3,85E-04 | 3,98E-03 |
| SEQ ID NO: 44 | hsa-miR-1908 | 1832 | 529 | 3,47 | 1,24 | 1,42E-04 | 1,98E-03 | 0,76 | 3,67E-02 | 1,07E-01 |
| SEQ ID NO: 19 | hsa-miR-24 | 655 | 1797 | 0,36 | -1,01 | 1,83E-04 | 2,35E-03 | 0,16 | 2,95E-04 | 3,24E-03 |
| SEQ ID NO: 53 | hsa-miR-222 | 253 | 548 | 0,46 | -0,77 | 2,20E-04 | 2,67E-03 | 0,23 | 1,47E-03 | 1,07E-02 |
| SEQ ID NO: 330 | hsa-miR-1268 | 513 | 215 | 2,39 | 0,87 | 6,62E-04 | 6,14E-03 | 0,67 | 4,57E-01 | 6,08E-01 |
| SEQ ID NO: 70 | hsa-miR-144* | 134 | 451 | 0,30 | -1,21 | 6,72E-04 | 6,17E-03 | 0,20 | 5,61E-04 | 5,09E-03 |
| SEQ ID NO: 75 | hsa-let-7f | 123 | 573 | 0,21 | -1,54 | 3,29E-03 | 2,06E-02 | 0,27 | 2,82E-03 | 1,59E-02 |
| SEQ ID NO: 86 | hsa-miR-424 | 114 | 365 | 0,31 | -1,17 | 4,70E-03 | 2,52E-02 | 0,26 | 1,34E-03 | 9,88E-03 |
| SEQ ID NO: 232 | hsa-miR-149* | 1184 | 467 | 2,54 | 0,93 | 6,98E-03 | 3,20E-02 | 0,68 | 3,32E-01 | 5,03E-01 |
| SEQ ID NO: 17 | hsa-miR-107 | 500 | 1039 | 0,48 | -0,73 | 9,86E-03 | 4,11E-02 | 0,26 | 1,16E-02 | 4,54E-02 |
| SEQ ID NO: 15 | hsa-miR-30d | 13000 | 6756 | 1,92 | 0,65 | 2,40E-10 | 2,69E-08 | 0,92 | 2,57E-07 | 1,30E-05 |
| SEQ ID NO: 45 | hsa-miR-1260 | 4200 | 2360 | 1,78 | 0,58 | 2,06E-09 | 1,78E-07 | 0,87 | 1,59E-05 | 3,34E-04 |
| SEQ ID NO: 52 | hsa-miR-25 | 8175 | 4755 | 1,72 | 0,54 | 5,22E-09 | 3,22E-07 | 0,88 | 1,43E-04 | 1,84E-03 |
| SEQ ID NO: 64 | hsa-miR-151-5p | 3301 | 6578 | 0,50 | -0,69 | 3,75E-06 | 1,01E-04 | 0,13 | 9,22E-06 | 2,15E-04 |
| SEQ ID NO: 60 | hsa-miR-484 | 9219 | 6406 | 1,44 | 0,36 | 4,91E-05 | 7,99E-04 | 0,82 | 5,45E-04 | 5,05E-03 |
| SEQ ID NO: 18 | hsa-miR-320b | 5652 | 3623 | 1,56 | 0,44 | 1,65E-04 | 2,23E-03 | 0,78 | 3,89E-04 | 3,98E-03 |
| SEQ ID NO: 244 | hsa-miR-23a | 4406 | 6406 | 0,69 | -0,37 | 2,00E-04 | 2,48E-03 | 0,23 | 2,45E-03 | 1,41E-02 |
| SEQ ID NO: 25 | hsa-miR-106b | 5928 | 11107 | 0,53 | -0,63 | 2,37E-04 | 2,84E-03 | 0,22 | 1,35E-04 | 1,76E-03 |
| SEQ ID NO: 23 | hsa-miR-93 | 2112 | 3453 | 0,61 | -0,49 | 2,53E-04 | 2,91E-03 | 0,20 | 2,41E-03 | 1,41E-02 |
| SEQ ID NO: 331 | hsa-miR-92a | 13000 | 9820 | 1,32 | 0,28 | 3,86E-04 | 4,06E-03 | 0,75 | 9,87E-03 | 4,03E-02 |
| SEQ ID NO: 5 | hsa-miR-103 | 2306 | 4200 | 0,55 | -0,60 | 4,29E-04 | 4,46E-03 | 0,16 | 5,50E-04 | 5,05E-03 |
| SEQ ID NO: 59 | hsa-miR-339-5p | 1283 | 736 | 1,74 | 0,56 | 5,85E-04 | 5,67E-03 | 0,72 | 2,37E-01 | 3,94E-01 |
| SEQ ID NO: 55 | hsa-miR-181a | 1066 | 633 | 1,68 | 0,52 | 7,10E-04 | 6,38E-03 | 0,74 | 2,54E-01 | 4,14E-01 |
| SEQ ID NO: 33 | hsa-miR-130a | 765 | 1340 | 0,57 | -0,56 | 7,41E-04 | 6,53E-03 | 0,18 | 2,17E-03 | 1,32E-02 |
| SEQ ID NO: 27 | hsa-miR-320a | 24753 | 19074 | 1,30 | 0,26 | 8,74E-04 | 7,47E-03 | 0,72 | 1,14E-02 | 4,47E-02 |
| SEQ ID NO: 32 | hsa-miR-660 | 253 | 456 | 0,55 | -0,59 | 9,85E-04 | 8,33E-03 | 0,24 | 4,41E-03 | 2,21E-02 |
| SEQ ID NO: 54 | hsa-miR-1280 | 7722 | 4667 | 1,65 | 0,50 | 1,06E-03 | 8,70E-03 | 0,72 | 4,64E-04 | 4,55E-03 |
| SEQ ID NO: 88 | hsa-miR-152 | 154 | 255 | 0,60 | -0,51 | 2,52E-03 | 1,75E-02 | 0,22 | 9,76E-04 | 7,65E-03 |
| SEQ ID NO: 31 | hsa-miR-16 | 11107 | 15762 | 0,70 | -0,35 | 2,75E-03 | 1,82E-02 | 0,21 | 3,55E-01 | 5,22E-01 |
| SEQ ID NO: 332 | hsa-miR-590-5p | 127 | 193 | 0,66 | -0,42 | 2,99E-03 | 1,92E-02 | 0,28 | 9,50E-03 | 3,90E-02 |

| | | | | | | | | | | |
|---|---|---|---|---|---|---|---|---|---|---|
| SEQ ID NO: 333 | hsa-miR-1228* | 2010 | 1276 | 1,58 | 0,45 | 3,32E-03 | 2,06E-02 | 0,67 | 8,96E-01 | 9,42E-01 |
| SEQ ID NO: 67 | hsa-miR-425 | 15762 | 13875 | 1,14 | 0,13 | 3,71E-03 | 2,23E-02 | 0,69 | 8,40E-02 | 1,94E-01 |
| SEQ ID NO: 334 | hsa-miR-361-5p | 523 | 688 | 0,76 | -0,27 | 3,72E-03 | 2,23E-02 | 0,29 | 5,43E-03 | 2,61E-02 |
| SEQ ID NO: 335 | hsa-miR-23b | 3793 | 4303 | 0,88 | -0,13 | 3,85E-03 | 2,29E-02 | 0,30 | 4,63E-02 | 1,25E-01 |
| SEQ ID NO: 336 | hsa-miR-1234 | 533 | 343 | 1,55 | 0,44 | 3,96E-03 | 2,32E-02 | 0,71 | 5,69E-01 | 7,00E-01 |
| SEQ ID NO: 112 | hsa-miR-584 | 247 | 137 | 1,80 | 0,59 | 4,25E-03 | 2,40E-02 | 0,63 | 9,62E-01 | 9,79E-01 |
| SEQ ID NO: 20 | hsa-miR-18a | 445 | 793 | 0,56 | -0,58 | 4,37E-03 | 2,42E-02 | 0,26 | 2,95E-03 | 1,63E-02 |
| SEQ ID NO: 337 | hsa-miR-92b | 420 | 279 | 1,50 | 0,41 | 4,96E-03 | 2,58E-02 | 0,67 | 5,25E-01 | 6,71E-01 |
| SEQ ID NO: 239 | hsa-miR-142-5p | 675 | 584 | 1,16 | 0,14 | 5,48E-03 | 2,70E-02 | 0,60 | 5,91E-01 | 7,15E-01 |
| SEQ ID NO: 48 | hsa-miR-500 | 453 | 317 | 1,43 | 0,36 | 5,53E-03 | 2,70E-02 | 0,67 | 7,46E-01 | 8,37E-01 |
| SEQ ID NO: 236 | hsa-miR-1274b | 706 | 1194 | 0,59 | -0,53 | 7,27E-03 | 3,28E-02 | 0,32 | 1,47E-02 | 5,43E-02 |
| SEQ ID NO: 51 | hsa-miR-342-3p | 5384 | 3453 | 1,56 | 0,44 | 9,77E-03 | 4,09E-02 | 0,71 | 1,59E-02 | 5,76E-02 |
| SEQ ID NO: 13 | hsa-miR-148b | 274 | 334 | 0,82 | -0,20 | 9,97E-03 | 4,12E-02 | 0,33 | 2,68E-02 | 8,45E-02 |
| SEQ ID NO: 338 | hsa-miR-29c | 689 | 506 | 1,36 | 0,31 | 1,22E-02 | 4,78E-02 | 0,64 | 6,87E-01 | 7,89E-01 |
| SEQ ID NO: 238 | hsa-miR-185 | 20492 | 24753 | 0,83 | -0,19 | 1,27E-02 | 4,86E-02 | 0,35 | 4,32E-02 | 1,18E-01 |
| SEQ ID NO: 159 | hsa-miR-199a-3p | 50 | 135 | 0,37 | -0,99 | 2,75E-08 | 1,48E-06 | 0,08 | 2,95E-06 | 8,77E-05 |
| SEQ ID NO: 153 | hsa-miR-146b-5p | 66 | 185 | 0,36 | -1,04 | 1,27E-05 | 2,80E-04 | 0,14 | 2,53E-04 | 2,88E-03 |
| SEQ ID NO: 106 | hsa-miR-26b | 91 | 488 | 0,19 | -1,68 | 1,57E-05 | 3,22E-04 | 0,14 | 6,11E-06 | 1,47E-04 |
| SEQ ID NO: 107 | hsa-miR-27a | 64 | 568 | 0,11 | -2,18 | 2,28E-05 | 4,38E-04 | 0,05 | 3,46E-10 | 7,47E-08 |
| SEQ ID NO: 108 | hsa-let-7g | 81 | 507 | 0,16 | -1,84 | 2,85E-05 | 5,13E-04 | 0,19 | 7,89E-04 | 6,68E-03 |
| SEQ ID NO: 127 | hsa-let-7d* | 175 | 70 | 2,49 | 0,91 | 2,79E-05 | 5,13E-04 | 0,68 | 1,28E-01 | 2,58E-01 |
| SEQ ID NO: 138 | hsa-miR-16-2* | 196 | 69 | 2,85 | 1,05 | 3,96E-05 | 6,66E-04 | 0,75 | 8,64E-02 | 1,96E-01 |
| SEQ ID NO: 339 | hsa-miR-1273 | 52 | 142 | 0,36 | -1,01 | 5,30E-05 | 8,32E-04 | 0,18 | 2,97E-04 | 3,24E-03 |
| SEQ ID NO: 160 | hsa-miR-525-5p | 51 | 105 | 0,49 | -0,72 | 5,66E-05 | 8,73E-04 | 0,16 | 4,22E-05 | 7,14E-04 |
| SEQ ID NO: 147 | hsa-miR-328 | 106 | 47 | 2,28 | 0,82 | 6,27E-05 | 9,49E-04 | 0,71 | 3,40E-02 | 1,01E-01 |
| SEQ ID NO: 116 | hsa-miR-143 | 57 | 298 | 0,19 | -1,66 | 7,25E-05 | 1,06E-03 | 0,11 | 3,72E-05 | 6,56E-04 |
| SEQ ID NO: 114 | hsa-miR-96 | 57 | 236 | 0,24 | -1,42 | 7,27E-05 | 1,06E-03 | 0,15 | 6,30E-04 | 5,67E-03 |
| SEQ ID NO: 250 | hsa-miR-342-5p | 62 | 208 | 0,30 | -1,22 | 2,01E-04 | 2,48E-03 | 0,22 | 4,17E-03 | 2,11E-02 |
| SEQ ID NO: 340 | hsa-miR-383 | 35 | 102 | 0,35 | -1,05 | 2,69E-04 | 3,05E-03 | 0,21 | 6,68E-04 | 5,82E-03 |
| SEQ ID NO: 105 | hsa-miR-27b | 57 | 211 | 0,27 | -1,31 | 3,38E-04 | 3,70E-03 | 0,11 | 3,50E-06 | 9,75E-05 |
| SEQ ID NO: 121 | hsa-miR-628-3p | 66 | 193 | 0,34 | -1,07 | 4,93E-04 | 5,01E-03 | 0,16 | 8,47E-05 | 1,18E-03 |
| SEQ ID NO: 341 | hsa-miR-636 | 161 | 73 | 2,21 | 0,79 | 7,25E-04 | 6,45E-03 | 0,68 | 1,88E-01 | 3,39E-01 |
| SEQ ID NO: 109 | hsa-miR-374a | 96 | 208 | 0,46 | -0,77 | 1,24E-03 | 9,89E-03 | 0,23 | 8,33E-04 | 6,91E-03 |
| SEQ ID NO: 119 | hsa-miR-541 | 52 | 110 | 0,48 | -0,74 | 1,26E-03 | 9,95E-03 | 0,22 | 1,79E-02 | 6,21E-02 |

| | | | | | | | | | | |
|---|---|---|---|---|---|---|---|---|---|---|
| SEQ ID NO: 150 | hsa-miR-523* | 53 | 144 | 0,37 | -1,00 | 2,42E-03 | 1,71E-02 | 0,25 | 1,73E-03 | 1,15E-02 |
| SEQ ID NO: 176 | hsa-miR-1911* | 50 | 109 | 0,46 | -0,78 | 4,10E-03 | 2,38E-02 | 0,21 | 2,12E-03 | 1,31E-02 |
| SEQ ID NO: 154 | hsa-miR-454 | 68 | 142 | 0,48 | -0,74 | 4,20E-03 | 2,39E-02 | 0,25 | 8,87E-02 | 1,97E-01 |
| SEQ ID NO: 125 | hsa-miR-18b | 83 | 178 | 0,47 | -0,76 | 4,82E-03 | 2,55E-02 | 0,28 | 3,08E-02 | 9,49E-02 |
| SEQ ID NO: 135 | hsa-miR-196a* | 53 | 140 | 0,38 | -0,97 | 4,91E-03 | 2,57E-02 | 0,20 | 1,23E-03 | 9,38E-03 |
| SEQ ID NO: 342 | hsa-miR-146a* | 102 | 50 | 2,04 | 0,71 | 5,44E-03 | 2,70E-02 | 0,73 | 2,02E-04 | 2,45E-03 |
| SEQ ID NO: 110 | hsa-miR-1301 | 84 | 184 | 0,45 | -0,79 | 5,64E-03 | 2,72E-02 | 0,25 | 8,35E-03 | 3,55E-02 |
| SEQ ID NO: 247 | hsa-miR-625 | 51 | 178 | 0,29 | -1,24 | 6,21E-03 | 2,91E-02 | 0,25 | 1,63E-03 | 1,14E-02 |
| SEQ ID NO: 343 | hsa-miR-548p | 116 | 21 | 5,56 | 1,72 | 8,04E-03 | 3,52E-02 | 0,71 | 3,82E-05 | 6,60E-04 |
| SEQ ID NO: 117 | hsa-miR-588 | 72 | 138 | 0,52 | -0,65 | 1,51E-04 | 2,07E-03 | 0,17 | 3,00E-03 | 1,65E-02 |
| SEQ ID NO: 344 | hsa-miR-641 | 57 | 102 | 0,55 | -0,59 | 1,78E-04 | 2,33E-03 | 0,21 | 2,68E-03 | 1,53E-02 |
| SEQ ID NO: 345 | hsa-miR-221* | 82 | 134 | 0,61 | -0,49 | 2,51E-04 | 2,91E-03 | 0,23 | 1,06E-02 | 4,23E-02 |
| SEQ ID NO: 120 | hsa-miR-1254 | 74 | 117 | 0,63 | -0,46 | 2,84E-04 | 3,15E-03 | 0,23 | 1,69E-03 | 1,14E-02 |
| SEQ ID NO: 140 | hsa-miR-33a | 61 | 102 | 0,60 | -0,51 | 4,86E-04 | 4,99E-03 | 0,21 | 4,87E-02 | 1,30E-01 |
| SEQ ID NO: 146 | hsa-miR-1251 | 99 | 155 | 0,64 | -0,45 | 5,41E-04 | 5,37E-03 | 0,23 | 1,64E-02 | 5,85E-02 |
| SEQ ID NO: 115 | hsa-miR-1272 | 69 | 128 | 0,54 | -0,63 | 7,81E-04 | 6,80E-03 | 0,25 | 1,73E-02 | 6,08E-02 |
| SEQ ID NO: 346 | hsa-miR-376a | 82 | 144 | 0,57 | -0,57 | 1,49E-03 | 1,16E-02 | 0,21 | 9,04E-04 | 7,29E-03 |
| SEQ ID NO: 139 | hsa-miR-96* | 74 | 132 | 0,56 | -0,58 | 2,40E-03 | 1,71E-02 | 0,27 | 6,07E-03 | 2,83E-02 |
| SEQ ID NO: 181 | hsa-miR-490-3p | 72 | 105 | 0,69 | -0,37 | 4,46E-03 | 2,42E-02 | 0,27 | 1,68E-02 | 5,95E-02 |
| SEQ ID NO: 124 | hsa-miR-1286 | 69 | 117 | 0,59 | -0,53 | 4,35E-03 | 2,42E-02 | 0,29 | 2,17E-02 | 7,32E-02 |
| SEQ ID NO: 126 | hsa-miR-550 | 57 | 112 | 0,51 | -0,67 | 4,86E-03 | 2,56E-02 | 0,29 | 2,28E-02 | 7,62E-02 |
| SEQ ID NO: 122 | hsa-miR-143* | 62 | 116 | 0,53 | -0,63 | 5,50E-03 | 2,70E-02 | 0,24 | 1,10E-02 | 4,32E-02 |
| SEQ ID NO: 253 | hsa-miR-21* | 104 | 65 | 1,59 | 0,46 | 6,15E-03 | 2,90E-02 | 0,68 | 2,44E-02 | 7,90E-02 |
| SEQ ID NO: 145 | hsa-miR-338-3p | 89 | 173 | 0,51 | -0,67 | 6,35E-03 | 2,96E-02 | 0,26 | 1,83E-03 | 1,20E-02 |
| SEQ ID NO: 134 | hsa-miR-1914* | 77 | 128 | 0,61 | -0,50 | 1,08E-02 | 4,35E-02 | 0,31 | 6,59E-03 | 2,95E-02 |
| SEQ ID NO: 316 | hsa-miR-205 | 62 | 18 | 3,48 | 1,25 | 7,87E-08 | 3,58E-06 | 0,87 | 1,15E-07 | 7,08E-06 |
| SEQ ID NO: 256 | hsa-miR-182* | 52 | 5 | 11,45 | 2,44 | 2,92E-07 | 1,15E-05 | 0,85 | 5,72E-07 | 2,47E-05 |
| SEQ ID NO: 167 | hsa-miR-129* | 57 | 6 | 8,98 | 2,20 | 9,78E-06 | 2,35E-04 | 0,86 | 2,39E-10 | 6,87E-08 |
| SEQ ID NO: 220 | hsa-miR-559 | 54 | 13 | 4,33 | 1,47 | 1,25E-05 | 2,80E-04 | 0,79 | 1,39E-06 | 4,79E-05 |
| SEQ ID NO: 257 | hsa-miR-136 | 51 | 3 | 18,06 | 2,89 | 1,35E-05 | 2,83E-04 | 0,78 | 2,17E-04 | 2,55E-03 |
| SEQ ID NO: 175 | hsa-miR-200c* | 59 | 22 | 2,74 | 1,01 | 1,34E-05 | 2,83E-04 | 0,87 | 3,36E-09 | 4,83E-07 |
| SEQ ID NO: 164 | hsa-miR-335* | 50 | 1 | 50,24 | 3,92 | 2,49E-05 | 4,67E-04 | 0,77 | 7,39E-05 | 1,12E-03 |
| SEQ ID NO: 212 | hsa-miR-323-3p | 52 | 8 | 6,67 | 1,90 | 3,12E-05 | 5,41E-04 | 0,80 | 5,27E-06 | 1,34E-04 |
| SEQ ID NO: 347 | hsa-miR-518b | 82 | 17 | 4,85 | 1,58 | 5,07E-05 | 8,11E-04 | 0,78 | 8,00E-07 | 3,14E-05 |

| | | | | | | | | | | |
|---|---|---|---|---|---|---|---|---|---|---|
| SEQ ID NO: 348 | hsa-miR-492 | 57 | 25 | 2,24 | 0,81 | 1,02E-04 | 1,45E-03 | 0,79 | 8,36E-08 | 5,55E-06 |
| SEQ ID NO: 349 | hsa-miR-518f | 64 | 31 | 2,03 | 0,71 | 1,68E-04 | 2,24E-03 | 0,73 | 9,28E-03 | 3,87E-02 |
| SEQ ID NO: 161 | hsa-miR-516b* | 56 | 13 | 4,27 | 1,45 | 1,96E-04 | 2,48E-03 | 0,76 | 6,84E-04 | 5,84E-03 |
| SEQ ID NO: 265 | hsa-miR-548m | 16 | 1 | 16,40 | 2,80 | 2,41E-04 | 2,85E-03 | 0,70 | 6,47E-04 | 5,70E-03 |
| SEQ ID NO: 350 | hsa-miR-200a* | 69 | 8 | 8,70 | 2,16 | 2,74E-04 | 3,07E-03 | 0,84 | 8,80E-10 | 1,52E-07 |
| SEQ ID NO: 222 | hsa-miR-1276 | 52 | 5 | 10,03 | 2,31 | 5,74E-04 | 5,63E-03 | 0,79 | 2,01E-05 | 4,14E-04 |
| SEQ ID NO: 351 | hsa-miR-135a* | 40 | 1 | 39,92 | 3,69 | 5,91E-04 | 5,67E-03 | 0,75 | 4,55E-04 | 4,52E-03 |
| SEQ ID NO: 185 | hsa-let-7c* | 51 | 1 | 51,33 | 3,94 | 6,14E-04 | 5,82E-03 | 0,83 | 1,28E-07 | 7,38E-06 |
| SEQ ID NO: 228 | hsa-miR-886-3p | 23 | 1 | 22,66 | 3,12 | 6,53E-04 | 6,12E-03 | 0,71 | 2,34E-03 | 1,38E-02 |
| SEQ ID NO: 177 | hsa-miR-517c | 43 | 1 | 43,28 | 3,77 | 6,80E-04 | 6,18E-03 | 0,76 | 4,83E-04 | 4,68E-03 |
| SEQ ID NO: 182 | hsa-miR-483-3p | 62 | 1 | 54,49 | 4,00 | 8,24E-04 | 7,11E-03 | 0,83 | 4,73E-09 | 5,83E-07 |
| SEQ ID NO: 323 | hsa-miR-561 | 51 | 14 | 3,59 | 1,28 | 9,96E-04 | 8,35E-03 | 0,73 | 8,20E-05 | 1,18E-03 |
| SEQ ID NO: 352 | hsa-miR-1308 | 50 | 1 | 34,49 | 3,54 | 1,13E-03 | 9,13E-03 | 0,74 | 6,76E-05 | 1,05E-03 |
| SEQ ID NO: 183 | hsa-miR-187 | 32 | 5 | 5,93 | 1,78 | 1,58E-03 | 1,22E-02 | 0,66 | 3,72E-02 | 1,08E-01 |
| SEQ ID NO: 353 | hsa-miR-384 | 66 | 18 | 3,61 | 1,28 | 1,72E-03 | 1,30E-02 | 0,77 | 2,97E-05 | 5,57E-04 |
| SEQ ID NO: 320 | hsa-miR-615-5p | 54 | 19 | 2,78 | 1,02 | 1,88E-03 | 1,41E-02 | 0,73 | 2,19E-03 | 1,32E-02 |
| SEQ ID NO: 209 | hsa-miR-548b-5p | 30 | 1 | 30,10 | 3,40 | 1,93E-03 | 1,44E-02 | 0,71 | 2,04E-03 | 1,28E-02 |
| SEQ ID NO: 186 | hsa-miR-649 | 30 | 2 | 14,76 | 2,69 | 2,04E-03 | 1,49E-02 | 0,69 | 7,61E-03 | 3,30E-02 |
| SEQ ID NO: 286 | hsa-miR-507 | 31 | 1 | 30,77 | 3,43 | 2,28E-03 | 1,65E-02 | 0,70 | 1,64E-03 | 1,14E-02 |
| SEQ ID NO: 202 | hsa-miR-454* | 52 | 7 | 7,13 | 1,96 | 2,31E-03 | 1,66E-02 | 0,82 | 6,18E-05 | 9,87E-04 |
| SEQ ID NO: 354 | hsa-miR-657 | 32 | 75 | 0,43 | -0,84 | 2,43E-03 | 1,71E-02 | 0,20 | 2,26E-03 | 1,34E-02 |
| SEQ ID NO: 355 | hsa-miR-662 | 67 | 5 | 12,66 | 2,54 | 2,56E-03 | 1,75E-02 | 0,86 | 2,08E-08 | 1,63E-06 |
| SEQ ID NO: 191 | hsa-miR-569 | 52 | 1 | 51,78 | 3,95 | 2,55E-03 | 1,75E-02 | 0,73 | 6,76E-04 | 5,84E-03 |
| SEQ ID NO: 313 | hsa-miR-655 | 36 | 16 | 2,25 | 0,81 | 2,71E-03 | 1,82E-02 | 0,72 | 1,87E-03 | 1,21E-02 |
| SEQ ID NO: 287 | hsa-miR-539 | 16 | 1 | 15,54 | 2,74 | 2,70E-03 | 1,82E-02 | 0,71 | 3,92E-04 | 3,98E-03 |
| SEQ ID NO: 356 | hsa-miR-1911 | 46 | 3 | 17,71 | 2,87 | 2,77E-03 | 1,82E-02 | 0,78 | 8,04E-05 | 1,18E-03 |
| SEQ ID NO: 357 | hsa-miR-337-3p | 31 | 1 | 30,77 | 3,43 | 2,97E-03 | 1,92E-02 | 0,72 | 3,11E-04 | 3,35E-03 |
| SEQ ID NO: 267 | hsa-miR-557 | 51 | 21 | 2,45 | 0,90 | 2,99E-03 | 1,92E-02 | 0,74 | 1,31E-03 | 9,84E-03 |
| SEQ ID NO: 358 | hsa-miR-98 | 8 | 1 | 7,97 | 2,08 | 3,26E-03 | 2,05E-02 | 0,62 | 7,86E-02 | 1,86E-01 |
| SEQ ID NO: 280 | hsa-miR-23a* | 30 | 1 | 30,05 | 3,40 | 3,72E-03 | 2,23E-02 | 0,66 | 9,98E-03 | 4,04E-02 |
| SEQ ID NO: 230 | hsa-miR-758 | 28 | 1 | 28,41 | 3,35 | 3,72E-03 | 2,23E-02 | 0,68 | 5,31E-03 | 2,59E-02 |
| SEQ ID NO: 359 | hsa-miR-429 | 50 | 11 | 4,68 | 1,54 | 4,13E-03 | 2,38E-02 | 0,70 | 2,45E-03 | 1,41E-02 |
| SEQ ID NO: 360 | hsa-miR-572 | 67 | 31 | 2,18 | 0,78 | 4,42E-03 | 2,42E-02 | 0,76 | 3,36E-03 | 1,82E-02 |
| SEQ ID NO: 361 | hsa-miR-30c-1* | 39 | 1 | 38,56 | 3,65 | 4,53E-03 | 2,44E-02 | 0,75 | 2,17E-05 | 4,36E-04 |

| | | | | | | | | | | |
|---|---|---|---|---|---|---|---|---|---|---|
| SEQ ID NO: 180 | hsa-miR-502-5p | 36 | 2 | 18,02 | 2,89 | 4,78E-03 | 2,55E-02 | 0,65 | 2,90E-02 | 9,07E-02 |
| SEQ ID NO: 362 | hsa-miR-211 | 9 | 1 | 8,73 | 2,17 | 5,18E-03 | 2,65E-02 | 0,69 | 1,04E-03 | 8,08E-03 |
| SEQ ID NO: 302 | hsa-miR-154 | 9 | 1 | 8,73 | 2,17 | 5,20E-03 | 2,65E-02 | 0,63 | 3,84E-02 | 1,10E-01 |
| SEQ ID NO: 363 | hsa-miR-548n | 44 | 12 | 3,53 | 1,26 | 5,29E-03 | 2,66E-02 | 0,66 | 5,27E-02 | 1,37E-01 |
| SEQ ID NO: 364 | hsa-miR-548k | 35 | 1 | 35,35 | 3,57 | 5,28E-03 | 2,66E-02 | 0,77 | 2,95E-05 | 5,57E-04 |
| SEQ ID NO: 231 | hsa-miR-9 | 17 | 1 | 17,46 | 2,86 | 5,42E-03 | 2,70E-02 | 0,75 | 5,17E-05 | 8,42E-04 |
| SEQ ID NO: 295 | hsa-miR-135b | 23 | 1 | 22,66 | 3,12 | 5,75E-03 | 2,76E-02 | 0,66 | 2,06E-02 | 7,02E-02 |
| SEQ ID NO: 319 | hsa-miR-372 | 50 | 24 | 2,12 | 0,75 | 6,05E-03 | 2,87E-02 | 0,72 | 5,35E-04 | 5,05E-03 |
| SEQ ID NO: 365 | hsa-miR-613 | 12 | 46 | 0,26 | -1,37 | 6,69E-03 | 3,10E-02 | 0,34 | 5,69E-02 | 1,47E-01 |
| SEQ ID NO: 264 | hsa-miR-493 | 42 | 20 | 2,09 | 0,74 | 7,57E-03 | 3,35E-02 | 0,67 | 1,60E-02 | 5,78E-02 |
| SEQ ID NO: 260 | hsa-let-7b* | 52 | 12 | 4,44 | 1,49 | 7,85E-03 | 3,46E-02 | 0,70 | 4,05E-03 | 2,06E-02 |
| SEQ ID NO: 366 | hsa-miR-99a* | 29 | 71 | 0,41 | -0,89 | 8,24E-03 | 3,57E-02 | 0,27 | 4,02E-03 | 2,05E-02 |
| SEQ ID NO: 217 | hsa-miR-148a* | 52 | 18 | 2,83 | 1,04 | 9,02E-03 | 3,85E-02 | 0,70 | 7,04E-03 | 3,09E-02 |
| SEQ ID NO: 281 | hsa-miR-302f | 34 | 1 | 34,05 | 3,53 | 9,35E-03 | 3,98E-02 | 0,71 | 3,69E-03 | 1,94E-02 |
| SEQ ID NO: 367 | hsa-miR-1248 | 50 | 4 | 13,03 | 2,57 | 1,01E-02 | 4,14E-02 | 0,79 | 1,53E-05 | 3,30E-04 |
| SEQ ID NO: 368 | hsa-miR-29b-1* | 10 | 1 | 9,57 | 2,26 | 1,01E-02 | 4,15E-02 | 0,61 | 6,37E-02 | 1,60E-01 |
| SEQ ID NO: 296 | hsa-miR-219-2-3p | 17 | 1 | 17,46 | 2,86 | 1,06E-02 | 4,28E-02 | 0,66 | 1,47E-02 | 5,43E-02 |
| SEQ ID NO: 369 | hsa-miR-184 | 28 | 58 | 0,48 | -0,73 | 1,09E-02 | 4,37E-02 | 0,28 | 6,96E-03 | 3,06E-02 |
| SEQ ID NO: 327 | hsa-miR-653 | 31 | 13 | 2,44 | 0,89 | 1,16E-02 | 4,61E-02 | 0,67 | 1,35E-02 | 5,10E-02 |
| SEQ ID NO: 184 | hsa-miR-519b-3p | 50 | 24 | 2,13 | 0,76 | 1,17E-02 | 4,64E-02 | 0,69 | 1,97E-03 | 1,25E-02 |
| SEQ ID NO: 370 | hsa-miR-566 | 80 | 32 | 2,47 | 0,90 | 1,20E-02 | 4,71E-02 | 0,67 | 3,23E-04 | 3,44E-03 |
| SEQ ID NO: 271 | hsa-miR-526b* | 23 | 1 | 23,30 | 3,15 | 1,20E-02 | 4,72E-02 | 0,65 | 2,49E-02 | 7,98E-02 |
| SEQ ID NO: 371 | hsa-miR-302b* | 32 | 3 | 10,47 | 2,35 | 1,26E-02 | 4,86E-02 | 0,69 | 7,72E-03 | 3,33E-02 |
| SEQ ID NO: 218 | hsa-miR-541* | 30 | 3 | 10,57 | 2,36 | 1,27E-02 | 4,86E-02 | 0,66 | 2,44E-02 | 7,90E-02 |
| SEQ ID NO: 372 | hsa-miR-623 | 57 | 42 | 1,36 | 0,31 | 3,50E-04 | 3,78E-03 | 0,73 | 2,19E-04 | 2,55E-03 |
| SEQ ID NO: 165 | hsa-miR-505 | 52 | 27 | 1,97 | 0,68 | 3,66E-04 | 3,90E-03 | 0,75 | 1,63E-04 | 2,07E-03 |
| SEQ ID NO: 373 | hsa-miR-122* | 57 | 29 | 1,96 | 0,67 | 5,25E-04 | 5,27E-03 | 0,73 | 2,06E-03 | 1,28E-02 |
| SEQ ID NO: 374 | hsa-miR-1233 | 71 | 54 | 1,31 | 0,27 | 1,01E-03 | 8,37E-03 | 0,71 | 1,88E-04 | 2,36E-03 |
| SEQ ID NO: 168 | hsa-miR-512-3p | 53 | 34 | 1,55 | 0,44 | 1,10E-03 | 8,92E-03 | 0,71 | 6,43E-04 | 5,70E-03 |
| SEQ ID NO: 318 | hsa-miR-132* | 1 | 1 | 1,38 | 0,33 | 1,32E-03 | 1,03E-02 | 0,70 | 1,30E-03 | 9,82E-03 |
| SEQ ID NO: 205 | hsa-miR-551b* | 56 | 28 | 1,98 | 0,68 | 1,61E-03 | 1,23E-02 | 0,72 | 4,16E-02 | 1,15E-01 |
| SEQ ID NO: 309 | hsa-miR-377* | 1 | 1 | 1,38 | 0,33 | 1,98E-03 | 1,46E-02 | 0,64 | 1,56E-02 | 5,71E-02 |
| SEQ ID NO: 215 | hsa-miR-409-3p | 59 | 42 | 1,41 | 0,35 | 2,71E-03 | 1,82E-02 | 0,68 | 1,82E-02 | 6,31E-02 |
| SEQ ID NO: 311 | hsa-miR-1244 | 1 | 1 | 1,00 | 0,00 | 3,00E-03 | 1,92E-02 | 0,67 | 6,42E-03 | 2,90E-02 |

| | | | | | | | | | | |
|---|---|---|---|---|---|---|---|---|---|---|
| SEQ ID NO: 328 | hsa-miR-29a* | 1 | 1 | 1,00 | 0,00 | 3,06E-03 | 1,94E-02 | 0,64 | 1,44E-02 | 5,38E-02 |
| SEQ ID NO: 375 | hsa-miR-606 | 57 | 35 | 1,62 | 0,48 | 3,34E-03 | 2,06E-02 | 0,69 | 5,39E-03 | 2,61E-02 |
| SEQ ID NO: 376 | hsa-miR-24-1* | 79 | 46 | 1,73 | 0,55 | 3,97E-03 | 2,32E-02 | 0,69 | 1,03E-04 | 1,42E-03 |
| SEQ ID NO: 315 | hsa-miR-196b | 1 | 1 | 1,00 | 0,00 | 3,93E-03 | 2,32E-02 | 0,65 | 9,47E-03 | 3,90E-02 |
| SEQ ID NO: 377 | hsa-miR-875-3p | 52 | 97 | 0,54 | -0,62 | 4,20E-03 | 2,39E-02 | 0,27 | 1,20E-02 | 4,68E-02 |
| SEQ ID NO: 306 | hsa-miR-26a-2* | 1 | 1 | 1,00 | 0,00 | 4,46E-03 | 2,42E-02 | 0,61 | 4,19E-02 | 1,16E-01 |
| SEQ ID NO: 378 | hsa-miR-873 | 52 | 83 | 0,62 | -0,48 | 4,42E-03 | 2,42E-02 | 0,27 | 3,63E-02 | 1,07E-01 |
| SEQ ID NO: 379 | hsa-miR-224 | 50 | 88 | 0,57 | -0,56 | 5,11E-03 | 2,64E-02 | 0,29 | 7,18E-02 | 1,74E-01 |
| SEQ ID NO: 380 | hsa-miR-1263 | 58 | 32 | 1,79 | 0,58 | 5,26E-03 | 2,66E-02 | 0,72 | 1,07E-03 | 8,24E-03 |
| SEQ ID NO: 190 | hsa-miR-656 | 51 | 38 | 1,37 | 0,31 | 5,65E-03 | 2,72E-02 | 0,63 | 1,84E-01 | 3,36E-01 |
| SEQ ID NO: 304 | hsa-miR-130a* | 46 | 57 | 0,80 | -0,22 | 6,06E-03 | 2,87E-02 | 0,30 | 8,62E-03 | 3,65E-02 |
| SEQ ID NO: 381 | hsa-miR-181a* | 73 | 97 | 0,75 | -0,28 | 6,72E-03 | 3,10E-02 | 0,30 | 3,32E-02 | 9,94E-02 |
| SEQ ID NO: 382 | hsa-miR-767-3p | 36 | 64 | 0,56 | -0,58 | 7,05E-03 | 3,22E-02 | 0,28 | 4,24E-04 | 4,25E-03 |
| SEQ ID NO: 324 | hsa-miR-548l | 1 | 1 | 1,00 | 0,00 | 7,20E-03 | 3,27E-02 | 0,67 | 2,95E-03 | 1,63E-02 |
| SEQ ID NO: 383 | hsa-miR-302d | 1 | 1 | 1,00 | 0,00 | 7,35E-03 | 3,30E-02 | 0,67 | 2,77E-03 | 1,57E-02 |
| SEQ ID NO: 384 | hsa-miR-937 | 52 | 43 | 1,21 | 0,19 | 7,50E-03 | 3,34E-02 | 0,63 | 8,91E-02 | 1,97E-01 |
| SEQ ID NO: 219 | hsa-miR-1290 | 1 | 1 | 1,00 | 0,00 | 7,49E-03 | 3,34E-02 | 0,63 | 3,99E-02 | 1,12E-01 |
| SEQ ID NO: 385 | hsa-miR-373* | 32 | 59 | 0,54 | -0,62 | 8,18E-03 | 3,56E-02 | 0,30 | 1,34E-02 | 5,09E-02 |
| SEQ ID NO: 386 | hsa-miR-1261 | 1 | 1 | 1,00 | 0,00 | 8,80E-03 | 3,80E-02 | 0,64 | 2,45E-02 | 7,90E-02 |
| SEQ ID NO: 274 | hsa-miR-220b | 71 | 37 | 1,90 | 0,64 | 8,91E-03 | 3,83E-02 | 0,73 | 2,17E-04 | 2,55E-03 |
| SEQ ID NO: 307 | hsa-miR-302a | 1 | 1 | 1,00 | 0,00 | 9,44E-03 | 3,99E-02 | 0,59 | 3,05E-01 | 4,70E-01 |
| SEQ ID NO: 162 | hsa-miR-199b-3p | 51 | 86 | 0,60 | -0,52 | 9,50E-03 | 4,00E-02 | 0,26 | 6,83E-03 | 3,02E-02 |
| SEQ ID NO: 387 | hsa-miR-188-5p | 96 | 61 | 1,56 | 0,45 | 9,97E-03 | 4,12E-02 | 0,64 | 2,25E-01 | 3,85E-01 |
| SEQ ID NO: 388 | hsa-miR-374a* | 30 | 23 | 1,28 | 0,25 | 1,02E-02 | 4,17E-02 | 0,65 | 1,09E-02 | 4,32E-02 |
| SEQ ID NO: 389 | hsa-miR-499-3p | 89 | 65 | 1,37 | 0,31 | 1,10E-02 | 4,38E-02 | 0,69 | 4,93E-04 | 4,73E-03 |
| SEQ ID NO: 390 | hsa-miR-622 | 53 | 92 | 0,58 | -0,55 | 1,23E-02 | 4,80E-02 | 0,27 | 1,92E-01 | 3,44E-01 |
| SEQ ID NO: 391 | hsa-miR-513b | 39 | 50 | 0,78 | -0,25 | 1,24E-02 | 4,81E-02 | 0,32 | 4,92E-03 | 2,43E-02 |

## Figure 15

| Set No. | SEQ-ID NO: | miRNA -identifiers | Acc | Spec | Sens |
|---|---|---|---|---|---|
| SH-1 | SEQ ID NO: 1, SEQ ID NO: 2, SEQ ID NO: 3 | hsa-miR-363, hsa-miR-151-3p, hsa-miR-17 | 94,2% | 95,1% | 93,3% |
| SH-2 | SEQ ID NO: 3, SEQ ID NO: 4, SEQ ID NO: 5 | hsa-miR-17, hsa-miR-20b, hsa-miR-103 | 94,4% | 92,8% | 96,1% |
| SH-3 | SEQ ID NO: 5, SEQ ID NO: 6, SEQ ID NO: 7 | hsa-miR-103, hsa-miR-106a, hsa-miR-101 | 95,2% | 96,7% | 93,6% |
| SH-4 | SEQ ID NO: 7, SEQ ID NO: 8, SEQ ID NO: 9 | hsa-miR-101, hsa-miR-20a, hsa-let-7d | 91,6% | 90,5% | 92,6% |
| SH-5 | SEQ ID NO: 9, SEQ ID NO: 10, SEQ ID NO: 11 | hsa-let-7d, hsa-miR-320d, hsa-miR-324-3p | 94,2% | 93,4% | 95,0% |
| SH-6 | SEQ ID NO: 11, SEQ ID NO: 12, SEQ ID NO: 13 | hsa-miR-324-3p, hsa-miR-15b, hsa-miR-148b | 88,9% | 94,4% | 83,4% |
| SH-7 | SEQ ID NO: 13, SEQ ID NO: 14, SEQ ID NO: 15 | hsa-miR-148b, hsa-miR-550*, hsa-miR-30d | 87,0% | 84,4% | 89,6% |
| SH-8 | SEQ ID NO: 15, SEQ ID NO: 16, SEQ ID NO: 17 | hsa-miR-30d, hsa-miR-26a, hsa-miR-107 | 86,8% | 87,0% | 86,5% |
| SH-9 | SEQ ID NO: 17, SEQ ID NO: 18, SEQ ID NO: 19 | hsa-miR-107, hsa-miR-320b, hsa-miR-24 | 93,3% | 94,4% | 92,3% |
| SH-10 | SEQ ID NO: 19, SEQ ID NO: 20, SEQ ID NO: 21 | hsa-miR-24, hsa-miR-18a, hsa-miR-320c | 90,3% | 89,9% | 90,8% |
| SH-11 | SEQ ID NO: 21, SEQ ID NO: 22, SEQ ID NO: 23 | hsa-miR-320c, hsa-miR-744, hsa-miR-93 | 90,3% | 91,2% | 89,3% |
| SH-12 | SEQ ID NO: 23, SEQ ID NO: 24, SEQ ID NO: 25 | hsa-miR-93, hsa-miR-30c, hsa-miR-106b | 79,0% | 81,7% | 76,4% |
| SH-13 | SEQ ID NO: 25, SEQ ID NO: 27, SEQ ID NO: 29 | hsa-miR-106b, hsa-miR-320a, hsa-miR-30e | 87,6% | 86,9% | 88,2% |
| SH-14 | SEQ ID NO: 27, SEQ ID NO: 28, SEQ ID NO: 29 | hsa-miR-320a, hsa-miR-720, hsa-miR-30e | 88,7% | 88,2% | 89,2% |

| | | | | | |
|---|---|---|---|---|---|
| SH-15 | SEQ ID NO: 29, SEQ ID NO: 30, SEQ ID NO: 31 | hsa-miR-30e, hsa-miR-664, hsa-miR-16 | 84,0% | 93,9% | 74,2% |
| SH-16 | SEQ ID NO: 1, SEQ ID NO: 2, SEQ ID NO: 3, SEQ ID NO: 4, SEQ ID NO: 5 | hsa-miR-363, hsa-miR-151-3p, hsa-miR-17, hsa-miR-20b, hsa-miR-103 | 95,8% | 98,0% | 93,6% |
| SH-17 | SEQ ID NO: 3, SEQ ID NO: 4, SEQ ID NO: 5, SEQ ID NO: 6, SEQ ID NO: 7 | hsa-miR-17, hsa-miR-20b, hsa-miR-103, hsa-miR-106a, hsa-miR-101 | 93,9% | 94,0% | 93,8% |
| SH-18 | SEQ ID NO: 5, SEQ ID NO: 6, SEQ ID NO: 7, SEQ ID NO: 8, SEQ ID NO: 9 | hsa-miR-103, hsa-miR-106a, hsa-miR-101, hsa-miR-20a, hsa-let-7d | 94,7% | 94,2% | 95,2% |
| SH-19 | SEQ ID NO: 6, SEQ ID NO: 7, SEQ ID NO: 8, SEQ ID NO: 9, SEQ ID NO: 10 | hsa-miR-106a, hsa-miR-101, hsa-miR-20a, hsa-let-7d, hsa-miR-320d | 95,5% | 98,1% | 92,9% |
| SH-20 | SEQ ID NO: 8, SEQ ID NO: 9, SEQ ID NO: 10, SEQ ID NO: 11, SEQ ID NO: 12 | hsa-miR-20a, hsa-let-7d, hsa-miR-320d, hsa-miR-324-3p, hsa-miR-15b | 97,9% | 98,0% | 97,8% |
| SH-21 | SEQ ID NO: 10, SEQ ID NO: 11, SEQ ID NO: 12, SEQ ID NO: 13, SEQ ID NO: 14 | hsa-miR-320d, hsa-miR-324-3p, hsa-miR-15b, hsa-miR-148b, hsa-miR-550* | 97,3% | #### | 94,6% |
| SH-22 | SEQ ID NO: 11, SEQ ID NO: 12, SEQ ID NO: 13, SEQ ID NO: 14, SEQ ID NO: 15, SEQ ID NO: 16 | hsa-miR-324-3p, hsa-miR-15b, hsa-miR-148b, hsa-miR-550*, hsa-miR-30d, hsa-miR-26a | 91,7% | 94,9% | 88,5% |
| SH-23 | SEQ ID NO: 14, SEQ ID NO: 15, SEQ ID NO: 16, SEQ ID NO: 17, SEQ ID NO: 18, SEQ ID NO: 19 | hsa-miR-550*, hsa-miR-30d, hsa-miR-26a, hsa-miR-107, hsa-miR-320b, hsa-miR-24 | 93,2% | 95,2% | 91,2% |
| SH-24 | SEQ ID NO: 17, SEQ ID NO: 18, SEQ ID NO: 19, SEQ ID NO: 20, SEQ ID NO: 21, SEQ ID NO: 22 | hsa-miR-107, hsa-miR-320b, hsa-miR-24, hsa-miR-18a, hsa-miR-320c, hsa-miR-744 | 94,3% | 95,4% | 93,2% |
| SH-25 | SEQ ID NO: 20, SEQ ID NO: 21, SEQ ID NO: 22, SEQ ID NO: 23, SEQ ID NO: 24, SEQ ID NO: 25 | hsa-miR-18a, hsa-miR-320c, hsa-miR-744, hsa-miR-93, hsa-miR-30c, hsa-miR-106b | 90,5% | 87,6% | 93,3% |
| SH-26 | SEQ ID NO: 23, SEQ ID NO: 24, SEQ ID NO: 25, SEQ ID NO: 26, SEQ ID NO: 27, SEQ ID NO: 28 | hsa-miR-93, hsa-miR-30c, hsa-miR-106b, hsa-miR-182, hsa-miR-320a, hsa-miR-720 | 94,8% | 94,0% | 95,6% |
| SH-27 | SEQ ID NO: 26, SEQ ID NO: 27, SEQ ID NO: 28, SEQ ID NO: 29, SEQ ID NO: 30, SEQ ID NO: 31 | hsa-miR-182, hsa-miR-320a, hsa-miR-720, hsa-miR-30e, hsa-miR-664, hsa-miR-16 | 94,2% | 92,3% | 96,2% |

| | | | | | |
|---|---|---|---|---|---|
| SH-28 | SEQ ID NO: 29, SEQ ID NO: 30, SEQ ID NO: 31, SEQ ID NO: 32, SEQ ID NO: 33, SEQ ID NO: 34 | hsa-miR-30e, hsa-miR-664, hsa-miR-16, hsa-miR-660, hsa-miR-130a, hsa-miR-148a | 85,8% | 88,9% | 82,7% |
| SH-29 | SEQ ID NO: 32, SEQ ID NO: 33, SEQ ID NO: 34, SEQ ID NO: 35, SEQ ID NO: 36, SEQ ID NO: 37 | hsa-miR-660, hsa-miR-130a, hsa-miR-148a, hsa-miR-93*, hsa-miR-423-5p, hsa-miR-17* | 90,8% | 94,4% | 87,1% |
| SH-30 | SEQ ID NO: 35, SEQ ID NO: 36, SEQ ID NO: 37, SEQ ID NO: 38, SEQ ID NO: 39, SEQ ID NO: 40 | hsa-miR-93*, hsa-miR-423-5p, hsa-miR-17*, hsa-let-7a, hsa-miR-331-3p, hsa-miR-192 | 90,9% | 91,3% | 90,6% |
| SH-31 | SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10 | hsa-miR-363, hsa-miR-151-3p, hsa-miR-17, hsa-miR-20b, hsa-miR-103, hsa-miR-106a, hsa-miR-101, hsa-miR-20a, hsa-let-7d, hsa-miR-320d | 97,7% | 99,7% | 95,7% |
| SH-32 | SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16 | hsa-miR-106a, hsa-miR-101, hsa-miR-20a, hsa-let-7d, hsa-miR-320d, hsa-miR-324-3p, hsa-miR-15b, hsa-miR-148b, hsa-miR-550*, hsa-miR-30d, hsa-miR-26a | 97,2% | 99,0% | 95,5% |
| SH-33 | SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21 | hsa-miR-15b, hsa-miR-148b, hsa-miR-550*, hsa-miR-30d, hsa-miR-26a, hsa-miR-107, hsa-miR-320b, hsa-miR-24, hsa-miR-18a, hsa-miR-320c | 96,2% | 99,0% | 93,4% |
| SH-34 | SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26 | hsa-miR-107, hsa-miR-320b, hsa-miR-24, hsa-miR-18a, hsa-miR-320c, hsa-miR-744, hsa-miR-93, hsa-miR-30c, hsa-miR-106b, hsa-miR-182 | 93,2% | 91,9% | 94,6% |
| SH-35 | SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31 | hsa-miR-744, hsa-miR-93, hsa-miR-30c, hsa-miR-106b, hsa-miR-182, hsa-miR-320a, hsa-miR-720, hsa-miR-30e, hsa-miR-664, hsa-miR-16 | 94,6% | 93,4% | 95,7% |

| | | | | | |
|---|---|---|---|---|---|
| SH-36 | SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36 | hsa-miR-320a, hsa-miR-720, hsa-miR-30e, hsa-miR-664, hsa-miR-16, hsa-miR-660, hsa-miR-130a, hsa-miR-148a, hsa-miR-93*, hsa-miR-423-5p | 92,0% | 92,9% | 91,1% |
| SH-37 | SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41 | hsa-miR-660, hsa-miR-130a, hsa-miR-148a, hsa-miR-93*, hsa-miR-423-5p, hsa-miR-17*, hsa-let-7a, hsa-miR-331-3p, hsa-miR-192, hsa-miR-29b | 92,2% | 91,7% | 92,6% |
| SH-38 | SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46 | hsa-miR-17*, hsa-let-7a, hsa-miR-331-3p, hsa-miR-192, hsa-miR-29b, hsa-miR-30a, hsa-miR-28-5p, hsa-miR-1908, hsa-miR-1260, hsa-miR-197 | 88,5% | 93,0% | 84,0% |
| SH-39 | SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51 | hsa-miR-30a, hsa-miR-28-5p, hsa-miR-1908, hsa-miR-1260, hsa-miR-197, hsa-miR-532-3p, hsa-miR-500, hsa-miR-223, hsa-miR-195, hsa-miR-342-3p | 87,2% | 92,6% | 81,9% |
| SH-40 | SEQ ID NO: 1, SEQ ID NO: 3, SEQ ID NO: 5, SEQ ID NO: 7, SEQ ID NO: 9 | hsa-miR-363, hsa-miR-17, hsa-miR-103, hsa-miR-101, hsa-let-7d | 96,6% | 97,5% | 95,6% |
| SH-41 | SEQ ID NO: 4, SEQ ID NO: 6, SEQ ID NO: 8, SEQ ID NO: 10, SEQ ID NO: 12 | hsa-miR-20b, hsa-miR-106a, hsa-miR-20a, hsa-miR-320d, hsa-miR-15b | 96,6% | 96,7% | 96,4% |
| SH-42 | SEQ ID NO: 8, SEQ ID NO: 10, SEQ ID NO: 12, SEQ ID NO: 14, SEQ ID NO: 16 | hsa-miR-20a, hsa-miR-320d, hsa-miR-15b, hsa-miR-550*, hsa-miR-26a | 98,0% | 98,5% | 97,4% |

# Figure 16

| Set No. | SEQ-ID NO: | miRNA -identifiers | Acc | Spec | Sens |
|---|---|---|---|---|---|
| SL-1 | SEQ ID NO: 10, SEQ ID NO: 2, SEQ ID NO: 21 | hsa-miR-320d, hsa-miR-151-3p, hsa-miR-320c | 95,6% | 97,4% | 93,8% |
| SL-2 | SEQ ID NO: 21, SEQ ID NO: 6, SEQ ID NO: 3 | hsa-miR-320c, hsa-miR-106a, hsa-miR-17 | 93,9% | 95,5% | 92,4% |
| SL-3 | SEQ ID NO: 3, SEQ ID NO: 1, SEQ ID NO: 12 | hsa-miR-17, hsa-miR-363, hsa-miR-15b | 91,1% | 94,8% | 87,4% |
| SL-4 | SEQ ID NO: 12, SEQ ID NO: 14, SEQ ID NO: 69 | hsa-miR-15b, hsa-miR-550*, hsa-miR-126 | 96,1% | 96,7% | 95,6% |
| SL-5 | SEQ ID NO: 69, SEQ ID NO: 7, SEQ ID NO: 8 | hsa-miR-126, hsa-miR-101, hsa-miR-20a | 91,4% | 92,6% | 90,2% |
| SL-6 | SEQ ID NO: 8, SEQ ID NO: 9, SEQ ID NO: 4 | hsa-miR-20a, hsa-let-7d, hsa-miR-20b | 90,6% | 88,3% | 93,0% |
| SL-7 | SEQ ID NO: 4, SEQ ID NO: 18, SEQ ID NO: 24 | hsa-miR-20b, hsa-miR-320b, hsa-miR-30c | 90,8% | 92,8% | 88,7% |
| SL-8 | SEQ ID NO: 24, SEQ ID NO: 26, SEQ ID NO: 68 | hsa-miR-30c, hsa-miR-182, hsa-miR-144 | 92,1% | 95,5% | 88,7% |
| SL-9 | SEQ ID NO: 68, SEQ ID NO: 74, SEQ ID NO: 71 | hsa-miR-144, hsa-let-7i, hsa-miR-374b | 92,0% | 91,6% | 92,3% |
| SL-10 | SEQ ID NO: 71, SEQ ID NO: 72, SEQ ID NO: 70 | hsa-miR-374b, hsa-miR-21, hsa-miR-144* | 85,9% | 87,8% | 83,9% |
| SL-11 | SEQ ID NO: 70, SEQ ID NO: 22, SEQ ID NO: 29 | hsa-miR-144*, hsa-miR-744, hsa-miR-30e | 89,7% | 94,1% | 85,2% |
| SL-12 | SEQ ID NO: 29, SEQ ID NO: 19, SEQ ID NO: 44 | hsa-miR-30e, hsa-miR-24, hsa-miR-1908 | 84,5% | 88,9% | 80,1% |
| SL-13 | SEQ ID NO: 44, SEQ ID NO: 232, SEQ ID NO: 20 | hsa-miR-1908, hsa-miR-149*, hsa-miR-18a | 83,5% | 81,6% | 85,3% |
| SL-14 | SEQ ID NO: 232, SEQ ID NO: 30, SEQ ID NO: 20 | hsa-miR-149*, hsa-miR-664, hsa-miR-18a | 83,9% | 88,1% | 79,7% |

| | | | | | |
|---|---|---|---|---|---|
| SL-15 | SEQ ID NO: 20, SEQ ID NO: 75, SEQ ID NO: 65 | hsa-miR-18a, hsa-let-7f, hsa-miR-574-5p | 81,0% | 71,6% | 90,3% |
| SL-16 | SEQ ID NO: 10, SEQ ID NO: 2, SEQ ID NO: 21, SEQ ID NO: 6, SEQ ID NO: 3 | hsa-miR-320d, hsa-miR-151-3p, hsa-miR-320c, hsa-miR-106a, hsa-miR-17 | 98,8% | #### | 97,5% |
| SL-17 | SEQ ID NO: 21, SEQ ID NO: 6, SEQ ID NO: 3, SEQ ID NO: 1, SEQ ID NO: 12 | hsa-miR-320c, hsa-miR-106a, hsa-miR-17, hsa-miR-363, hsa-miR-15b | 94,0% | 95,2% | 92,7% |
| SL-18 | SEQ ID NO: 3, SEQ ID NO: 1, SEQ ID NO: 12, SEQ ID NO: 14, SEQ ID NO: 69 | hsa-miR-17, hsa-miR-363, hsa-miR-15b, hsa-miR-550*, hsa-miR-126 | 95,2% | 94,8% | 95,6% |
| SL-19 | SEQ ID NO: 1, SEQ ID NO: 12, SEQ ID NO: 14, SEQ ID NO: 69, SEQ ID NO: 7 | hsa-miR-363, hsa-miR-15b, hsa-miR-550*, hsa-miR-126, hsa-miR-101 | 94,2% | 94,8% | 93,6% |
| SL-20 | SEQ ID NO: 14, SEQ ID NO: 69, SEQ ID NO: 7, SEQ ID NO: 8, SEQ ID NO: 9 | hsa-miR-550*, hsa-miR-126, hsa-miR-101, hsa-miR-20a, hsa-let-7d | 93,2% | 90,8% | 95,6% |
| SL-21 | SEQ ID NO: 7, SEQ ID NO: 8, SEQ ID NO: 9, SEQ ID NO: 4, SEQ ID NO: 18 | hsa-miR-101, hsa-miR-20a, hsa-let-7d, hsa-miR-20b, hsa-miR-320b | 93,2% | 95,7% | 90,6% |
| SL-22 | SEQ ID NO: 8, SEQ ID NO: 9, SEQ ID NO: 4, SEQ ID NO: 18, SEQ ID NO: 24, SEQ ID NO: 26 | hsa-miR-20a, hsa-let-7d, hsa-miR-20b, hsa-miR-320b, hsa-miR-30c, hsa-miR-182 | 94,2% | 95,6% | 92,8% |
| SL-23 | SEQ ID NO: 18, SEQ ID NO: 24, SEQ ID NO: 26, SEQ ID NO: 68, SEQ ID NO: 74, SEQ ID NO: 71 | hsa-miR-320b, hsa-miR-30c, hsa-miR-182, hsa-miR-144, hsa-let-7i, hsa-miR-374b | 93,3% | 94,5% | 92,1% |
| SL-24 | SEQ ID NO: 68, SEQ ID NO: 74, SEQ ID NO: 71, SEQ ID NO: 72, SEQ ID NO: 70, SEQ ID NO: 22 | hsa-miR-144, hsa-let-7i, hsa-miR-374b, hsa-miR-21, hsa-miR-144*, hsa-miR-744 | 92,5% | 96,6% | 88,4% |
| SL-25 | SEQ ID NO: 72, SEQ ID NO: 70, SEQ ID NO: 22, SEQ ID NO: 29, SEQ ID NO: 19, SEQ ID NO: 44 | hsa-miR-21, hsa-miR-144*, hsa-miR-744, hsa-miR-30e, hsa-miR-24, hsa-miR-1908 | 90,4% | 94,8% | 86,0% |
| SL-26 | SEQ ID NO: 29, SEQ ID NO: 19, SEQ ID NO: 44, SEQ ID NO: 77, SEQ ID NO: 232, SEQ ID NO: 30 | hsa-miR-30e, hsa-miR-24, hsa-miR-1908, hsa-miR-183, hsa-miR-149*, hsa-miR-664 | 86,8% | 89,6% | 83,9% |
| SL-27 | SEQ ID NO: 77, SEQ ID NO: 232, SEQ ID NO: 30, SEQ ID NO: 20, SEQ ID NO: 75, SEQ ID NO: 65 | hsa-miR-183, hsa-miR-149*, hsa-miR-664, hsa-miR-18a, hsa-let-7f, hsa-miR-574-5p | 88,2% | 90,0% | 86,3% |

| | | | | | |
|---|---|---|---|---|---|
| SL-28 | SEQ ID NO: 20, SEQ ID NO: 75, SEQ ID NO: 65, SEQ ID NO: 91, SEQ ID NO: 15, SEQ ID NO: 233 | hsa-miR-18a, hsa-let-7f, hsa-miR-574-5p, hsa-miR-532-5p, hsa-miR-30d, hsa-miR-130b | 85,1% | 86,0% | 84,1% |
| SL-29 | SEQ ID NO: 91, SEQ ID NO: 15, SEQ ID NO: 233, SEQ ID NO: 41, SEQ ID NO: 13, SEQ ID NO: 23 | hsa-miR-532-5p, hsa-miR-30d, hsa-miR-130b, hsa-miR-29b, hsa-miR-148b, hsa-miR-93 | 87,6% | 84,7% | 90,6% |
| SL-30 | SEQ ID NO: 41, SEQ ID NO: 13, SEQ ID NO: 23, SEQ ID NO: 55, SEQ ID NO: 11, SEQ ID NO: 32 | hsa-miR-29b, hsa-miR-148b, hsa-miR-93, hsa-miR-181a, hsa-miR-324-3p, hsa-miR-660 | 92,7% | 96,4% | 89,0% |
| SL-31 | SEQ ID NO: 10, SEQ ID NO: 2, SEQ ID NO: 21, SEQ ID NO: 6, SEQ ID NO: 3, SEQ ID NO: 1, SEQ ID NO: 12, SEQ ID NO: 14, SEQ ID NO: 69, SEQ ID NO: 7 | hsa-miR-320d, hsa-miR-151-3p, hsa-miR-320c, hsa-miR-106a, hsa-miR-17, hsa-miR-363, hsa-miR-15b, hsa-miR-550*, hsa-miR-126, hsa-miR-101 | 97,4% | 99,9% | 94,9% |
| SL-32 | SEQ ID NO: 1, SEQ ID NO: 12, SEQ ID NO: 14, SEQ ID NO: 69, SEQ ID NO: 7, SEQ ID NO: 8, SEQ ID NO: 9, SEQ ID NO: 4, SEQ ID NO: 18, SEQ ID NO: 24, SEQ ID NO: 26 | hsa-miR-363, hsa-miR-15b, hsa-miR-550*, hsa-miR-126, hsa-miR-101, hsa-miR-20a, hsa-let-7d, hsa-miR-20b, hsa-miR-320b, hsa-miR-30c, hsa-miR-182 | 96,3% | 98,4% | 94,2% |
| SL-33 | SEQ ID NO: 9, SEQ ID NO: 4, SEQ ID NO: 18, SEQ ID NO: 24, SEQ ID NO: 26, SEQ ID NO: 68, SEQ ID NO: 74, SEQ ID NO: 71, SEQ ID NO: 72, SEQ ID NO: 70 | hsa-let-7d, hsa-miR-20b, hsa-miR-320b, hsa-miR-30c, hsa-miR-182, hsa-miR-144, hsa-let-7i, hsa-miR-374b, hsa-miR-21, hsa-miR-144* | 94,8% | 94,9% | 94,7% |
| SL-34 | SEQ ID NO: 68, SEQ ID NO: 74, SEQ ID NO: 71, SEQ ID NO: 72, SEQ ID NO: 70, SEQ ID NO: 22, SEQ ID NO: 29, SEQ ID NO: 19, SEQ ID NO: 44, SEQ ID NO: 77 | hsa-miR-144, hsa-let-7i, hsa-miR-374b, hsa-miR-21, hsa-miR-144*, hsa-miR-744, hsa-miR-30e, hsa-miR-24, hsa-miR-1908, hsa-miR-183 | 93,2% | 97,7% | 88,6% |
| SL-35 | SEQ ID NO: 22, SEQ ID NO: 29, SEQ ID NO: 19, SEQ ID NO: 44, SEQ ID NO: 77, SEQ ID NO: 232, SEQ ID NO: 30, SEQ ID NO: 20, SEQ ID NO: 75, SEQ ID NO: 65 | hsa-miR-744, hsa-miR-30e, hsa-miR-24, hsa-miR-1908, hsa-miR-183, hsa-miR-149*, hsa-miR-664, hsa-miR-18a, hsa-let-7f, hsa-miR-574-5p | 90,8% | 92,4% | 89,2% |

| | | | | | |
|---|---|---|---|---|---|
| SL-36 | SEQ ID NO: 232, SEQ ID NO: 30, SEQ ID NO: 20, SEQ ID NO: 75, SEQ ID NO: 65, SEQ ID NO: 91, SEQ ID NO: 15, SEQ ID NO: 233, SEQ ID NO: 41, SEQ ID NO: 13 | hsa-miR-149*, hsa-miR-664, hsa-miR-18a, hsa-let-7f, hsa-miR-574-5p, hsa-miR-532-5p, hsa-miR-30d, hsa-miR-130b, hsa-miR-29b, hsa-miR-148b | 90,3% | 88,7% | 91,9% |
| SL-37 | SEQ ID NO: 91, SEQ ID NO: 15, SEQ ID NO: 233, SEQ ID NO: 41, SEQ ID NO: 13, SEQ ID NO: 23, SEQ ID NO: 55, SEQ ID NO: 11, SEQ ID NO: 32, SEQ ID NO: 51 | hsa-miR-532-5p, hsa-miR-30d, hsa-miR-130b, hsa-miR-29b, hsa-miR-148b, hsa-miR-93, hsa-miR-181a, hsa-miR-324-3p, hsa-miR-660, hsa-miR-342-3p | 94,6% | 97,2% | 92,1% |
| SL-38 | SEQ ID NO: 23, SEQ ID NO: 55, SEQ ID NO: 11, SEQ ID NO: 32, SEQ ID NO: 51, SEQ ID NO: 234, SEQ ID NO: 54, SEQ ID NO: 43, SEQ ID NO: 28, SEQ ID NO: 235 | hsa-miR-93, hsa-miR-181a, hsa-miR-324-3p, hsa-miR-660, hsa-miR-342-3p, hsa-miR-30b, hsa-miR-1280, hsa-miR-28-5p, hsa-miR-720, hsa-miR-1271 | 93,6% | 96,6% | 90,6% |
| SL-39 | SEQ ID NO: 234, SEQ ID NO: 54, SEQ ID NO: 43, SEQ ID NO: 28, SEQ ID NO: 235, SEQ ID NO: 38, SEQ ID NO: 33, SEQ ID NO: 92, SEQ ID NO: 236, SEQ ID NO: 97 | hsa-miR-30b, hsa-miR-1280, hsa-miR-28-5p, hsa-miR-720, hsa-miR-1271, hsa-let-7a, hsa-miR-130a, hsa-miR-1226*, hsa-miR-1274b, hsa-miR-1225-3p | 95,8% | 97,3% | 94,2% |
| SL-40 | SEQ ID NO: 10, SEQ ID NO: 21, SEQ ID NO: 3, SEQ ID NO: 12, SEQ ID NO: 69 | hsa-miR-320d, hsa-miR-320c, hsa-miR-17, hsa-miR-15b, hsa-miR-126 | 96,1% | 98,0% | 94,3% |
| SL-41 | SEQ ID NO: 6, SEQ ID NO: 1, SEQ ID NO: 14, SEQ ID NO: 7, SEQ ID NO: 9 | hsa-miR-106a, hsa-miR-363, hsa-miR-550*, hsa-miR-101, hsa-let-7d | 95,6% | 95,6% | 95,6% |
| SL-42 | SEQ ID NO: 14, SEQ ID NO: 7, SEQ ID NO: 9, SEQ ID NO: 18, SEQ ID NO: 26 | hsa-miR-550*, hsa-miR-101, hsa-let-7d, hsa-miR-320b, hsa-miR-182 | 94,6% | 94,6% | 94,6% |

# Figure 17

| Set No. | SEQ-ID NO: | miRNA -identifiers | Acc | Spec | Sens |
|---|---|---|---|---|---|
| SC-1 | SEQ ID NO: 2, SEQ ID NO: 10, SEQ ID NO: 24 | hsa-miR-151-3p, hsa-miR-320d, hsa-miR-30c | 94,8% | 97,6% | 92,1% |
| SC-2 | SEQ ID NO: 24, SEQ ID NO: 11, SEQ ID NO: 16 | hsa-miR-30c, hsa-miR-324-3p, hsa-miR-26a | 90,2% | 94,3% | 86,1% |
| SC-3 | SEQ ID NO: 16, SEQ ID NO: 35, SEQ ID NO: 9 | hsa-miR-26a, hsa-miR-93*, hsa-let-7d | 89,8% | 89,7% | 89,9% |
| SC-4 | SEQ ID NO: 9, SEQ ID NO: 14, SEQ ID NO: 28 | hsa-let-7d, hsa-miR-550*, hsa-miR-720 | 90,1% | 89,2% | 91,0% |
| SC-5 | SEQ ID NO: 28, SEQ ID NO: 21, SEQ ID NO: 1 | hsa-miR-720, hsa-miR-320c, hsa-miR-363 | 89,2% | 89,5% | 88,9% |
| SC-6 | SEQ ID NO: 1, SEQ ID NO: 49, SEQ ID NO: 47 | hsa-miR-363, hsa-miR-223, hsa-miR-532-3p | 90,1% | 93,8% | 86,5% |
| SC-7 | SEQ ID NO: 47, SEQ ID NO: 6, SEQ ID NO: 3 | hsa-miR-532-3p, hsa-miR-106a, hsa-miR-17 | 91,8% | 91,3% | 92,3% |
| SC-8 | SEQ ID NO: 3, SEQ ID NO: 7, SEQ ID NO: 69 | hsa-miR-17, hsa-miR-101, hsa-miR-126 | 91,6% | 94,1% | 89,1% |
| SC-9 | SEQ ID NO: 69, SEQ ID NO: 42, SEQ ID NO: 4 | hsa-miR-126, hsa-miR-30a, hsa-miR-20b | 93,3% | 93,6% | 93,1% |
| SC-10 | SEQ ID NO: 4, SEQ ID NO: 68, SEQ ID NO: 26 | hsa-miR-20b, hsa-miR-144, hsa-miR-182 | 92,9% | 95,5% | 90,4% |
| SC-11 | SEQ ID NO: 26, SEQ ID NO: 30, SEQ ID NO: 71 | hsa-miR-182, hsa-miR-664, hsa-miR-374b | 90,3% | 94,8% | 85,8% |
| SC-12 | SEQ ID NO: 71, SEQ ID NO: 43, SEQ ID NO: 74 | hsa-miR-374b, hsa-miR-28-5p, hsa-let-7i | 79,9% | 73,4% | 86,4% |
| SC-13 | SEQ ID NO: 74, SEQ ID NO: 72, SEQ ID NO: 77 | hsa-let-7i, hsa-miR-21, hsa-miR-183 | 82,7% | 85,1% | 80,4% |
| SC-14 | SEQ ID NO: 72, SEQ ID NO: 12, SEQ ID NO: 77 | hsa-miR-21, hsa-miR-15b, hsa-miR-183 | 89,7% | 97,5% | 81,9% |

| | | | | | |
|---|---|---|---|---|---|
| SC-15 | SEQ ID NO: 77, SEQ ID NO: 38, SEQ ID NO: 76 | hsa-miR-183, hsa-let-7a, hsa-miR-18a* | 83,7% | 84,7% | 82,8% |
| SC-16 | SEQ ID NO: 2, SEQ ID NO: 10, SEQ ID NO: 24, SEQ ID NO: 11, SEQ ID NO: 16 | hsa-miR-151-3p, hsa-miR-320d, hsa-miR-30c, hsa-miR-324-3p, hsa-miR-26a | 97,3% | 98,1% | 96,4% |
| SC-17 | SEQ ID NO: 24, SEQ ID NO: 11, SEQ ID NO: 16, SEQ ID NO: 35, SEQ ID NO: 9 | hsa-miR-30c, hsa-miR-324-3p, hsa-miR-26a, hsa-miR-93*, hsa-let-7d | 94,0% | 93,5% | 94,5% |
| SC-18 | SEQ ID NO: 16, SEQ ID NO: 35, SEQ ID NO: 9, SEQ ID NO: 14, SEQ ID NO: 28 | hsa-miR-26a, hsa-miR-93*, hsa-let-7d, hsa-miR-550*, hsa-miR-720 | 93,6% | 93,4% | 93,8% |
| SC-19 | SEQ ID NO: 35, SEQ ID NO: 9, SEQ ID NO: 14, SEQ ID NO: 28, SEQ ID NO: 21 | hsa-miR-93*, hsa-let-7d, hsa-miR-550*, hsa-miR-720, hsa-miR-320c | 94,7% | 94,0% | 95,5% |
| SC-20 | SEQ ID NO: 14, SEQ ID NO: 28, SEQ ID NO: 21, SEQ ID NO: 1, SEQ ID NO: 49 | hsa-miR-550*, hsa-miR-720, hsa-miR-320c, hsa-miR-363, hsa-miR-223 | 94,8% | 97,6% | 91,9% |
| SC-21 | SEQ ID NO: 21, SEQ ID NO: 1, SEQ ID NO: 49, SEQ ID NO: 47, SEQ ID NO: 6 | hsa-miR-320c, hsa-miR-363, hsa-miR-223, hsa-miR-532-3p, hsa-miR-106a | 95,8% | 96,4% | 95,3% |
| SC-22 | SEQ ID NO: 1, SEQ ID NO: 49, SEQ ID NO: 47, SEQ ID NO: 6, SEQ ID NO: 3, SEQ ID NO: 7 | hsa-miR-363, hsa-miR-223, hsa-miR-532-3p, hsa-miR-106a, hsa-miR-17, hsa-miR-101 | 92,8% | 96,5% | 89,1% |
| SC-23 | SEQ ID NO: 6, SEQ ID NO: 3, SEQ ID NO: 7, SEQ ID NO: 69, SEQ ID NO: 42, SEQ ID NO: 4 | hsa-miR-106a, hsa-miR-17, hsa-miR-101, hsa-miR-126, hsa-miR-30a, hsa-miR-20b | 92,4% | 93,7% | 91,2% |
| SC-24 | SEQ ID NO: 69, SEQ ID NO: 42, SEQ ID NO: 4, SEQ ID NO: 68, SEQ ID NO: 26, SEQ ID NO: 30 | hsa-miR-126, hsa-miR-30a, hsa-miR-20b, hsa-miR-144, hsa-miR-182, hsa-miR-664 | 93,0% | 95,0% | 90,9% |
| SC-25 | SEQ ID NO: 68, SEQ ID NO: 26, SEQ ID NO: 30, SEQ ID NO: 71, SEQ ID NO: 43, SEQ ID NO: 74 | hsa-miR-144, hsa-miR-182, hsa-miR-664, hsa-miR-374b, hsa-miR-28-5p, hsa-let-7i | 90,4% | 94,7% | 86,0% |
| SC-26 | SEQ ID NO: 71, SEQ ID NO: 43, SEQ ID NO: 74, SEQ ID NO: 29, SEQ ID NO: 72, SEQ ID NO: 12 | hsa-miR-374b, hsa-miR-28-5p, hsa-let-7i, hsa-miR-30e, hsa-miR-21, hsa-miR-15b | 91,3% | 98,7% | 84,0% |
| SC-27 | SEQ ID NO: 29, SEQ ID NO: 72, SEQ ID NO: 12, SEQ ID NO: 77, SEQ ID NO: 38, SEQ ID NO: 76 | hsa-miR-30e, hsa-miR-21, hsa-miR-15b, hsa-miR-183, hsa-let-7a, hsa-miR-18a* | 89,3% | 95,0% | 83,6% |

| | | | | | |
|---|---|---|---|---|---|
| SC-28 | SEQ ID NO: 77, SEQ ID NO: 38, SEQ ID NO: 76, SEQ ID NO: 8, SEQ ID NO: 41, SEQ ID NO: 22 | hsa-miR-183, hsa-let-7a, hsa-miR-18a*, hsa-miR-20a, hsa-miR-29b, hsa-miR-744 | 90,7% | 91,3% | 90,0% |
| SC-29 | SEQ ID NO: 8, SEQ ID NO: 41, SEQ ID NO: 22, SEQ ID NO: 44, SEQ ID NO: 19, SEQ ID NO: 53 | hsa-miR-20a, hsa-miR-29b, hsa-miR-744, hsa-miR-1908, hsa-miR-24, hsa-miR-222 | 90,5% | 92,6% | 88,4% |
| SC-30 | SEQ ID NO: 44, SEQ ID NO: 19, SEQ ID NO: 53, SEQ ID NO: 330, SEQ ID NO: 70, SEQ ID NO: 75 | hsa-miR-1908, hsa-miR-24, hsa-miR-222, hsa-miR-1268, hsa-miR-144*, hsa-let-7f | 91,6% | 94,4% | 88,7% |
| SC-31 | SEQ ID NO: 2, SEQ ID NO: 10, SEQ ID NO: 24, SEQ ID NO: 11, SEQ ID NO: 16, SEQ ID NO: 35, SEQ ID NO: 9, SEQ ID NO: 14, SEQ ID NO: 28, SEQ ID NO: 21 | hsa-miR-151-3p, hsa-miR-320d, hsa-miR-30c, hsa-miR-324-3p, hsa-miR-26a, hsa-miR-93*, hsa-let-7d, hsa-miR-550*, hsa-miR-720, hsa-miR-320c | 97,0% | 97,9% | 96,1% |
| SC-32 | SEQ ID NO: 35, SEQ ID NO: 9, SEQ ID NO: 14, SEQ ID NO: 28, SEQ ID NO: 21, SEQ ID NO: 1, SEQ ID NO: 49, SEQ ID NO: 47, SEQ ID NO: 6, SEQ ID NO: 3, SEQ ID NO: 7 | hsa-miR-93*, hsa-let-7d, hsa-miR-550*, hsa-miR-720, hsa-miR-320c, hsa-miR-363, hsa-miR-223, hsa-miR-532-3p, hsa-miR-106a, hsa-miR-17, hsa-miR-101 | 94,4% | 95,2% | 93,7% |
| SC-33 | SEQ ID NO: 49, SEQ ID NO: 47, SEQ ID NO: 6, SEQ ID NO: 3, SEQ ID NO: 7, SEQ ID NO: 69, SEQ ID NO: 42, SEQ ID NO: 4, SEQ ID NO: 68, SEQ ID NO: 26 | hsa-miR-223, hsa-miR-532-3p, hsa-miR-106a, hsa-miR-17, hsa-miR-101, hsa-miR-126, hsa-miR-30a, hsa-miR-20b, hsa-miR-144, hsa-miR-182 | 91,8% | 94,0% | 89,6% |
| SC-34 | SEQ ID NO: 69, SEQ ID NO: 42, SEQ ID NO: 4, SEQ ID NO: 68, SEQ ID NO: 26, SEQ ID NO: 30, SEQ ID NO: 71, SEQ ID NO: 43, SEQ ID NO: 74, SEQ ID NO: 29 | hsa-miR-126, hsa-miR-30a, hsa-miR-20b, hsa-miR-144, hsa-miR-182, hsa-miR-664, hsa-miR-374b, hsa-miR-28-5p, hsa-let-7i, hsa-miR-30e | 90,1% | 93,2% | 86,9% |
| SC-35 | SEQ ID NO: 30, SEQ ID NO: 71, SEQ ID NO: 43, SEQ ID NO: 74, SEQ ID NO: 29, SEQ ID NO: 72, SEQ ID NO: 12, SEQ ID NO: 77, SEQ ID NO: 38, SEQ ID NO: 76 | hsa-miR-664, hsa-miR-374b, hsa-miR-28-5p, hsa-let-7i, hsa-miR-30e, hsa-miR-21, hsa-miR-15b, hsa-miR-183, hsa-let-7a, hsa-miR-18a* | 89,2% | 97,8% | 80,5% |

| | | | | | |
|---|---|---|---|---|---|
| SC-36 | SEQ ID NO: 72, SEQ ID NO: 12, SEQ ID NO: 77, SEQ ID NO: 38, SEQ ID NO: 76, SEQ ID NO: 8, SEQ ID NO: 41, SEQ ID NO: 22, SEQ ID NO: 44, SEQ ID NO: 19 | hsa-miR-21, hsa-miR-15b, hsa-miR-183, hsa-let-7a, hsa-miR-18a*, hsa-miR-20a, hsa-miR-29b, hsa-miR-744, hsa-miR-1908, hsa-miR-24 | 90,9% | 97,7% | 84,2% |
| SC-37 | SEQ ID NO: 8, SEQ ID NO: 41, SEQ ID NO: 22, SEQ ID NO: 44, SEQ ID NO: 19, SEQ ID NO: 53, SEQ ID NO: 330, SEQ ID NO: 70, SEQ ID NO: 75, SEQ ID NO: 86 | hsa-miR-20a, hsa-miR-29b, hsa-miR-744, hsa-miR-1908, hsa-miR-24, hsa-miR-222, hsa-miR-1268, hsa-miR-144*, hsa-let-7f, hsa-miR-424 | 91,2% | 93,7% | 88,8% |
| SC-38 | SEQ ID NO: 53, SEQ ID NO: 330, SEQ ID NO: 70, SEQ ID NO: 75, SEQ ID NO: 86, SEQ ID NO: 232, SEQ ID NO: 17, SEQ ID NO: 15, SEQ ID NO: 45, SEQ ID NO: 52 | hsa-miR-222, hsa-miR-1268, hsa-miR-144*, hsa-let-7f, hsa-miR-424, hsa-miR-149*, hsa-miR-107, hsa-miR-30d, hsa-miR-1260, hsa-miR-25 | 92,3% | 95,4% | 89,2% |
| SC-39 | SEQ ID NO: 232, SEQ ID NO: 17, SEQ ID NO: 15, SEQ ID NO: 45, SEQ ID NO: 52, SEQ ID NO: 64, SEQ ID NO: 60, SEQ ID NO: 18, SEQ ID NO: 244, SEQ ID NO: 25 | hsa-miR-149*, hsa-miR-107, hsa-miR-30d, hsa-miR-1260, hsa-miR-25, hsa-miR-151-5p, hsa-miR-484, hsa-miR-320b, hsa-miR-23a, hsa-miR-106b | 91,4% | 92,7% | 90,1% |
| SC-40 | SEQ ID NO: 2, SEQ ID NO: 24, SEQ ID NO: 16, SEQ ID NO: 9, SEQ ID NO: 28 | hsa-miR-151-3p, hsa-miR-30c, hsa-miR-26a, hsa-let-7d, hsa-miR-720 | 94,2% | 95,8% | 92,5% |
| SC-41 | SEQ ID NO: 11, SEQ ID NO: 35, SEQ ID NO: 14, SEQ ID NO: 21, SEQ ID NO: 49 | hsa-miR-324-3p, hsa-miR-93*, hsa-miR-550*, hsa-miR-320c, hsa-miR-223 | 94,5% | 93,9% | 95,1% |
| SC-42 | SEQ ID NO: 14, SEQ ID NO: 21, SEQ ID NO: 49, SEQ ID NO: 6, SEQ ID NO: 7 | hsa-miR-550*, hsa-miR-320c, hsa-miR-223, hsa-miR-106a, hsa-miR-101 | 94,8% | 96,0% | 93,5% |

## Figure 18

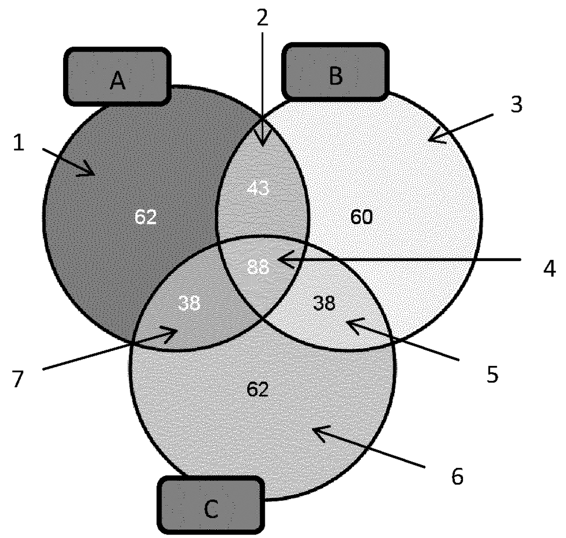


**A: Healthy control versus Sarcoidosis**

**B: Lung cancer versus Sarcoidosis**

**C: Sarcoidosis versus COPD**

**List 1**: SEQ ID NOs: 34, 36, 39, 40, 50, 56, 57, 58, 61, 62, 66, 79, 80, 81, 83, 84, 85, 89, 90, 93, 94, 96, 98, 99, 100, 101, 102, 104, 113, 128, 129, 132, 133, 142, 143, 144, 148, 151, 155, 156, 163, 166, 169, 170, 171, 173, 179, 193, 195, 196, 199, 200, 201, 203, 206, 214, 216, 221, 223, 225, 226, and 229

**List 2**: SEQ ID NOs: 37, 46, 63, 65, 73, 78, 82, 87, 91, 92, 95, 97, 103, 111, 118, 123, 130, 131, 136, 137, 141, 149, 152, 157, 158, 172, 174, 178, 187, 188, 189, 192, 194, 197, 198, 204, 207, 208, 210, 211, 213, 224, and 227

**List 3**: SEQ ID NOs: 233, 234, 235, 237, 240, 241, 242, 243, 245, 246, 248, 249, 251, 252, 254, 255, 258, 259, 261, 262, 263, 266, 268, 269, 270, 272, 273, 275, 276, 277, 278, 279, 282, 283, 284, 285, 288, 289, 290, 291, 292, 293, 294, 297, 298, 299, 300, 301, 303, 305, 308, 310, 312, 314, 317, 321, 322, 325, 326, and 329

**List 4**: SEQ ID NOs: 1, 2, 3, 4, 5, 6, 7, 8, 9, 10, 11, 12, 13, 14, 15, 18, 19, 20, 21, 22, 23, 24, 25, 26, 27, 28, 29, 30, 32, 33, 38, 41, 42, 43, 44, 45, 47, 51, 54, 55, 64, 68, 69, 70, 71, 72, 74, 75, 76, 77, 105, 106, 107, 108, 109, 110, 112, 114, 115, 116, 120, 121, 125, 126, 134, 138, 145, 153, 154, 159, 161, 162, 164, 165, 167, 168, 175, 177, 183, 184, 185, 186, 190, 191, 209, 218, 219, and 228

**List 5**: SEQ ID NOs: 232, 236, 238, 239, 244, 247, 250, 253, 256, 257, 260, 264, 265, 267, 271, 274, 280, 281, 286, 287, 295, 296, 302, 304, 306, 307, 309, 311, 313, 315, 316, 318, 319, 320, 323, 324, 327, and 328

**List 6**: SEQ ID NOs: 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, and 391

**List 7**: SEQ ID NO: 16, 17, 31, 35, 48, 49, 52, 53, 59, 60, 67, 86, 88, 117, 119, 122, 124, 127, 135, 139, 140, 146, 147, 150, 160, 176, 180, 181, 182, 202, 205, 212, 215, 217, 220, 222, 230, and 231

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61437293 B


### Non-patent literature cited in the description


- **BONS et al.** *Respiratory Medicine,* 20 July 2007, vol. 101 (8), 1687-1695
- A multilingual glossary of biotechnological terms: (IUPAC Recommendations). Helvetica Chimica Acta. 1995
- Molecular Cloning: A Laboratory Manual. Cold Spring Harbor Laboratory Press, 1989
- **KELLER A ; LEIDINGER P ; BORRIES A ; WENDSCHLAG A ; WUCHERPFENNIG F ; SCHEFFLER M ; HUWER H ; LENHOF HP ; MEESE E.** miRNAs in lung cancer - studying complex fingerprints in patient's blood cells by microarray experiments. *BMC Cancer,* 2009, vol. 9, 353
- **VORWERK S ; GANTER K ; CHENG Y ; HOHEISEL J ; STAHLER PF ; BEIER M.** Microfluidic-based enzymatic on-chip labeling of miRNAs. *N Biotechnol,* 2008, vol. 25 (2-3), 142-149
- **BOLSTAD BM ; IRIZARRY RA ; ASTRAND M ; SPEED TP.** A comparison of normalization methods for high density oligonucleotide array data based on variance and bias. *Bioinformatics,* 2003, vol. 19 (2), 185-193
- **BENJAMINI Y ; DRAI D ; ELMER G ; KAFKAFI N ; GOLANI I.** Controlling the false discovery rate in behavior genetics research. *Behav Brain Res,* 2001, vol. 125 (1-2), 279-284
- **HOCHBERG Y.** A sharper bonferroni procedure for multiple tests of significance. *Biometrica,* 1988, vol. 75, 185-193
- **VAPNIK V.** The nature of statistical learning theory. Spinger, 2000
- R: A Language and Environment for Statistical Computing. *Vienna: R Foundation for Statistical Computing,* 2008